(19) Europäisches Patentamt
European Patent Office
Office européen des brevets

(11) Numéro de publication : **0 465 369 B1**

# (12) FASCICULE DE BREVET EUROPEEN

(45) Date de publication du fascicule du brevet : **13.04.94 Bulletin 94/15**

(21) Numéro de dépôt : **91401858.5**

(22) Date de dépôt : **04.07.91**

(51) Int. Cl.$^5$ : **C07C 327/30,** A61K 31/17, C07C 323/44, C07D 295/215, C07D 295/32, C07C 331/24, C07D 243/08, C07D 243/24, C07D 213/75, A61K 31/40, A61K 31/535

## (54) Nouveaux dérivés N-substitués d'alpha-mercapto alkylamines, leur procédé de préparation et les intermédiaires obtenus, leur application à titre de médicaments et les compositions les renfermant.

(30) Priorité : **05.07.90 FR 9008539**

(43) Date de publication de la demande : **08.01.92 Bulletin 92/02**

(45) Mention de la délivrance du brevet : **13.04.94 Bulletin 94/15**

(84) Etats contractants désignés : **AT BE CH DE DK ES FR GB GR IT LI LU NL SE**

(56) Documents cités :
**EP-A- 0 066 415**
**FR-A- 2 331 559**
**BIOCHEMICAL AND BIOPHYSICAL RESEARCH COMMUNICATIONS, vol. 143, no. 1, 27 février 1987, pages 44-51, Academic Press, Inc., Duluth, Minnesota, US; J.R. LULY et al.: "Modified peptides which display potent and specific inhibition of human renin"**

(73) Titulaire : **ROUSSEL-UCLAF**
**35, Boulevard des Invalides**
**F-75007 Paris (FR)**

(72) Inventeur : **Clemence, François**
**2, rue Turgot**
**F-75009 Paris (FR)**
Inventeur : **Le Martret, Odile**
**42, Avenue de Versailles**
**F-75016 Paris (FR)**
Inventeur : **Petit, Francis**
**119, Avenue Carnot**
**F-93140 Bondy (FR)**

(74) Mandataire : **Bourgouin, André et al**
**Département des Brevets ROUSSEL UCLAF**
**111, route de Noisy B.P. no 9**
**F-93230 Romainville (FR)**

EP 0 465 369 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés N-substitués d'alpha-mercapto alkylamines, leur procédé de préparation, leur application à titre de médicaments et les compositions les renfermant.

Des composés semblables sont révéles dans le document EP-A-66415.

La présente invention a pour objet les produits de formule ($I_A$):

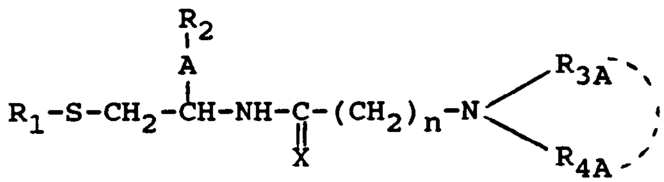

($I_A$)

dans laquelle :

n représente les valeurs 0 à 4,

$R_1$ représente un atome d'hydrogène ou un radical $R_5$ - CO - dans lequel $R_5$ représente :

- un radical alkyle, alkényle ou alkynyle linéaire ou ramifié renfermant au plus 6 atomes de carbone et éventuellement substitué,
- un radical monocyclique comprenant 5 à 7 chaînons ou un radical constitué de cycles condensés comprenant 8 à 10 chaînons, ces radicaux étant saturés ou insaturés, renfermant éventuellement un ou plusieurs hétéroatomes, identiques ou différents, choisis parmi les atomes d'oxygène, d'azote et de soufre et étant éventuellement substitués,
- un radical

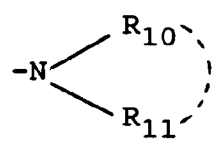

dans lequel $R_{10}$ et $R_{11}$ sont tels que :

**ou bien** $R_{10}$ et $R_{11}$ forment avec l'atome d'azote auxquels ils sont liés un radical monocyclique comprenant 5 à 7 chaînons ou un radical constitué de cycles condensés comprenant 8 à 10 chaînons, ces radicaux renfermant éventuellement un autre ou plusieurs autres hétéroatomes, identiques ou différents, choisis parmi les atomes d'oxygène, d'azote et de soufre, et étant éventuellement substitués,

**ou bien** $R_{10}$ et $R_{11}$, identiques ou différents, représentent :

- un atome d'hydrogène ;
- un radical alkyle, alkényle ou alkynyle linéaire ou ramifié renfermant au plus 6 atomes de carbone et éventuellement substitué ;
- un radical aryle dans lequel le radical aryle représente un radical monocyclique comprenant 5 à 7 chaînons ou constitué de cycles condensés comprenant 8 à 10 chaînons, ces radicaux renfermant éventuellement un ou plusieurs hétéroatomes, identiques ou différents, choisis parmi les atomes d'oxygène, d'azote et de soufre, et étant éventuellement substitués ;

A représente :

- soit un radical alkylène ou alkénylène linéaire ou ramifié renfermant au plus 6 atomes de carbone et éventuellement substitué par un radical hydroxyle ou alcoxy renfermant au plus 6 atomes de carbone,
- soit une simple liaison,

$R_2$ représente un radical monocyclique comprenant 5 à 7 chaînons ou un radical constitué de cycles condensés comprenant 8 à 10 chaînons, ces radicaux étant saturés ou insaturés, renfermant éventuellement un ou plusieurs hétéroatomes, identiques ou différents, choisis parmi les atomes d'oxygène, d'azote et de soufre et étant éventuellement substitués,

X représente un atome d'oxygène ou de soufre,

$R_{3A}$ et $R_{4A}$ sont tels que :

**ou bien** $R_{3A}$ et $R_{4A}$ forment avec l'atome d'azote auxquels ils sont liés un radical monocyclique comprenant 5 à 7 chaînons ou un radical constitué de cycles condensés comprenant 8 à 10 chaînons, ces radicaux renfermant éventuellement un autre ou plusieurs autres hétéroatomes, identiques ou différents, choisis parmi les atomes

d'oxygène, d'azote et de soufre, et étant éventuellement substitués,

**ou bien** $R_{3A}$ et $R_{4A}$, identiques ou différents, représentent :

- un atome d'hydrogène ;
- un radical hydroxyle ; alcoxy, acyle ou acyloxy renfermant au plus 6 atomes de carbone ; carboxy, salifié ou estérifié ; cyano ;
- un radical alkyle, alkényle ou alkynyle linéaire ou ramifié renfermant au plus 6 atomes de carbone et éventuellement substitué ;
- un radical $R_c$ ou $R_c$-O- dans lesquels le radical $R_c$ représente un radical aliphatique ou arylique, monocyclique comprenant 5 à 7 chaînons ou constitué de cycles condensés comprenant 8 à 10 chaînons, ces radicaux renfermant éventuellement un ou plusieurs hétéroatomes, identiques ou différents, choisis parmi les atomes d'oxygène, d'azote et de soufre, et étant éventuellement substitués ;
- le radical

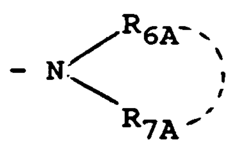 ou 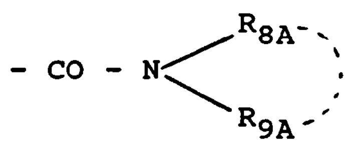

dans lesquels $R_{6A}$, $R_{7A}$, $R_{8A}$ et $R_{9A}$, identiques ou différents, représentent un atome d'hydrogène, un radical hydroxyle, alkyle, alcoxy, acyloxy ou acyle, ces radicaux renfermant au plus 6 atomes de carbone, carboxy libre, salifié ou estérifié, phénoxy, phényle, benzyle, phénéthyle, azépine, pipéridyle, morpholine, pyrrolidinyle, pipérazinyle, éventuellement substitués sur le second atome d'azote par un radical hydroxyle, alkyle, alcoxy ou phényle, ces trois derniers radicaux étant eux-mêmes éventuellement substitués par un ou plusieurs radicaux choisis dans les atomes d'halogène, le radical hydrogène, trifluorométhyle, méthoxy, éthoxy, méthyle et éthyle,

ou bien d'une part $R_{6A}$ et $R_{7A}$ et d'autre part $R_{8A}$ et $R_{9A}$ forment respectivement avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux hydroxyle, trifluorométhyle, alkyle et alcoxy, ces radicaux renfermant au plus 6 atomes de carbone,

lesdits produits de formule ($I_A$) étant sous toutes les formes isomères possibles racémiques, énantiomères et diastéréoisomères,

ainsi que les sels d'addition avec les acides minéraux et organiques desdits produits de formule ($I_A$).

L'invention a également pour objet les produits de formule ($I_A$) telle que définie ci-dessus répondant à la formule (I) :

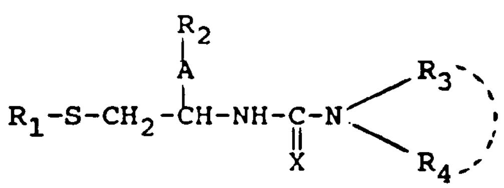 (I)

dans laquelle :

$R_1$ représente un atome d'hydrogène ou un radical $R_8$ - CO - dans lequel $R_8$ représente :

- un radical alkyle, alkényle ou alkynyle linéaire ou ramifié renfermant au plus 6 atomes de carbone et éventuellement substitué,
- un radical monocyclique comprenant 5 à 7 chaînons ou un radical constitué de cycles condensés comprenant 8 à 10 chaînons, ces radicaux étant saturés ou insaturés, renfermant éventuellement un ou plusieurs hétéroatomes, identiques ou différents, choisis parmi les atomes d'oxygène, d'azote et de soufre et étant éventuellement substitués,
- un radical

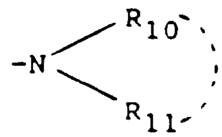

dans lequel $R_{10}$ et $R_{11}$ sont tels que :

**ou bien** $R_{10}$ et $R_{11}$ forment avec l'atome d'azote auxquels ils sont liés un radical monocyclique comprenant 5 à 7 chaînons ou un radical constitué de cycles condensés comprenant 8 à 10 chaînons, ces radicaux renfermant éventuellement un autre ou plusieurs autres hétéroatomes, identiques ou différents, choisis parmi les atomes d'oxygène, d'azote et de soufre, et étant éventuellement substitués,

**ou bien** $R_{10}$ et $R_{11}$, identiques ou différents, représentent :

- un atome d'hydrogène ;
- un radical alkyle, alkényle ou alkynyle linéaire ou ramifié renfermant au plus 6 atomes de carbone et éventuellement substitué ;
- un radical aryle dans lequel le radical aryle représente un radical monocyclique comprenant 5 à 7 chaînons ou constitué de cycles condensés comprenant 8 à 10 chaînons, ces radicaux renfermant éventuellement un ou plusieurs hétéroatomes, identiques ou différents, choisis parmi les atomes d'oxygène, d'azote et de soufre, et étant éventuellement substitués ;

A représente :

- soit un radical alkylène ou alkénylène linéaire ou ramifié renfermant au plus 6 atomes de carbone et éventuellement substitué par un radical hydroxyle ou alcoxy renfermant au plus 6 atomes de carbone,
- soit une simple liaison,

$R_2$ représente un radical monocyclique comprenant 5 à 7 chaînons ou un radical constitué de cycles condensés comprenant 8 à 10 chaînons, ces radicaux étant saturés ou insaturés, renfermant éventuellement un ou plusieurs hétéroatomes, identiques ou différents, choisis parmi les atomes d'oxygène, d'azote et de soufre et étant éventuellement substitués,

X représente un atome d'oxygène ou de soufre,

$R_3$ et $R_4$ sont tels que : **ou bien** $R_3$ et $R_4$ forment avec l'atome d'azote auxquels ils sont liés un radical monocyclique comprenant 5 à 7 chaînons ou un radical constitué de cycles condensés comprenant 8 à 10 chaînons, ces radicaux renfermant éventuellement un autre ou plusieurs autres hétéroatomes, identiques ou différents, choisis parmi les atomes d'oxygène, d'azote et de soufre, et étant éventuellement substitués,

**ou bien** $R_3$ et $R_4$, identiques ou différents, représentent :

- un atome d'hydrogène ;
- un radical hydroxyle ; alcoxy, acyle ou acyloxy renfermant au plus 6 atomes de carbone ; carboxy, salifié ou estérifié ; cyano ;
- un radical alkyle, alkényle ou alkynyle linéaire ou ramifié renfermant au plus 6 atomes de carbone et éventuellement substitué ;
- un radical aryle ou aryloxydans lesquels le radical aryle représente un radical monocyclique comprenant 5 à 7 chaînons ou constitué de cycles condensés comprenant 8 à 10 chaînons, ces radicaux renfermant éventuellement un ou plusieurs hétéroatomes, identiques ou différents, choisis parmi les atomes d'oxygène, d'azote et de soufre, et étant éventuellement substitués ;
- le radical

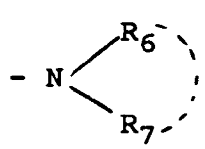

ou

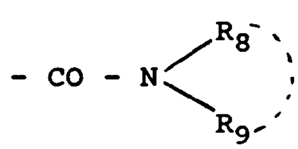

dans lesquels $R_6$, $R_7$, $R_8$ et $R_9$, identiques ou différents, représentent un atome d'hydrogène, un radical hydroxyle, alkyle, alcoxy, acyloxy ou acyle, ces radicaux renfermant au plus 6 atomes de carbone, carboxy libre, salifié ou estérifié, phényle, benzyle, phénéthyle, azépine, pipéridyle, morpholine, pyrrolidinyle, pipérazinyle, éventuellement substitués sur le second atome d'azote par un radical hydroxyle, alkyle, alcoxy ou phényle, ces trois derniers radicaux étant eux-mêmes éventuellement substitués par un ou plusieurs radicaux choisis dans les atomes d'halogène, le radical hydrogène, trifluorométhyle, méthoxy, éthoxy, méthyle et éthyle,

ou bien d'une part $R_8$ et $R_7$ et d'autre part $R_8$ et $R_9$ forment respectivement avec l'atome d'azote auxquels

ils sont liés un radical imidazolyle, pyrrolyle, pyrrolinyle, pyrrolidinyle, pyridyle, pipéridinyle, pyrimidinyle, pyridazinyle,
pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux hydroxyle, trifluorométhyle, alkyle et alcoxy, ces radicaux renfermant au plus 6 atomes de carbone,
lesdits produits de formule (I) étant sous toutes les formes isomères possibles racémiques, énantiomères et diastéréoisomères,
ainsi que les sels d'addition avec les acides minéraux et organiques desdits produits de formule (I).

Dans les produits de formule ($I_A$) et (I) et dans ce qui suit :

- le terme radical alkyle linéaire ou ramifié désigne de préférence les radicaux méthyle, éthyle, propyle, isopropyle, butyle, isobutyle, sec-butyle et tert-butyle mais peut également représenter un radical pentyle ou hexyle et particulièrement isopentyle et isohexyle,
- le terme radical alkényle linéaire ou ramifié désigne de préférence un radical vinyle, allyle, 1-propényle, butényle et particulièrement 1-butènyle, ou pentényle,
- le terme radical alkynyle linéaire ou ramifié désigne de préférence un radical éthynyle, propargyle, butynyle ou pentynyle,
- les termes radical monocyclique et radical constitué de cycles condensés désignent des radicaux carbocyclique ou hétérocyclique saturés ou insaturés étant entendu que les radicaux hétérocycliques tels que définis ci-dessus peuvent renfermer un ou plusieurs hétéroatomes choisis parmi les atomes d'oxygène, d'azote ou de soufre et que lorsque ces radicaux hétérocycliques comportent plus d'un hétéroatome, les hétéroatomes de ces radicaux hétérocycliques peuvent être identiques ou différents :
- le terme radical monocyclique désigne de préférence les radicaux qui renferment 5 à 7 chaînons :

parmi les radicaux monocycliques carbocycliques saturés, on peut citer, par exemple, les radicaux cyclohexyle et cyclopentyle ;

parmi les radicaux monocycliques carbocycliques insaturés, on peut citer, par exemple, les radicaux cyclopentènyle, cyclohexènyle, cyclopentadiényle, cyclohexadiényle et les radicaux aryles carbocycliques comme le radical phényle ;

parmi les radicaux monocycliques hétérocycliques saturés, on peut citer, par exemple, les radicaux pyrrolidinyle, imidazolidinyle, pyrazolidinyle , pipéridyle, pipérazinyle, morpholinyle, thiomorpholinyle, azépinyle ;

parmi les radicaux monocycliques hétérocycliques insaturés, on peut citer les radicaux aryliques, par exemple, les radicaux thiényle, furyle, pyrannyle, pyrrolyle, imidazolyle, pyrazolyle, pyridyle, pyrazinyle, pyrimidinyle, pyridazinyle, thiazolyle, oxazolyle, furazannyle, pyrrolinyle tel que delta 2-pyrrolinyle, imidazolinyle tel que delta 2-imidazolinyle, pyrazolinyle tel que delta 3-pyrazolinyle ainsi que les isomères de position du ou des hétéroatomes que ces radicaux peuvent renfermer tels que, par exemple, les radicaux isothiazolyle ou isoxazolyle,

- le terme radical constitué de cycles condensés désigne de préférence les radicaux qui renferment 8 à 10 chaînons :
  parmi les radicaux constitués de cycles condensés carbocycliques saturés, on peut citer, par exemple, le bicyclo[4,4,0]décyle, le bicyclo[4,4,1]undécyle,
  parmi les radicaux constitués de cycles condensés carbocycliques insaturés, on peut citer les radicaux aryliques, par exemple, les radicaux naphtyle, indényle, phénanthryle, parmi les radicaux constitués de cycles condensés hétérocycliques saturés, on peut citer, par exemple, le 1-oxa-1 spiro[4,5]décane, le tétrahydropyranne-2-spirocyclohexane, le cyclohexanespiro-2'-(tétrahydrofuranne) ou le 1,10-diaza anthr-4-yle ;
  parmi les radicaux constitués de cycles condensés hétérocycliques insaturés, on peut citer, par exemple, le benzothiényle, le naphto[2,3-b]thiényle, l'indanyle, l'indényle, le thianthrényle, l'isobenzofurannyle, le chroményle, le xanthényle, le phénoxathiinyle, l'indolizinyle, l'isoindolyle, le 3H-indolyle, l'indolyle, l'indazolyle, le purinyle, le quinolizinyle, l'isoquinolyle, le quinolyle, le phtalazinyle, le naphtyridinyle, le quinoxalinyle, le quinazolinyle, le cinnolinyle, le ptéridinyle, le carbazolyle, le béta-carbolinyle, l'acridinyle, le phénazinyle, le phénothiazinyle, le phénoxazinyle, l'indolinyle, l'isoindolinyle ou encore les systèmes polycycliques condensés constitués de monocycliques hétérocycliques tels que définis, par exemple, ci-dessus comme par exemple le furo[2,3-b]pyrrole ou le thiéno[2,3-b]furanne,
- le terme alkylène désigne de préférence le radical méthylène, éthylène, propylène, triméthylène, tétraméthylène, pentaméthylène, hexaméthylène, hydroxyméthylène, hydroxypropylène, méthoxypropylène, 2-méthoxy tétraméthylène,
- le terme alkénylène désigne de préférence le radical vinylène, propénylène, buténylène tel que par

exemple le radical 2-butènylène, hydroxyéthynylène, 1-méthoxy 2-butènylène,

- le terme radical alcoxy linéaire ou ramifié désigne de préférence les radicaux méthoxy ou éthoxy, mais peut aussi représenter un radical propoxy, isopropoxy, butoxy linéaire, secondaire ou tertiaire,
- le terme radical acyle ayant au plus 6 atomes de carbone désigne de préférence un radical formyle, acétyle, propionyle, butyryle ou benzoyle, mais également un radical valéryle, hexanoyle, acryloyle, crotonoyle ou carbamoyle,
- le terme radical acyloxy désigne par exemple un radical dans lequel le radical acyle a les valeurs indiquées ci-dessus et désigne de préférence un radical formyloxy, acétyloxy, propionyloxy, butyryloxy ou benzoyloxy,
- le terme radical aryle désigne les radicaux aryles tels que définis ci-dessus, soit les radicaux insaturés, monocycliques ou constitués de cycles condensés, carbocycliques ou hétérocycliques, étant entendu que les radicaux hétérocycliques peuvent renfermer un ou plusieurs hétéroatomes choisis parmi les atomes d'oxygène, d'azote ou de soufre et que lorsque ces radicaux hétérocycliques comportent plus d'un hétéroatome, les hétéroatomes de ces radicaux hétérocycliques peuvent être identiques ou différents : comme exemples de tels radicaux aryle, on peut citer les radicaux phényle, naphtyle, thiényle tel que 2-thiényle et 3-thiényle, furyle tel que 2-furyle, pyridyle tel que 3-pyridyle, pyrimidyle, pyrrolyle, thiazolyle, isothiazolyle, diazolyle, triazolyle, tétrazolyle, thiadiazolyle, thiatriazolyle, oxazolyle, oxadiazolyle, 3- ou 4-isoxazolyle ; des groupes hétérocycliques condensés contenant au moins un hétéro-atome choisi parmi le soufre, l'azote et l'oxygène, par exemple benzothiényle tel que 3-benzothiényle, benzofuryle, benzopyrrolyle, benzimidazolyle, benzoxazolyle, thionaphtyle, indolyle ou purinyle ;
- le terme radical aryloxy désigne un radical dans lequel le radical aryle a la signification indiquée ci-dessus et désigne ainsi, par exemple, les radicaux phénoxy ou pyridyloxy.

Le ou les radicaux carboxy des produits de formule (I) peuvent être salifiés ou estérifiés par les groupements divers connus de l'homme de métier parmi lesquels on peut citer, par exemple des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,

- les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle, phénéthyle, chrorobenzyle, méthoxybenzyle ou trifluorométhylbenzyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

Les radicaux alkyle, alkényle, alkynyle, les radicaux monocyclique ou constitué de cycles condensés, aryle et aryloxy tels que définis ci-dessus peuvent ne pas être substitués ou porter un ou plusieurs substituants choisis, par exemple, dans le groupe formé par :

- les atomes d'halogène, tel que chloro ou bromo, comme dans, par exemple, le groupe 2-bromoéthyle ou o-chloro-phényle ;
- le radical hydroxyle ;
- les radicaux alkyle, tel que alkyle inférieur, par exemple méthyle, éthyle, ou également isopropyle ou ter-butyle ;
- alkyle substitué tel que par exemple trihaloalkyle comme dans trifluorométhyle ou tel que par exemple le radical N-méthylpyrrolyle ; le radical 3- ou 4-isoxazolyle substitué, par exemple, 3-aryl-5-méthylisoxazol-4-yle, le groupe aryle étant par exemple, un groupe phényle ou halophényle ;
- alkényle tel que, par exemple, vinyle ou allyle ;
- alkynyle tel que, par exemple, propargyle ou éthynyle ;
- aryle tel que défini ci-dessus, soit un radical monocyclique ou constitué de cycles condensés carbocyclique ou hétérocyclique, étant entendu que les radicaux hétérocycliques tels que définis ci-dessus peuvent renfermer un ou plusieurs hétéroatomes choisis parmi les atomes d'oxygène, d'azote ou de soufre

et que lorsque ces radicaux hétérocycliques comportent plus d'un hétéroatome, les hétéroatomes de ces radicaux hétérocycliques peuvent être identiques ou différents, ce radical hétérocyclique pouvant être lié par un atome de carbone ou, le cas échéant, par un atome d'azote tel que par exemple le radical 3- ou 4-isoxazolyle substitué, par exemple, 3-aryl-5-méthylisoxazol-4-yle, le groupe aryle étant par exemple, un groupe phényle ou halophényle ;

- arylalkyle dans lequel le radical aryle est tel que défini ci-dessus comme par exemple benzyle ;
- cycloalkyle, tels que par exemple cyclopropyle, cyclopentyle ou cyclohexyle et cycloalkényle tels que par exemple cyclohexényle, ces radicaux étant éventuellement substitués, parmi lesquels on peut citer le diméthyl-1,3 cyclohexène ;
- alcoxy, tel que défini ci-dessus par exemple méthoxy, éthoxy, propoxy ou isopropoxy comme dans par exemple les groupes méthoxyméthyle ou 1-éthoxyéthyle ;
- alcoxy substitué tel que trihaloalcoxy comme, par exemple, trifluorométhoxy ;
- aryloxy, par exemple phénoxy ;
- aralcoxy, par exemple benzyloxy ;
- mercapto ;
- alkylthio, par exemple méthylthio ou éthylthio ;
- alkylthio substitué tel que trihaloalkylthio comme, par exemple, trifluorométhylthio ;
- arylthio comme par exemple phénylthio ;
- aralkylthio comme par exemple benzylthio ;
- amino comme dans, par exemple, le groupe 2-aminoéthyle ;
- amino substitué par un ou deux radicaux choisis par exemple parmi les radicaux alkyle, alkényle, aryle et arylalkyle tels que définis ci-dessus comme par exemple monoalkylamino dans, par exemple, méthylamino ou éthylamino, comme par exemple dialkylamino dans, par exemple, diméthylamino ;
- un radical hydrocarboné hétérocyclique comprenant 5 à 7 chaînons ou constitué de cycles condensés comprenant 8 à 10 chaînons, ces radixaux pouvant renfermer un ou plusieurs hétéroatomes identiques ou différents, choisis parmi les atomes d'oxygène, d'azote ou de soufre, et étant éventuellement substitués, ce radical hétérocyclique pouvant être lié par un atome de carbone ou le cas échénat par un atome d'azote, par exemple le radical pipéridinyle, morpholinyle, pipérazinyle, méthylpipérazinyle ;
- nitro ;
- cyano ;
- azido ;
- carboxy ;
- carboxy salifié ou estérifié, par exemple méthoxycarbonyle ou éthoxycarbonyle ;
- formyle ;
- acyle, par exemple acétyle, propionyle ou benzoyle ;
- acyle substitué par exemple par un radical amino tel que défini ci-dessus ou par un radical cyclique lié au radical acyle par un atome d'azote, ce radical cyclique pouvant renfermer éventuellement un ou plusieurs hétéroatomes choisis parmi les atomes d'azote, d'oxygène ou de soufre et tel que défini ci-dessus ; on peut également citer les radicaux chloroacétyle ;
- acyloxy, par exemple acétoxy ou propionyloxy ;
- carbamoyle ;
- carbamoyle substitué par exemple un groupe N-monoalkyl inférieur carbamoyle, tel que N-méthylcarbamoyle, N-éthylcarbamoyle, un groupe N,N-dialkyl inférieur carbamoyle, tel que N,N-diméthylcarbamoyle, N,N-diéthylcarbamoyle; un groupe N-(hydroxyalkyl inférieur)carbamoyle, tel que N-(hydroxyméthyl)carbamoyle, N-(hydroxyéthyl)carbamoyle, un groupe carbamoylalkyle inférieur, tel que carbamoylméthyle, carbamoyléthyle ;
- phtalimido ;
- acylamido, par exemple acétamido ou benzamido ; alcoxycarbonylamino, par exemple méthoxycarbonylamino ou éthoxycarbonylamino ; ou aralcoxycarbonylamino, par exemple benzyloxycarbonylamino.

Dans les produits de formule (I) telle que définie ci-dessus,

le radical

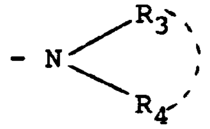

désigne, de préférence :

- lorsque $R_3$ et $R_4$ forment ensemble avec l'atome d'azote auquel ils sont liés un hétérocycle, un radical pyrrolyle, imidazolyle, pyridyle, pyrazinyle, pyrimidyle, indolyle, indolinyle, purinyle, quinolyle, pyrrolidinyle, pipéridyle, pipéridino, morpholino, pipérazinyle ; ces radicaux peuvent être éventuellement substitués par les substituants déjà mentionnés précédemment et en particulier par un ou plusieurs radicaux choisis parmi les atomes de chlore et de fluor,les radicaux méthyle, éthyle, isopropyle, tert-butyle, méthoxy, éthoxy, propoxy, benzoyle, méthoxycarbonyle, éthoxycarbonyle, comme par exemple dans méthylpipérazinyle, éthylpipérazinyle, propylpipérazinyle, phénylpipérazinyle ou benzylpipérazinyle : dans ces deux derniers radicaux, les radicaux phényle et benzyle peuvent être substitués comme indiqué précédemment dans les radicaux aryle, arylalkyle et arylalkényle, comme par exemple dans chlorophényle ou trifluorophényle ;
- lorsque $R_3$ et $R_4$ forment sur l'atome d'azote auquel ils sont liés des radicaux monoalkyl- ou dialkylamino, $R_3$ et $R_4$ désignent des radicaux alkyles linéaires ou ramifiés renferment de 1 à 6 atomes de carbone et en particulier des radicaux méthyle, éthyle, isopropyle, trifluorométhyle, pentafluoroéthyle, hydroxyméthyle, hydroxyéthyle, méthoxyméthyle, méthoxyéthyle, éthoxyéthyle ; les radicaux alkényle tels que définis ci-dessus et représentés de préférence par les radicaux vinyle et allyle ; les radicaux acyle choisis de préférence parmi les radicaux acétyle, propionyle, butyryle, valéryle ou carbamoyle ; les radicaux aryle ou arylalkyle tels que définis ci-dessus, carbocycliques ou hétérocycliques et en particulier phényle, benzyle, phénéthyle, naphtyle, indolyle, indolinyle, thiényle, furyle, pyrrolyle, pyridyle, pyrrolidinyle, pipéridino, morpholino, pipérazinyle, ces radicaux pouvant être substitués par un ou plusieurs radicaux tels que définis ci-dessus comme par exemple dans méthylpipérazinyle, fluorométhylpipérazinyle, éthylpipérazinyle, propylpipérazinyle, phénylpipérazinyle ou benzylpipérazinyle,

Le radical

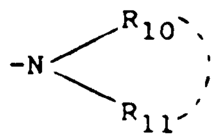

tel que défini ci-dessus dans le radical $R_1$ désigne de préférence les valeurs indiquées ci-dessus pour

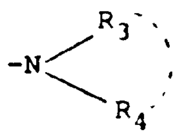

lorsque $R_{10}$ et $R_{11}$ forment ensemble avec l'atome d'azote auquel ils sont liés un hétérocycle ou lorsque $R_{10}$ et $R_{11}$ forment sur l'atome d'azote auquel ils sont liés des racidaux monoalkyl- ou dialkylamino.

Les radicaux

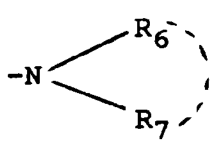

et

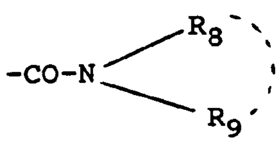

désignent de préférence les radicaux dans lesquels $R_6$, $R_7$, $R_8$ et $R_9$ ont les significations indiquées précédemment pour les radicaux alkyle, alcoxy, acyloxy, acyle ou carboxy salifié ou estérifié.

L'invention a notamment pour objet les produits de formule ($I_A$) et (I) telle que définie ci-dessus caractérisés en ce que le ou les substituants, identiques ou différents que peuvent porter :

a) les radicaux alkyle, alkényle, alkynyle,

b) les radicaux monocyclique ou constitué de cycles condensés, aryle et aryloxy,

sont choisis dans le groupe formé par :

- les atomes d'halogène ; les radicaux hydroxyle ; cyano ; mercapto ; nitro ; acyle et acyloxy ayant au plus 6 atomes de carbone ; carboxy libre, salifié ou estérifié ; alcoxycarbonyle ;
- les radicaux alkoxy linéaires et ramifiés renfermant au plus 6 atomes de carbone ;

- les radicaux alkyle, alkényle, alkynyle, alkylthio, cycloalkyle, cycloalkényle, les radicaux hydrocarbonés hétérocycliques, aryle, aryloxy, arylthio, phénylalkyle, phénylalcoxy, eux-mêmes éventuellement substitués par un ou plusieurs substituants identiques ou différents choisis parmi les atomes d'halogène, les radicaux hydroxyle, trifluorométhyle, nitro, alkyle, alkényle, aryle, alcoxy et acyle, ces radicaux renfermant au plus 6 atomes de carbone, carboxy libre, salifié ou estérifié, acylamido dans lequel le radical acyle renferme au plus 6 atomes de carbone, carbamoyle et amino, ces radicaux étant éventuellement substitués sur l'atome d'azote par un ou deux radicaux identiques ou différents choisis parmi les radicaux hydroxyle, alkyle et alcoxy renfermant au plus 6 atomes de carbone ;
- le radical acylamido dans lequel le radical acyle renferme au plus 6 atomes de carbone ; les radicaux carbamoyle et amino, ces radicaux étant éventuellement substitués sur l'atome d'azote par un ou deux radicaux identiques ou différents choisis parmi les radicaux hydroxyle, alkyle, alkényle et alcoxy renfermant au plus 6 atomes de carbone ; lesdits produits de formule ($I_A$) et (I) étant sous toutes les formes isomères possibles racémiques, énantiomères et diastéréoisomères, ainsi que les sels d'addition avec les acides minéraux et organiques desdits produits de formule ($I_A$) et (I).

Dans les produits de formule ($I_A$) et (I) et dans ce qui suit :

- le terme atome d'halogène désigne de préférence l'atome de chlore, mais peut aussi représenter un atome de fluor, de brome ou d'iode,
- le terme cycloalkyle désigne de préférence les radicaux cyclopropyle, cyclopentyle ou cyclohexyle ;
- le terme cycloalkényle désigne de préférence les radicaux cyclopentényle et cyclohexényle, ces radicaux étant éventuellement substitués ainsi qu'il est indiqué ci-dessus tel que par exemple dans le diméthyl-1,3 cyclohexènyle,
- le terme phénylalkyle désigne de préférence les radicaux benzyle et phénéthyle,
- le terme phénylalcoxy désigne de préférence les radicaux benzyloxy et phénéthoxy,
- le terme acylamido désigne de préférence les radicaux acétamido et benzoylamido,

Comme exemples de radicaux alkyle substitués par un radical aryle, on peut citer, par exemple, les radicaux benzyle, diphénylméthyle, triphénylméthyle, naphtylméthyle, indénylméthyle, thiénylméthyle tel que 2-thiénylméthyle, furylméthyle tel que furfuryle, pyridylméthyle, pyrimidylméthyle ou pyrrolylméthyle, étant entendu que dans la liste non exhaustive d'exemples de radicaux telle que citée ci-dessus, le radical alkyle peut être représenté tout aussi également par les radicaux éthyle, propyle ou butyle tel que, par exemple, dans le radical phénéthyle ;

Comme exemples de radicaux alkényle substitués par un radical aryle, on peut citer, par exemple, les exemples donnés ci-dessus de radicaux arylalkyle dans lesquels le radical alkyle est remplacé par un radical alkényle tel que par exemple dans les radicaux phénylvinyle ou phénylallyle, étant entendu que dans ces radicaux le radical phényle peut être remplacé tout aussi également par un radical naphtyle, pyridyle ou encore par exemple l'un des radicaux aryles tels que définis ci-dessus.

Le radical aryle tel que défini ci-dessus, carbocyclique ou hétérocyclique, désigne de préférence phényle, benzyle, phénéthyle, naphtyle, indolyle, indolizinyle, thiényle, furyle, pyrrolyle, pyridyle.

Le radical hydrocarboné aliphatique hétérocyclique tel que défini ci-dessus désigne de préférence pyrrolidinyle, pipéridino, morpholino, pipérazinyle, ces radicaux pouvant être substitués par un ou plusieurs radicaux tels que définis ci-dessus comme par exemple dans méthylpipérazinyle, fluorométhylpipérazinyle, éthylpipérazinyle, propylpipérazinyle, phénylpipérazinyle ou benzylpipérazinyle.

Les radicaux amino et carbamoyle désignent les radicaux dans lesquels l'atome d'azote peut être substitué par un ou deux radicaux choisis parmi les radicaux tels que définis précédemment :
à titre d'exemple et de façon non exhaustive, on peut citer comme radical carbamoyle substitué le groupe N-monoalkyl inférieur carbamoyle, par exemple, N-méthylcarbamoyle, N-éthylcarbamoyle ; le groupe N,N-dialkyl inférieur carbamoyle, par exemple, N,N-diméthylcarbamoyle, N,N-diéthylcarbamoyle ;
le groupe N-(hydroxyalkyl inférieur)carbamoyle, par exemple, N-(hydroxyméthyl)carbamoyle, N-(hydroxyéthyl)carbamoyle ; le groupe carbamoylalkyle inférieur, par exemple carbamoylméthyle, carbamoyléthyle.
Le radical amino substitué peut être par exemple un radical monoalkyl- ou dialkylamino dans lequel le radical alkyle est choisi parmi les radicaux méthyle, éthyle ou isopropyle.
Des exemples d'un tel radical amino sustitué sont donnés dans la partie expérimentale ci-aprés.

L'invention a particulièrement pour objet les produits de formule ($I_A$) telle que définie ci-dessus répondant à la formule ($I'_A$) :

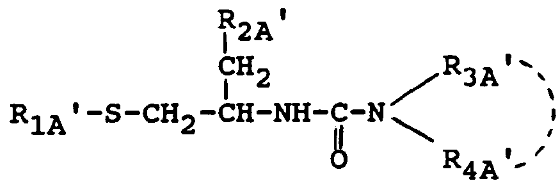

(I'A)

dans laquelle :

$R_{1A}$' représente un atome d'hydrogène ou un radical $R_{5A}$'-CO- dans lequel $R_{5A}$' représente un radical phényle, cycloalkyle, cycloalkényle, alkyle ou alkényle renfermant au plus 6 atomes de carbone, ces radicaux étant éventuellement substitués, ou un radical

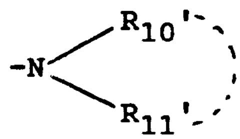

dans lequel $R_{10}$' et $R_{11}$' sont tels que :

**ou bien** $R_{10}$' et $R_{11}$' forment avec l'atome d'azote auxquels ils sont liés un radical imidazolyle, pyrrolyle, pyrrolinyle, pyrrolidinyle, pyridyle, pipéridinyle, pyrimidinyle, piridazinyle, pyrazinyle, pipérazinyle, phénylpipérazinyle, méthylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, mercapto, trifluorométhyle, alkyle, alkényle, alkynyle, alkylthio, alcoxy et phényle éventuellement substitué par un ou plusieurs radicaux choisis parmi les atomes d'halogène et les radicaux trifluorométhyle, hydroxyle, alkyle et alcoxy, les radicaux alkyle et alcoxy renfermant au plus 6 atomes de carbone,

**ou bien** $R_{10}$' et $R_{11}$', identiques ou différents, représentent :

- un atome d'hydrogène ;
- un radical alkyle, alkényle ou alkynyle linéaire ou ramifié renfermant au plus 6 atomes de carbone et éventuellement substitué,
- un radical phényle, benzyle, naphtyle, indényle, imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux hydroxyle, mercapto, trifluorométhyle, les radicaux alkyle, alkényle, alkynyle, alkylthio et alcoxy renfermant au plus 6 atomes de carbone,

$R_2$' représente un radical phényle, naphtyle, benzyle, phénéthyle, indolyle, cycloalkyle, cycloalkényle, ces radicaux étant éventuellement substitués,

$R_{3A}$' et $R_{4A}$' sont tels que :

ou bien $R_{3A}$' et $R_{4A}$' forment avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux hydroxyle, trifluorométhyle, alkyle, alkényle, alkynyle, alcoxy et phényle éventuellement substitué par un ou plusieurs radicaux choisis parmi les atomes d'halogène et les radicaux trifluorométhyle, hydroxyle, alkyle et alcoxy, les radicaux alkyle et alcoxy renfermant au plus 6 atomes de carbone et étant éventuellement substitué par un radical phényle,

ou bien $R_{3A}$' et $R_{4A}$', identiques ou différents, représentent :

- un atome d'hydrogène ;
- un radical hydroxyle ; alcoxy, acyloxy ou acyle renfermant au plus 6 atomes de carbone ; carboxy, salifié ou estérifié ; cyano ;
- un radical alkyle, alkényle ou alkynyle linéaire ou ramifié renfermant au plus 6 atomes de carbone et éventuellement substitué,
- un radical phényle, benzoyle, naphtyle, indényle, imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, diazépine, benzodiazépine, indolyle, ces radicaux étant éventuellement substitués par un ou plusieurs radicaux identiques ou différents choisis parmi les atomes d'halogène, les radicaux hydroxyle, trifluorométhyle, les radicaux alkyle et alcoxy renfermant au plus 6 atomes de carbone, eux-mêmes éventuellement substitués par un ou plusieurs radicaux choisis parmi les atomes d'halogène, les radicaux hydroxyle, alkyle, alcoxy et carboxy, libre, salifié ou estérifié par un radical alkyle, aryle ou arylalkyle,

- le radical

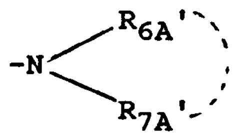 ou 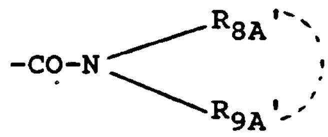

dans lesquels $R_{6A}$', $R_{7A}$', $R_{8A}$' et $R_{9A}$', identiques ou différents, représentent un atome d'hydrogène, un radical hydroxyle, alkyle, alcoxy, acyloxy ou acyle, ces radicaux renfermant au plus 6 atomes de carbone, carboxy libre, salifié ou estérifié, phényle, benzyle, phénéthyle, azépinyle, pipéridyle, morpholine, pyrrolidinyle, pipérazinyle,

ou bien d'une part $R_{6A}$' et $R_{7A}$' et d'autre part $R_{6A}$' et $R_{9A}$' forment respectivement avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux hydroxyle, trifluorométhyle, alkyle et alcoxy, ces radicaux renfermant au plus 6 atomes de carbone, étant entendu que le ou les éventuels substituants, identiques ou différents, que peuvent porter les radicaux que peuvent représenter $R_{1A}$', $R_{2A}$' et $R_{5A}$' sont choisis dans le groupe formé par :

- les atomes d'halogène ; les radicaux hydroxyle ; cyano ; nitro ; acyle et acyloxy ayant au plus 6 atomes de carbone ; benzoyle ; carboxy libre, salifié ou estérifié ;
- les radicaux alkoxy linéaires et ramifiés renfermant au plus 6 atomes de carbone ;
- les radicaux alkyle, alkényle, cyclopentyle, cyclohexyle, cyclopentényle, cyclohexényle, phényle, naphtyle, indolyle, phénoxy, benzyle, phénéthyle, benzyloxy, eux-mêmes éventuellement substitués par un ou plusieurs substituants identiques ou différents choisis parmi les atomes d'halogène, les radicaux hydroxyle, trifluorométhyle, nitro, alkyle, alkényle, alcoxy, ces radicaux renfermant au plus 6 atomes de carbone, formyle, acétyle, benzoyle, carboxy libre, salifié ou estérifié, acétamido, benzoylamido, carbamoyle et amino, ces radicaux étant éventuellement substitués sur l'atome d'azote par un ou deux radicaux identiques ou différents choisis parmi les radicaux hydroxyle, méthyle, éthyle, propyle, méthoxy, éthoxy et propoxy ;
- le radical acylamido dans lequel le radical acyle renferme au plus 6 atomes de carbone ; les radicaux carbamoyle et amino, ces radicaux étant éventuellement substitués sur l'atome d'azote par un ou deux radicaux identiques ou différents choisis parmi les radicaux hydroxyle, alkyle, alkényle, alcoxy et acyle renfermant au plus 6 atomes de carbone ; lesdits produits de formule ($I'_A$) étant sous toutes les formes isomères possibles racémiques, énantiomères et diastéréoisomères, ainsi que les sels d'addition avec les acides minéraux et organiques desdits produits de formule ($I'_A$).

L'invention a aussi particulièrement pour objet les produits de formule ($I_A$) et (I) telle que définie ci-dessus répondant à la formule (I') :

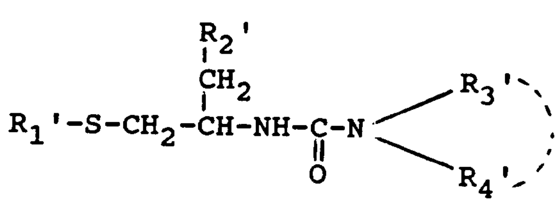

(I')

dans laquelle :

$R_1$' représente un atome d'hydrogène ou un radical $R_6$' - CO - dans lequel $R_5$' représente un radical phényle, cycloalkyle, cycloalkényle, alkyle ou alkényle renfermant au plus 6 atomes de carbone, ces radicaux étant éventuellement substitués, ou un radical

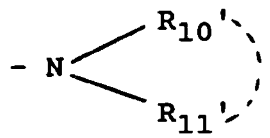

dans lequel $R_{10}$' et $R_{11}$' sont tels que :

**ou bien** $R_{10}$' et $R_{11}$' forment avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, trifluorométhyle, alkyle, alcoxy et phényle éventuellement substitué par un ou plusieurs radicaux choisis parmi les atomes d'halogène et les radicaux trifluorométhyle, hydroxyle, alkyle et alcoxy, les radicaux alkyle et alcoxy renfermant au plus 6 atomes de carbone,

**ou bien** $R_{10}$' et $R_{11}$', identiques ou différents, représentent :

- un atome d'hydrogène ;
- un radical alkyle, alkényle ou alkynyle linéaire ou ramifié renfermant au plus 6 atomes de carbone et éventuellement substitué,
- un radical phényle, benzoyle, naphtyle, indényle, imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, trifluorométhyle, les radicaux alkyle et alcoxy renfermant au plus 6 atomes de carbone,

$R_2$' représente un radical phényle, naphtyle, benzyle, phénéthyle, indolyle, cycloalkyle, cycloalkényle, ces radicaux étant éventuellement substitués,

$R_3$' et $R_4$' sont tels que :

ou bien $R_3$' et $R_4$' forment avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, trifluorométhyle, alkyle, alcoxy et phényle éventuellement substitué par un ou plusieurs radicaux choisis parmi les atomes d'halogène et les radicaux trifluorométhyle, hydroxyle, alkyle et alcoxy, les radicaux alkyle et alcoxy renfermant au plus 6 atomes de carbone,

ou bien $R_3$' et $R_4$', identiques ou différents, représentent :

- un atome d'hydrogène ;
- un radical hydroxyle ; alcoxy, acyloxy ou acyle renfermant au plus 6 atomes de carbone ; carboxy, salifié ou estérifié ; cyano ;
- un radical alkyle, alkényle ou alkynyle linéaire ou ramifié renfermant au plus 6 atomes de carbone et éventuellement substitué,
- un radical phényle, benzoyle, naphtyle, indényle, imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, trifluorométhyle, les radicaux alkyle et alcoxy renfermant au plus 6 atomes de carbone,
- le radical

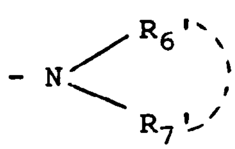 ou 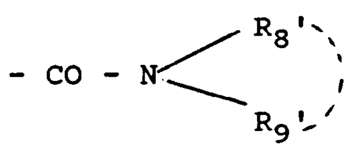

dans lesquels $R_6$', $R_7$', $R_8$' et $R_9$', identiques ou différents, représentent un atome d'hydrogène, un radical hydroxyle, alkyle, alcoxy, acyloxy ou acyle, ces radicaux renfermant au plus 6 atomes de carbone, carboxy libre, salifié ou estérifié, phényle, benzyle, phénéthyle, azépine, pipéridyle, morpholine, pyrrolidinyle, pipérazinyle,

ou bien d'une part $R_6$ et $R_7$ et d'autre part $R_6$ et $R_9$ forment respectivement avec l'atome d'azote auxquels ils sont liés un radical imidazolyle, pyrrolyle, pyrrolinyle, pyrrolidinyle, pyridyle, pipéridinyle,

pyrimidinyle, pyridazinyle, pyrazinyle, pipérazinyle, phénylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, trifluorométhyle, alkyle et alcoxy, ces radicaux renfermant au plus 6 atomes de carbone, étant entendu que le ou les éventuels substituants, identiques ou différents, que peuvent porter les radicaux que peuvent représenter $R_1$', $R_2$' et $R_5$' sont choisis dans le groupe formé par :

- les atomes d'halogène ; les radicaux hydroxyle ; cyano ; nitro ; acyle et acyloxy ayant au plus 6 atomes de carbone ; benzoyle ; carboxy libre, salifié ou estérifié ;
- les radicaux alkoxy linéaires et ramifiés renfermant au plus 6 atomes de carbone ;
- les radicaux alkyle, alkényle, cyclopentyle, cyclohexyle, cyclopentényle, cyclohexényle, phényle, naphtyle, indolyle, phénoxy, benzyle, phénéthyle, benzyloxy, eux-mêmes éventuellement substitués par un ou plusieurs substituants identiques ou différents choisis parmi les atomes d'halogène, les radicaux hydroxyle, trifluorométhyle, nitro, alkyle, alkényle, alcoxy, ces radicaux renfermant au plus 6 atomes de carbone, formyle, acétyle, benzoyle, carboxy libre, salifié ou estérifié, acétamido, benzoylamido, carbamoyle et amino, ces radicaux étant éventuellement substitués sur l'atome d'azote par un ou deux radicaux identiques ou différents choisis parmi les radicaux hydroxyle, méthyle, éthyle, propyle, méthoxy, éthoxy et propoxy ;
- le radical acylamido dans lequel le radical acyle renferme au plus 6 atomes de carbone ; les radicaux carbamoyle et amino, ces radicaux étant éventuellement substitués sur l'atome d'azote par un ou deux radicaux identiques ou différents choisis parmi les radicaux hydroxyle, alkyle, alkényle, alcoxy et acyle renfermant au plus 6 atomes de carbone ; lesdits produits de formule (I') étant sous toutes les formes isomères possibles racémiques, énantiomères et diastéré-oisomères, ainsi que les sels d'addition avec les acides minéraux et organiques desdits produits de formule (I').

L'invention a tout particulièrement pour objet les produits de formule ($I'_A$) telle que définie ci-dessus caractérisés en ce que :

$R_1$' représente un atome d'hydrogène ou un radical acétyle,

$R_2$' représente un radical phényle,

$R_3$' et $R_4$' sont tels que :

ou bien $R_3$' et $R_4$' forment avec l'atome d'azote auxquels ils sont liés un radical pipéridinyle, morpholinyle, diméthylmorpholinyle, thiomorpholinyle, pyrrolidinyle, azépine, diazépine éventuellement substitué sur le second atome d'azote par un radical alkyle renfermant 1 ou 2 atomes de carbone, pipérazinyle éventuellement substitué sur le second atome d'azote soit par un radical phényle éventuellement substitué par un atome d'halogène ou un radical trifluorométhyle, soit par un radical alkyle, alkényle ou alkynyle renfermant 1 à 4 atomes de carbone éventuellement substitué par un radical phényle ou acyle, soit par un radical acyle renfermant au plus 6 atomes de carbone,

**ou bien** $R_3$' et $R_4$', identiques ou différents, représentent un atome d'hydrogène ; un radical hydroxyle ; phényle ; phényle substitué par un radical carboxy, pipéridinyle éventuellement substitué sur l'atome d'azote par un radical benzyle, pipérazinyle éventuellement substitué sur le second atome d'azote par un radical alkyle renfermant 1 ou 2 atomes de carbone, carbamoyle ; alcoxy renfermant au plus 4 atomes de carbone ; alkyle renfermant au plus 4 atomes de carbone éventuellement substitué par un ou plusieurs radicaux, identiques ou différents, choisis parmi les radicaux hydroxyle, alcoxy, carboxy libre, salifié ou estérifié, amino, alkylamino, dialkylamino, phényle, morpholinyle, alkylthio renfermant au plus 4 atomes de carbone, éventuellement substitué par un radical carboxy libre, salifié ou estérifié, benzodiazépine éventuellement substitué, lesdits produits de formule ($I'_A$) étant sous toutes les formes isomères possibles racémiques, énantiomères et diastéréoisomères, ainsi que les sels d'addition avec les acides minéraux et organiques desdits produits de formule ($I'_A$).

L'invention a également tout particulièrement pour objet les produits de formule (I') telle que définie cidessus caractérisés en ce que :

$R_1$' représente un atome d'hydrogène ou un radical acétyle,

$R_2$' représente un radical phényle,

$R_3$' et $R_4$' sont tels que :

**ou bien** $R_3$' et $R_4$' forment avec l'atome d'azote auxquels ils sont liés un radical pipéridinyle, morpholinyle, diméthylmorpholinyle, thiomorpholinyle, pyrrolidinyle, azépine, pipérazinyle éventuellement substitué sur le second atome d'azote par un radical alkyle renfermant 1 ou 2 atomes de carbone ou un radical phényle luimême éventuellement substitué par un atome d'halogène ou un radical trifluorométhyle,

**ou bien** $R_3$' et $R_4$', identiques ou différents, représentent un atome d'hydrogène ; un radical hydroxyle ; phényle ; carbamoyle ; alcoxy renfermant au plus 4 atomes de carbone ; alkyle renfermant au plus 4 atomes de carbone éventuellement substitué par un ou plusieurs radicaux, identiques ou différents, choisis parmi les radicaux hydroxyle, alcoxy, carboxy libre, salifié ou estérifié, lesdits produits de formule (I') étant sous toutes

les formes isomères possibles racémiques, énantiomères et diastéré-oisomères, ainsi que les sels d'addition avec les acides minéraux et organiques desdits produits de formule (I').

Parmi les produits objet de l'invention, peuvent être cités tout particulièrement : les produits de formule (I) répondant aux formules suivantes :
le chlorhydrate de (±) 4-méthyl-N-[2-mercapto 1-(phénylméthyl) éthyl] 1-pipérazinecarboxamide,
le (±) N-[2-mercapto 1-(phénylméthyl) éthyl] 4-morpholinecarboxamide,
le (±) N-[2-mercapto 1-(phénylméthyl) éthyl] 1-pyrrolidinecarboxamide,
le (±) N-[[[2-mercapto 1-(phénylméthyl) éthyl] amino] carbonyl] glycine,
le N,N-bis(2-méthoxy éthyl)-N'-[2-mercapto 1-(phénylméthyl) éthyl] urée,
le (±) cis-2,6-diméthyl N-[2-mercapto 1-(phényl méthyl) éthyl] 4-morpholinecarboxamide (isomère A),
le (±) N-[2-mercapto 1-(phénylméthyl) éthyl] 4-thiomorpholinecarboxamide,
le (±) N-[2-mercapto 1-(phénylméthyl) éthyl] N'-méthoxy urée,
le (±) N-hydroxy N'-[2-mercapto 1-(phénylméthyl) éthyl] N-méthyl urée,
le (±) N-(1-mercaptométhyl) 2-(phényléthyl) 4-méthyl 1-pipérazine acétamide,
ainsi que leurs sels avec les acides minéraux ou organiques.

L'invention a également pour objet un procédé de préparation de produits de formule ($I_A$) telle que définie ci-dessus caractérisé en ce que :

- pour préparer les produits de formule ($I_A$) dans laquelle n = 0, l'on soumet un composé de formule (II):

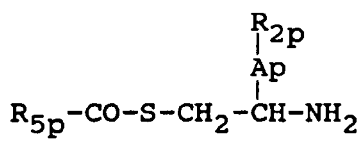

(II)

dans laquelle $R_{5p}$, $R_{2p}$ et Ap ont les significations indiquées ci-dessus respectivement pour $R_5$, $R_2$ et A dans lesquelles les éventuelles fonctions réactives sont éventuellement protégées par des groupements protecteurs, à l'action d'un réactif tel que le phosgène ou le thiophosgène pour obtenir respectivement l'isocyanate ou l'isothiocyanate correspondant de formule (III) :

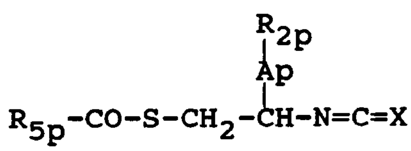

(III)

dans laquelle $R_{5p}$, $R_{2p}$ et Ap ont les significations indiquées précédemment et X représente un atome d'oxygène ou un atome de soufre, que l'on fait réagir avec un composé de formule (IV) :

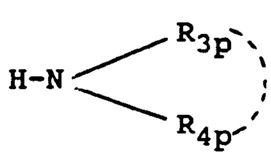

(IV)

dans laquelle $R_{3p}$ et $R_{4p}$ ont les significations indiquées ci-dessus respectivement pour $R_3$ et $R_4$ dans lesquelles les éventuelles fonctions réactives sont éventuellement protégées par des groupements protecteurs, pour obtenir un produit de formule (V):

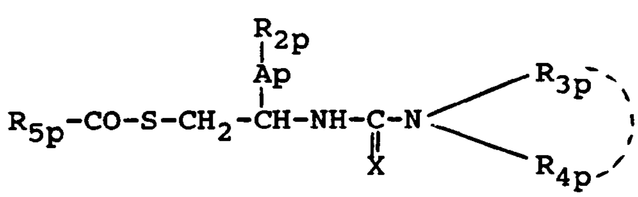

(V)

dans laquelle $R_{5p}$, $R_{2p}$, Ap, $R_{3p}$, $R_{4p}$ et X ont les significations indiquées précédemment, que l'on soumet, si nécessaire et si désiré, une réaction d'élimination du radical $R_{5p}$-CO- pour obtenir un produit de formule

(VI) :

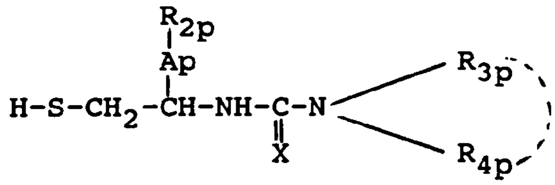

(VI)

dans laquelle $R_{2p}$, Ap, $R_{3p}$, $R_{4p}$ et X ont les significations indiquées précédemment,

- pour préparer les produits de formule ($I_A$) dans laquelle n est différent de 0 et X représente un atome d'oxygène, l'on soumet un produit de formule (II') :

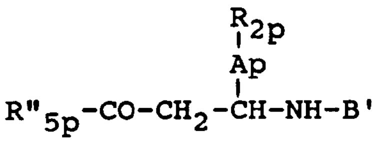

(II')

dans laquelle B' représente un atome d'hydrogène ou un groupe protecteur de la fonction amine et R"$_5$ représente un radical alkyle renfermant de 1 à 4 atomes de carbone substitué par un radical

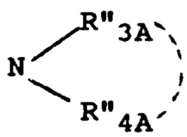

dans lequel R"$_{3A}$ et R"$_{4A}$ ont la valeur indiquée ci-dessus respectivement pour $R_{3A}$ et $R_{4A}$ à l'action le cas échéant d'un agent d'élimination du groupement protecteur porté par la fonction amine, puis à l'action d'un agent alcalin pour obtenir par transposition moléculaire un produit de formule (VII) :

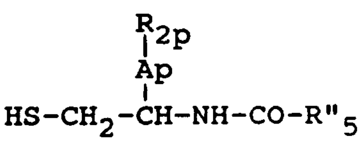

(VII)

dans laquelle $R_{2p}$, $A_p$ et R"$_5$ ont la signification indiquée ci-dessus, produit de formule (V), (VI) et (VII) que l'on traite, si désiré et si nécessaire, à l'une ou plusieurs des réactions suivantes, dans un ordre quelconque :

- une réaction d'élimination des groupements protecteurs que peuvent porter les fonctions réactives protégées,
- une réaction de salification par un acide minéral ou organique pour obtenir le sel correspondant,
- une réaction de réduction de fonction carboxy esterifiée en fonction alcool,
- une réaction de saponification de fonction ester en fonction acide,
- une réaction de transformation de fonction cyano en fonction acide,
- une réaction de transformation de fonction alcoxy en fonction hydroxyle,
- une réaction d'estérification, salification ou carbamoylation de fonction acide,
- une réaction d'estérification de la fonction thiol par un acide ou une fonction acide, ou par un isocyanate ou par un chlorure de carbamoyle.

lesdits produits de formule (I) ainsi obtenus étant sous toutes les formes isomères possibles racémiques, énantiomères et diastéréoisomères.

Dans des conditions préférentielles de mise en oeuvre de l'invention, le procédé ci-dessus est réalisé de la manière suivante :

le composé de formule (II) peut être soumis à l'action d'un chloroformiate tel que par exemple et de préférence le chloroformiate de trichlorométhyle ou diphosgène pour obtenir l'isocyanate correspondant de formule (III)

dans laquelle X représente un atome d'oxygène ;
le composé de formule (II) peut également être soumis à l'action d'un dérivé soufré tel que par exemple et de préférence le thiophosgène pour obtenir l'isothiocyanate correspondant de formule (III) dans laquelle X représente un atome de soufre ;
La réaction d'obtention du produit de formule (III) est réalisée par le mélange du composé de formule (II) avec le réactif correspondant par exemple du phosgène ou du thiophosgène dans un solvant tel que, par exemple, le toluène ou le chlore de méthylène, le mélange étant porté au reflux pendant une durée d'environ une heure et demie.

La réaction d'addition du composé de formule (IV) sur le produit de formule (III) pour obtenir le produit correspondant de formule (V) est réalisée en plaçant le produit de formule (III) dans un solvant tel que par exemple le chlorure de méthylène ou le toluène en présence du composé de formule (IV), le mélange étant de préférence refroidi à une température d'environ 0°C puis agité à la température ambiante pendant une durée d'environ deux heures.

Si nécessaire et si désiré, le produit obtenu de formule (V) peut être soumis à une réaction d'élimination du groupement acyle $R_{5p}$-CO pour obtenir le produit correspondant de formule (VI) : la réaction d'élimination du groupement acyle peut être obtenu, par exemple, en plaçant le produit de formule (V) dans un solvant tel que, par exemple, le tétrahydrofuranne ou le chlorure de méthylène en présence d'hydrate d'hydrazine de préférence à une température d'environ 0°C pendant une durée d'environ une heure ou encore en milieu acide ou alcalin.

L'élimination du groupement protecteur que peut porter la fonction amine du produit de formule (II') est effectuée dans les conditions usuelles connues de l'homme du métier, comme indiqué ci-dessus.

On utilise de préférence l'acide trifluoroacétique.

L'agent alcalin responsable de la transposition moléculaire peut être par exemple une base minérale telle que la soude ou la potasse, un alcoolate tel que le méthylate ou l'éthylate de sodium ou encore un carbonate tel que le bicarbonate de sodium.

Selon les valeurs de $R_{5p}$, $R_{2p}$, Ap, $R_{3p}$ ou $R_{4p}$, les produits de formules (V), (VI) et (VII) constituent ou non des produits de formule ($I_A$).

Les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :

- les groupements hydroxyle peuvent être protégés par exemple par les radicaux alkyle, triméthylsilyle, dihydropyranne, méthoxyméthyle ou tétrahydropyrannyle,
- les groupements amino peuvent être protégés par exemple par les radicaux acétyle, trityle, benzyle, ter butoxycarbonyle, phtalimido ou d'autres radicaux connus dans la chimie des peptides,
- les groupements acyles tel que le groupement formyle peuvent être protégés par exemple sous forme de cétals cycliques ou non cycliques tels que le diméthyl ou diéthylcétal ou l'éthylènedioxy ;
- les fonctions acide des produits décrits ci-dessus peuvent être, si désiré, amidifiées par une amine primaire ou secondaire par exemple en présence de chlorure de méthylène dans du chlorhydrate de 1-éthyl-3-(diméthylaminopropyl) arbodiimide à la température ambiante,
- les groupements carboxy peuvent être protégés par exemple sous forme d'esters formés avec les esters facilement clivables tels que les esters benzyliques ou ter butyliques ou des esters connus dans la chimie des peptides.

L'élimination de ces groupements protecteurs est effectuée dans les conditions usuelles connues de l'homme de métier notamment l'hydrolyse acide effectuée avec un acide tel que l'acide chlorhydrique, benzène sulfonique ou para-toluène sulfonique, formique ou trifluoroacétique.

Le groupement phtalimido est éliminé par l'hydrazine.

On trouvera une liste de différents groupements protecteurs utilisables par exemple dans le brevet BF 2 499 995.

Les produits décrits ci-dessus peuvent, si désiré, faire l'objet de réactions de salification par un acide minéral ou organique réalisées selon les méthodes usuelles connues de l'homme de métier pour obtenir le sel correspondant.

Les éventuelles fonctions carboxy estérifiées des produits décrits ci-dessus peuvent être, si désiré, réduites en fonction alcool par les méthodes connues de l'homme de métier et notamment par de l'hydrure de lithium et d'aluminium dans un solvant tel que par exemple le tétrahydrofurane ou encore le dioxane ou l'éther éthylique.

Les éventuelles transformations de fonctions ester en fonction acide des produits décrits ci-dessus peuvent être, si désiré, réalisées dans les conditions usuelles connues de l'homme de métier notamment par hydrolyse acide ou alcaline par exemple par de la soude ou de la potasse en milieu alcoolique tel que, par exemple, dans du méthanol ou encore par de l'acide chlorhydrique ou sulfurique.

Les éventuelles fonctions cyano des produits décrits ci-dessus peuvent être, si désiré, transformées en fonction acide dans les conditions usuelles connues de l'homme de métier par exemple par une double hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux.

Les éventuelles fonctions alcoxy telles que notamment méthoxy des produits décrits ci-dessus peuvent être, si désiré, transformées en fonction alcool dans les conditions usuelles connues de l'homme de métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide acétique au reflux.

Les éventuelles fonctions acides des produits décrits ci-dessus peuvent être, si désiré, estérifiées, salifiées ou encore amidifiées dans les conditions usuelles connues de l'homme de métier par exemple par action sur l'acide ou une forme activée de celui-ci d'un alcool ou d'une amine.

Les formes optiquement actives des produits de formule (I) peuvent être préparées par dédoublement des racémiques selon les méthodes usuelles.

Les composés de formule (I) tels que définis ci-dessus ainsi que leurs sels d'addition avec les acides présentent d'intéressantes propriétés pharmacologiques.

Ils présentent en particulier de trés intéressantes propriétés inhibitrices de l'enképhalinase et sont doués d'une trés bonne activité analgésique. Ils présentent aussi des propriétés psychotropes et notamment antidépressives et anxiolytiques.

L'enképhalinase est une dipeptidylcarboxypeptidase qui hydrolyse spécifiquement la méthionine et la leucine enképhaline entre le troisième et le quatrième acide aminé libérant ainsi un tripeptide TYR-GLY-GLY ( Swerts, J.P., Perdrisot, R., Patey, G., de la Baume, S., and Schwartz, J.C., Europ. J. Pharmacol. (1979); 57, 279).

L'enképhalinase participe ainsi directement à la dégradation des enképhalines, ligands naturels endogènes des récepteurs opiacés. Les composés de l'invention, qui retardent la dégradation des enképhalines, stimulent donc les réactions de défense de l'organisme contre la douleur.

Certains des composés de l'invention sont des inhibiteurs de l'endopeptidase neutre EC 3.4.24.11. Cette enzyme est impliquée en particulier dans la dégradation des enképhalines et du peptide natriurétique auriculaire (ou ANF). L'ANF est un peptide vasodilatateur, diurétique et natriurétique puissant. L'inhibition de l'endepeptidase neutre EC 3.4.24.11 par ces composés de l'invention peut conduire ainsi à une potentialisation des effets biologiques de l'ANF. Certains composés de l'invention conduisent en particulier à des effets hémodynémiques, diurétiques et natriurétiques.

Ces propriétés justifient leur application en thérapeutique et l'invention a également pour objet à titre de médicaments, les produits tels que définis par la formule (I) ci-dessus,
lesdits produits de formule (I) étant sous toutes les formes isoméres possibles racémiques énantiomères et diastéréoisomères,
ainsi que les sels d'addition avec les acides minéraux ou organiques pharmaceutiquement acceptables desdits produits de formule (I).

L'invention a particulièrement pour objet à titre de médicaments, les produits tels que définis par la formule (I') ci-dessus, lesdits produits de formule (I') étant sous toutes les formes isoméres possibles racémiques énantiomères et diastéréoisomères, ainsi que les sels d'addition avec les acides minéraux ou organiques pharmaceutiquement acceptables desdits produits de formule (I').

L'invention a plus particulièrement pour objet à titre de médicaments, des produits de formule (I') telle que définie ci-dessus caractérisés en ce que :
$R_1$' représente un atome d'hydrogène ou un radical acétyle,
$R_2$' représente un radical phényle,
$R_3$' et $R_4$' sont tels que :

**ou bien** $R_3$' et $R_4$' forment avec l'atome d'azote auxquels ils sont liés un radical pipéridinyle, morpholinyle, diméthylmorpholinyle, thiomorpholinyle, pyrrolidinyle, azépine, pipérazinyle éventuellement substitué sur le second atome d'azote par un radical alkyle renfermant 1 ou 2 atomes de carbone ou un radical phényle lui-même éventuellement substitué par un atome d'halogène ou un radical trifluorométhyle,

**ou bien** $R_3$' et $R_4$', identiques ou différents, représentent un atome d'hydrogène ; un radical hydroxyle ; phényle ; carbamoyle ; alcoxy renfermant au plus 4 atomes de carbone ; alkyle renfermant au plus 4 atomes de carbone éventuellement substitué par un ou plusieurs radicaux, identiques ou différents, choisis parmi les radicaux hydroxyle, alcoxy, carboxy libre, salifié ou estérifié, ces produits étant sous toutes les formes isomères possibles racémiques énantiomères et diastéréoisomères, ainsi que les sels d'addition avec les acides minéraux ou organiques pharmaceutiquement acceptables de ces produits.

L'invention a tout particulièrement pour objet, à titre de médicaments, les produits de formule (I) suivants : les produits de formule (I) répondant aux formules suivantes : le chlorhydrate de (±) 4-méthyl-N-[2-mercapto 1-(phénylméthyl) éthyl] 1-pipérazinecarboxamide,
le (±) N-[2-mercapto 1-(phénylméthyl) éthyl] 4-morpholinecarboxamide,
le (±) N-[2-mercapto 1-(phénylméthyl) éthyl] 1-pyrrolidinecarboxamide,
le (±) N-[[[2-mercapto 1-(phénylméthyl) éthyl] amino] carbonyl] glycine,
le N,N-bis(2-méthoxy éthyl)-N'-[2-mercapto 1-(phénylméthyl) éthyl] urée,
le (±) cis-2,6-diméthyl N-[2-mercapto 1-(phényl méthyl) éthyl] 4-morpholinecarboxamide (isomère A),
le (±) N-[2-mercapto 1-(phénylméthyl) éthyl] 4-thiomorpholinecarboxamide,
le (±) N-[2-mercapto 1-(phénylméthyl) éthyl] N'-méthoxy urée,
le (±) N-hydroxy N'-[2-mercapto 1-(phénylméthyl) éthyl] N-méthyl urée,
le (±) N-(1-mercaptométhyl) 2-(phénylméthyl) 4-méthyl pipérazine acétamide,
ainsi que leurs sels d'addition avec les acides minéraux ou organiques pharmaceutiquement acceptables.

Les médicaments, objet de l'invention, peuvent être utilisés dans le traitement des algies musculaires, articulaires et nerveuses, des affections rhumatismales, des douleurs dentaires, des zonas et des migraines, ainsi que dans le traitement des maladies inflammatoires, notamment des arthroses, des lumbagos et aussi à titre de traitement complémentaire dans les états infectieux et fébriles. Ils peuvent aussi être utilisés pour traiter les états dépressifs.

Certains composés de l'invention présentent une utilité dans le traitement de l'insuffisance cardiaque, de l'insuffisance rénale, l'hypertension associée ou non à une hyperréninehémie, l'hyperaldostéroniome, les oedèmes de différentes origines, le glaucome. Ces composés pourraient également présenter un intérêt thérapeutique dans le traitement des troublesgastro-intestinaux (dont en particulier diarrhées et colon irritable) ainsi que les troubles cognitifs.

L'invention s'étend aux compositions pharmaceutiques renfermant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration, de l'affection traitée et du sujet en cause.

La posologie usuelle, variable selon le produit utilisé, le sujet traité et l'affection en cause, peut être, par exemple, de 20 mg à 2 g par jour chez l'adulte, par voie orale.

Les composés de départ de formule (II') :

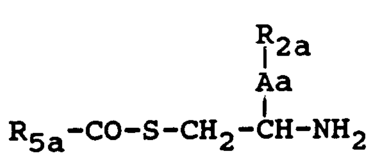

(II')

dans laquelle $R_{5a}$ représente un radical acétyle, Aa représente un radical méthylène et $R_{2a}$ représente un radical phényle peuvent être préparés ainsi qu'il est indiqué dans la demande de brevet numéro PCT/FR87/00367.

La présente invention concerne également un nouveau procédé de préparation des produits de départ de formule (II) telle que définie ci-dessus, caractérisé en ce que l'on soumet un composé de formule (IIa) :

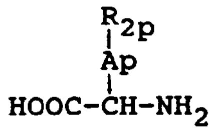

(IIa)

dans laquelle $R_{2p}$ et Ap ont les significations indiquées ci-dessus respectivement pour $R_2$ et A dans lesquelles les éventuelles fonctions réactives sont éventuellement protégées par des groupements protecteurs, à l'action d'un agent réducteur de la fonction acide, pour obtenir l'alcool correspondant de formule (IIb) :

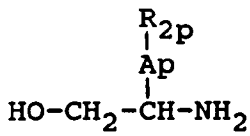

(IIb)

dans laquelle $R_{2p}$ et Ap ont les significations indiquées précédemment, que l'on cyclise sous l'action d'un dérivé carbonylé, pour obtenir l'oxazolidone correspondant de formule (IIc) :

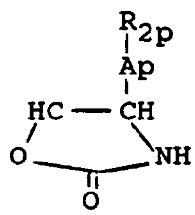

(IIc)

dans laquelle $R_{2p}$ et Ap ont les significations indiquées précédemment, que l'on soumet à l'action d'un agent protecteur, pour obtenir un produit de formule (IId) :

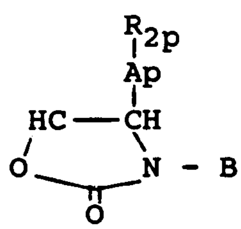

(IId)

dans laquelle $R_{2p}$ et Ap ont les significations indiquées précédemment et B représente un groupement protecteur de la fonction amine, que l'on fait réagir avec un composé de formule (IIe) :

$R_{5p}$-CO-SH (IIe)

dans laquelle $R_{5p}$ a la signification indiquée ci-dessus pour $R_5$ dans laquelle les éventuelles fonctions réactives sont éventuellement protégées par des groupements protecteurs, pour obtenir un composé de formule (IIf) :

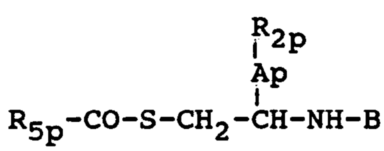

(IIf)

dans laquelle $R_{2p}$, $R_{5p}$, Ap et B ont les significations indiquées précédemment, que l'on soumet, si désiré, à une réaction d'élimination de B, lesdits produits de formule (II) ainsi obtenus étant sous toutes les formes isomères possibles racémiques, énantiomères et diastéréoisomères.

Dans des conditions préférentielles de mise en oeuvre de l'invention, le procédé ci-dessus est réalisé de la manière suivante :

- le composé de formule (IIa) peut être soumis à l'action d'un agent réducteur de la fonction acide selon les méthodes usuelles connues de l'homme de métier telle que par exemple en présence d'hydrure de lithium et d'aluminium dans un solvant tel que par exemple le tétrahydrofuranne ;

- le composé de formule (IIb) ainsi obtenu peut être cyclisé en l'oxazolidone correspondant par exemple en présence de carbodiimidazole dans un solvant tel que par exemple le tétrahydrofuranne : le mélange est porté de préférence à une température d'environ 40 à 60°C pendant une durée d'environ deux heures sous agitation ;
- l'oxazolidone de formule (IIc) ainsi obtenu peut être soumis à l'action d'un agent bloquant B de l'atome d'hydrogène tel que par exemple le di-tertbutyloxycarbonyl en solution dans un solvant tel que l'acétonitrile de préférence en présence d'un catalyseur tel que par exemple le DMAP, le mélange étant de préférence laissé sous agitation pendant une durée variable d'environ 18 heures à la température ambiante ;
- on fait réagir l'oxazolidone ainsi bloqué de formule (IId) avec un composé de formule (IIe) activé par exemple sous forme de sel de sodium ou de potassium tel que par exemple le thioacétate de potassium de préférence en présence d'eau dans un solvant tel que par exemple le diméthylformamide : le mélange est portée de préférence à une température d'environ 100°C pendant une durée d'environ deux heures ;
- le composé de formule (IIf) ainsi obtenu peut être soumis à une réaction d'élimination du groupement bloquant B tel que par exemple le butyloxycarbonyl par des méthodes usuelles connues de l'homme de métier telle que par exemple par barbotage dans un solvant tel que par exemple l'acétate d'éthyle ou l'éther de préférence sous agitation pendant une durée d'environ deux heures et en présence d'un excés d'acide chlorhydrique gazeux pour obtenir le composé correspondant de formule (II) ou encore par l'acide trifluoroacétique par exemple le chlorure de méthylène.

Le composé de formule (IIa) tel que défini ci-dessus peut être un acide aminé naturel tel que la phénylalanine, la tyrosine, le tryptophane que l'on peut trouver sous forme de produit commercialisé par exemple par FLUKA. Les autres produits de formule (IIa) peuvent être préparés par des méthodes connues de l'homme de métier en utilisant par exemple la glycine comme produit de départ, ou peut être préparé selon les méthodes usuelles connues de l'homme de métier.

Les réactifs agissant sur les produits de départ de formule (II), tels que le diphosgène ou le thiophosgène, pour obtenir les produits correspondant de formule (III) peuvent être trouvés sous forme de produit commmercialisé par exemple par FLUKA.

La présente invention a enfin pour objet à titre de produits industriels nouveaux, et notamment à titre de produits intermédiaires nécessaires à la préparation des produits de formule (I), les composés de formule (III).

Les exemples suivants illustrent l'invention sans toutefois la limiter.

**Préparation de : Ethanethioate de S-(2-isocyanato 3-phényl propyle).**

**Stade A : (±) bêta-amino benzènepropanol**

A un mélange agité de 18,4 g d'hydrure d'aluminium et de lithium et 1000 cm3 de tétrahydrofuranne on ajoute sans dépasser 30°C 30 g de (D,L) Phénylalanine. On chauffe 20 minutes au reflux. On refroidit et ajoute 18,4 cm3 d'eau puis 18,4 cm3 d'hydroxyde de sodium en solution à 15 % et 55,2 cm3 d'eau. On filtre l'insoluble, le lave avec du tétrahydrofuranne. Le filtrat est séché puis évaporé à sec sous pression réduite. On recristallise le résidu obtenu dans un mélange acétate d'éthyle-hexane. On obtient 21,2 g de produit recherché F=68-71°C.

Spectre IR ($CHCl_3$)

| | |
|---|---|
| OH | 3620 $cm^{-1}$ |
| $NH_2$ | 3370 $cm^{-1}$ |
| Aromatiques | 1601-1495 $cm^{-1}$ |
| $NH_2$ def | 1580 $cm^{-1}$ |

**Stade B : (±) 4-(phénylméthyl) 2-oxazolidinone**

A une solution de 1,51 g du produit obtenu au stade A, ci-dessus, dans 50 cm3 de tétrahydrofuranne on ajoute 2,4 g de carbonyldiimidazole. On agite 2 heures à 40/45°C et concentre le mélange sous pression réduite. On chromatographie le résidu sur silice, éluant chlorure de méthylène. On obtient 1,2 g du produit recherché F=66°C. Spectre IR ($CHCl_3$)

| | |
|---|---|
| =C-NH | 3452 $cm^{-1}$ |
| C=O | 1760 $cm^{-1}$ |
| Aromatiques | 1495 $cm^{-1}$ |

**Stade C : (±) 2-oxo 4-(phénylméthyl) 3-oxazolidinecarboxylate de (1,1-diméthyl éthyle)**

A une solution de 17,9 g de produit obtenu comme au stade B ci-dessus, dans 150 cm3 d'acétonitrile, on

ajoute 100 mg 4-diméthyl aminopyridine puis 25,5 cm3 de dicarbonate ditertbutyle. On agite 18 heures. On essore le précipité et on obtient 11,4 g du produit recherché F=119°C par concentration des liqueurs mères on recueille encore 3,7 g de produit recherché et 3,4 g supplémentaire par chromatographie sur silice de L'extrait sec du filtrat.

RMN $CDCl_3$ 250 MHz
t-butyl 1,58 ppm
= 2,8 ppm
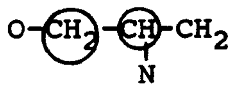 = 4,05 à 4,55 ppm
Aromatique 7,15 ppm

**Stade D : (±) Ethanethioate de S-[2-[((1,1-diméthyléthoxy) carbonyl) amino] 3-phényl propyle]**

A une solution de 2,77 g du produit obtenu au stade C, ci-dessus, dans 28 cm3 de diméthyl formamide. On ajoute 3,42 g de thioacétate de potassium et 0,180 cm3 d'eau. On chauffe pendant 2 heures 30 à 85/87°C. On verse dans 150 cm3 d'eau, extrait avec de l'éther, lave à l'eau, sèche, filtre et concentre sous pression réduite. Après chromatographie sur silice (éluant chlorure de méthylène-acétate d'éthyle 1-1) on obtient 2,6 g du produit recherché F=68-70°C.

Spectre de RMN ppm
t.Bu 1,40 ppm
S-CO-$CH_3$ 2,36 ppm
Les $CH_2$ 2,73 à 3,10 4,8 H
>CH-NH-CO 3,98 ppm 1 H
4,62 m 1H
7,15 à 7,34 ppm 6,5 H 4 aromatiques

**Stade E : (±) chlorhydrate de éthanethioate de S-[2-amino-3-phényl propyle]**

Dans une solution de 1,5 g du produit obtenu au stade C, ci-dessus, dans 30 cm3 d'éther éthylique, on fait barboter de l'acide chlorhydrique gaz pendant 2 heures la température s'élève à 30°C puis se stabilise à 10°C. On agite 3 heures après la fin du barbotage. On essore le précipité ; le lave à l'éther et obtient, après séchage sous pression réduite,
0,78 g de produit attendu F=40°C.

| | |
|---|---|
| RMN ($CDCl_3$) | 250 MHz |
| $CH_3$ | 2,40 ppm |
| 2 $CH_2$ | 3,0 à 3,5 ppm |
| $NH_2$ -CH< | 3,75 ppm |
| $NH_2$ | 8,6 ppm |
| H aromatiques | 7,29 ppm |

**Stade F : Ethanethioate de S-[2-isocyanato-3-phényl propyle]**

On chauffe au reflux pendant 1 heure 30 minutes un mélange de 2,45 g du produit obtenu au stade E, 60 cm3 de toluène, 0,9 cm3 chloroformiate de trichlorométhyle (diphosgène) et 0,125 g de charbon actif (acticarbone 3 SA). On refroidit, filtre, lave avec du toluène et concentre le filtrat à sec sous pression réduite. On obtient 2,5 g de produit recherché utilisé tel quel au départ des exemples. Après chromatographie sur silice, éluant chlorure de méthylène on obtient 1,5 g de produit purifié. Spectre IR ($CHCl_3$)

| | |
|---|---|
| N=C=O | 2265 $cm^{-1}$ |
| >C=O | 1695 $cm^{-1}$ |
| Aromatique | 1600 $cm^{-1}$ |
| | 1580 $cm^{-1}$ |
| | 1496 $cm^{-1}$ |

**Exemple 1 : Ethanethioate de S-[2-(3-méthoxy ureido) 3-phényl propyle].**

A un mélange agité de 2,35 g de l'isocyanate obtenu au stade F de la préparation (ci-dessus) avec 1,252 g de chlorhydrate de O-méthylhydroxylamine et 100 cm3 de chlorure de méthylène, on ajoute à 0°C 2,09 cm3 de triéthylamine en solution dans 20 cm3 de chlorure de méthylène. On agite 2 heures à température ambiante. On verse le mélange réactionnel dans un mélange d'eau et d'acide chlorhydrique 2N à pH1. On décante, lave la phase organique à l'eau, sèche, filtre et évapore à sec. On obtient 3 g d'extrait sec que l'on empâte dans l'éther. Après essorage on obtient 2 g de produit recherché F = 110-112°C.

RMN $CDCl_3$

| | |
|---|---|
| $S-CO-CH_3$ | 2,36 ppm (s) |
| $S-\underline{CH_2}-CH$ | 2,85 ppm (dd) |
| $C_6H_5-\underline{CH_2}-CH$ | 3,00 ppm (dd) |
| | 3,06 ppm (m) |
| $X-CH_3$ | 3,89 ppm (s) |
| 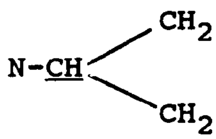 | 4,2 ppm (m) |
| $\underline{NH}-CH$ | 5,86 ppm (d) |
| Aromatiques | 7,2 à 7,3 ppm (m) |

**Exemple 2 : (±) N-[2-mercapto 1-(phénylméthyl) éthyl] N'-méthoxy-urée.**

A une solution de 1,7 g du composé obtenu à l'exemple 1 dans 50 cm3 de tétrahydrofuranne, on ajoute 0,320 cm3 d'hydrate d'hydrazine à 0°C. On agite 1 heure 30 à 0°C. On évapore à sec sous pression réduite et chromatographie le résidu (éluant chlorure de méthylène-méthanol 100-1,5). On obtient 1,05 g du produit recherché F=70°C/72°C.

Analyse pour $C_{11}H_{16}N_2O_2S$ = 240,3

| | | | | |
|---|---|---|---|---|
| % calculés | C=54,98 | H=6,71 | N=11,66 | S=13,34 |
| % trouvés | 54,8 | 6,5 | 11,5 | 13,4 |

Spectre de RMN ($CDCl_3$)

| | |
|---|---|
| $CH_2-\underline{CH}$ | 1,36 ppm |
| $\underline{CH_2}-CH$ | 2,60 ppm à 2,81 ppm |
| $C_6H_5-\underline{CH_2}-CH$ | 2,94 ppm |
| $0-CH_3$ | 3,63 ppm |
| >$\underline{CH}$-NH-CO | 4,24 ppm |
| >CH-$\underline{NH}$-CO | 5,86 ppm |

7,18 à 7,35 ppm H aromatiques

**Exemple 3 : Ethanethioate de S-[2-(3-méthyl 3-méthoxy uréido) 3-phényl propyle.**

On opère comme à l'exemple 1 à partir de 2,35 g de l'isocyanate obtenu selon la préparation 1 en utilisant 1,463 cm3 de chlorhydrate de N,O diméthylhydroxylamine et 2,3 cm3 de triéthyl amine. On obtient 2,2 g du produit recherché F=68/70°C.

| Spectre RMN ($CDCl_3$) | ppm |
|---|---|
| $COCH_3$ | 2,36 |
| $CON-CH_3$ | 3,05 |
| $CONOCH_3$ | 3,55 |
| 4,14 | $N-\underline{CH}-CH_2$<br>$CH_2$ |
| $C_6H_5-\underline{CH_2}-CH$<br>et<br>$S-\underline{CH_2}-CH$ | 2,81-2,97-3,05 |
| $\underline{NH}-CH$ | 5,92 |
| Aromatiques | 7,14 à 7,37 |

**Exemple 4 : (±) N-[2-mercapto 1-(phénylméthyl) éthyl] N'-méthoxy N'-méthyl urée.**

On opère comme à l'exemple 2 à partir de 1,758 g du produit obtenu à l'exemple 3 en utilisant 0,320 cm3 d'hydrate d'hydrazine on obtient, après sublimation à 100°C sous pression réduite, 860 mg du produit pur attendu F= 50°C.

Analyse pour $C_{12}H_{18}N_2O_2S$ = 254,4

| | | | | |
|---|---|---|---|---|
| % calculés | C=56,67 | H=7,13 | N=11,01 | S=12,61 |
| % trouvés | 56,7 | 7,1 | 10,8 | 12,5 |

| RMN $CDCl_3$ | ppm |
|---|---|
| $\underline{SH}-CH_2$ | 1,34 |
| $SH-\underline{CH_2}-CH$ | 2,58 à 2,78 |
| $C_6H_5-\underline{CH_2}-CH$ | 2,92 |
| $\underset{\underset{O}{\|}}{C}-N-\underline{CH_3}$ | 3,06 |
| $O-CH_3$ | 3,58 |
| $C_6H_5-CH_2-\underline{CH}-NH-\underset{\underset{O}{\|}}{C}-$ | 4,17 |
| $\underset{\underset{O}{\|}}{C}-\underline{NH}-CH$ | 5,94 |
| Aromatiques | 7,15 à 7,45 |

**Exemple 5 : Ethanethioate de S-[-2-(3-méthoxy-3-hydroxy uréido) 3-phényl propyle].**

On opère comme à l'exemple I à partir de 2,35 g de l'isocyanate obtenu à la préparation 1 en utilisant 1,225 g de chlorhydrate de N-méthyl hydroxylamine et 2,09 cm3 de triéthylamine. On obtient 1,9 g du produit recherché. Rf=0,36 (éluant acétate d'éthyle-n hexane 7-3).

| RMN $CDCl_3$ | ppm |
|---|---|
| N-CH-$CH_2$ | 4,1 |
| $CH_2$ | |
| $COCH_3$ | 2,34 ppm |
| S-$CH_2$-CH | |
| et | 2,3 à 3,1 |
| $C_6H_5$-$CH_2$-CH | |
| $CONCH_3$ | 3,07 |
| | 3,08 |
| NH-CH | 6,11 |
| | 6,18 |
| Aromatiques | 7,1 à 7,4 |

**Exemple 6 :(±) N-hydroxy N'-[2-mercapto 1-(phénylméthyl) éthyl] N-méthyl urée.**

On opère comme à l'exemple 2 à partir de 1,7 g du produit obtenu à l'exemple 5 en utilisant 0,320 cm3 d'hydrate d'hydazine. On obtient 1,2 g du produit recherché.

| Analyse pour $C_{11}H_{16}N_2O_2S$ = 240,3 | | | | |
|---|---|---|---|---|
| % calculés | C=54,98 | H=6,71 | N=11,66 | S=13,34 |
| % trouvés | 55,2 | 6,8 | 11,6 | 13,4 |

| RMN ($CDCl_3$) | ppm |
|---|---|
| $CH_2$-SH | 1,35 |
| $CH_2$-SH | 2,50 à 2,70 |
| $C_6H_5$-$CH_2$-CH | 2,75 à 2,95 |
| N-$CH_3$ | 3,06 |
| CH-NH-CO | 4,12 |
| CH-NH-CO | 6,19 |
| 7,15 à 7,35 | aromatiques |
| 1 H mobile | 8,05 |

**Exemple 7 : (±) éthanethioate de B-[2-[[[(2-hydroxy éthyl) méthyl amino] carbonyl] amino] 3-phényl propyle].**

On opère comme à l'exemple 1 à partir de 2,35 g de l'isocyanate obtenu à la préparation 1 en utilisant 1,1 cm3 de 2-méthylaminoéthanol. On obtient, après chromatographie sur silice (éluant acétate d'éthyle-n hexane 9-1) 2 g de produit recherché.

| Spectre IR | CHCl$_3$ | |
|---|---|---|
| OH | 3620 cm$^{-1}$ | |
| NH | 3425 cm$^{-1}$ | |
| 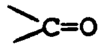 | 1682 cm$^{-1}$ | 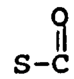 |
| | 1636 cm$^{-1}$ | 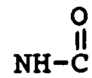 |
| Amide II | 1526 cm$^{-1}$ | |
| | 1600 cm$^{-1}$ | |
| | 1496 cm$^{-1}$ | |

**Exemple 8 : (±) N-(2-hydroxy éthyl) N'-[2-mercapto-1-(phénylméthyl) éthyl]-N-méthyl urée.**

On opère comme à l'exemple 2 à partir de 2 g du produit obtenu à l'exemple 7 en utilisant 0,360 cm3 d'hydrate d'hydrazine. On obtient après empâtage dans l'hexane 1,2 g de produit F=86°C que l'on recristallise dans l'éther isopropylique. On recueille 0,7 g du produit recherché F=86°C.

Analyse pour $C_{13}H_{20}N_2O_2S$ = 268,38

| | | | | |
|---|---|---|---|---|
| % calculés | C=58,18 | H=7,51 | N=10,438 | S=11,95 |
| % trouvés | 58,5 | 7,8 | 10,5 | 11,7 |

| Spectre IR | | |
|---|---|---|
| OH | 3620 cm$^{-1}$ | |
| NH | 3448 cm$^{-1}$ | |
| C=O | 1636 cm$^{-1}$ | 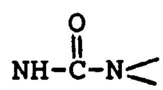 |
| Amide II | 1518 cm$^{-1}$ | |
| Aromatiques | 1497 cm$^{-1}$ | |

**Exemple 9 : (±) éthanethioate de S-[2-[[[bis(2-méthoxy éthyl) amino] carbonyl] amino] 3-phényl propyle].**

On opère comme à l'exemple I à partir de 2,35 g de l'isocyanate obtenu selon la préparation 1 en utilisant 2 g de di-(méthoxyméthylamine). On obtient après chromatographie sur silice (éluant chlorure de méthylène-acétate d'éthyle 7-3) 2 g de produit recherché.

| Spectre IR - CHCl$_3$ | | |
|---|---|---|
| =C-NH | 3341 cm$^{-1}$ | |
| C=O | 1689 cm$^{-1}$ | 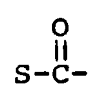 |

| | | |
|---|---|---|
| | 1644 $cm^{-1}$ | 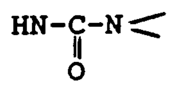 |
| Amide II | 1535 $cm^{-1}$ | |

**Exemple 10 : N,N-bis(2-méthoxy éthyl)-N'-[2-mercapto 1-(phénylméthyl) éthyl] urée.**

On opère comme à l'exemple 2 à partir de 2 g du produit obtenu à l'exemple 9 en utilisant 0,290 cm3 d'hydrate d'hydrazine. On obtient après chromatographie sur silice (éluant chlorure de méthylène acétate d'éthyle 7-3) 1,5 g du produit attendu.

Analyse pour $C_{16}H_{26}N_2O_3S$ = 326,46

| | | | | |
|---|---|---|---|---|
| % calculés | C=58,87 | H=8,03 | N=8,58 | S=9,821 |
| % trouvés | 58,9 | 8,2 | 8,6 | 9,7 |

| Spectre IR $CHCl_3$ | |
|---|---|
| SH | 2570 $cm^{-1}$ |
| =C-NH | 3340 $cm^{-1}$ |
| C=O | 1642 $cm^{-1}$ |
| Amide II | 1532 $cm^{-1}$ |

**Exemple 11 : N-[[[2-(acétylthio) 1-(phénylméthyl) éthyl] amino] carbonyl]-glycinate de méthyle.**

On opère comme à l'exemple 1 à partir de 2,35 g du produit obtenu à la préparation I en utilisant 1,9 g de chlorhydrate de glycine méthyl ester et 2,1 cm3 de triéthylamine. On obtient après chromatographie sur silice (éluant chlorure de méthylène-acétate d'éthyle 7-3) et empâtage dans le n hexane : 2,1 g du produit recherché F=102°C.

| Spectre IR | $CHCl_3$ |
|---|---|
| =C-NH | 3425 $cm^{-1}$ |
| >C=O | 1476 $cm^{-1}$ |
| 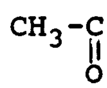 | 1440 $cm^{-1}$ |
| Autre C=O | 1684 $cm^{-1}$ |
| Amide II | 1534 $cm^{-1}$ |

**Exemple 12 : (±) N-[[[2-mercapto 1-(phénylméthyl) éthyl] amino] carbonyl] glycine.**

A une solution de 1,6 g du produit obtenu à l'exemple 11, dans 30 cm3 de méthanol, on ajoute 10 cm3 de soude N à 0°C. On agite 6 heures à température ambiante, dilue à l'eau et acidifie avec de l'acide chlorhydrique N. On extrait avec du chlorure de méthylène, lave à l'eau, sèche et évapore à sec sous pression réduite. On obtient, après empâtage dans l'hexane 0,65 g du produit recherché F=110°C.

Analyse pour $C_{12}H_{16}N_2O_3S$ = 268,336

| | | | | |
|---|---|---|---|---|
| % calculés | C=53,713 | H=6,01 | N=10,439 | S=11,949 |
| % trouvés | 53,8 | 6,1 | 10,3 | 11,9 |

Spectre IR Nujol

| | |
|---|---|
| Absorption NH/OH | |
| C=O | 1758 $cm^{-1}$ |
| | 1745 $cm^{-1}$ |
| Aromatique | 1594 $cm^{-1}$ |
| Amide II | 1585 $cm^{-1}$ |
| | 1510 $cm^{-1}$ |
| | 1495 $cm^{-1}$ |

**Exemple 13 : Ethanethioate de S-(2-uréido 3-phényl propyle).**

On opère comme à l'exemple 1 à partir de 3 g de l'isocyanate obtenu à la préparation I et en faisant barboter de l'ammoniae pendant 45 minutes dans le milieu à 0°C. on dilue à l'eau, extrait avec du chlorure de méthylène, lave, sèche et évapore à sec sous pression réduite. Après chromatographie sur silice (éluant acétate d'éthyle n-hexane 9-1). On obtient 1,6 g de produit recherché F=90°C.

Spectre IR $CHCl_3$

| | |
|---|---|
| Absence de N=C=O | |
| =C-$NH_2$ | 3511 $cm^{-1}$ |
| =C-NH- | 3418 $cm^{-1}$ |
| | 3384 $cm^{-1}$ |
| C=O | 1681 $cm^{-1}$ |
| $NH_2$ dif | 1599 $cm^{-1}$ |
| Amide II | 1525 $cm^{-1}$ |
| Aromatique | 1497 $cm^{-1}$ |

**Exemple 14 : (±) N-[2-mercapto 1-(phénylméthyl) éthyl] urée.**

On opère comme à l'exemple 2 à partir de 1,2 g du produit obtenu à l'exemple 13 en utilisant 0,270 cm3 d'hydrate d'hydrazine. On obtient après recristallisation dans l'éther isopropylique : 0,65 g de produit recherché F=118°C.

| Analyse pour $C_{10}H_{14}N_2OS$ = 210,3 | | | | |
|---|---|---|---|---|
| % calculés | C=57,114 | H=6,71 | N=13,32 | S=15,246 |
| % trouvés | 56,9 | 6,7 | 13,0 | 15,1 |

Spectre IR

| | |
|---|---|
| NH/$NH_2$ | 3515 $cm^{-1}$ |
| | 3419 $cm^{-1}$ |
| C=O complexe | 1679 $cm^{-1}$ |
| $NH_2$ def | 1597 $cm^{-1}$ |
| Amide II | 1518 $cm^{-1}$ |
| Aromatique | 1497 $cm^{-1}$ |
| SH | 2575 $cm^{-1}$ |

**Exemple 15 : Ethanethioate de S-[2-(3-phényl uréido) 3-phényl propyle].**

On opère comme à l'exemple 1 à partir de 3 g d'isocyanate obtenu comme à la préparation 1 en utilisant 1,39 $cm^{-1}$ d'aniline. On obtient après chromatographie sur silice (éluant acétate d'éthyle-n hexane 5-5), 3 g de produit recherché.

Spectre IR $CHCl_3$

| | |
|---|---|
| Absence de N=C=O | |
| CNH | 3427 $cm^{-1}$ |
| | 3367 $cm^{-1}$ |
| C=O Complexe | 1682 $cm^{-1}$ |
| Amide II | 1544 $cm^{-1}$ |
| | 1527 $cm^{-1}$ |
| Aromatique | 1599 $cm^{-1}$ |
| | 1499 $cm^{-1}$ |

**Exemple 16 : (±) N-[2-mercapto 1-(phénylméthyl) éthyl] N'-phényl urée.**

On opère comme à l'exemple 2 à partir de 2 g du produit obtenu à l'exemple 15 en utilisant 0,330 cm3 d'hydrate d'hydrazine. On obtient, après chromatographie sur silice (éluant acétate d'éthyle-n hexane 6-4) 1,1 g de produit que l'on empâte dans l'hexane puis recristallise dans un mélange éther isopropylique-acétate d'éthyle (1-1). On recueille 0,82 g de produit recherché F=138°C.

Analyse pour $C_{16}H_{18}N_2OS$ = 286,398

| | | | | |
|---|---|---|---|---|
| % calculés | C=67,101 | H=6,33 | N=9,78 | S=11,195 |
| % trouvés | 66,9 | 6,3 | 9,6 | 11,2 |

RMN-$CDCl_3$ (250 MHz) ppm

| | |
|---|---|
| SH-$CH_2$ | 1,33 |
| HS-<u>$CH_2$</u>-CH | 2,67 |
| $C_6H_5$-<u>$CH_2$</u>-CH | 2,88 |
| $CH_2$-CH(-$CH_2$)-N-CO | 4,25 |
| Aromatiques | 7,05 à 7,30 |

**Exemple 17 : Ethanethioate de S-[2-[3-bis(propyl) ureido] 3-phényl propyle].**

On opère comme à l'exemple 1 à partir de 3 g de l'isocyanate obtenu selon la préparation I en utilisant 1,94 cm3 de dipropylamine. On obtient 3 g de produit recherché F=60°C.

**Exemple 18 : (±) N,N-dipropyl N'-[2-mercapto 1-(phénylméthyl) éthyl] urée.**

On opère comme à l'exemple 2 à partir de 1,5 g du produit obtenu à l'exemple 17. On obtient 680 mg du produit recherché F=68°C.

**Exemple 19 : Ethanethioate de S-[2-[3,3-bis(2-hydroxy éthyl) uréido] 3-phényl propyle].**

On opère comme à l'exemple 1 à partir de 2,35 g d'isocyanate obtenu selon la préparation I en utilisant 1,25 cm3 de diéthanolamine. On obtient, après chromatographie sur silice (éluant chlorure de méthylène-méthanol (95-5)) 2,5 g du produit recherché.

Spectre IR ($CHCl_3$)

| | |
|---|---|
| OH | 3620 $cm^{-1}$ |
| NH | 3348 $cm^{-1}$ |
| C=O | 1687 $cm^{-1}$ |
| | 1627 $cm^{-1}$ |

| | |
|---|---|
| Amide II | 1531 $cm^{-1}$ |
| Aromatique | 1497 $cm^{-1}$ |

**Exemple 20 : (±) N,N-bis(2-hydroxy éthyl) N'-[2-mercapto 1-(phénylméthyl) éthyl] urée.**

On opère comme à l'exemple 2 à partir de 2 g du produit obtenu à l'exemple 19 en utilisant 0,358 cm3 d'hydrate d'hydrazine. On obtient après chromatographie sur silice (éluant chlorure de méthylène-méthanol 9-1) 0,95 g du produit recherché.

| Analyse pour $C_{14}H_{22}N_2O_3S$ = 298,41 | | | | |
|---|---|---|---|---|
| % calculés | C=56,35 | H=7,43 | N=9,38 | S=10,745 |
| % trouvés | 56,2 | 7,7 | 9,2 | 10,7 |

RMN $CDCl_3$ (250 MHz) ppm

| | |
|---|---|
| $\underline{SH}-CH_2$ | 1,38 |
| $HS-\underline{CH_2}-CH$ | 2,66 |
| $C_6H_5\underline{CH_2}CH$ | 2,89 |
| $NCH_2CH_2O$ | 3,37 |
| | 3,72 |
| Proton | 3,82 |
| $CH_2-\underline{CH}NCO$ (avec $CH_2$ en branche sur CH) | 4,13 |
| NH-CH | 6,04 |
| Aromatiques | 7,2 à 7,4 |

**Exemple 21 : Ethanethioate de S-[2-[[(1-pyrrolidinyl) carbonyl] amino] 3-phényl propyle].**

On opère comme à l'exemple 1 à partir de 2,35 g du produit obtenu à la préparation I en utilisant 1,25 cm3 de pyrrolidine. On obtient 1,8 g de produit recherché F=100-102°C.

RMN $CDCl_3$

| | |
|---|---|
| S-Ac | 2,35 ppm |
| les $CH_2$ en béta de N | 1,38 ppm |
| les $CH_2$ en alpha de N | 3,26 ppm |
| $C_6H_5-CH_2$ et $CH_2-S$ | 2,6 à 3,1 ppm |
| $\underline{CH}$-NH | 4,16 ppm |
| $\underline{NH}$ | 4,56 ppm |
| H aromatique | 7,2 à 7,3 ppm |

**Exemple 22 : (±) N-[2-mercapto 1-(phénylméthyl) éthyl] 1-pyrrolidinecarboxamide.**

On opère comme à l'exemple 2 à partir de 1,7 g du produit obtenu à l'exemple 21 en utilisant 0,290 cm3 d'hydrate hydrazine. On obtient 860 mg du produit recherché F=92-94°C.

Analyse pour $C_{14}H_{20}N_2OS$ = 264,4

| | | | | |
|---|---|---|---|---|
| % calculés | C=63,6 | H=7,6 | N=10,6 | S=12,13 |
| % trouvés | 63,4 | 7,7 | 10,3 | 12,0 |

RMN ($CDCl_3$) ppm

| | |
|---|---|
| $CH_2$-SH | 1,31 |
| $CH_2$ en 3 et 4 | 1,89 |
| $CH_2$ en 2 et 5 | 3,30 |
| <u>$CH_2$</u>-SH | 2,67 |
| $C_6H_5-CH_2$ | 2,91 |
| $CH_2-\underset{NH-CO}{\underset{\vert}{\underline{CH}}}-CH_2$ | 4,24 |
| <u>NH</u>-CO | 4,40 |
| Aromatiques | 7,15 à 7,35 |

**<u>Exemple 23 :</u> (±) Ethanethioate de S-[2-[[(hexahydro-1H-1-azépinyl) carbonyle] amino] 3-phényl propyle].**

On opère comme à l'exemple 1 à partir de 2,35 g de l'isocyanate obtenu selon la préparation I en utilisant 2,52 cm3 d'hexaméthylénémine. On obtient, après chromatographie sur silice (éluant chlorure de méthylène-acétate d'éthyle 7-3) et empâtage dans l'hexane : 2,5 g du produit recherché F=97°C.

Spectre IR

| | |
|---|---|
| $S-\overset{O}{\overset{\Vert}{C}}-Me$ | 1681 $cm^{-1}$ |
| =C-NH | 3435 $cm^{-1}$ |
| C=O | 1634 $cm^{-1}$ |

**<u>Exemple 24 :</u> (±) hexahydro-N-[2-mercapto 1-(phénylméthyl) éthyl] 1H-azépine-1-carboxamide.**

On opère comme à l'exemple 2 à partir de 1,5 g du produit obtenu à l'exemple 23 en utilisant 0,260 cm3 d'hydrate d'hydrazine. On obtient, après recristallisation dans l'éther isopropylique 0,7 g de produit recherché F=102°C.

Analyse pour $C_{16}H_{24}N_2OS$ = 292,446

| | | | | |
|---|---|---|---|---|
| % calculés | C=65,713 | H=8,27 | N=9,58 | S=10,964 |
| % trouvés | 65,9 | 8,4 | 9,5 | 11,1 |

<u>Spectre IR</u> ($CHCl_3$)

| | |
|---|---|
| C=O | 1633 $cm^{-1}$ |
| Amide II | 1589 $cm^{-1}$ |
| =C-NH | 3344 $cm^{-1}$ |

**Exemple 25 : Ethanethioate de S-[2-[[(1-pipéridinyl) carbonyl] amino] 3-phényl propyle].**

On opère comme à l'exemple 1 à partir de 1,81 g de l'isocyanate obtenu selon la préparation I en utilisant 1,74 cm3 de pyridine. On obtient 1,775 g de produit recherché F=114-116°C.

| RMN ($CDCl_3$) | ppm |
|---|---|
| S-$COCH_3$ | 2,35 |
| $CH_2$-N-CO | 3,25 |
| $CH_2$ cycliques | 1,51 |
| $C_6H_5$-$CH_2$ | 2,74 |
| et | |
| $CH2_2$ S | 2,91 à 3,20 |
| NH-CH | 4,15 |
| NH-CH | 4,83 |
| Aromatiques | 7,15 à 7,35 |

**Exemple 26 : (±) N-[2-mercapto 1-(phénylméthyl) éthyl] 1-pyridinecarboxamide.**

On opère comme à l'exemple 2 à partir de 1,6 g du produit obtenu à l'exemple 25 en utilisant 0,290 cm3 d'hydrate d'hydrazine. On obtient après chromatographie sur silice (éluant chlorure de méthylène-acétate d'éthyle 85-15) : 900 mg de produit recherché F=115-1170°C après empâtage dans l'éther.

Analyse pour $C_{15}H_{22}N_2OS$ = 278,4

| | | | | |
|---|---|---|---|---|
| % calculés | C=64,71 | H=7,96 | N=10,06 | S=11,52 |
| % trouvés | 64,7 | 8,1 | 10,0 | 11,4 |

| RMN ($CDCl_3$) | |
|---|---|
| SH-$CH_2$ | |
| $CH_2$ en 3,4 et 5 (pipéridine) | 1,53 |
| $C_6H_5$-$CH_2$- <br> CH-$CH_2$ | 2,87 à 3,00 |
| $CH_2$ en 2 et 6 | 3,28 |
| CH-NH-CO | 4,23 |
| CH-NH-CO | 4,64 |
| Aromatiques | 7,20 à 7,33 |

**Exemple 27 : Ethanethioate de S-[2-[[(4-thiomorpholinyl) carbonyl] amino] 3-phényl éthyle].**

On opère comme à l'exemple 1 à partir de 1,8 g de l'isocyanate obtenu selon la préparation I en utilisant 1,76 cm3 de thiomorpholine. On obtient 2 g de produit recherché.

| RMN | |
|---|---|
| S-AC | 2,36 ppm |

| | |
|---|---|
| les $CH_2$-S cycliques | 2,53 ppm |
| les $CH_2$-N-C(=O) | 3,61 ppm |
| $C_6H_5$-<u>$CH_2$</u> et <u>$CH_2$</u>-S-Ac | 2,75 ppm 1 H |
| | 2,91 à 3,11 ppm 3 H |
| NH-<u>CH</u>< | 4,15 ppm |
| <u>NH</u>-CH | 4,93 ppm |
| Phényle | 7,20 à 7,34 ppm |

**<u>Exemple 28 :</u> (±) N-[2-mercapto 1-(phénylméthyl) éthyl] 4-thiomorpholinecarboxamide.**

On opère comme à l'exemple 2 à partir de 1,6 g du produit obtenu à l'exemple 27 en utilisant 0,290 cm3 d'hydrate d'hydrazine. On obtient 1,15 g du produit recherché F=98-100°C.

Analyse pour $C_{14}H_{20}N_2OS_2$ = 296,5

| | | | | |
|---|---|---|---|---|
| % calculés | C=56,72 | H=6,80 | N=9,45 | S=21,63 |
| % trouvés | 57,0 | 6,9 | 9,4 | 21,5 |

| RMN (CDCl$_3$) | ppm |
|---|---|
| <u>SH</u> $CH_2$ | 1,31 |
| $C_6H_5$-<u>$CH_2$</u>-CH(-<u>$CH_2$</u>) | 2,53 |
| $CH_2$-<u>$CH_2$</u>-S-<u>$CH_2$</u>-$CH_2$ | 2,69-2,9 |
| $CH_2$-<u>$CH_2$</u>-N-<u>$CH_2$</u>-$CH_2$ | 3,63 |
| $CH_2$-<u>CH</u>-$CH_2$ (NH-CO) | 4,24 |
| $CH_2$-CH-$CH_2$ (<u>NH</u>-CO) | 4,6 |
| Aromatiques | 7,19 à 7,35 |

**<u>Exemple 29 :</u> Ethanethioate de S-[2-[[(4-morpholinyl) carbonyl) amino] 3-phényl propyle].**

On opère comme à l'exemple 1 à partir de 2,2 g d'isocyanate obtenu comme à la préparation I en utilisant 1,8 cm3 de morpholine. On obtient après chromatographie sur silice éluant chlorure de méthylène-méthanol 100-1) 1,9 g de produit recherché F=105°C.

| Spectre IR (CHCl$_3$) | |
|---|---|
| NH complexe | 3428 $cm^{-1}$ |
| C=O | 1675 |
| | 1645 |

| | |
|---|---|
| Aromatique | 1603 |
| + | 1580 |
| Amide II | 1522 |
| | 1497 |

**Exemple 30 : (±) N-[2-mercapto 1-(phénylméthyl) éthyl] 4-morpholinecarboxamide.**

On opère comme à l'exemple 2 à partir de 1,9 g du produit obtenu à l'exemple 29 en utilisant 0,34 cm3 d'hydrate d'hydrazine. On obtient, après chromatographie sur silice (éluant chlorure de méthylène-méthanol 100-1,5) 1,5 g du produit recherché F=84°C.

| | |
|---|---|
| Spectre IR | ($CHCl_3$) |
| SH | 2575 $cm^{-1}$ |
| =C-NH | 3457 $cm^{-1}$ |
| C=O | 1644 $cm^{-1}$ |
| Amide II | 1509 $cm^{-1}$ |

**Exemple 31 : Ethanethioate de S-[2-[[[4-(cis-2,6-diméthyl) morpholinyl] carbonyl] amino] 3-phényl propyle.**

On opère comme à l'exemple 1 à partir de 4,7 g de l'isocyanate obtenu selon la préparation I et en utilisant 3,7 cm3 de 2,6 diméthyl morpholine. On obtient 1,2 g de produit (isomère A) isolé de l'éther PF=120-122°C. Après chromatographie des liqueurs mères (éluant acétate d'éthyle-hexane (7-3)). On obtient à nouveau 2,5 g de l'isomère A PF=120-122°C et 1 g de produit PF=98-100°C (isomère B).

| | | |
|---|---|---|
| RMN - isomère A | ($CDCl_3$ 250 MHz) | |
| $\underline{CH_3}$-CH | 1,18 ppm (d,J=6 Hz) | |
| S-CO-$\underline{CH_3}$ | 2,35 ppm (s) | |
| $\underline{NH}$-CH | 4,91 ppm (d) | |
| CO-N $\underline{CH}$-$CH_2$ (CH–$CH_2$) | 4,17 ppm (m) | |
| 3,4 à 3,7 (m) | 4 H | |
| 2,88 à 3,13 (m) | 3 H | autres protons |
| 2,73 (dd) | 1 H | non aromatiques |
| 2,45 (m) | 2 H | |
| 7,15 à 7,35 (m) | aromatiques | |

**Exemple 32 : (±) cis-2,6-diméthyl N-[2-mercapto 1-(phényl méthyl) éthyl] 4-morpholinecarboxamide (isomère A).**

On opère comme à l'exemple 2 à partir de 2,1 g du produit, isomère A, obtenu à l'exemple 31 en utilisant 0,320 cm3 d'hydrate d'hydrazine. On obtient 1,3 g du produit recherché F=120-122°C.

Analyse pour $C_{16}H_{24}N_2O_2S$ = 308,45

| | | | | |
|---|---|---|---|---|
| % calculés | C=62,31 | H=7,84 | N=9,08 | S=10,4 |
| % trouvés | 62,3 | 8,0 | 9,0 | 10,5 |

| RMN (CDCl3) | ppm |
|---|---|
| les $\underline{CH_3}$-CH | 1,18-1,19 |
| $\underline{SH}$-$CH_2$ | 1,31 |
| $C_6H_5\underline{CH_2}$-CH-$\underline{CH_2}$-S | 4,49 |
| et | 2,68 |
| N-$\underline{CH_2}$-CH | 2,91 |
| et | 3,65 |
| O-$\underline{CH}$(CH_3)-$CH_2$ | 3,52 |
| N-$\underline{CH}$(CH_2)-$CH_2$ | 4,25 |

**Exemple 33 : (±) Ethanethioate de S-[2-[[(4-méthyl-1-pipérazinyl) carbonyl] amino] 3-phényl propyle].**

On opère comme à l'exemple 1 à partir de 2,35 g de l'isocyanate obtenu selon la préparation I et en utilisant 1,44 cm3 de N-méthyl piprazine. On obtient après chromatographie sur silice, éluant chlorure de méthylène-méthanol (9-1) 3,2 g du produit recherché F=78°C.

| Spectre IR | ($CHCl_3$) |
|---|---|
| NH | 3430 $cm^{-1}$ |
| C=O | 1676 $cm^{-1}$ |
| | 1639 $cm^{-1}$ |
| Amide II | 1520 $cm^{-1}$ |

**Exemple 34 : Chlorhydrate de (±) 4-méthyl-N-[2-mercapto 1-(phénylméthyl) éthyl] 1-pipérazinecarboxamide.**

On opère comme à l'exemple 2 à partir de 2 g du produit obtenu à l'exemple 33 en utilisant 0,36 cm3 d'hydrate d'hydrazine. Après chromatographie sur silice (éluant chlorure de méthylène-méthanol 9-1). On obtient 1,2 g de produit sous forme de base. Obtention du chlorhydrate. On dissout 1,2 g du produit ci-dessus dans 10 cm3 d'acétate éthyle et ajoute une solution éthanolique saturée d'acide chlorhydrique jusqu'à pH 1. On obtient 1 g du chlorhydrate recherché PF=170°C. Après recristallisation dans l'acétate d'éthyle-isopropanol on recueille 0,75 g de produit F=176°C.

Analyse pour $C_{15}H_{24}ClN_3OS$ = 329,895

| | | | | | |
|---|---|---|---|---|---|
| % calculés | C=54,61 | H=7,33 | N=12,74 | Cl=10,746 | S=9,72 |
| % trouvés | 54,5 | 7,5 | 12,6 | 11,0 | 9,6 |

| Spectre IR | $CHCl_3$ |
|---|---|
| NH | 3430 $cm^{-1}$ |
| >C=O | 1676 $cm^{-1}$ |
| | 1639 $cm^{-1}$ |
| Amide II | 1520 $cm^{-1}$ |

**Exemple 35 : (±) Ethanethioate de S-[2-[[(4-phényl) 1-pipérazinyl) carbonyl] amino] 3-phényl propyle].**

On opère comme à l'exemple 1 à partir de 2,35 g de l'isocyanate obtenu selon la préparation I en utilisant 2 cm3 de 1 phényl pipérazine. Après chromatographie sur silice (éluant chlorure de méthylène-acétate d'éthyle 9-1). On obtient 2,94 g de produit recherché F=100°C.

| Spectre IR | ($CHCl_3$) |
|---|---|
| NH | 3430 $cm^{-1}$ |

| | |
|---|---|
| C=O | 1674 $cm^{-1}$ |
| | 1642 $cm^{-1}$ |
| Aromatique | 1600 $cm^{-1}$ |
| | 1581 $cm^{-1}$ |
| | 1495 $cm^{-1}$ |

**<u>Exemple 36 :</u> (±) N-[2-mercapto 1-(phénylméthyl) éthyl] 4-phényl 1-pipérazinecarboxamide.**

On opère comme à l'exemple 2 à partir de 1,5 g du produit obtenu à l'exemple 35 en utilisant 0,22 cm3 d'hydrate d'hydrazine. Après chromatographie sur silice (éluant chlorure de méthylène-acétate d'éthyle 9-1) on obtient 0,7 g de produit attendu F=122°C après empâtage dans l'heptane.

Analyse pour $C_{20}H_{25}N_3OS$ = 355,5

| | | | | |
|---|---|---|---|---|
| % calculés | C=67,572 | H=7,088 | N=11,82 | S=9,019 |
| % trouvés | 67,9 | 7,2 | 11,9 | 8,9 |

RMN ($CDCl_3$) ppm

$SH-CH_2$ 1,32

$>SHCH_2CH$ 2,6 1

$C_6H_5-\underline{CH_2}-CH$ à 3,0

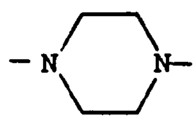 3,16 3,50

$CH_2-CH-NCO$ (H encerclé), $CH_2$ 4,27

$\underline{NH}-CH$ 4,72

Aromatiques 6,93

7,2 à 7,35

**<u>Exemple 37 :</u> (±) Ethanethioate de S-[2[[[4-[3-(trifluorométhyl) phényl] 1-pipérazinyl] carbonyl] amino] 3-phényl propyle].**

On opère comme à l'exemple 1 à partir de 2,35 g de l'isocyanate obtenu selon la préparation I en utilisant 2,81 cm3 de 4-[3-(trifluorométhyl) phényl] pipérazine. On obtient 3,9 g de produit recherché F=78-80°C cristallisé de l'hexane.

| RMN | ($CDCl_3$) | ppm |
|---|---|---|
| S-Ac | | 2,37 (s) |
| 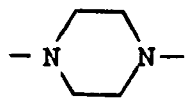 | | 3,20 (m) 4 H<br>3,49 (m) 4 H |
| $C_6H_5-CH_2$ et $S-CH_2$ | | 2,70 à 3,2 (m) |
| $CH_2-\underline{CH}-CH_2$ | | 4,17 (m) |
| $\underline{NH}-CO$ | | 5,06 (d) |
| Aromatiques | | 7,03 à 7,40 (m) 4 H |

**Exemple 38 : (±) N-[2-mercapto 1-(phénylméthyl) éthyl] 4-[3-(trifluorométhyl) phényl] 1-pipérazinecarboxamide.**

On opère comme à l'exemple 2 à partir de 2,3 g du produit obtenu à l'exemple 37 en utilisant 0,266 cm3 d'hydrate d'hydrazine. On obtient 1,4 g du produit recherché F=130-132°C.

Analyse pour $C_{21}H_{24}F_3N_3OS$ = 423,5

| | | | | | |
|---|---|---|---|---|---|
| % calculés | C=59,56 | H=5,71 | N=9,92 | S=7,57 | F=13,46 |
| % trouvés | 59,6 | 5,8 | 9,9 | 7,6 | 13,6 |

| RMN | ($CDCl_3$) | ppm |
|---|---|---|
| SH | | 1,33 |
| $SH-\underline{CH_2}$ | | 2,6 à 2,80 |
| $C_6H_5-CH_2$ | | 2,80 à 3,05 |
| 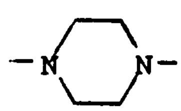 | | 3,20<br>3,51 |
| -CH- | | 4,25 |
| NH-CO | | |
| $-CH-\underline{NH}-CO$ | | 4,72 |

**Exemple 39 : (±) Ethanethioate de S-[2-[[[4-(4-chlorophényl) 1-pipérazinyl] carbonyl] amino] 3-phényl propyle].**

On opère comme à l'exemple 1 à partir de 2,35 g d'isocyanate obtenu selon la préparation I en utilisant 2,55 g de p-chlorophényl piprazine. On obtient 2,8 g (après empâtage dans l'hexane) du produit recherché. Après chromatographie sur silice (éluant acétate d'éthyle-hexane 7-3) on recueille 2,5 g de produit attendu F=140°C.

| | |
|---|---|
| Spectre IR | $CHCl_3$ |
| =C-NH | 3430 $cm^{-1}$ |
| C=O | 1676 $cm^{-1}$ |

| | |
|---|---|
| | 1644 $cm^{-1}$ |
| Aromatique | 1598-1572-1496 $cm^{-1}$ |
| Amide II | 1522 $cm^{-1}$ |

**<u>Exemple 40 :</u> (±) 4-(4-chloro phényl) N-[2-mercapto 1-(phénylméthyl) éthyl] 1-pipérazinecarboxamide.**

On opère comme à l'exemple 2 à partir de 2,5 g du produit obtenu à l'exemple 39 en utilisant 0,340 cm3 d'hydrate d'hydrazine. On obtient, cristallisé de l'hexane 1,8 g de produit recherché F=140°C. Après chromatographie sur silice (éluant acétate d'éthyle-hexane 1-1) on recueille 0,8 g du produit attendu F=148°C.

Analyse pour $C_{20}H_{24}N_3ClOS$ = 389,951

| | | | | | |
|---|---|---|---|---|---|
| % calculés | C=61,6 | H=6,2 | N=10,77 | Cl=9,091 | S=8,22 |
| % trouvés | 61,5 | 6,1 | 10,7 | 9,3 | 8,3 |

Spectre IR

| | |
|---|---|
| SH | 2580 $cm^{-1}$ |
| =C-NH | 3450 $cm^{-1}$ |
| C=O | 1645 $cm^{-1}$ |
| Aromatique + Amide II | 1598-1509-1497 $cm^{-1}$ |

En opérant comme aux exemples 1 et 2 respectifs, à partir de l'isocyanate obtenu au stade F de la préparation, et des réactifs appropriés, on a obtenu les produits des exemples 41 à 58.

<u>Exemple 41</u> : **Ethanethioate de S-[2-(3,3-diméthyl uréido) 3-phénylpropyle].**

F = 70°C.

<u>Exemple 42</u> : **(±) N,N-diméthyl N'-[1-(mercaptométhyl) 2-phényléthyl]** urée.

F ≈ 60°C.

<u>Exemple 43</u> : **Ethanethioate de S-[2-[3-(2-phényléthyl) uréido] 3-phénylpropyle].**

<u>Exemple 44</u> : **(±) N-[[1-(mercaptométhyl) 2-phényléthyl] N'-(2-phényléthyl) urée.**

F ≈ 62°C.

<u>Exemple 45</u> : **Ethanethioate de S-[2-(3-méthyl uréido) 3-phénylpropyle].**

F = 80°C.

<u>Exemple 46</u> : **(±) N-[1-(mercaptométhyl) 2-phényléthyl] N'-méthyl) urée.**

F = 66°C.

**Exemple 47 : Ethanethioate de S-[2-[[(4-hydroxyéthyl 1-pipérazinyl) carbonyl] amino] 3-phénylpropyle].**

**Exemple 48 : (±) 4-(2-hydroxyéthyl) N-[1-(mercaptométhyl) 2-phényléthyl] 1-pipérazinecarboxamide et son chlorhydrate.**

Le chlorhydrate est obtenu comme indiqué à l'exemple 34.
F = 210°C.

**Exemple 49 : Ethanethioate de S-[2-[[(4-éthyl 1-pipérazinyl) carbonyl] amino] 3-phénylpropyle].**

**Exemple 50 : (±) 4-éthyl N-[1-(mercaptométhyl) 2-phényléthyl] 1-pipérazinecarboxamide et son chlorhydrate.**

Le chlorhydrate est obtenu comme indiqué à l'exemple 34.
F = 198°C.

**Exemple 51 : Ethanethioate de S-[2-[[(4-phénylméthyl 1-pipérazinyl) carbonyl] amino] 3-phénylpropyle].**

**Exemple 52 : (±) N-[1-(mercaptométhyl) 2-phényléthyl] 4-(phényléthyl) 1-pipérazinecarboxamide et son oxalate.**

L'oxalate est préparé à partir de la base dissoute dans l'acétate d'éthyle par addition d'une solution d'acide oxalique dans un mélange acétate d'éthyle-méthanol.
F = 192°C.

**Exemple 53 : Ethanethioate de (±) S-[2-[[(2-benzoylhydrazo) carbonyl] amino] 3-phénylpropyle].**

F = 122°C.

**Exemple 54 : (±) 2-benzoylhydrazide de l'acide [1-(mercaptométhyl) 2-phényléthyl] carbamique.**

F = 158°C.

**Exemple 55 : Ethanethioate de (±) S-[2-[[[(2,3-dihydro 2-oxo 5-phényl 1H-1,4-benzodiazepin-3-yl) amino] carbonyl] amino] 3-phénylpropyle].**

F = 210°C.

**Exemple 56 : N-(2,3-dihydro 1-méthyl 2-oxo 5-phényl 1H-1,4-benzodiazépin-3-yl) N'-[1-(mercaptométhyl) 2-phényléthyl] urée.**

F = 235°C.

**Exemple 57 : (±) N-[[[1-[(acétylthio) méthyl] 2-phényléthyl] amino] carbonyl] glycine.**

F = 120°C.

**Exemple 58 : Ethanethioate de S-[2-[[(4-propyl 1-pipérazinyl) carbonyl] amino] 3-phénylpropyle].**

**Exemple 59 : (±) N-[1-(mercaptométhyl) 2-phényléthyl] 4-propyl 1-pipérazinecarboxamide et son chlorhydrate.**

On agite pendant 24 heures à température ambiante 2,5 g de produit obtenu à l'exemple 58 dans 20 $cm^3$ d'éthanol et 4 $cm^3$ d'une solution éthanolique d'acide chlorhydrique (6,6N), évapore le solvant, reprend dans l'acétate d'éthyle, laisse cristalliser et essore. On reprend le résidu dans l'isopropanol à chaud, glace, essore et sèche à 60°C sous pression réduite. On recueille 1,2 g de produit attendu sous forme de chlorhydrate.

F = 192°C.

En opérant comme aux exemples 1 à 58 respectifs, à partir de l'isocyanate obtenu au stade F de la préparation et des réactifs appropriés, on a obtenu les produits des exemples 60 à 79.

**Exemple 60 : Ethanethioate de (±) S-[2-[[[4-(3-phényl 2-propényl) 1-pipérazinyl] carbonyl] amino] 3-phénylpropyle].**

**Exemple 61 : (±) N-[1-(mercaptométhyl) 2-phényléthyl] 4-(3-phényl 2-propényl) 1-pipérazinecarboxamide et son chlorhydrate.**

F = 211°C.

**Exemple 62 : Ethanethioate de S-[2-[[(hexahydro 1H-1,4-diazépin-1-yl) carbonyl] amino] 3-phénylpropyle].**

**Exemple 63 : (±) hexahydro N-[1-(mercaptométhyl) 2-phényléthyl] 1H-1,4-diazépin 1-carboxamide et son chlorhydrate.**

F = 165°C.

**Exemple 64 : Ethanethioate de S-[2-[[[4-(2-propényl) 1-pipérazinyl] carbonyl] amino] 3-phénylpropyle].**

F = 80°C.

**Exemple 65 : (±) N-[1-(mercaptométhyl) 2-phényléthyl] 4-(2-propényl) 1-pipérazinecarboxamide et son chlorhydrate.**

F = 190°C.

**Exemple 66 : Ethanethioate de S-[2-[[[4-(1-méthyléthyl) 1-pipérazinyl] carbonyl] amino] 3-phénylpropyle].**

**Exemple 67 : (±) N-[1-(mercaptométhyl) 2-phényléthyl] 4-(1-méthyléthyl) 1-pipérazinecarboxamide et son chlorhydrate.**

F = 202°C.

**Exemple 68 : Ethanethioate de (±) S-[2-[[[4-(2-propynyl) 1-pipérazinyl] carbonyl] amino] 3-phénylpropyle].**

**Exemple 69 : (±) N-[1-(mercaptométhyl) 2-phényléthyl] 4-(2-propynyl) 1-pipérazinecarboxamide et son chlorhydrate.**

F = 170°C.

**Exemple 70 : (±) 4-[[[1-[(acétylthio) méthyl] 2-phényléthyl] amino] carbonyl] 1-pipérazinecarboxylate d'éthyle.**

F = 84°C.

**Exemple 71 : (±) 4-[[[1-(mercaptométhyl) 2-phényléthyl] amino] carbonyl] pipérazine 1-carboxylate d'éthyle.**

La base est obtenue par addition d'une solution aqueuse saturée en bicarbonate de sodium au résidu huileux de chlorhydrate.
F = 121°C.

**Exemple 72 : (±) 4-[[[1-[(acétylthio) méthyl] 2-phényléthyl] amino] carbonyl] 1-pipérazine acétate d'éthyle.**

**Exemple 73 : (±) 4-[[[1-(mercaptométhyl) 2-phényléthyl] amino] carbonyl] 1-pipérazine acétate d'éthyle et son oxalate.**

L'oxalate est préparé à partir de la base comme indiqué à l'exemple 52 ; la base étant obtenue par addition de carbonate de potassium au résidu huileux contenant le chlorhydrate.

**Exemple 74 : Ethanethioate de S-[2-[[[4-(2-phényléthyl) 1-pipérazinyl] carbonyl] amino] 3-phénylpropyle].**

F + 100°C.

**Exemple 75 : (±) N-[1-(mercaptométhyl) 2-phényléthyl] 4-(2-phényléthyl) 1-pipérazinecarboxamide et son chlorhydrate.**

F = 215°C.

**Exemple 76 : Ethanethioate de (±) S-[2-[[[[1-(phénylméthyl) 4-pipéridinyl] amino] carbonyl] amino] 3-phénylpropyle].**

**Exemple 77 : (±) N-[1-(mercaptométhyl) 2-phényléthyl] N'-[1-(phénylméthyl) 4-pipéridinyl] urée et son chlorhydrate.**

F = 212°C.

**Exemple 78 : (±) Ethanethioate de S-[2-[[[2-(diéthylamino) éthyl] amino] carbonyl] amino] 3-phénylpropyle].**

F = 52-54°C.

**Exemple 79 : (±) N-[2-(diéthylamino) éthyl] N'-[1-(mercaptométhyl) 2-phényléthyl] urée.**

La base est obtenue par addition d'une solution aqueuse saturée de bicarbonate de sodium au résidu huileux de chlorhydrate.

Dans les exemples 80 à 93 le produit de départ est identique à celui obtenu au stade E de la préparation. La synthèse de l'isocyanate, identique à celle indiquée au stade F, est effectuée in situ.

**Exemple 80 : (±) N-[[[1-[(acétylthio) méthyl] 2-phényléthyl] amino] carbonyl] glycinate de phénylméthyle.**

F = 80-82°C.

**Exemple 81 : (±) N-[[[1-(mercaptométhyl) 2-phényléthyl] amino] carbonyl] glycinate de phénylméthyle.**

F = 116-118°C.

L'élimination du groupement acyle a été effectuée au moyen d'hydrate d'hydrazine.

**Exemple 82 : (±) 3-[[[[1-[(acétylthio) méthyl] 2-phényléthyl] amino] carbonyl] amino] propanoate de méthyle.**

F = 60-62°C.

**Exemple 83 : Acide (±) 3-[[[[(1-mercaptométhyl) 2-phényléthyl] amino] carbonyl] amino] propanoïque.**

On refroidit à 0°C 1,89 g de produit préparé à l'exemple 81 dans 18 $cm^3$ de méthanol, ajoute 11,2 $cm^3$ de

soude N et agite 16 heures à température ambiante. On ajoute 5,6 $cm^3$ de soude N, agite 1 heure, évapore le solvant sous pression réduite, verse le milieu réactionnel dans 25 $cm^3$ d'acide chlorhydrique 2N additionné de 25 $cm^3$ d'eau et extrait à l'acétate d'éthyle. Après concentration à sec sous pression réduite, on reprend le résidu dans le chlorure de méthylène, concentre à sec de nouveau et cristallise le résidu dans l'éther. Après recristallisation dans l'acétate d'éthyle acidifié par de l'acide chlorhydrique 2N, on obtient 0,74 g de produit attendu. F = 88-90°C.

**Exemple 84 : Acide (±) 3-[[[[1-[(acétylthio) méthyl] 2-phényléthyl] amino] carbonyl] amino] benzoïque.**

F = 198-200°C.

**Exemple 85 : Acide (±) 3-[[[[(1-mercaptométhyl) 2-phényléthyl] amino] carbonyl] amino] benzoïque.**

F = 178-180°C.

L'élimination du groupement acyle est effectuée comme indiqué à l'exemple 83.

**Exemple 86 : Acide (±) 2-[[[[1-[(acétylthio) méthyl] 2-phényléthyl] amino] carbonyl] amino] benzoïque.**

F = 178-180°C.

**Exemple 87 : Acide (±) 2-[[[[(1-mercaptométhyl) 2-phényléthyl] amino] carbonyl] amino] benzoïque.**

F = 162-164°C.

L'élimination du groupement acyle est effectuée comme indiqué à l'exemple 83.

**Exemple 88 : Acide (±) 4-[[[[1-[(acétylthio) méthyl] 2-phényléthyl] amino] carbonyl] amino] benzoïque.**

F = 218-220°C.

**Exemple 89 : Acide (±) 4-[[[[(1-mercaptométhyl) 2-phényléthyl] amino] carbonyl] amino] benzoïque.**

F = 160-162°C.

L'élimination du groupement acyle est effectuée comme indiqué à l'exemple 83.

**Exemple 90 : Ethanethioate de (±) S-[2-[[[[2-(4-morpholinyl) éthyl] amino] carbonyl] amino] 3-phénylpropyle].**

F = 78-80°C.

**Exemple 91 : (±) N-[1-(mercaptométhyl) 2-phényléthyl] N'-[2-(4-morpholinyl) éthyl] urée.**

F = 88-90°C.

L'élimination du groupement acyle est effectuée comme indiqué à l'exemple 58 et la base est obtenue comme indiqué à l'exemple 71.

**Exemple 92 : (±) Ethanethioate de S-[2-[[(1-pipérazinyl) carbonyl] amino] 3-phénylpropyle].**

**Exemple 93 : (±) N-[1-(mercaptométhyl) 2-phényléthyl] 1-pipérazinecarboxamide sous forme de chlorhydrate.**

F = 238-240°C.

**Exemple 94 : (±) N-3-[[[1-(mercaptométhyl) 2-phényléthyl] amino] carbonyl] glycinate de méthyle.**

On agite 16 heures à température ambiante 2,04 g de produit obtenu à l'exemple 11 dans 5 $cm^3$ de mé-

thanol en présence de 20 $cm^3$ de solution méthanolique d'acide chlorhydrique. On concentre, reprend l'huile obtenue dans du chlorure de méthylène, lave à l'eau, sèche et élimine le solvant sous pression réduite. On reprend dans un mélange hexane-éther, essore, sèche sous pression réduite à température ambiante et obtient 1,442 g de produit attendu. F = 70-72°C.

**Exemple 95 : (±) N-[[[2-[[(phénylamino) carbonyl] thio] 1-(phénylméthyl) éthyl] amino] carbonyl] glycinate de méthyle.**

On mélange à température ambiante 1,35 g de produit obtenu à l'exemple 94, 15 $cm^3$ de chlorure de méthylène et 0,52 $cm^3$ de phényl isocyanate, agite 24 heures, ajoute 0,2 $cm^3$ de phényl isocyanate, agite 12 heures, ajoute encore 0,2 $cm^3$ du réatif et agite 12 heures supplémentaires. On élimine le solvant sous pression réduite, reprend le résidu dans un mélange acétate-d'éthyle-chlorure de méthylène (2-8) et obtient après filtration puis recristallisation dans l'acétate d'éthyle 540 mg de produit attendu. F = 152-154°C.

**Exemple 96 : (±) N-[[[2-[[(phénylamino) carbonyl] thio] 1-(phénylméthyl) éthyl] amino] carbonyl] glycine.**

On refroidit à 0°C 1,11 g du produit préparé à l'exemple 12 dans 15 $cm^3$ de chlorure de méthylène, ajoute 1,2 $cm^3$ de triéthylamine et 0,49 $cm^3$ de phényl isocyanate dissous dans 5 $cm^3$ de chlorure de méthylène. On agite 1 heure, verse le milieu réactionnel dans une solution comprenant 10 $cm^3$ d'eau et 8 $cm^3$ d'acide chlorhydrique 2N, filtre le précipité, le redissout dans l'acétate d'éthyle. Après élimination du solvant sous pression réduite, on récupère 1,52 g de produit brut que l'on recristallise dans l'acétate d'éthyle. On obtient 0,983 g de produit attendu. F = 179-181°C.

**Exemple 97 : (±) Phénylthiocarbamate de S-[2-[[(4-méthyl 1-pipérazinyl) carbonyl] amino] 3-phénylpropyle] et son chlorhydrate.**

On opère comme à l'exemple 96 en utilisant au départ 1,3 g du chlorhydrate obtenu à l'exemple 34 et 0,64 $cm^3$ de phényl isocyanate en purifiant la base par chromatographie sur silice (éluant : chlorure de méthylène-méthanol 9-1). On obtient 1,15 g de produit attendu. F = 120°C.

Preparation du chlorhydrate.

On dissout 1 g de base dans 10 $cm^3$ d'acétate d'éthyle, ajoute une solution d'acide chlorhydrique dans l'acétate d'éthyle jusqu'à pH acide, chauffe au reflux, ajoute de l'acétonitrile jusqu'à dissolution, glace, essore et sècje sous pression réduite à 80°C. On obtient 0,78 g de chlorhydrate attendu. F = 214°C.

**Exemple 98 : (±) (3,4-dichlorophényl) carbamothioate de S-[2-[[(4-méthyl 1-pipérazinyl) carbonyl] amino] 3-phénylpropyle] et son chlorhydrate.**

On opère comme à l'exemple 97 à partir de 1 g de produit de l'exemple 34 sous forme de base et 0,56 g de 3,4-dichlorophényl isocyanate. On obtient 1,4 g de produit attendu sous forme de base. F = 160°C. 1,2 g sont transformés en chlorhydrate. On obtient 0,8 g de chlorhydrate attendu. F = 200°C.

**Exemple 99 : (±) (4-méthoxyphényl) carbamothioate de S-[2-[[(4-méthyl 1-pipérazinyl) carbonyl] amino] 3-phénylpropyle] (E)-butènedioate [1,2].**

On opère comme à l'exemple 97 à partir de 1 g de produit obtenu à l'exemple 34 sous forme de base et 0,42 $cm^3$ de 4-méthoxyphényl isocyanate. On obtient 1,1 g de produit sous forme de base. F = 160°C.

Préparation du fumarate.

On dissout 500 mg de base dans 30 $cm^3$ d'isopropanol et ajoute 0,14 g d'acide fumarique dans 10 $cm^3$ d'isopropanol. On glace, essore et sèche sous pression réduite à 80°C. on obtient 0,6 g de fumarate attendu. F = 198°C.

**<u>Exemple 100</u> : (±) Chlorhydrate de éthanethioate de 2-[[(4-méthyl 1-pipérazinyl) carbonyl] amino] 3-phénylpropyle.**

On dissout 1,2 g de produit obtenu à l'exemple 33 dans l'acétate d'éthyle, ajoute une solution d'acide chlorhydrique 2N dans l'acétate d'éthyle, concentre sous pression rédcuite, reprend dans l'acétone, essore les cristaux et les sèche sous pression réduite. On obtient 1,16 g de chlorhydrate attendu. F = 184-186°C.

**<u>Exemple 101</u> : (S)-éthanethioate de 2-[[(4-méthyl 1-pipérazinyl) carbonyl] amino] 3-phénylpropyle et son chlorhydrate.**

On opère comme à l'exemple 1 à partir de 2,5 g de (S)-éthanethioate de S-[2-isocyanato 3-phénylpropyle] et de 1,44 $cm^3$ de N-méthyl pipérazine. On obtient 2,1 g de produit attendu sous forme de base. F = 68-70°C.

<u>Formation du chlorhydrate.</u>

On opère comme à l'exemple 100 à partir de 1,2 g de base. On récupère 1,1 g de chlorhydrate attendu. F = 188-190°C.

<u>Preparation du (S)-éthanethioate de S-[2-isocyanato 3-phénylpropyle].</u>

On opère comme à la préparation 1, stades A à F, en utilisant au départ la (L)-phénylamine.

**<u>Exemple 102</u> : (S) (-) N-[1-(mercaptométhyl) 2-phényléthyl] 4-méthyl 1-pipérazinecarboxamide.**

On opère comme à l'exemple 2, en utilisant au départ 1,675 g de produit obtenu à l'exemple 101 sous forme de base et 0,266 $cm^3$ d'hydrate d'hydrazine. On obtient 460 mg de produit attendu. F = 68-70°C.

**<u>Exemple 103</u> : (R)-éthanethioate de 2-[[(4-méthyl 1-pipérazinyl) carbonyl] amino] 3-phénylpropyle et son chlorhydrate.**

On opère comme à l'exemple 101 à partir de 2,45 g d'isomère (R). On obtient 2,73 g de produit attendu sous forme de base. F = 68-70°C. A partir de 1,2 g de base, on obtient 1 g de chlorhydrate attendu. F = 188-190°C.

<u>Préparation du (R)-éthanethioate de S-(2-isocyanato 3-phénylpropyle) utilisé au départ de l'exemple 103.</u>

**Stade A** : N-t-Boc-D-phénylalaninol.

On agite 6,07 g d'hydrure d'aluminium lithium dans 200 $cm^3$ de tétrahydrofuranne, ajoute 21,2 g de N-t-Boc-D-phénylalanine dans 250 $cm^3$ de tétrahydrofuranne à une température inférieure à 45°C. On agite 1 heure, refroidit le mélange à température ambiante, ajoute lentement 30 $cm^3$ d'un mélange tétrahydrofuranne-eau (1-1), puis 50 $cm^3$ d'une solution aqueuse saturée de tartrate de sodium de potassium. On filtre, lave au tétrahydrofuranne, concentre le filtrat sous pression réduite, reprend le résidu dans l'eau, extrait au chlorure de méthylène, élimine le solvant et cristallise le résidu dans l'hexane. Après séchage, on obtient 12,2 g de produit attendu. F = 94-96°C.

**Stade B** : (R) [1-[[[(4-méthylphényl) sulfonyl] oxy] méthyl] 2-phényléthyl] carbamate de 1,1-diméthyléthyle.

On dissout 11,3 g de produit obtenu au stade A dans 110 $cm^3$ de chlorure de méthylène, ajoute 8,25 g de 4-diméthylaminopyridine puis 10,68 g de chlorure de tosyle et agite 2 heures à température ambiante. On verse le milieu réactionnel dans 150 $cm^3$ d'une solution aqueuse d'acide chlorhydrique 2N jusqu'à pH 1, sépare la phase organique, la sèche, élimine les sovlants sous pression réduite et obtient 18,2 g de produit attendu. F = 110-112°C.

**Stade C** : Ethanethioate de (R) S-[2-[[(1,1-diméthyléthoxy) carbonyl] amino] 3-phénylpropyle].

On ajoute à température ambiante 7,36 g de thioacétate de potassium à une solution de 17,415 g de produit obtenu au stade B dans 170 $cm^3$ de diméthylformamide. On agite 2 heures, verse le milieu réactionnel dans l'eau, extrait à l'éther, élimine le solvant sous pression réduite, chromatographie le résidu sur silice (éluant : acétate d'éthyle-n-hexane 8-2) et obtient 9 g de produit attendu. F= 92-94°C.

**Stade D** : Chlorhydrate de (R) ethanethioate de S-(2-amino 3-phénylpropyle).

On ajoute 85 $cm^3$ d'une solution d'acide chlorhydrique 4N dans l'acétate d'éthyle à 8,5 g de produit obtenu au stade C en solution dans 30 $cm^3$ d'acétate d'éthyle. On agite 2 heures, élimine les solvants, reprend le résidu

dans l'éther, essore les cristaux formés, les sèche sous pression réduite et obtient 6,4 g de produit attendu. F = 170-172°C.

**Stade E** : Ethanethioate de S-(2-isocyanato 3-phénylpropyle).

On opère comme indiqué au stade F de la préparation 1 à partir de 2,45 g de produit obtenu au stade D. On récupère 2,5 g de produit attendu utilisé tel quel pour l'exemple 103.

**Exemple 104 : (R) (+) N-[1-(mercaptométhyl) 2-phényléthyl] 4-méthyl 1-pipérazinecarboxamide et son chlorhydrate.**

On opère comme à l'exemple 59 à partir de 2,35 g de produit obtenu à l'exemple 103 sous forme de base. Après addition d'une solution aqueuse de bicarbonate de sodium, on obtient 1,520 g de produit attendu sous forme de base. F = 68-70°C. En opérant de la même manière mais sous addition de bicarbonate de sodium, on obtient 1,848 g de chlorhydrate attendu. F ≈ 170°C.

**Exemple 105 : Bis éthanethioate de (±) S,S'-[2,2'-[carbonyl-bis-(imino)] bis-(3-phénylpropyle)].**

On opère comme à l'exemple 1 à partir de 2,35 g d'isocyanate préparé au stade F de la préparation et 2,45 g de (±) chlorhydrate d'éthanethioate de S-(2-amino 3-phénylpropyle) préparé au stade E de la préparation. On obtient 1,4 g de produit attendu. F = 144-146°C.

**Exemple 106 : N,N'-bis [1-(mercaptométhyl) 2-phényléthyl] urée.**

On opère comme à l'exemple 2 à partir de 1,776 g de produit obtenu comme à l'exemple 105 et 0,426 $cm^3$ d'hydrate d'hydrazine. On obtient 1,250 g de produit attendu. F = 136-138°C.

**Exemple 107 : Ethanethioate de S-[2-[[[(3-pyridinyl) amino] carbonyl] amino] 3-phénylpropyle].**

On opère comme à l'exemple 1 à partir de 3 g d'isocyanate préparé comme au stade F de la préparation et 1,2 g de 3-aminopyridine. On obtient après purification sur silice (éluant : chlorure de méthylène) 2,5 g de produit attendu.

**Exemple 108 : (±) N-[1-(mercaptométhyl) 2-phényléthyl] N'-(3-pyridinyl) urée.**

On opère comme à l'exemple 2 à partir de 1,5 g de produit obtenu à l'exemple 107 et 0,24 $cm^3$ d'hydrate d'hydrazine. On obtient 0,78 g de produit attendu. F = 149°C.

**Exemple 109 : (±) Ethanethioate de S-[2-[[1-(4-méthylpipérazinyl) carbonyl] amino] 3-phénylpropyle].**

On opère comme à l'exemple 1 à partir de 3,7 g de thiocyanate préparé comme au stade F de la préparation et 2,4 $cm^3$ de 1-amino 4-méthylpipérazine. On obtient environ 5 g de produit sous forme de base que l'on transforme en chlorhydrate par addition d'une solution d'acide chlorhydrique dans l'acétate d'éthyle. On obtient le dichlorhydrate que l'on transforme en monochlorhydrate par addition d'une solution aqueuse de bicarbonate de soude. Après cristallisation dans l'acétate d'éthyle, on obtient 1,5 g de produit attendu. F = 158-160°C.

**Exemple 110 : (±) N-[1-(mercaptométhyl) 2-phényléthyl] N'-(4-méthyl 1-pipérazinyl) urée et son chlorhydrate.**

On opère comme à l'exemple 59 à partir de 2 g de produit obtenu comme à l'exemple 109. On obtient 1 g de produit attendu sous forme de base puis 0,56 g de chlorhydrate. F = 198-200°C.

**Exemple 111 : (±) Ethanethioate de S-[2-[[hexahydro 1H-1-(4-méthylazépinyl) carbonyl] amino] 3-phénylpropyle].**

On opère comme à l'exemple 1 à partir de 3 g d'isocyanate préparé au stade F de la préparation et 1,6 g de N-méthyl homopipérazine. On obtient 2,6 g de produit attendu.

**Exemple 112 : (±) hexahydro N-[(2-mercaptométhyl) 2-phényléthyl] 4-méthyl 1H-1,4-diazépin-1-carboxamide et son chlorhydrate.**

On opère comme à l'exemple 59 à partir de 2 g de produit obtenu à l'exemple 111. On obtient 1,3 g de produit attendu sous forme de base 1,2 g de base sont transformés en chlorhydrate. On obtient 0,7 g de chlorhydrate attendu. F = 162°C.

**Exemple 113 : (±) N-[1-(mercaptométhyl) 2-phényléthyl] 4- méthyl 1-pipérazinecarboxamide, dichlorhydrate, trihydrate.**

On agite pendant 4 heures à température ambiante 2 g de 4-méthyl 1-pipérazine éthanethioate de (±) S-[2-[[(1,1-diméthyléthoxy) carbonyl amino] 3-phénylpropyle] dans 20 $cm^3$ de chlorure de méthylène en présence de 4 $cm^3$ d'acide trifluoroacétique. On évapore les solvants sous pression réduite. On obtient 2,4 g de résidu huileux que l'on dissout dans l'eau, on alcalinise avec une solution aqueuse saturée en bicarbonate de sodium, extrait à l'acétate d'éthyle, sèche et évapore le solvant. On récupère 1,1 g de produit que l'on transforme en chlorhydrate par addition d'une solution éthanolique d'acide chlorhydrique. On obtient 0,6 g de produit attendu. F = 170°C.

Preparation du produit de départ de l'exemple 113 : 4-méthyl 1-pipérazine ethanethioate de (±) S-[2-[[(1,1-diméthyléthoxy) carbonyl] amino] 3-phénylpropyle].

On agite pendant 20 heures 2,5 g d'acide 2-(t-butoxycarbonylamino) phényl propanoïque dans 125 $cm^3$ de chlorure de méthylène avec 1,77 g d'acide 4-méthyl 1-pipérazine acétique (Journal of Pharmaceutical Sciences Vol. 63 n° 12 (Déc. 1974) 1883) et 1,47 g de chlorhydrate de N-(3-diméthylaminopropylà N'-éthylcarbodiimide et 0,57 g de diméthylaminopyridine. On dilue à l'eau, extrait au chlorure de méthylène, lave à l'eau, sèche et évapore le solvant. On récupère ainsi 3 g de produit brut. Après chromatographie sur silice (éluant : chlorure de méthylène-méthanol 9-1), on recueille 2,25 g de produit attendu. F = 104°C.

**Préparation des réactifs**.

A) **Préparation du dichlorhydrate d'allyl pipérazine utilisé à l'exemple 64.**

Stade a : N-allyl N-carboxylate d'éthylpipérazine.

On chauffe au reflux 15 g de N-carboxylate d'éthylpipérazine dans 150 $cm^3$ d'éthanol en présence de 8,5 $cm^3$ de bromure d'allyle et 13,1 g de carbamate de potassium. On dilue à l'eau, extrait à l'acétate d'éthyle, évapore le solvant, chromatographie le résidu sur silice (éluant : acétate d'éthyle-triéthylamine 9-1) et obtient 15,5 g de produit attendu.

Stade b : dichlorhydrate d'allylpipérazine.

On chauffe 24 heures au reflux 8 g de produit du stade A dans 26 $cm^3$ d'eau et 26 $cm^3$ d'acide chlorhydrique. On évapore le solvant, cristallise dans un mélange acétate d'éthyle-éther isopropylique et obtient 4,79 g de produit attendu. F = 230°C.

B) **Préparation du dichlorhydrate de 1-isopropylpipérazine utilisé à l'exemple 66.**

Stade a : N-isopropyl N-carboxylate d'éthylpipérazine.

On chauffe 24 heures au reflux 10 $cm^3$ de pipérazine N-carboxylate d'éthyle dans 100 $cm^3$ d'éthanol en présence de 10,37 g de carbonate de potassium et 7,05 $cm^3$ de bromopropane. On dilue à l'eau, extrait au chlorure de méthylène, évapore le solvant, chromatographie le résidu sur silice (éluant : acétate d'éthyle-triéthylamine 9-1) et obtient 9 g de produit attendu.

Stade b : dichlorhydrate 1-isopropylpipérazine.

On opère comme au stade b de la préparation A à partir de 7 g de produit préparé au stade a. On obtient 5 g de produit attendu. F > 250°C.

C) **Préparation du 1-(2-propynyl) pipérazine utilisé à l'exemple 68.**

Stade a : N-(2-propynyl) pipérazine N-carboxylate d'éthyle.

On opère comme à la préparation B stade a) à partir de 13,8 $cm^3$ de pipérazine N-carboxylate d'éthyle, 13,1 g de carbonate de potassium et 8,4 $cm^3$ de bromure de porpargyle. Onobtient 14 g de produit attendu.

Stade b : 1-(2-propynyl) pipérazine.

On chauffe 4 heures au reflux 2,67 g de produit du stade a) dans 5 $cm^3$ d'éthanol, 0,66 $cm^3$ d'eau et 4,4 g de potasse. On extrait à l'acétate d'éthyle, évapore le solvant, reprend le résidu dans l'eau, extrait de nouveau avec un mélange tétrahydrofuranne-acétate d'éthyle 4-6 et obtient 0,2 g de produit attendu.

**Exemple 114 de composition pharmaceutique**

On a préparé des comprimés répondant à la formule suivante :

| | |
|---|---|
| Produit de l'exemple ...................... | 100 mg |
| Excipient pour un comprimé terminé à......... | 400 mg |

(détail de l'excipient : lactose, talc, amidon, stéarate de magnésium)

**ETUDE BIOLOGIQUE**

**1) Détermination de l'effet inhibiteur de l'enképhalinase.**

L'activité de l'enképhalinase est déterminée à partir d'une fraction membranaire rénale de rat. Les reins sont prélevés sur glace et homogénéisés en tampon HEPES 0.05M pH 7,5 contenant du chlorure de magnésium (50 fois le volume). Après une première centrifugation à 1500 g, la fraction particulaire est obtenue à l'issue d'une centrifugation à 15000 g de 20 minutes. Le culot est ensuite lavé et suspendu en tampon HEPES et conservé à -20°C. Une aliquote de cette préparation membranaire est mise en présence d'octyl béta-D-glucopyranoside (50 mM en centrifugation finale) pendant 15 minutes à 4°C. Après centrifugation à 100 000 g pendant une heure, le surnageant est prélevé et congelé par aliquotes à -20°C. La concentration en protéines est déterminée par la méthode au bleu de Comassie.

Une aliquote de la fraction protéinique ainsi préparée est pré-incubée à 25°C pendant 10 minutes en tampon HEPES 0.05M pH 7,5 ou en présence du produit à étudier. Le substrat ajouté est une enképhaline non naturelle de formule SuccinylAla-Ala-Phe-amino-méthylcoumarine (référence 1) (40 micromolaire en concentration finale), l'incubation se poursuit à 37°C pendant 30 minutes. La réaction est stoppée par chauffage à 95°C pendant 5 minutes et chaque incubat est centrifugé. Une solution d'aminopeptidase M(4 micromolaire en concentration finale) et de thiorphan (référence 2) ($10^{-5}$M) est ajoutée au surnageant et l'ensemble est porté à 37°C pendant une heure. La réaction est stoppée comme précédemment décrit et la fluoroescence de la 7-amino-méthyl coumarine ainsi produite déterminée. L'effet des produits à tester est déterminé par le calcul de la $CI_{50}$, concentration qui inhibe de 50 % l'hydrolyse du substrat.

**Référence 1** : R.A. Munford, P.A. Pierzchala, A-W Strauss and M. Zimmerman Purification of a membrane-bound metalloendo peptidase from porcine kidney that degrades peptides hormones. Proc. Natl. Acad. Sci 78, n° 11, p. 6623.

**Référence 2** : Inhibiteur de l'enképhalinase décrit à l'exemple 20 du B.F. 2 480 747.

<u>Résultats</u> :

| Produit de l'exemple | $CI_{50}$ en $10^{-8}$ M |
|---|---|
| 6 | 0,35 |
| 17 | 4,8 |
| 16 | 7 |
| 1 | 8,7 |
| 15 | 10 |
| 18 | 18 |

**2) Etude de l'activité analgésique chez la souris.**

<u>Etirements provoqués par l'acide acétique chez la souris.</u>

Le test employé est basé sur le fait signalé par R. KOSTER et Coll., (Fed. Proc. (1959), <u>1B</u>, 412) selon lequel l'injection intrapéritonéale d'acide acétique provoque, chez la souris, des mouvements répétés d'étirement, de torsion pouvant persister pendant plus de 6 heures. Les analgésiques préviennent ou diminuent ce syndrome qui peut être considéré comme l'extériorisation d'une douleur abdominale diffuse. On utilise une solution d'acide acétique à 1 % dans l'eau administrée sous un volume de 10 ml/kg.

Le produit étudié est administré par voie buccale une demi-heure avant l'injection d'acide acétique, les souris étant à jeun depuis 6 heures au minimum.

Les étirements sont observés et comptés pour chaque souris pendant une période d'observation de 15 minutes.

Les résultats sont exprimés au moyen de la $DA_{50}$, c'est-à-dire la dose qui permet d'obtenir une diminution de 50 % du nombre des étirements par rapport aux animaux témoins.

| Produit | $DA_{50}$ en mg/kg PO |
|---|---|
| 18 | 33 |
| 16 | 50 |
| 1 | 71 |
| 11 | 85 |

**3) Test de la plaque chaude.**

Des souris males (22 g) sont placées une par une sur une plaque de cuivre maintenue à 56°C à l'aide d'un bain-marie thermostaté. La réaction à la douleur se manifeste par le léchage d'une ou des deux pattes antérieures. Le temps de réaction au stimulus thermique est noté et l'on ne retient que les animaux réagissant entre 4,5 et 6,5 secondes.

Le temps de réaction à la douleur est mesuré 30 minutes après l'administration par voie orale.

Les variations du temps de réaction sont exprimées en pourcentage du temps initial. On détermine graphiquement ou par la méthode des moindres carrés la $DA_{50}$ et la $DA_{100}$, ou doses qui augmentent le temps

réaction de 50 et 100 %, compte tenu des variations du groupe témoin.

| PRODUIT | en mg/kg/po | |
|---|---|---|
| | DA 50 | DA 100 |
| Ex 18 | 33 | 100 |
| Ex 5 | 100 | 200 |
| Ex 11 | 100 | - |

Les composés sont des inhibiteurs de l'endopeptidase neutre EC 3.4.24.11. Cette enzyme est impliquée en particulier dans la dégradation des enképhalines et du peptide natriurétique auriculaire (ou ANF). L'ANF est un peptide vasodilatateur, diurétique et natriurétique puissant. L'inhibition de l'endopeptidase neutre EC 3.4.24.11 par les composés décrits peut conduire ainsi à une potentialisation des effets biologiques de l'ANF. En particulier, les effets hémodynémiques, diurétiques et natriurétiques de ces composés pourraient présenter une utilité dans le traitement de l'insuffisance cardiaque, de l'insuffisance rénale, l'hypertension associée ou non à une hyperréninehémie, l'hyperaldostéroniome, les oedèmes de différentes origines, le glaucome. Ces composés pourraient également présenter un intérêt thérapeutique dans le traitement des troublesgastro-intestinaux (dont en particulier diarrhées et colon irritable) ainsi que les troubles cognitifs.

## Revendications

### Revendications pour les Etats contractants suivants : AT, BE, CH, DE, DK, FR, GB, IT, LI, LU, NL, SE

**1.** Produits de formule ($I_A$):

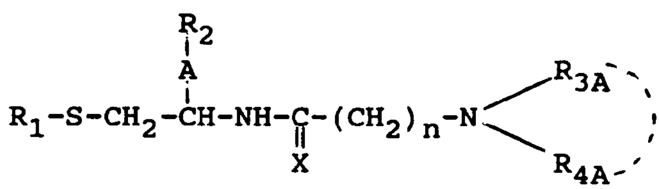

($I_A$)

dans laquelle :

n représente les valeurs 0 à 4,

$R_1$ représente un atome d'hydrogène ou un radical $R_5$ - CO - dans lequel $R_5$ représente :

- un radical alkyle, alkényle ou alkynyle linéaire ou ramifié renfermant au plus 6 atomes de carbone et éventuellement substitué,
- un radical monocyclique comprenant 5 à 7 chaînons ou un radical constitué de cycles condensés comprenant 8 à 10 chaînons, ces radicaux étant saturés ou insaturés, renfermant éventuellement un ou plusieurs hétéroatomes, identiques ou différents, choisis parmi les atomes d'oxygène, d'azote et de soufre et étant éventuellement substitués,
- un radical

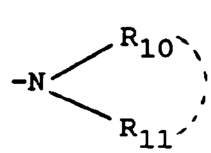

dans lequel $R_{10}$ et $R_{11}$ sont tels que :

**ou bien** $R_{10}$ et $R_{11}$ forment avec l'atome d'azote auxquels ils sont liés un radical monocyclique comprenant 5 à 7 chaînons ou un radical constitué de cycles condensés comprenant 8 à 10 chaînons, ces radicaux renfermant éventuellement un autre ou plusieurs autres hétéroatomes, identiques ou différents, choisis parmi les atomes d'oxygène, d'azote et de soufre, et étant éventuellement substitués,

**ou bien** $R_{10}$ et $R_{11}$, identiques ou différents, représentent :

- un atome d'hydrogène ;
- un radical alkyle, alkényle ou alkynyle linéaire ou ramifié renfermant au plus 6 atomes de carbone et éventuellement substitué ;
- un radical aryle dans lequel le radical aryle représente un radical monocyclique comprenant 5 à 7 chaînons ou constitué de cycles condensés comprenant 8 à 10 chaînons, ces radicaux renfermant éventuellement un ou plusieurs hétéroatomes, identiques ou différents, choisis parmi les atomes d'oxygène, d'azote et de soufre, et étant éventuellement substitués ;

A représente :

- soit un radical alkylène ou alkénylène linéaire ou ramifié renfermant au plus 6 atomes de carbone et éventuellement substitué par un radical hydroxyle ou alcoxy renfermant au plus 6 atomes de carbone,
- soit une simple liaison,

$R_2$ représente un radical monocyclique comprenant 5 à 7 chaînons ou un radical constitué de cycles condensés comprenant 8 à 10 chaînons, ces radicaux étant saturés ou insaturés, renfermant éventuellement un ou plusieurs hétéroatomes, identiques ou différents, choisis parmi les atomes d'oxygène, d'azote et de soufre et étant éventuellement substitués,

X représente un atome d'oxygène ou de soufre,

$R_{3A}$ et $R_{4A}$ sont tels que :

**ou bien** $R_{3A}$ et $R_{4A}$ forment avec l'atome d'azote auxquels ils sont liés un radical monocyclique comprenant 5 à 7 chaînons ou un radical constitué de cycles condensés comprenant 8 à 10 chaînons, ces radicaux renfermant éventuellement un autre ou plusieurs autres hétéroatomes, identiques ou différents, choisis parmi les atomes d'oxygène, d'azote et de soufre, et étant éventuellement substitués,

**ou bien** $R_{3A}$ et $R_{4A}$, identiques ou différents, représentent :

- un atome d'hydrogène ;
- un radical hydroxyle ; alcoxy, acyle ou acyloxy renfermant au plus 6 atomes de carbone ; carboxy, salifié ou estérifié ; cyano ;
- un radical alkyle, alkényle ou alkynyle linéaire ou ramifié renfermant au plus 6 atomes de carbone et éventuellement substitué ;
- un radical $R_c$ ou $R_c$-O- dans lesquels le radical $R_c$ représente un radical aliphatique ou arylique, monocyclique comprenant 5 à 7 chaînons ou constitué de cycles condensés comprenant 8 à 10 chaînons, ces radicaux renfermant éventuellement un ou plusieurs hétéroatomes, identiques ou différents, choisis parmi les atomes d'oxygène, d'azote et de soufre, et étant éventuellement substitués ;
- le radical

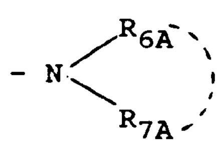

ou

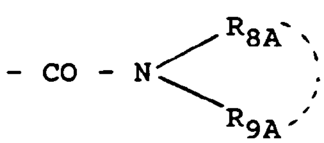

dans lesquels $R_{6A}$, $R_{7A}$, $R_{8A}$ et $R_{9A}$, identiques ou différents, représentent un atome d'hydrogène, un radical hydroxyle, alkyle, alcoxy, acyloxy ou acyle, ces radicaux renfermant au plus 6 atomes de carbone, carboxy libre, salifié ou estérifié, phénoxy, phényle, benzyle, phénéthyle, azépine, pipéridyle, morpholine, pyrrolidinyle, pipérazinyle, éventuellement substitués sur le second atome d'azote par un radical hydroxyle, alkyle, alcoxy ou phényle, ces trois derniers radicaux étant eux-mêmes éventuellement substitués par un ou plusieurs radicaux choisis dans les atomes d'halogène, le radical hydroxyle, trifluorométhyle, méthoxy, éthoxy, méthyle et éthyle,

ou bien d'une part $R_{6A}$ et $R_{7A}$ et d'autre part $R_{8A}$ et $R_{9A}$ forment respectivement avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux

hydroxyle, trifluorométhyle, alkyle et alcoxy, ces radicaux renfermant au plus 6 atomes de carbone, lesdits produits de formule ($I_A$) étant sous toutes les formes isomères possibles racémiques, énantiomères et diastéréoisomères,

ainsi que les sels d'addition avec les acides minéraux et organiques desdits produits de formule ($I_A$).

**2.** Produits de formule ($I_A$) telle que définie à la revendication 1 répondant à la formule (I) :

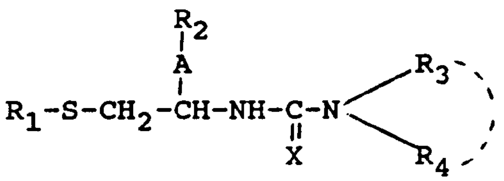

(I)

dans laquelle :

$R_1$ représente un atome d'hydrogène ou un radical $R_5$ - CO - dans lequel $R_5$ représente :

- un radical alkyle, alkényle ou alkynyle linéaire ou ramifié renfermant au plus 6 atomes de carbone et éventuellement substitué,
- un radical monocyclique comprenant 5 à 7 chaînons ou un radical constitué de cycles condensés comprenant 8 à 10 chaînons, ces radicaux étant saturés ou insaturés, renfermant éventuellement un ou plusieurs hétéroatomes, identiques ou différents, choisis parmi les atomes d'oxygène, d'azote et de soufre et étant éventuellement substitués,
- un radical

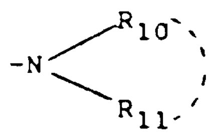

dans lequel $R_{10}$ et $R_{11}$ sont tels que :

**ou bien** $R_{10}$ et $R_{11}$ forment avec l'atome d'azote auxquels ils sont liés un radical monocyclique comprenant 5 à 7 chaînons ou un radical constitué de cycles condensés comprenant 8 à 10 chaînons, ces radicaux renfermant éventuellement un autre ou plusieurs autres hétéroatomes, identiques ou différents, choisis parmi les atomes d'oxygène, d'azote et de soufre, et étant éventuellement substitués,

**ou bien** $R_{10}$ et $R_{11}$, identiques ou différents, représentent :

- un atome d'hydrogène ;
- un radical alkyle, alkényle ou alkynyle linéaire ou ramifié renfermant au plus 6 atomes de carbone et éventuellement substitué ;
- un radical aryle dans lequel le radical aryle représente un radical monocyclique comprenant 5 à 7 chaînons ou constitué de cycles condensés comprenant 8 à 10 chaînons, ces radicaux renfermant éventuellement un ou plusieurs hétéroatomes, identiques ou différents, choisis parmi les atomes d'oxygène, d'azote et de soufre, et étant éventuellement substitués ;

A représente :

- soit un radical alkylène ou alkénylène linéaire ou ramifié renfermant au plus 6 atomes de carbone et éventuellement substitué par un radical hydroxyle ou alcoxy renfermant au plus 6 atomes de carbone,
- soit une simple liaison, $R_2$ représente un radical monocyclique comprenant 5 à 7 chaînons ou un radical constitué de cycles condensés comprenant 8 à 10 chaînons, ces radicaux étant saturés ou insaturés, renfermant éventuellement un ou plusieurs hétéroatomes, identiques ou différents, choisis parmi les atomes d'oxygène, d'azote et de soufre et étant éventuellement substitués,

  X représente un atome d'oxygène ou de soufre,

  $R_3$ et $R_4$ sont tels que :

**ou bien** $R_3$ et $R_4$ forment avec l'atome d'azote auxquels ils sont liés un radical monocyclique comprenant 5 à 7 chaînons ou un radical constitué de cycles condensés comprenant 8 à 10 chaînons, ces radicaux renfermant éventuellement un autre ou plusieurs autres hétéroatomes, identiques ou différents, choisis parmi les atomes d'oxygène, d'azote et de soufre, et étant éventuellement substitués,

**ou bien** $R_3$ et $R_4$, identiques ou différents, représentent :

- un atome d'hydrogène ;
- un radical hydroxyle ; alcoxy, acyle ou acyloxy renfermant au plus 6 atomes de carbone ; carboxy, salifié ou estérifié ; cyano ;
- un radical alkyle, alkényle ou alkynyle linéaire ou ramifié renfermant au plus 6 atomes de carbone et éventuellement substitué ;
- un radical aryle ou aryloxydans lesquels le radical aryle représente un radical monocyclique comprenant 5 à 7 chaînons ou constitué de cycles condensés comprenant 8 à 10 chaînons, ces radicaux renfermant éventuellement un ou plusieurs hétéroatomes, identiques ou différents, choisis parmi les atomes d'oxygène, d'azote et de soufre, et étant éventuellement substitués ;
- le radical

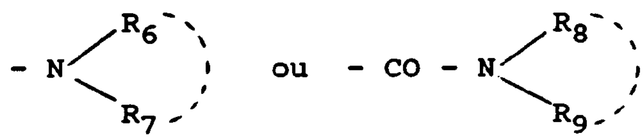

dans lesquels $R_6$, $R_7$, $R_8$ et $R_9$, identiques ou différents, représentent un atome d'hydrogène, un radical hydroxyle, alkyle, alcoxy, acyloxy ou acyle, ces radicaux renfermant au plus 6 atomes de carbone, carboxy libre, salifié ou estérifié, phényle, benzyle, phénéthyle, azépine, pipéridyle, morpholine, pyrrolidinyle, pipérazinyle, éventuellement substitués sur le second atome d'azote par un radical hydroxyle, alkyle, alcoxy ou phényle, ces trois derniers radicaux étant eux-mêmes éventuellement substitués par un ou plusieurs radicaux choisis dans les atomes d'halogène, le radical hydrogène, trifluorométhyle, méthoxy, éthoxy, méthyle et éthyle,
ou bien d'une part $R_6$ et $R_7$ et d'autre part $R_8$ et $R_9$ forment respectivement avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux hydroxyle, trifluorométhyle, alkyle et alcoxy, ces radicaux renfermant au plus 6 atomes de carbone,
lesdits produits de formule (I) étant sous toutes les formes isomères possibles racémiques, énantiomères et diastéréoisomères, ainsi que les sels d'addition avec les acides minéraux et organiques desdits produits de formule (I).

**3.** Produits de formules ($I_A$) et (I) telle que définies aux revendications 1 et 2, caractérisés en ce que le ou les substituants, identiques ou différents que peuvent porter :
   a) les radicaux alkyle, alkényle, alkynyle,
   b) les radicaux monocyclique ou constitué de cycles condensés, aryle et aryloxy,

sont choisis dans le groupe formé par :
- les atomes d'halogène ; les radicaux hydroxyle ; cyano ; mercapto ; nitro ; acyle et acyloxy ayant au plus 6 atomes de carbone ; carboxy libre, salifié ou estérifié ; alcoxycarbonyle ;
- les radicaux alkoxy linéaires et ramifiés renfermant au plus 6 atomes de carbone ;
- les radicaux alkyle, alkényle, alkynyle, alkylthio, cycloalkyle, cycloalkényle, les radicaux hydrocarbonés hétérocycliques, aryle, aryloxy, arylthio, phénylalkyle, phénylalcoxy, eux-mêmes éventuellement substitués par un ou plusieurs substituants identiques ou différents choisis parmi les atomes d'halogène, les radicaux hydroxyle, trifluorométhyle, nitro, alkyle, alkényle, aryle, alcoxy et acyle, ces radicaux renfermant au plus 6 atomes de carbone, carboxy libre, salifié ou estérifié, acylamido dans lequel le radical acyle renferme au plus 6 atomes de carbone, carbamoyle et amino, ces radicaux étant éventuellement substitués sur l'atome d'azote par un ou deux radicaux identiques ou différents choisis parmi les radicaux hydroxyle, alkyle et alcoxy renfermant au plus 6 atomes de carbone ;
- le radical acylamido dans lequel le radical acyle renferme au plus 6 atomes de carbone ; les radicaux carbamoyle et amino, ces radicaux étant éventuellement substitués sur l'atome d'azote par un ou deux radicaux identiques ou différents choisis parmi les radicaux hydroxyle, alkyle, alkényle et alcoxy renfermant au plus 6 atomes de carbone ; lesdits produits de formule ($I_A$) et (I) étant sous toutes les formes isomères possibles racémiques, énantiomères et diastéréoisomères, ainsi que les sels d'addition avec les acides minéraux et organiques desdits produits de formule ($I_A$) et (I).

**4.** Produits de formule ($I_A$) telle que définie à la revendication 1, répondant à la formule ($I'_A$) :

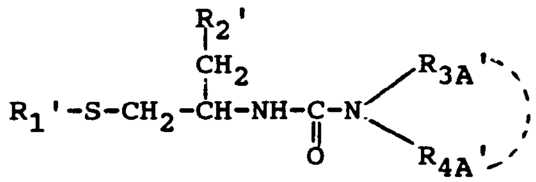

(I'A)

dans laquelle :

$R_{1A}$' représente un atome d'hydrogène ou un radical $R_{5A}$'-CO- dans lequel $R_{5A}$' représente un radical phényle, cycloalkyle, cycloalkényle, alkyle ou alkényle renfermant au plus 6 atomes de carbone, ces radicaux étant éventuellement substitués, ou un radical

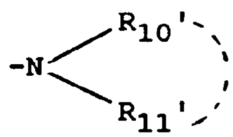

dans lequel $R_{10}$' et $R_{11}$' sont tels que :

**ou bien** $R_{10}$' et $R_{11}$' forment avec l'atome d'azote auxquels ils sont liés un radical imidazolyle, pyrrolyle, pyrrolinyle, pyrrolidinyle, pyridyle, pipéridinyle, pyrimidinyle, piridazinyle, pyrazinyle, pipérazinyle, phénylpipérazinyle, méthylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, mercapto, trifluorométhyle, alkyle, alkényle, alkynyle, alkylthio, alcoxy et phényle éventuellement substitué par un ou plusieurs radicaux choisis parmi les atomes d'halogène et les radicaux trifluorométhyle, hydroxyle, alkyle et alcoxy, les radicaux alkyle et alcoxy renfermant au plus 6 atomes de carbone,

**ou bien** $R_{10}$' et $R_{11}$', identiques ou différents, représentent :

- un atome d'hydrogène ;
- un radical alkyle, alkényle ou alkynyle linéaire ou ramifié renfermant au plus 6 atomes de carbone et éventuellement substitué,
- un radical phényle, benzyle, naphtyle, indényle, imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux hydroxyle, mercapto, trifluorométhyle, les radicaux alkyle, alkényle, alkynyle, alkylthio et alcoxy renfermant au plus 6 atomes de carbone,

$R_2$' représente un radical phényle, naphtyle, benzyle, phénéthyle, indolyle, cycloalkyle, cycloalkényle, ces radicaux étant éventuellement substitués,

$R_{3A}$' et $R_{4A}$' sont tels que :

ou bien $R_{3A}$' et $R_{4A}$' forment avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux hydroxyle, trifluorométhyle, alkyle, alkényle, alkynyle, alcoxy et phényle éventuellement substitué par un ou plusieurs radicaux choisis parmi les atomes d'halogène et les radicaux trifluorométhyle, hydroxyle, alkyle et alcoxy, les radicaux alkyle et alcoxy renfermant au plus 6 atomes de carbone et étant éventuellement substitué par un radical phényle,

ou bien $R_{3A}$' et $R_{4A}$', identiques ou différents, représentent :

- un atome d'hydrogène ;
- un radical hydroxyle ; alcoxy, acyloxy ou acyle renfermant au plus 6 atomes de carbone ; carboxy, salifié ou estérifié ; cyano ;
- un radical alkyle, alkényle ou alkynyle linéaire ou ramifié renfermant au plus 6 atomes de carbone et éventuellement substitué,
- un radical phényle, benzoyle, naphtyle, indényle, imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, diazépine, benzodiazépine, indolyle, ces radicaux étant éventuellement substitués par un ou plusieurs radicaux identiques ou différents choisis parmi les atomes d'halogène, les radicaux hydroxyle, trifluorométhyle, les radicaux alkyle et alcoxy renfermant au plus 6 atomes de carbone, eux-mêmes éventuellement substitués par un ou plusieurs radicaux choisis parmi les atomes d'halogène, les radicaux hydroxyle, alkyle, alcoxy et carboxy, libre, salifié ou estérifié par un radical alkyle, aryle ou arylalkyle,

- le radical

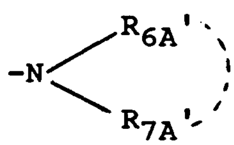 ou 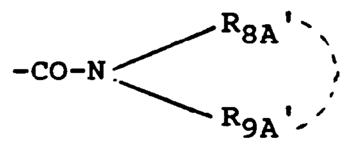

dans lesquels $R_{6A}$', $R_{7A}$', $R_{6A}$' et $R_{9A}$', identiques ou différents, représentent un atome d'hydrogène, un radical hydroxyle, alkyle, alcoxy, acyloxy ou acyle, ces radicaux renfermant au plus 6 atomes de carbone, carboxy libre, salifié ou estérifié, phényle, benzyle, phénéthyle, azépinyle, pipéridyle, morpholine, pyrrolidinyle, pipérazinyle,
ou bien d'une part $R_{6A}$' et $R_{7A}$' et d'autre part $R_{6A}$' et $R_{9A}$' forment respectivement avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux hydroxyle, trifluorométhyle, alkyle et alcoxy, ces radicaux renfermant au plus 6 atomes de carbone, étant entendu que le ou les éventuels substituants, identiques ou différents, que peuvent porter les radicaux que peuvent représenter $R_1$', $R_2$' et $R_5$' sont choisis dans le groupe formé par :

- les atomes d'halogène ; les radicaux hydroxyle ; cyano ; nitro ; acyle et acyloxy ayant au plus 6 atomes de carbone ; benzoyle ; carboxy libre, salifié ou estérifié ;
- les radicaux alkoxy linéaires et ramifiés renfermant au plus 6 atomes de carbone ;
- les radicaux alkyle, alkényle, cyclopentyle, cyclohexyle, cyclopentényle, cyclohexényle, phényle, naphtyle, indolyle, phénoxy, benzyle, phénéthyle, benzyloxy, eux-mêmes éventuellement substitués par un ou plusieurs substituants identiques ou différents choisis parmi les atomes d'halogène, les radicaux hydroxyle, trifluorométhyle, nitro, alkyle, alkényle, alcoxy, ces radicaux renfermant au plus 6 atomes de carbone, formyle, acétyle, benzoyle, carboxy libre, salifié ou estérifié, acétamido, benzoylamido, carbamoyle et amino, ces radicaux étant éventuellement substitués sur l'atome d'azote par un ou deux radicaux identiques ou différents choisis parmi les radicaux hydroxyle, méthyle, éthyle, propyle, méthoxy, éthoxy et propoxy ;
- le radical acylamido dans lequel le radical acyle renferme au plus 6 atomes de carbone ; les radicaux carbamoyle et amino, ces radicaux étant éventuellement substitués sur l'atome d'azote par un ou deux radicaux identiques ou différents choisis parmi les radicaux hydroxyle, alkyle, alkényle, alcoxy et acyle renfermant au plus 6 atomes de carbone ; lesdits produits de formule ($I'_A$) étant sous toutes les formes isomères possibles racémiques, énantiomères et diastéréoisomères, ainsi que les sels d'addition avec les acides minéraux et organiques desdits produits de formule ($I'_A$).

**5.** Produits de formule ($I_A$) et (I) telle que définie aux revendications 1 et 2, répondant à la formule (I') :

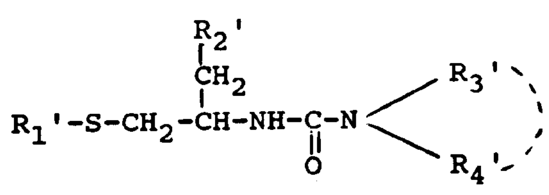

(I')

dans laquelle :
$R_1$' représente un atome d'hydrogène ou un radical $R_6$' - CO - dans lequel $R_6$' représente un radical phényle, cycloalkyle, cycloalkényle, alkyle ou alkényle renfermant au plus 6 atomes de carbone, ces radicaux étant éventuellement substitués, ou un radical

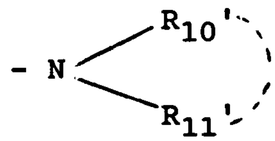

dans lequel $R_{10}$' et $R_{11}$' sont tels que :

**ou bien** $R_{10}$' et $R_{11}$' forment avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, trifluorométhyle, alkyle, alcoxy et phényle éventuellement substitué par un ou plusieurs radicaux choisis parmi les atomes d'halogène et les radicaux trifluorométhyle, hydroxyle, alkyle et alcoxy, les radicaux alkyle et alcoxy renfermant au plus 6 atomes de carbone,

**ou bien** $R_{10}$' et $R_{11}$', identiques ou différents, représentent :

- un atome d'hydrogène ;
- un radical alkyle, alkényle ou alkynyle linéaire ou ramifié renfermant au plus 6 atomes de carbone et éventuellement substitué,
- un radical phényle, benzoyle, naphtyle, indényle, imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, trifluorométhyle, les radicaux alkyle et alcoxy renfermant au plus 6 atomes de carbone,

$R_2$' représente un radical phényle, naphtyle, benzyle, phénéthyle, indolyle, cycloalkyle, cycloalkényle, ces radicaux étant éventuellement substitués,

$R_3$' et $R_4$' sont tels que :

ou bien $R_3$' et $R_4$' forment avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, trifluorométhyle, alkyle, alcoxy et phényle éventuellement substitué par un ou plusieurs radicaux choisis parmi les atomes d'halogène et les radicaux trifluorométhyle, hydroxyle, alkyle et alcoxy, les radicaux alkyle et alcoxy renfermant au plus 6 atomes de carbone,

ou bien $R_3$' et $R_4$', identiques ou différents, représentent :

- un atome d'hydrogène ;
- un radical hydroxyle ; alcoxy, acyloxy ou acyle renfermant au plus 6 atomes de carbone ; carboxy, salifié ou estérifié ; cyano ;
- un radical alkyle, alkényle ou alkynyle linéaire ou ramifié renfermant au plus 6 atomes de carbone et éventuellement substitué,
- un radical phényle, benzoyle, naphtyle, indényle, imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, trifluorométhyle, les radicaux alkyle et alcoxy renfermant au plus 6 atomes de carbone,
- le radical

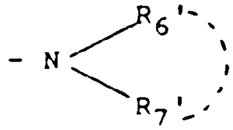 ou 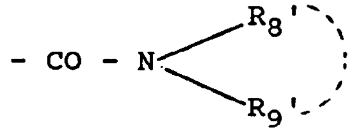

dans lesquels $R_6$', $R_7$', $R_6$' et $R_9$', identiques ou différents, représentent un atome d'hydrogène, un radical hydroxyle, alkyle, alcoxy, acyloxy ou acyle, ces radicaux renfermant au plus 6 atomes de carbone, carboxy libre, salifié ou estérifié, phényle, benzyle, phénéthyle, azépine, pipéridyle, morpholine, pyrrolidinyle, pipérazinyle,

ou bien d'une part $R_6$ et $R_7$ et d'autre part $R_8$ et $R_9$ forment respectivement avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, trifluorométhyle, alkyle et alcoxy, ces radicaux renfermant au plus 6 atomes de carbone, étant entendu que le ou les éventuels substituants, identiques ou différents, que peuvent porter les radicaux que peuvent représenter $R_1$', $R_2$' et $R_5$' sont choisis dans le groupe formé par :

- les atomes d'halogène ; les radicaux hydroxyle ; cyano ; nitro ; acyle et acyloxy ayant au plus 6 atomes de carbone ; benzoyle ; carboxy libre, salifié ou estérifié ;
- les radicaux alkoxy linéaires et ramifiés renfermant au plus 6 atomes de carbone ;
- les radicaux alkyle, alkényle, cyclopentyle, cyclohexyle, cyclopentényle, cyclohexényle, phényle, naphtyle, indolyle, phénoxy, benzyle, phénéthyle, benzyloxy, eux-mêmes éventuellement substitués par un ou plusieurs substituants identiques ou différents choisis parmi les atomes d'halogène, les radicaux hydroxyle, trifluorométhyle, nitro, alkyle, alkényle, alcoxy, ces radicaux renfermant au plus 6 atomes de carbone, formyle, acétyle, benzoyle, carboxy libre, salifié ou estérifié, acétamido, benzoylamido, carbamoyle et amino, ces radicaux étant éventuellement substitués sur l'atome d'azote par un ou deux radicaux identiques ou différents choisis parmi les radicaux hydroxyle, méthyle, éthyle, propyle, méthoxy, éthoxy et propoxy ;
- le radical acylamido dans lequel le radical acyle renferme au plus 6 atomes de carbone ; les radicaux carbamoyle et amino, ces radicaux étant éventuellement substitués sur l'atome d'azote par un ou deux radicaux identiques ou différents choisis parmi les radicaux hydroxyle, alkyle, alkényle, alcoxy et acyle renfermant au plus 6 atomes de carbone ; lesdits produits de formule (I') étant sous toutes les formes isomères possibles racémiques, énantiomères et diastéréoisomères, ainsi que les sels d'addition avec les acides minéraux et organiques desdits produits de formule (I').

**6.** Produits de formule ($I'_A$) telle que définie à la revendication 4, caractérisés en ce que :
$R_1$' représente un atome d'hydrogène ou un radical acétyle,
$R_2$' représente un radical phényle,
$R_{3A}$' et $R_{4A}$' sont tels que :

ou bien $R_{3A}$' et $R_{4A}$' forment avec l'atome d'azote auxquels ils sont liés un radical pipéridinyle, morpholinyle, diméthylmorpholinyle, thiomorpholinyle, pyrrolidinyle, azépine, diazépine éventuellement substitué sur le second atome d'azote par un radical alkyle renfermant 1 ou 2 atomes de carbone, pipérazinyle éventuellement substitué sur le second atome d'azote soit par un radical phényle éventuellement substitué par un atome d'halogène ou un radical trifluorométhyle, soit par un radical alkyle, alkényle ou alkynyle renfermant 1 à 4 atomes de carbone éventuellement substitué par un radical phényle ou acyle, soit par un radical acyle renfermant au plus 6 atomes de carbone,

**ou bien** $R_{3A}$' et $R_{4A}$', identiques ou différents, représentent un atome d'hydrogène ; un radical hydroxyle ; phényle ; phényle substitué par un radical carboxy, pipéridinyle éventuellement substitué sur l'atome d'azote par un radical benzyle, pipérazinyle éventuellement substitué sur le second atome d'azote par un radical alkyle renfermant 1 ou 2 atomes de carbone, carbamoyle ; alcoxy renfermant au plus 4 atomes de carbone ; alkyle renfermant au plus 4 atomes de carbone éventuellement substitué par un ou plusieurs radicaux, identiques ou différents, choisis parmi les radicaux hydroxyle, alcoxy, carboxy libre, salifié ou estérifié, amino, alkylamino, dialkylamino, phényle, morpholinyle, alkylthio renfermant au plus 4 atomes de carbone, éventuellement substitué par un radical carboxy libre, salifié ou estérifié, benzodiazépine éventuellement substitué, lesdits produits de formule ($I'_A$) étant sous toutes les formes isomères possibles racémiques, énantiomères et diastéréoisomères, ainsi que les sels d'addition avec les acides minéraux et organiques desdits produits de formule ($I'_A$).

**7.** Produits de formule (I') telle que définie à la revendication 5, caractérisés en ce que :
$R_1$' représente un atome d'hydrogène ou un radical acétyle,
$R_2$' représente un radical phényle,
$R_3$' et $R_4$' sont tels que :

**ou bien** $R_3$' et $R_4$' forment avec l'atome d'azote auxquels ils sont liés un radical pipéridinyle, morpholinyle, diméthylmorpholinyle, thiomorpholinyle, pyrrolidinyle, azépine, pipérazinyle éventuellement substitué sur le second atome d'azote par un radical alkyle renfermant 1 ou 2 atomes de carbone ou un radical phényle lui-même éventuellement substitué par un atome d'halogène ou un radical trifluorométhyle,

**ou bien** $R_3$' et $R_4$', identiques ou différents, représentent un atome d'hydrogène ; un radical hydroxyle ; phényle ; carbamoyle ; alcoxy renfermant au plus 4 atomes de carbone ; alkyle renfermant au plus 4 atomes de carbone éventuellement substitué par un ou plusieurs radicaux, identiques ou différents, choisis parmi les radicaux hydroxyle, alcoxy, carboxy libre, salifié ou estérifié,
lesdits produits de formule (I') étant sous toutes les formes isomères possibles racémiques, énantiomères et diastéréoisomères, ainsi que les sels d'addition avec les acides minéraux et organiques desdits produits de formule (I').

8. Les produits de formule (IA) répondant aux formules suivantes :
le chlorhydrate de (±) 4-méthyl-N-[2-mercapto 1-(phénylméthyl) éthyl] 1-pipérazinecarboxamide,
le (±) N-[2-mercapto 1-(phénylméthyl) éthyl] 4-morpholinecarboxamide,
le (±) N-[2-mercapto 1-(phénylméthyl) éthyl] 1-pyrrolidinecarboxamide,
le (±) N-[[[2-mercapto 1-(phénylméthyl) éthyl] amino] carbonyl glycine,
le N,N-bis(2-méthoxy éthyl)-N'-[2-mercapto 1-(phénylméthyl) éthyl] urée,
le (±) cis-2,6-diméthyl N-[2-mercapto 1-(phényl méthyl) éthyl] 4-morpholinecarboxamide (isomère A),
le (±) N-[2-mercapto 1-(phénylméthyl) éthyl] 4-thiomorpholinecarboxamide,
le (±) N-[2-mercapto 1-(phénylméthyl) éthyl] N'-méthoxy urée,
le (±) N-hydroxy N'-[2-mercapto 1-(phénylméthyl) éthyl] N-méthyl urée,
le (±) N-(1-mercaptométhyl) 2-(phényléthyl) 4-méthyl 1-pipérazine acétamide,
ainsi que leurs sels avec les acides minéraux ou organiques.

9. Procédé de préparation de produits de formule ($I_A$) telle que définie à la revendication 1, caractérisé en ce que :
- pour préparer les produits de formule ($I_A$) dans laquelle n = 0, l'on soumet un composé de formule (II):

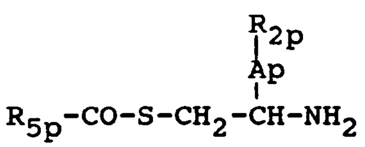

(II)

dans laquelle $R_{5p}$, $R_{2p}$ et Ap ont les significations indiquées ci-dessus respectivement pour $R_5$, $R_2$ et A dans lesquelles les éventuelles fonctions réactives sont éventuellement protégées par des groupements protecteurs, à l'action d'un réactif tel que le phosgène ou le thiophosgène pour obtenir respectivement l'isocyanate ou l'isothiocyanate correspondant de formule (III) :

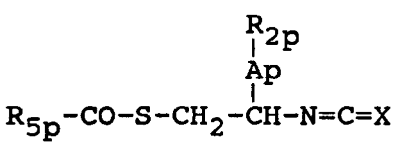

(III)

dans laquelle $R_{5p}$, $R_{2p}$ et Ap ont les significations indiquées précédemment et X représente un atome d'oxygène ou un atome de soufre, que l'on fait réagir avec un composé de formule (IV) :

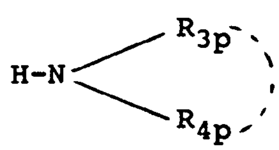

(IV)

dans laquelle $R_{3p}$ et $R_{4p}$ ont les significations indiquées ci-dessus respectivement pour $R_3$ et $R_4$ dans lesquelles les éventuelles fonctions réactives sont éventuellement protégées par des groupements protecteurs, pour obtenir un produit de formule (V):

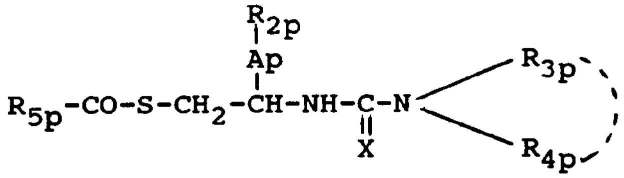

(V)

dans laquelle $R_{5p}$, $R_{2p}$, Ap, $R_{3p}$, $R_{4p}$ et X ont les significations indiquées précédemment, que l'on soumet, si nécessaire et si désiré, à une réaction d'élimination du radical $R_{5p}$-CO- pour obtenir un produit de formule (VI) :

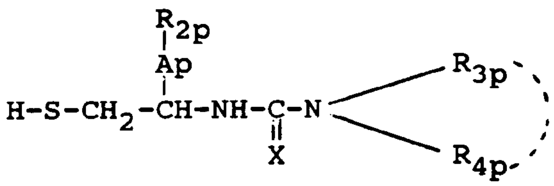

(VI)

dans laquelle $R_{2p}$, Ap, $R_{3p}$, $R_{4p}$ et X ont les significations indiquées précédemment,

- pour préparer les produits de formule ($I_A$) dans laquelle n est différent de 0 et X représente un atome d'oxygène, l'on soumet un produit de formule (II') :

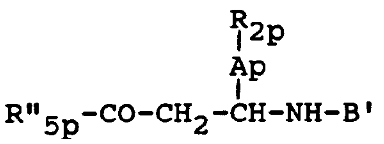

(II')

dans laquelle B' représente un atome d'hydrogène ou un groupe protecteur de la fonction amine et R"$_5$ représente un radical alkyle renfermant de 1 à 4 atomes de carbone substitué par un radical

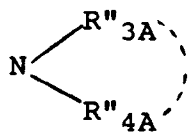

dans lequel R"$_{3A}$ et R"$_{4A}$ ont la valeur indiquée ci-dessus respectivement pour $R_{3A}$ et $R_{4A}$ à l'action le cas échéant d'un agent d'élimination du groupement protecteur porté par la fonction amine, puis à l'action d'un agent alcalin pour obtenir par transposition moléculaire un produit de formule (VII) :

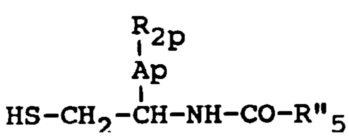

(VII)

dans laquelle $R_{2p}$, $A_p$ et R"$_5$ ont la signification indiquée ci-dessus, produit de formule (V), (VI) et (VII) que l'on traite, si désiré et si nécessaire, à l'une ou plusieurs des réactions suivantes, dans un ordre quelconque :

- une réaction d'élimination des groupements protecteurs que peuvent porter les fonctions réactives protégées,
- une réaction de salification par un acide minéral ou organique pour obtenir le sel correspondant,
- une réaction de réduction de fonction carboxy esterifiée en fonction alcool,
- une réaction de saponification de fonction ester en fonction acide,
- une réaction de transformation de fonction cyano en fonction acide,
- une réaction de transformation de fonction alcoxy en fonction hydroxyle,
- une réaction d'estérification, salification ou carbamoylation de fonction acide,

- une réaction d'estérification de la fonction thiol par un acide ou une fonction acide, ou par un isocyanate ou par un chlorure de carbamoyle.
  lesdits produits de formule (I) ainsi obtenus étant sous toutes les formes isomères possibles racémiques, énantiomères et diastéréoisomères.

**10.** A titre de médicaments, les produits tels que définis par la formule ($I_A$) de la revendication 1, lesdits produits de formule ($I_A$) étant sous tous les formes isoméres possibles racémiques énantiomères et diastéréoisomères,
ainsi que les sels d'addition avec les acides minéraux ou organiques pharmaceutiquement acceptables desdits produits de formule ($I_A$).

**11.** A titre de médicaments, les produits de formule ($I_A$) tels que définis aux revendications 2 à 8, ainsi que les sels d'addition avec les acides minéraux ou organiques pharmaceutiquement acceptables desdits produits de formule ($I_A$).

**12.** Les compositions pharmaceutiques contenant à titre de principe actif, l'un au moins des médicaments tels que définis à l'une quelconque des revendications 11 et 12.

**13.** Procédé de préparation des produits de départ de formule (II) telle que définie à la revendication 9, caractérisé en ce que l'on soumet un composé de formule (IIa) :

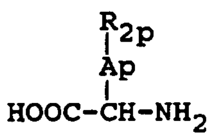

(IIa)

dans laquelle $R_{2p}$ et Ap ont les significations indiquées à la revendication 1 respectivement pour $R_2$ et A dans lesquelles les éventuelles fonctions réactives sont éventuellement protégées par des groupements protecteurs, à l'action d'un agent réducteur de la fonction acide, pour obtenir l'alcool correspondant de formule (IIb) :

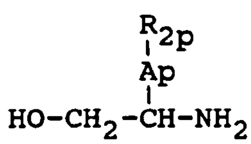

(IIb)

dans laquelle $R_{2p}$ et Ap ont les significations indiquées précédemment, que l'on cyclise sous l'action d'un dérivé carbonylé, pour obtenir l'oxazolidone correspondant de formule (IIc) :

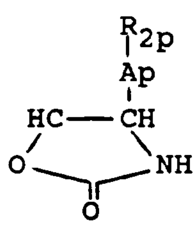

(IIc)

dans laquelle $R_{2p}$ et Ap ont les significations indiquées précédemment, que l'on soumet à l'action d'un agent protecteur, pour obtenir un produit de formule (IId) :

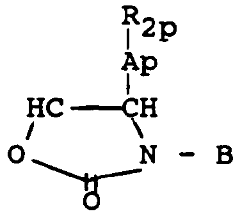

(IId)

dans laquelle $R_{2p}$ et Ap ont les significations indiquées précédemment et B représente un groupement protecteur de la fonction amine, que l'on fait réagir avec un composé de formule (IIe) :

$R_{5p}$-CO-SH (IIe)

dans laquelle $R_{5p}$ a la signification indiquée ci-dessus pour $R_5$ dans laquelle les éventuelles fonctions réactives sont éventuellement protégées par des groupements protecteurs, pour obtenir un composé de formule (IIf) :

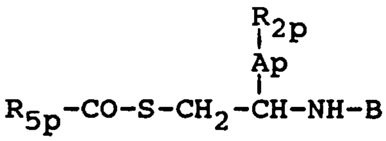

(IIf)

dans laquelle $R_{2p}$, $R_{5p}$, Ap et B ont les significations indiquées précédemment, que l'on soumet, si désiré, à une réaction d'élimination de B, lesdits produits de formule (II) ainsi obtenus étant sous toutes les formes isomères possibles racémiques, énantiomères et diastéréoisomères.

**14.** A titre de produits industriels nouveaux, les composés de formule (III).

**Revendications pour l'Etat contractant suivant : ES**

**1.** Procédé pour préparer des produits de formule ($I_A$):

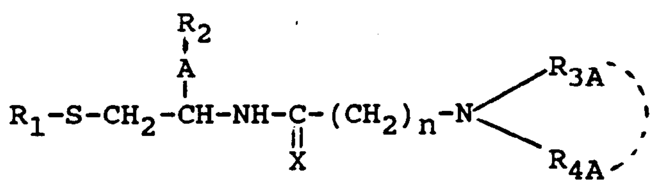

($I_A$)

dans laquelle :
n représente les valeurs 0 à 4,
$R_1$ représente un atome d'hydrogène ou un radical $R_5$ - CO - dans lequel $R_5$ représente :

- un radical alkyle, alkényle ou alkynyle linéaire ou ramifié renfermant au plus 6 atomes de carbone et éventuellement substitué,
- un radical monocyclique comprenant 5 à 7 chaînons ou un radical constitué de cycles condensés comprenant 8 à 10 chaînons, ces radicaux étant saturés ou insaturés, renfermant éventuellement un ou plusieurs hétéroatomes, identiques ou différents, choisis parmi les atomes d'oxygène, d'azote et de soufre et étant éventuellement substitués,
- un radical

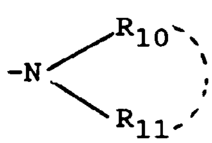

dans lequel $R_{10}$ et $R_{11}$ sont tels que :

**ou bien** $R_{10}$ et $R_{11}$ forment avec l'atome d'azote auxquels ils sont liés un radical monocyclique comprenant 5 à 7 chaînons ou un radical constitué de cycles condensés comprenant 8 à 10 chaînons, ces radicaux renfermant éventuellement un autre ou plusieurs autres hétéroatomes, identiques ou différents, choisis parmi les atomes d'oxygène, d'azote et de soufre, et étant éventuellement substitués,

**ou bien** $R_{10}$ et $R_{11}$, identiques ou différents, représentent :

- un atome d'hydrogène ;
- un radical alkyle, alkényle ou alkynyle linéaire ou ramifié renfermant au plus 6 atomes de carbone et éventuellement substitué ;
- un radical aryle dans lequel le radical aryle représente un radical monocyclique comprenant 5 à 7 chaînons ou constitué de cycles condensés comprenant 8 à 10 chaînons, ces radicaux renfermant éventuellement un ou plusieurs hétéroatomes, identiques ou différents, choisis parmi les atomes d'oxygène, d'azote et de soufre, et étant éventuellement substitués ; A représente :
- soit un radical alkylène ou alkénylène linéaire ou ramifié renfermant au plus 6 atomes de carbone et éventuellement substitué par un radical hydroxyle ou alcoxy renfermant au plus 6 atomes de carbone,
- soit une simple liaison,

$R_2$ représente un radical monocyclique comprenant 5 à 7 chaînons ou un radical constitué de cycles condensés comprenant 8 à 10 chaînons, ces radicaux étant saturés ou insaturés, renfermant éventuellement un ou plusieurs hétéroatomes, identiques ou différents, choisis parmi les atomes d'oxygène, d'azote et de soufre et étant éventuellement substitués,

X représente un atome d'oxygène ou de soufre,

$R_{3A}$ et $R_{4A}$ sont tels que : **ou bien** $R_{3A}$ et $R_{4A}$ forment avec l'atome d'azote auxquels ils sont liés un radical monocyclique comprenant 5 à 7 chaînons ou un radical constitué de cycles condensés comprenant 8 à 10 chaînons, ces radicaux renfermant éventuellement un autre ou plusieurs autres hétéroatomes, identiques ou différents, choisis parmi les atomes d'oxygène, d'azote et de soufre, et étant éventuellement substitués,

**ou bien** $R_{3A}$ et $R_{4A}$, identiques ou différents, représentent :

- un atome d'hydrogène ;
- un radical hydroxyle ; alcoxy, acyle ou acyloxy renfermant au plus 6 atomes de carbone ; carboxy, salifié ou estérifié ; cyano ;
- un radical alkyle, alkényle ou alkynyle linéaire ou ramifié renfermant au plus 6 atomes de carbone et éventuellement substitué ;
- un radical $R_c$ ou $R_c$-O- dans lesquels le radical $R_c$ représente un radical aliphatique ou arylique, monocyclique comprenant 5 à 7 chaînons ou constitué de cycles condensés comprenant 8 à 10 chaînons, ces radicaux renfermant éventuellement un ou plusieurs hétéroatomes, identiques ou différents, choisis parmi les atomes d'oxygène, d'azote et de soufre, et étant éventuellement substitués ;
- le radical

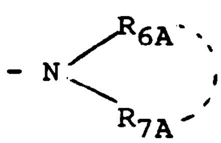

ou

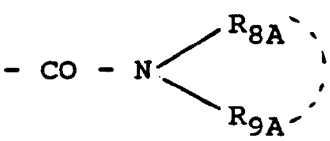

dans lesquels $R_{6A}$, $R_{7A}$, $R_{8A}$ et $R_{9A}$, identiques ou différents, représentent un atome d'hydrogène, un radical hydroxyle, alkyle, alcoxy, acyloxy ou acyle, ces radicaux renfermant au plus 6 atomes de carbone, carboxy libre, salifié ou estérifié, phénoxy, phényle, benzyle, phénéthyle, azépine, pipéridyle, morpholine, pyrrolidinyle, pipérazinyle, éventuellement substitués sur le second atome d'azote par un radical hydroxyle, alkyle, alcoxy ou phényle, ces trois derniers radicaux étant eux-mêmes éventuellement substitués par un ou plusieurs radicaux choisis dans les atomes d'halogène, le radical hydroxyle, trifluorométhyle, méthoxy, éthoxy, méthyle et éthyle,

ou bien d'une part $R_{6A}$ et $R_{7A}$ et d'autre part $R_{6A}$ et $R_{9A}$ forment respectivement avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux hydroxyle, trifluorométhyle, alkyle et alcoxy, ces radicaux renfermant au plus 6 atomes de carbone,

lesdits produits de formule ($I_A$) étant sous toutes les formes isomères possibles racémiques, énantiomères

et diastéréoisomères,
ainsi que les sels d'addition avec les acides minéraux et organiques desdits produits de formule ($I_A$), caractérisé en ce que :

- pour préparer les produits de formule ($I_A$) dans laquelle n = 0, l'on soumet un composé de formule (II):

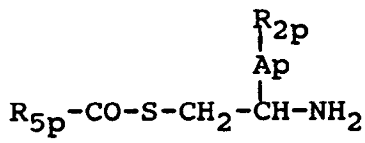

(II)

dans laquelle $R_{5p}$, $R_{2p}$ et Ap ont les significations indiquées ci-dessus respectivement pour $R_5$, $R_2$ et A dans lesquelles les éventuelles fonctions réactives sont éventuellement protégées par des groupements protecteurs, à l'action d'un réactif tel que le phosgène ou le thiophosgène pour obtenir respectivement l'isocyanate ou l'isothiocyanate correspondant de formule (III) :

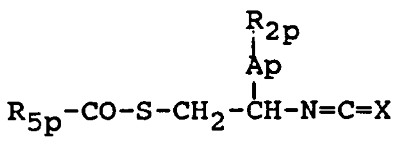

(III)

dans laquelle $R_{5p}$, $R_{2p}$ et Ap ont les significations indiquées précédemment et X représente un atome d'oxygène ou un atome de soufre, que l'on fait réagir avec un composé de formule (IV) :

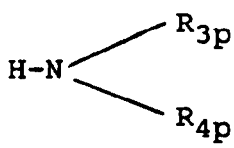

(IV)

dans laquelle $R_{3p}$ et $R_{4p}$ ont les significations indiquées ci-dessus respectivement pour $R_3$ et $R_4$ dans lesquelles les éventuelles fonctions réactives sont éventuellement protégées par des groupements protecteurs, pour obtenir un produit de formule (V):

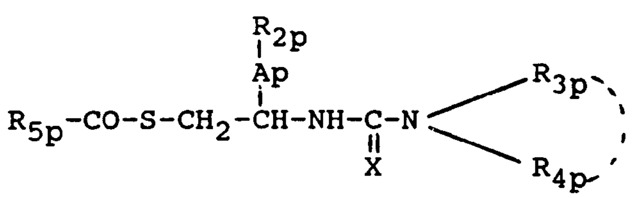

(V)

dans laquelle $R_{5p}$, $R_{2p}$, Ap, $R_{3p}$, $R_{4p}$ et X ont les significations indiquées précédemment, que l'on soumet, si nécessaire et si désiré, à une réaction d'élimination du radical $R_{5p}$-CO- pour obtenir un produit de formule (VI) :

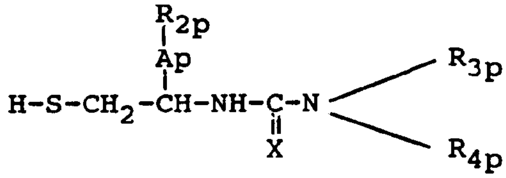

(VI)

dans laquelle $R_{2p}$, Ap, $R_{3p}$, $R_{4p}$ et X ont les significations indiquées précédemment,

- pour préparer les produits de formule ($I_A$) dans laquelle n est différent de 0 et X représente un atome d'oxygène, l'on soumet un produit de formule (II') :

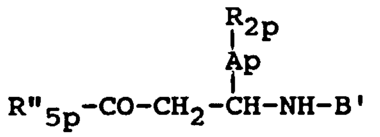

(II')

dans laquelle B' représente un atome d'hydrogène ou un groupe protecteur de la fonction amine et R"$_5$ représente un radical alkyle renfermant de 1 à 4 atomes de carbone substitué par un radical

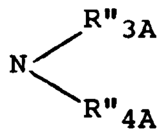

dans lequel R"$_{3A}$ et R"$_{4A}$ ont la valeur indiquée ci-dessus respectivement pour $R_{3A}$ et $R_{4A}$ à l'action le cas échéant d'un agent d'élimination du groupement protecteur porté par la fonction amine, puis à l'action d'un agent alcalin pour obtenir par transposition moléculaire un produit de formule (VII) :

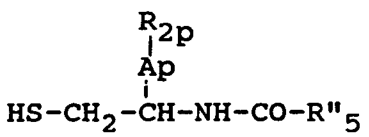

(VII)

dans laquelle $R_{2p}$, $A_p$ et R"$_5$ ont la signification indiquée ci-dessus, produit de formule (V), (VI) et (VII) que l'on traite, si désiré et si nécessaire, à l'une ou plusieurs des réactions suivantes, dans un ordre quelconque :

- une réaction d'élimination des groupements protecteurs que peuvent porter les fonctions réactives protégées,
- une réaction de salification par un acide minéral ou organique pour obtenir le sel correspondant,
- une réaction de réduction de fonction carboxy esterifiée en fonction alcool,
- une réaction de saponification de fonction ester en fonction acide,
- une réaction de transformation de fonction cyano en fonction acide,
- une réaction de transformation de fonction alcoxy en fonction hydroxyle,
- une réaction d'estérification, salification ou carbamoylation de fonction acide,
- une réaction d'estérification de la fonction thiol par un acide ou une fonction acide, ou par un isocyanate ou par un chlorure de carbamoyle.

lesdits produits de formule (I) ainsi obtenus étant sous toutes les formes isomères possibles racémiques, énantiomères et diastéréoisomères.

**2.** Procédé selon la revendication 1, caractérisé en ce que le ou les substituants, identiques ou différents que peuvent porter :

a) les radicaux alkyle, alkényle, alkynyle,

b) les radicaux monocyclique ou constitué de cycles condensés, aryle et aryloxy,

sont choisis dans le groupe formé par :

- les atomes d'halogène ; les radicaux hydroxyle ; cyano ; mercapto ; nitro ; acyle et acyloxy ayant au plus 6 atomes de carbone ; carboxy libre, salifié ou estérifié ; alcoxycarbonyle ;
- les radicaux alkoxy linéaires et ramifiés renfermant au plus 6 atomes de carbone ;
- les radicaux alkyle, alkényle, alkynyle, alkylthio, cycloalkyle, cycloalkényle, les radicaux hydrocarbonés hétérocycliques, aryle, aryloxy, arylthio, phénylalkyle, phénylalcoxy, eux-mêmes éventuellement substitués par un ou plusieurs substituants identiques ou différents choisis parmi les atomes d'halogène, les radicaux hydroxyle, trifluorométhyle, nitro, alkyle, alkényle, aryle, alcoxy et acyle, ces radicaux renfermant au plus 6 atomes de carbone, carboxy libre, salifié ou estérifié, acylamido dans lequel le radical acyle renferme au plus 6 atomes de carbone, carbamoyle et amino, ces radicaux étant éventuellement substitués sur l'atome d'azote par un ou deux radicaux identiques ou différents choisis parmi les radicaux hydroxyle, alkyle et alcoxy renfermant au plus 6 atomes de carbone ;
- le radical acylamido dans lequel le radical acyle renferme au plus 6 atomes de carbone ; les radicaux

carbamoyle et amino, ces radicaux étant éventuellement substitués sur l'atome d'azote par un ou deux radicaux identiques ou différents choisis parmi les radicaux hydroxyle, alkyle, alkényle et alcoxy renfermant au plus 6 atomes de carbone.

3. Procédé selon la revendication 1 ou 2, caractérisé en ce que l'on utilise au départ des produits de formule (II) et (IV) choisis de manière telle que l'on prépare un produit répondant à la formule ($I'_A$) :

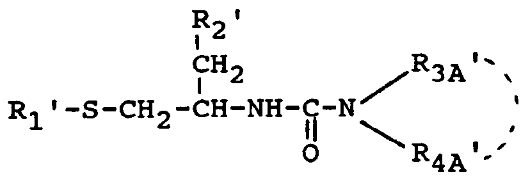

($I'_A$)

dans laquelle :
$R_{1A}$' représente un atome d'hydrogène ou un radical $R_{5A}$'-CO- dans lequel $R_{5A}$' représente un radical phényle, cycloalkyle, cycloalkényle, alkyle ou alkényle renfermant au plus 6 atomes de carbone, ces radicaux étant éventuellement substitués, ou un radical

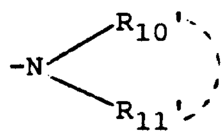

dans lequel $R_{10}$' et $R_{11}$' sont tels que :
**ou bien** $R_{10}$' et $R_{11}$' forment avec l'atome d'azote auxquels ils sont liés un radical imidazolyle, pyrrolyle, pyrrolinyle, pyrrolidinyle, pyridyle, pipéridinyle, pyrimidinyle, piridazinyle, pyrazinyle, pipérazinyle, phénylpipérazinyle, méthylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, mercapto, trifluorométhyle, alkyle, alkényle, alkynyle, alkylthio, alcoxy et phényle éventuellement substitué par un ou plusieurs radicaux choisis parmi les atomes d'halogène et les radicaux trifluorométhyle, hydroxyle, alkyle et alcoxy, les radicaux alkyle et alcoxy renfermant au plus 6 atomes de carbone,
**ou bien** $R_{10}$' et $R_{11}$', identiques ou différents, représentent :

- un atome d'hydrogène ;
- un radical alkyle, alkényle ou alkynyle linéaire ou ramifié renfermant au plus 6 atomes de carbone et éventuellement substitué,
- un radical phényle, benzyle, naphtyle, indényle, imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux hydroxyle, mercapto, trifluorométhyle, les radicaux alkyle, alkényle, alkynyle, alkylthio et alcoxy renfermant au plus 6 atomes de carbone,

$R_2$' représente un radical phényle, naphtyle, benzyle, phénéthyle, indolyle, cycloalkyle, cycloalkényle, ces radicaux étant éventuellement substitués,
$R_{3A}$' et $R_{4A}$' sont tels que :
ou bien $R_{3A}$' et $R_{4A}$' forment avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux hydroxyle, trifluorométhyle, alkyle, alkényle, alkynyle, alcoxy et phényle éventuellement substitué par un ou plusieurs radicaux choisis parmi les atomes d'halogène et les radicaux trifluorométhyle, hydroxyle, alkyle et alcoxy, les radicaux alkyle et alcoxy renfermant au plus 6 atomes de carbone et étant éventuellement substitué par un radical phényle, ou bien $R_{3A}$' et $R_{4A}$', identiques ou différents, représentent :

- un atome d'hydrogène ;
- un radical hydroxyle ; alcoxy, acyloxy ou acyle renfermant au plus 6 atomes de carbone ; carboxy,

salifié ou estérifié ; cyano ;

- un radical alkyle, alkényle ou alkynyle linéaire ou ramifié renfermant au plus 6 atomes de carbone et éventuellement substitué,
- un radical phényle, benzoyle, naphtyle, indényle, imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, diazépine, benzodiazépine, indolyle, ces radicaux étant éventuellement substitués par un ou plusieurs radicaux identiques ou différents choisis parmi les atomes d'halogène, les radicaux hydroxyle, trifluorométhyle, les radicaux alkyle et alcoxy renfermant au plus 6 atomes de carbone, eux-mêmes éventuellement substitués par un ou plusieurs radicaux choisis parmi les atomes d'halogène, les radicaux hydroxyle, alkyle, alcoxy et carboxy, libre, salifié ou estérifié par un radical alkyle, aryle ou arylalkyle,
- le radical

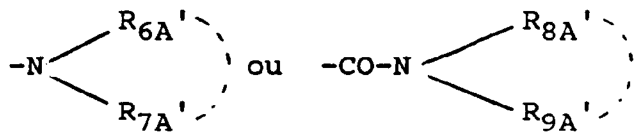

dans lesquels $R_{6A}$', $R_{7A}$', $R_{6A}$' et $R_{9A}$', identiques ou différents, représentent un atome d'hydrogène, un radical hydroxyle, alkyle, alcoxy, acyloxy ou acyle, ces radicaux renfermant au plus 6 atomes de carbone, carboxy libre, salifié ou estérifié, phényle, benzyle, phénéthyle, azépinyle, pipéridyle, morpholine, pyrrolidinyle, pipérazinyle, ou bien d'une part $R_{6A}$' et $R_{7A}$' et d'autre part $R_{6A}$' et $R_{9A}$' forment respectivement avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux hydroxyle, trifluorométhyle, alkyle et alcoxy, ces radicaux renfermant au plus 6 atomes de carbone, étant entendu que le ou les éventuels substituants, identiques ou différents, que peuvent porter les radicaux que peuvent représenter $R_1$', $R_2$' et $R_6$' sont choisis dans le groupe formé par :

- les atomes d'halogène ; les radicaux hydroxyle ; cyano ; nitro ; acyle et acyloxy ayant au plus 6 atomes de carbone ; benzoyle ; carboxy libre, salifié ou estérifié ;
- les radicaux alkoxy linéaires et ramifiés renfermant au plus 6 atomes de carbone ;
- les radicaux alkyle, alkényle, cyclopentyle, cyclohexyle, cyclopentényle, cyclohexényle, phényle, naphtyle, indolyle, phénoxy, benzyle, phénéthyle, benzyloxy, eux-mêmes éventuellement substitués par un ou plusieurs substituants identiques ou différents choisis parmi les atomes d'halogène, les radicaux hydroxyle, trifluorométhyle, nitro, alkyle, alkényle, alcoxy, ces radicaux renfermant au plus 6 atomes de carbone, formyle, acétyle, benzoyle, carboxy libre, salifié ou estérifié, acétamido, benzoylamido, carbamoyle et amino, ces radicaux étant éventuellement substitués sur l'atome d'azote par un ou deux radicaux identiques ou différents choisis parmi les radicaux hydroxyle, méthyle, éthyle, propyle, méthoxy, éthoxy et propoxy ;
- le radical acylamido dans lequel le radical acyle renferme au plus 6 atomes de carbone ; les radicaux carbamoyle et amino, ces radicaux étant éventuellement substitués sur l'atome d'azote par un ou deux radicaux identiques ou différents choisis parmi les radicaux hydroxyle, alkyle, alkényle, alcoxy et acyle renfermant au plus 6 atomes de carbone ; lesdits produits de formule ($I'_A$) étant sous toutes les formes isomères possibles racémiques, énantiomères et diastéréoisomères, ainsi que les sels d'addition avec les acides minéraux et organiques desdits produits de formule ($I'_A$).

**4.** Procédé selon l'une quelconque des revendications 1 à 3, caractérisé en ce que l'on utilise au départ un produit de formule (II) dans laquelle $R_{5p}$ représente un radical méthyle et $R_{2p}$ représente un radical phényle et un produit de formule (IV) dans laquelle $R_{3p}$ et $R_{4p}$ sont tels que

ou bien $R_{3p}$ et $R_{4p}$ forment avec l'atome d'azote auxquels ils sont liés un radical pipéridinyle, morpholinyle, diméthylmorpholinyle, thiomorpholinyle, pyrrolidinyle, azépine, diazépine éventuellement substitué sur le second atome d'azote par un radical alkyle renfermant 1 ou 2 atomes de carbone, pipérazinyle éventuellement substitué sur le second atome d'azote soit par un radical phényle éventuellement substitué par un atome d'halogène ou un radical trifluorométhyle, soit par un radical alkyle, alkényle ou alkynyle renfermant 1 à 4 atomes de carbone éventuellement substitué par un radical phényle ou acyle, soit par un radical acyle renfermant au plus 6 atomes de carbone,

**ou bien** $R_{3p}$ et $R_{4p}$, identiques ou différents, représentent un atome d'hydrogène ; un radical hydroxyle ; phényle ; phényle substitué par un radical carboxy, pipéridinyle éventuellement substitué sur l'atome d'azote par un radical benzyle, pipérazinyle éventuellement substitué sur le second atome d'azote par un radical alkyle renfermant 1 ou 2 atomes de carbone, carbamoyle ; alcoxy renfermant au plus 4 atomes de carbone ; alkyle renfermant au plus 4 atomes de carbone éventuellement substitué par un ou plusieurs radicaux, identiques ou différents, choisis parmi les radicaux hydroxyle, alcoxy, carboxy libre, salifié ou estérifié, amino, alkylamino, dialkylamino, phényle, morpholinyle, alkylthio renfermant au plus 4 atomes de carbone, éventuellement substitué par un radical carboxy libre, salifié ou estérifié, benzodiazépine éventuellement substitué et dans lesquels les fonctions réactives sont éventuellement protégées.

**5.** Procédé selon l'une quelconque des revendications 1 à 4, caractérisé en ce que l'on utilise au départ des produits de formule (II) et (IV) choisis de manière telle que l'on prépare un produit répondant aux formules suivantes :
le chlorhydrate de (±) 4-méthyl-N-[2-mercapto 1-(phénylméthyl) éthyl] 1-pipérazinecarboxamide,
le (±) N-[2-mercapto 1-(phénylméthyl) éthyl] 4-morpholinecarboxamide,
le (±) N-[2-mercapto 1-(phénylméthyl) éthyl] 1-pyrrolidinecarboxamide,
le (±) N-[[[2-mercapto 1-(phénylméthyl) éthyl] amino] carbonyl] glycine,
le N,N-bis(2-méthoxy éthyl)-N'-[2-mercapto 1-(phénylméthyl) éthyl] urée,
le (±) cis-2,6-diméthyl N-[2-mercapto 1-(phényl méthyl) éthyl] 4-morpholinecarboxamide (isomère A),
le (±) N-[2-mercapto 1-(phénylméthyl) éthyl] 4-thiomorpholinecarboxamide,
le (±) N-[2-mercapto 1-(phénylméthyl) éthyl] N'-méthoxy urée,
le (±) N-hydroxy N'-[2-mercapto 1-(phénylméthyl) éthyl] N-méthyl urée,
le (±) N-(1-mercaptométhyl) 2-(phényléthyl) 4-méthyl 1-pipérazine acétamide,
ainsi que leurs sels avec les acides minéraux ou organiques.

**6.** Procédé de préparation des produits de départ de formule (II) telle que définie à la revendication 1, caractérisé en ce que l'on soumet un composé de formule (IIa) :

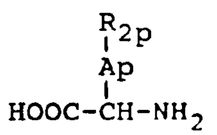

(IIa)

dans laquelle $R_{2p}$ et Ap ont les significations indiquées à la revendication 1 respectivement pour $R_2$ et A dans lesquelles les éventuelles fonctions réactives sont éventuellement protégées par des groupements protecteurs, à l'action d'un agent réducteur de la fonction acide, pour obtenir l'alcool correspondant de formule (IIb) :

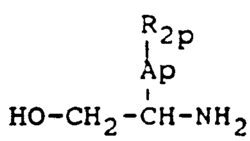

(IIb)

dans laquelle $R_{2p}$ et Ap ont les significations indiquées précédemment, que l'on cyclise sous l'action d'un dérivé carbonylé, pour obtenir l'oxazolidone correspondant de formule (IIc) :

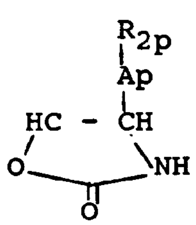

(IIc)

dans laquelle $R_{2p}$ et Ap ont les significations indiquées précédemment, que l'on soumet à l'action d'un agent protecteur, pour obtenir un produit de formule (IId) :

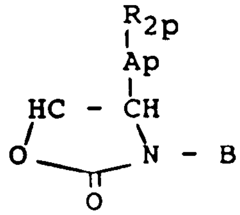

(IId)

dans laquelle $R_{2p}$ et Ap ont les significations indiquées précédemment et B représente un groupement protecteur de la fonction amine, que l'on fait réagir avec un composé de formule (IIe) :

$R_{5p}$-CO-SH (IIe)

dans laquelle $R_{5p}$ a la signification indiquée ci-dessus pour $R_5$ dans laquelle les éventuelles fonctions réactives sont éventuellement protégées par des groupements protecteurs, pour obtenir un composé de formule (IIf) :

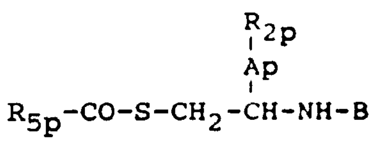

(IIf)

dans laquelle $R_{2p}$, $R_{5p}$, Ap et B ont les significations indiquées précédemment, que l'on soumet, si désiré, à une réaction d'élimination de B, lesdits produits de formule (II) ainsi obtenus étant sous toutes les formes isomères possibles racémiques, énantiomères et diastéréoisomères.

**Revendications pour l'Etat contractant suivant : GR**

1. Procédé pour préparer des produits de formule ($I_A$):

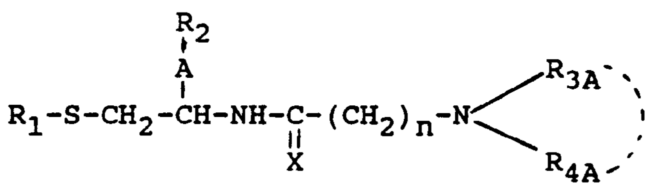

($I_A$)

dans laquelle :
n représente les valeurs 0 à 4,
$R_1$ représente un atome d'hydrogène ou un radical $R_5$ - CO - dans lequel $R_5$ représente :

- un radical alkyle, alkényle ou alkynyle linéaire ou ramifié renfermant au plus 6 atomes de carbone et éventuellement substitué,
- un radical monocyclique comprenant 5 à 7 chaînons ou un radical constitué de cycles condensés comprenant 8 à 10 chaînons, ces radicaux étant saturés ou insaturés, renfermant éventuellement un ou plusieurs hétéroatomes, identiques ou différents, choisis parmi les atomes d'oxygène, d'azote et de soufre et étant éventuellement substitués,
- un radical

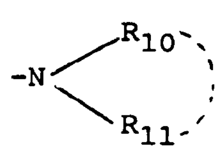

dans lequel $R_{10}$ et $R_{11}$ sont tels que :

**ou bien** $R_{10}$ et $R_{11}$ forment avec l'atome d'azote auxquels ils sont liés un radical monocyclique comprenant 5 à 7 chaînons ou un radical constitué de cycles condensés comprenant 8 à 10 chaînons, ces radicaux renfermant éventuellement un autre ou plusieurs autres hétéroatomes, identiques ou différents, choisis parmi les atomes d'oxygène, d'azote et de soufre, et étant éventuellement substitués,

**ou bien** $R_{10}$ et $R_{11}$, identiques ou différents, représentent :

- un atome d'hydrogène ;
- un radical alkyle, alkényle ou alkynyle linéaire ou ramifié renfermant au plus 6 atomes de carbone et éventuellement substitué ;
- un radical aryle dans lequel le radical aryle représente un radical monocyclique comprenant 5 à 7 chaînons ou constitué de cycles condensés comprenant 8 à 10 chaînons, ces radicaux renfermant éventuellement un ou plusieurs hétéroatomes, identiques ou différents, choisis parmi les atomes d'oxygène, d'azote et de soufre, et étant éventuellement substitués ;

A représente :

- soit un radical alkylène ou alkénylène linéaire ou ramifié renfermant au plus 6 atomes de carbone et éventuellement substitué par un radical hydroxyle ou alcoxy renfermant au plus 6 atomes de carbone,
- soit une simple liaison,

$R_2$ représente un radical monocyclique comprenant 5 à 7 chaînons ou un radical constitué de cycles condensés comprenant 8 à 10 chaînons, ces radicaux étant saturés ou insaturés, renfermant éventuellement un ou plusieurs hétéroatomes, identiques ou différents, choisis parmi les atomes d'oxygène, d'azote et de soufre et étant éventuellement substitués,

X représente un atome d'oxygène ou de soufre,

$R_{3A}$ et $R_{4A}$ sont tels que :

**ou bien** $R_{3A}$ et $R_{4A}$ forment avec l'atome d'azote auxquels ils sont liés un radical monocyclique comprenant 5 à 7 chaînons ou un radical constitué de cycles condensés comprenant 8 à 10 chaînons, ces radicaux renfermant éventuellement un autre ou plusieurs autres hétéroatomes, identiques ou différents, choisis parmi les atomes d'oxygène, d'azote et de soufre, et étant éventuellement substitués,

**ou bien** $R_{3A}$ et $R_{4A}$, identiques ou différents, représentent :

- un atome d'hydrogène ;
- un radical hydroxyle ; alcoxy, acyle ou acyloxy renfermant au plus 6 atomes de carbone ; carboxy, salifié ou estérifié ; cyano ;
- un radical alkyle, alkényle ou alkynyle linéaire ou ramifié renfermant au plus 6 atomes de carbone et éventuellement substitué ;
- un radical $R_c$ ou $R_c$-O- dans lesquels le radical $R_c$ représente un radical aliphatique ou arylique, monocyclique comprenant 5 à 7 chaînons ou constitué de cycles condensés comprenant 8 à 10 chaînons, ces radicaux renfermant éventuellement un ou plusieurs hétéroatomes, identiques ou différents, choisis parmi les atomes d'oxygène, d'azote et de soufre, et étant éventuellement substitués ;
- le radical

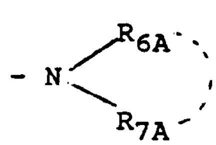

ou

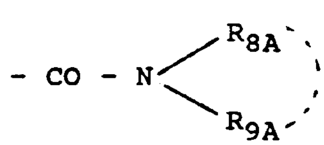

dans lesquels $R_{6A}$, $R_{7A}$, $R_{8A}$ et $R_{9A}$, identiques ou différents, représentent un atome d'hydrogène, un radical hydroxyle, alkyle, alcoxy, acyloxy ou acyle, ces radicaux renfermant au plus 6 atomes de carbone, carboxy libre, salifié ou estérifié, phénoxy, phényle, benzyle, phénéthyle, azépine, pipéridyle, morpholine, pyrrolidinyle, pipérazinyle, éventuellement substitués sur le second atome d'azote par un radical hydroxyle, alkyle, alcoxy ou phényle, ces trois derniers radicaux étant eux-mêmes éventuellement substitués par un ou plusieurs radicaux choisis dans les atomes d'halogène, le radical hydroxyle, trifluorométhyle, méthoxy, éthoxy, méthyle et éthyle,

ou bien d'une part $R_{6A}$ et $R_{7A}$ et d'autre part $R_{6A}$ et $R_{9A}$ forment respectivement avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux hydroxyle, trifluorométhyle, alkyle et alcoxy, ces radicaux renfermant au plus 6 atomes de carbone,
lesdits produits de formule ($I_A$) étant sous toutes les formes isomères possibles racémiques, énantiomères et diastéréoisomères,
ainsi que les sels d'addition avec les acides minéraux et organiques desdits produits de formule ($I_A$), caractérisé en ce que :

- pour préparer les produits de formule ($I_A$) dans laquelle n = 0, l'on soumet un composé de formule (II):

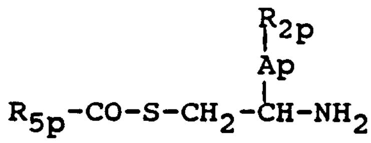

(II)

dans laquelle $R_{5p}$, $R_{2p}$ et Ap ont les significations indiquées ci-dessus respectivement pour $R_5$, $R_2$ et A dans lesquelles les éventuelles fonctions réactives sont éventuellement protégées par des groupements protecteurs, à l'action d'un réactif tel que le phosgène ou le thiophosgène pour obtenir respectivement l'isocyanate ou l'isothiocyanate correspondant de formule (III) :

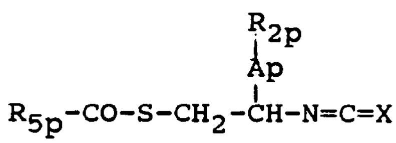

(III)

dans laquelle $R_{5p}$, $R_{2p}$ et Ap ont les significations indiquées précédemment et X représente un atome d'oxygène ou un atome de soufre, que l'on fait réagir avec un composé de formule (IV) :

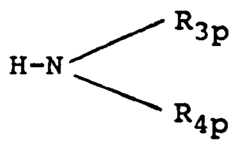

(IV)

dans laquelle $R_{3p}$ et $R_{4p}$ ont les significations indiquées ci-dessus respectivement pour $R_3$ et $R_4$ dans lesquelles les éventuelles fonctions réactives sont éventuellement protégées par des groupements protecteurs, pour obtenir un produit de formule (V):

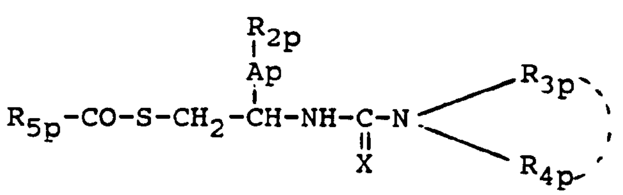

(V)

dans laquelle $R_{5p}$, $R_{2p}$, Ap, $R_{3p}$, $R_{4p}$ et X ont les significations indiquées précédemment, que l'on soumet, si nécessaire et si désiré, à une réaction d'élimination du radical $R_{5p}$-CO- pour obtenir un produit de formule (VI) :

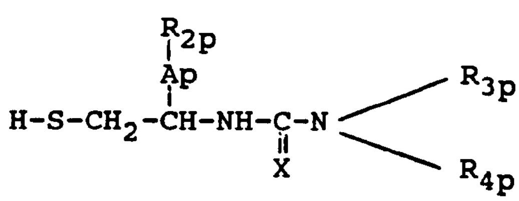

(VI)

dans laquelle $R_{2p}$, Ap, $R_{3p}$, $R_{4p}$ et X ont les significations indiquées précédemment,

- pour préparer les produits de formule ($I_A$) dans laquelle n est différent de 0 et X représente un atome d'oxygène, l'on soumet un produit de formule (II') :

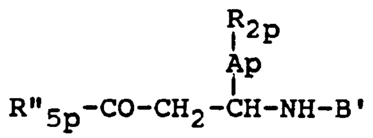

(II')

dans laquelle B' représente un atome d'hydrogène ou un groupe protecteur de la fonction amine et $R''_5$ représente un radical alkyle renfermant de 1 à 4 atomes de carbone substitué par un radical

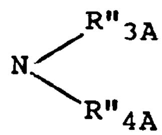

dans lequel $R''_{3A}$ et $R''_{4A}$ ont la valeur indiquée ci-dessus respectivement pour $R_{3A}$ et $R_{4A}$ à l'action le cas échéant d'un agent d'élimination du groupement protecteur porté par la fonction amine, puis à l'action d'un agent alcalin pour obtenir par transposition moléculaire un produit de formule (VII) :

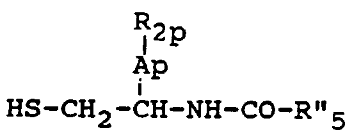

(VII)

dans laquelle $R_{2p}$, $A_p$ et $R''_5$ ont la signification indiquée ci-dessus, produit de formule (V), (VI) et (VII) que l'on traite, si désiré et si nécessaire, à l'une ou plusieurs des réactions suivantes, dans un ordre quelconque :

- une réaction d'élimination des groupements protecteurs que peuvent porter les fonctions réactives protégées,
- une réaction de salification par un acide minéral ou organique pour obtenir le sel correspondant,
- une réaction de réduction de fonction carboxy esterifiée en fonction alcool,
- une réaction de saponification de fonction ester en fonction acide,
- une réaction de transformation de fonction cyano en fonction acide,
- une réaction de transformation de fonction alcoxy en fonction hydroxyle,
- une réaction d'estérification, salification ou carbamoylation de fonction acide,
- une réaction d'estérification de la fonction thiol par un acide ou une fonction acide, ou par un isocyanate ou par un chlorure de carbamoyle.

lesdits produits de formule (I) ainsi obtenus étant sous toutes les formes isomères possibles racémiques, énantiomères et diastéréoisomères.

**2.** Procédé selon la revendication 1, caractérisé en ce que le ou les substituants, identiques ou différents que peuvent porter :

a) les radicaux alkyle, alkényle, alkynyle,

b) les radicaux monocyclique ou constitué de cycles condensés, aryle et aryloxy,

sont choisis dans le groupe formé par :

- les atomes d'halogène ; les radicaux hydroxyle ; cyano ; mercapto ; nitro ; acyle et acyloxy ayant au plus 6 atomes de carbone ; carboxy libre, salifié ou estérifié ; alcoxycarbonyle ;
- les radicaux alkoxy linéaires et ramifiés renfermant au plus 6 atomes de carbone ;
- les radicaux alkyle, alkényle, alkynyle, alkylthio, cycloalkyle, cycloalkényle, les radicaux hydrocarbonés hétérocycliques, aryle, aryloxy, arylthio, phénylalkyle, phénylalcoxy, eux-mêmes éventuellement substitués par un ou plusieurs substituants identiques ou différents choisis parmi les atomes d'halogène, les radicaux hydroxyle, trifluorométhyle, nitro, alkyle, alkényle, aryle, alcoxy et acyle, ces radicaux renfermant au plus 6 atomes de carbone, carboxy libre, salifié ou estérifié, acylamido dans lequel le radical acyle renferme au plus 6 atomes de carbone, carbamoyle et amino, ces radicaux

étant éventuellement substitués sur l'atome d'azote par un ou deux radicaux identiques ou différents choisis parmi les radicaux hydroxyle, alkyle et alcoxy renfermant au plus 6 atomes de carbone ;

- le radical acylamido dans lequel le radical acyle renferme au plus 6 atomes de carbone ; les radicaux carbamoyle et amino, ces radicaux étant éventuellement substitués sur l'atome d'azote par un ou deux radicaux identiques ou différents choisis parmi les radicaux hydroxyle, alkyle, alkényle et alcoxy renfermant au plus 6 atomes de carbone.

3. Procédé selon la revendication 1 ou 2, caractérisé en ce que l'on utilise au départ des produits de formule (II) et (IV) choisis de manière telle que l'on prépare un produit répondant à la formule ($I'_A$) :

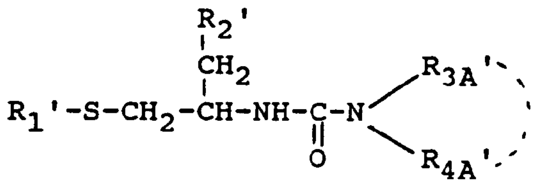

($I'_A$)

dans laquelle :

$R_{1A}$' représente un atome d'hydrogène ou un radical $R_{5A}$'-CO- dans lequel $R_{5A}$' représente un radical phényle, cycloalkyle, cycloalkényle, alkyle ou alkényle renfermant au plus 6 atomes de carbone, ces radicaux étant éventuellement substitués, ou un radical

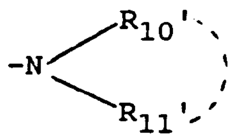

dans lequel $R_{10}$' et $R_{11}$' sont tels que :

**ou bien** $R_{10}$' et $R_{11}$' forment avec l'atome d'azote auxquels ils sont liés un radical imidazolyle, pyrrolyle, pyrrolinyle, pyrrolidinyle, pyridyle, pipéridinyle, pyrimidinyle, piridazinyle, pyrazinyle, pipérazinyle, phénylpipérazinyle, méthylpipérazinyle, pipéridyle, oxazolyle, morpholinyle et thiomorpholinyle, azépine, indolyle, ces radicaux étant éventuellement substitués par un ou plusieurs radicaux identiques ou différents choisis parmi les atomes d'halogène, les radicaux hydroxyle, mercapto, trifluorométhyle, alkyle, alkényle, alkynyle, alkylthio, alcoxy et phényle éventuellement substitué par un ou plusieurs radicaux choisis parmi les atomes d'halogène et les radicaux trifluorométhyle, hydroxyle, alkyle et alcoxy, les radicaux alkyle et alcoxy renfermant au plus 6 atomes de carbone,

**ou bien** $R_{10}$' et $R_{11}$', identiques ou différents, représentent :

- un atome d'hydrogène ;
- un radical alkyle, alkényle ou alkynyle linéaire ou ramifié renfermant au plus 6 atomes de carbone et éventuellement substitué,
- un radical phényle, benzyle, naphtyle, indényle, imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux hydroxyle, mercapto, trifluorométhyle, les radicaux alkyle, alkényle, alkynyle, alkylthio et alcoxy renfermant au plus 6 atomes de carbone,

$R_2$' représente un radical phényle, naphtyle, benzyle, phénéthyle, indolyle, cycloalkyle, cycloalkényle, ces radicaux étant éventuellement substitués,

$R_{3A}$' et $R_{4A}$' sont tels que : ou bien $R_{3A}$' et $R_{4A}$' forment avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux hydroxyle, trifluorométhyle, alkyle, alkényle, alkynyle, alcoxy et phényle éventuellement substitué par un ou plusieurs radicaux choisis parmi les atomes d'halogène et les radicaux trifluorométhyle, hydroxyle, alkyle et alcoxy, les radicaux alkyle et alcoxy renfermant au plus 6 atomes de carbone et étant éventuellement substitué par un radical phényle,

ou bien $R_{3A}$' et $R_{4A}$', identiques ou différents, représentent :

- un atome d'hydrogène ;
- un radical hydroxyle ; alcoxy, acyloxy ou acyle renfermant au plus 6 atomes de carbone ; carboxy, salifié ou estérifié ; cyano ;
- un radical alkyle, alkényle ou alkynyle linéaire ou ramifié renfermant au plus 6 atomes de carbone et éventuellement substitué,
- un radical phényle, benzoyle, naphtyle, indényle, imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, diazépine, benzodiazépine, indolyle, ces radicaux étant éventuellement substitués par un ou plusieurs radicaux identiques ou différents choisis parmi les atomes d'halogène, les radicaux hydroxyle, trifluorométhyle, les radicaux alkyle et alcoxy renfermant au plus 6 atomes de carbone, eux-mêmes éventuellement substitués par un ou plusieurs radicaux choisis parmi les atomes d'halogène, les radicaux hydroxyle, alkyle, alcoxy et carboxy, libre, salifié ou estérifié par un radical alkyle, aryle ou arylalkyle,
- le radical

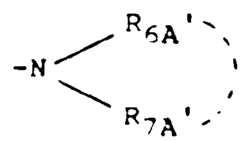
ou
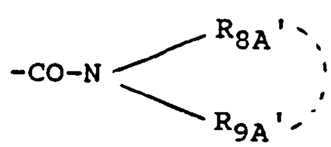

dans lesquels $R_{6A}$', $R_{7A}$', $R_{6A}$' et $R_{9A}$', identiques ou différents, représentent un atome d'hydrogène, un radical hydroxyle, alkyle, alcoxy, acyloxy ou acyle, ces radicaux renfermant au plus 6 atomes de carbone, carboxy libre, salifié ou estérifié, phényle, benzyle, phénéthyle, azépinyle, pipéridyle, morpholine, pyrrolidinyle, pipérazinyle, ou bien d'une part $R_{6A}$' et $R_{7A}$' et d'autre part $R_{6A}$' et $R_{9A}$' forment respectivement avec l'atome d'azote auxquels ils sont liés un radical imidazolyle, pyrrolyle, pyrrolinyle, pyrrolidinyle, pyridyle, pipéridinyle, pyrimidinyle, pyridazinyle, pyrazinyle, pipérazinyle, phénylpipérazinyle, pipéridyle, oxazolyle, morpholinyle et thiomorpholinyle, azépinyle, indolyle, ces radicaux étant éventuellement substitués par un ou plusieurs radicaux identiques ou différents choisis parmi les atomes d'halogène, les radicaux hydroxyle, trifluorométhyle, alkyle et alcoxy, ces radicaux renfermant au plus 6 atomes de carbone, étant entendu que le ou les éventuels substituants, identiques ou différents, que peuvent porter les radicaux que peuvent représenter $R_1$', $R_2$' et $R_6$' sont choisis dans le groupe formé par :
- les atomes d'halogène ; les radicaux hydroxyle ; cyano ; nitro ; acyle et acyloxy ayant au plus 6 atomes de carbone ; benzoyle ; carboxy libre, salifié ou estérifié ;
- les radicaux alkoxy linéaires et ramifiés renfermant au plus 6 atomes de carbone ;
- les radicaux alkyle, alkényle, cyclopentyle, cyclohexyle, cyclopentényle, cyclohexényle, phényle, naphtyle, indolyle, phénoxy, benzyle, phénéthyle, benzyloxy, eux-mêmes éventuellement substitués par un ou plusieurs substituants identiques ou différents choisis parmi les atomes d'halogène, les radicaux hydroxyle, trifluorométhyle, nitro, alkyle, alkényle, alcoxy, ces radicaux renfermant au plus 6 atomes de carbone, formyle, acétyle, benzoyle, carboxy libre, salifié ou estérifié, acétamido, benzoylamido, carbamoyle et amino, ces radicaux étant éventuellement substitués sur l'atome d'azote par un ou deux radicaux identiques ou différents choisis parmi les radicaux hydroxyle, méthyle, éthyle, propyle, méthoxy, éthoxy et propoxy ;
- le radical acylamido dans lequel le radical acyle renferme au plus 6 atomes de carbone ; les radicaux carbamoyle et amino, ces radicaux étant éventuellement substitués sur l'atome d'azote par un ou deux radicaux identiques ou différents choisis parmi les radicaux hydroxyle, alkyle, alkényle, alcoxy et acyle renfermant au plus 6 atomes de carbone ; lesdits produits de formule ($I'_A$) étant sous toutes les formes isomères possibles racémiques, énantiomères et diastéréoisomères, ainsi que les sels d'addition avec les acides minéraux et organiques desdits produits de formule ($I'_A$).

4. Procédé selon l'une quelconque des revendications 1 à 3, caractérisé en ce que l'on utilise au départ un produit de formule (II) dans laquelle $R_{5p}$ représente un radical méthyle et $R_{2p}$ représente un radical phényle et un produit de formule (IV) dans laquelle $R_{3p}$ et $R_{4p}$ sont tels que
ou bien $R_{3p}$ et $R_{4p}$ forment avec l'atome d'azote auxquels ils sont liés un radical pipéridinyle, morpholinyle, diméthylmorpholinyle, thiomorpholinyle, pyrrolidinyle, azépine, diazépine éventuellement substitué sur le second atome d'azote par un radical alkyle renfermant 1 ou 2 atomes de carbone, pipérazinyle éventuellement substitué sur le second atome d'azote soit par un radical phényle éventuellement substitué par un

atome d'halogène ou un radical trifluorométhyle, soit par un radical alkyle, alkényle ou alkynyle renfermant 1 à 4 atomes de carbone éventuellement substitué par un radical phényle ou acyle, soit par un radical acyle renfermant au plus 6 atomes de carbone,

**ou bien** $R_{3p}$ et $R_{4p}$, identiques ou différents, représentent un atome d'hydrogène ; un radical hydroxyle ; phényle ; phényle substitué par un radical carboxy, pipéridinyle éventuellement substitué sur l'atome d'azote par un radical benzyle, pipérazinyle éventuellement substitué sur le second atome d'azote par un radical alkyle renfermant 1 ou 2 atomes de carbone, carbamoyle ; alcoxy renfermant au plus 4 atomes de carbone ; alkyle renfermant au plus 4 atomes de carbone éventuellement substitué par un ou plusieurs radicaux, identiques ou différents, choisis parmi les radicaux hydroxyle, alcoxy, carboxy libre, salifié ou estérifié, amino, alkylamino, dialkylamino, phényle, morpholinyle, alkylthio renfermant au plus 4 atomes de carbone, éventuellement substitué par un radical carboxy libre, salifié ou estérifié, benzodiazépine éventuellement substitué et dans lesquels les fonctions réactives sont éventuellement protégées.

5. Procédé selon l'une quelconque des revendications 1 à 4, caractérisé en ce que l'on utilise au départ des produits de formule (II) et (IV) choisis de manière telle que l'on prépare un produit répondant aux formules suivantes :
le chlorhydrate de (±) 4-méthyl-N-[2-mercapto 1-(phénylméthyl) éthyl] 1-pipérazinecarboxamide,
le (±) N-[2-mercapto 1-(phénylméthyl) éthyl] 4-morpholinecarboxamide,
le (±) N-[2-mercapto 1-(phénylméthyl) éthyl] 1-pyrrolidinecarboxamide,
le (±) N-[[[2-mercapto 1-(phénylméthyl) éthyl] amino] carbonyl] glycine,
le N,N-bis(2-méthoxy éthyl)-N'-[2-mercapto 1-(phénylméthyl) éthyl] urée,
le (±) cis-2,6-diméthyl N-[2-mercapto 1-(phényl méthyl) éthyl] 4-morpholinecarboxamide (isomère A),
le (±) N-[2-mercapto 1-(phénylméthyl) éthyl] 4-thiomorpholinecarboxamide,
le (±) N-[2-mercapto 1-(phénylméthyl) éthyl] N'-méthoxy urée,
le (±) N-hydroxy N'-[2-mercapto 1-(phénylméthyl) éthyl] N-méthyl urée,
le (±) N-(1-mercaptométhyl) 2-(phényléthyl) 4-méthyl 1-pipérazine acétamide,
ainsi que leurs sels avec les acides minéraux ou organiques.

6. Procédé de préparation des produits de départ de formule (II) telle que définie à la revendication 1, caractérisé en ce que l'on soumet un composé de formule (IIa) :

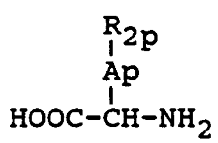

(IIa)

dans laquelle $R_{2p}$ et Ap ont les significations indiquées à la revendication 1 respectivement pour $R_2$ et A dans lesquelles les éventuelles fonctions réactives sont éventuellement protégées par des groupements protecteurs, à l'action d'un agent réducteur de la fonction acide, pour obtenir l'alcool correspondant de formule (IIb) :

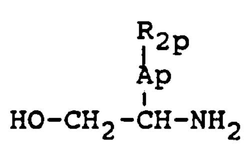

(IIb)

dans laquelle $R_{2p}$ et Ap ont les significations indiquées précédemment, que l'on cyclise sous l'action d'un dérivé carbonylé, pour obtenir l'oxazolidone correspondant de formule (IIc) :

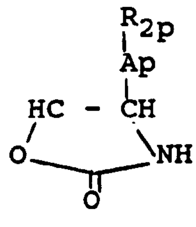

(IIc)

dans laquelle $R_{2p}$ et Ap ont les significations indiquées précédemment, que l'on soumet à l'action d'un agent protecteur, pour obtenir un produit de formule (IId) :

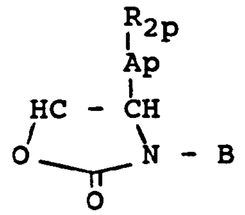

(IId)

dans laquelle $R_{2p}$ et Ap ont les significations indiquées précédemment et B représente un groupement protecteur de la fonction amine, que l'on fait réagir avec un composé de formule (IIe) :

$R_{5p}$-CO-SH (IIe)

dans laquelle $R_{5p}$ a la signification indiquée ci-dessus pour $R_5$ dans laquelle les éventuelles fonctions réactives sont éventuellement protégées par des groupements protecteurs, pour obtenir un composé de formule (IIf) :

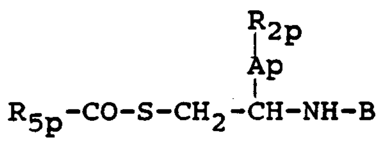

(IIf)

dans laquelle $R_{2p}$, $R_{5p}$, Ap et B ont les significations indiquées précédemment, que l'on soumet, si désiré, à une réaction d'élimination de B, lesdits produits de formule (II) ainsi obtenus étant sous toutes les formes isomères possibles racémiques, énantiomères et diastéréoisomères.

7. A titre de produits industriels nouveaux, les composés de formule (III).

## Claims

### Claims for the following Contracting States : AT, BE, CH, DE, DK, FR, GB, IT, LI, LU, NL, SE

1. Products of formula ($I_A$):

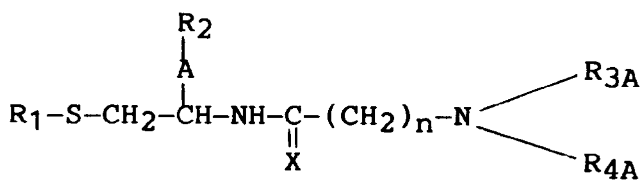

($I_A$)

in which:
n represents the values 0 to 4,
$R_1$ represents a hydrogen atom or an $R_5$ - CO - radical in which $R_5$ represents:
- a linear or branched alkyl, alkenyl or alkynyl radical containing at most 6 carbon atoms and optionally substituted,
- a monocyclic radical containing 5 to 7 members or a radical constituted by condensed rings containing 8 to 10 members, these radicals being saturated or unsaturated, optionally containing one or more identical or different heteroatoms, chosen from oxygen, nitrogen and sulphur atoms and being optionally substituted,
- an

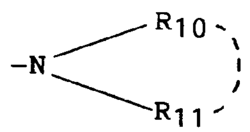

radical

in which $R_{10}$ and $R_{11}$ are such that:

**either** $R_{10}$ and $R_{11}$ form with the nitrogen atom to which they are linked a monocyclic radical containing 5 to 7 members or a radical constituted by condensed rings containing 8 to 10 members, these radicals optionally containing one or more other identical or different heteroatoms, chosen from oxygen, nitrogen and sulphur atoms, and being optionally substituted,

**or** $R_{10}$ and $R_{11}$, identical or different, represent:

- a hydrogen atom,
- a linear or branched alkyl, alkenyl or alkynyl radical containing at most 6 carbon atoms and optionally substituted,
- an aryl radical in which the aryl radical represents a monocyclic radical containing 5 to 7 members or constituted by condensed rings containing 8 to 10 members, these radicals optionally containing one or more identical or different heteroatoms, chosen from oxygen, nitrogen and sulphur atoms, and being optionally substituted,

A represents:

- either a linear or branched alkylene or alkenylene radical containing at most 6 carbon atoms and optionally substituted by a hydroxyl or alkoxy radical containing at most 6 carbon atoms,
- or a single bond,

$R_2$ represents a monocyclic radical containing 5 to 7 members or a radical constituted by condensed rings containing 8 to 10 members, these radicals being saturated or unsaturated, optionally containing one or more identical or different heteroatoms, chosen from oxygen, nitrogen and sulphur atoms and being optionally substituted,

X represents an oxygen or sulphur atom,

$R_{3A}$ and $R_{4A}$ are such that:

**either** $R_{3A}$ and $R_{4A}$ form with the nitrogen atom to which they are linked a monocyclic radical containing 5 to 7 members or a radical constituted by condensed rings containing 8 to 10 members, these radicals optionally containing one or more other identical or different heteroatoms, chosen from oxygen, nitrogen and sulphur atoms, and being optionally substituted,

**or** $R_{3A}$ and $R_{4A}$, identical or different, represent:

- a hydrogen atom,
- one of the following radicals: hydroxyl; alkoxy, acyl or acyloxy containing at most 6 carbon atoms; salified or esterified carboxy; cyano;
- a linear or branched alkyl, alkenyl or alkynyl radical containing at most 6 carbon atoms and optionally substituted;
- an $R_c$ or $R_c$-O- radical in which the $R_c$ radical represents a monocyclic aliphatic or aryl radical containing 5 to 7 members or constituted by condensed rings containing 8 to 10 members, these radicals optionally containing one or more identical or different heteroatoms, chosen from oxygen, nitrogen and sulphur atoms, and being optionally substituted,
- the

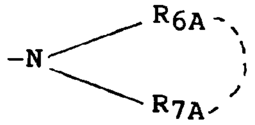 or 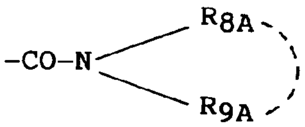

radical

in which $R_{6A}$, $R_{7A}$, $R_{8A}$ and $R_{9A}$, identical or different, represent a hydrogen atom, one or the following radicals: hydroxyl, alkyl, alkoxy, alkyloxy or acyl, these radicals containing at most 6 carbon atoms, free, salified or esterified carboxy, phenoxy, phenyl, benzyl, phenethyl, azepine, piperidyl, morpholine, pyrrolidinyl, piperazinyl optionally substituted on the second nitrogen atom by a hydroxyl, alkyl, alkoxy or phenyl radical, these last three radicals being themselves optionally substituted by one or

more radicals chosen from halogen atoms, hydroxyl, trifluoromethyl, methoxy, ethoxy, methyl and ethyl radicals, or on the one hand $R_{6A}$ and $R_{7A}$ and on the other hand $R_{8A}$ and $R_{9A}$ form respectively with the nitrogen atom to which they are linked, one of the following radicals: imidazolyl, pyrrolyl, pyrrolinyl, pyrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepinyl, indolyl, these radicals being optionally substituted by one or more identical or different radicals chosen from halogen atoms, hydroxyl, trifluoromethyl, alkyl and alkoxy radicals, these radicals containing at most 6 carbon atoms,
the said products of formula ($I_A$) being in all the possible racemic, enantiomeric and diastereoisomeric isomer forms, as well as the addition salts with mineral and organic acids of the said products of formula ($I_A$).

**2.** Products of formula ($I_A$) as defined in claim 1 corresponding to formula (I):

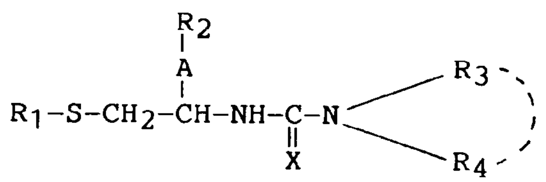

(I)

in which:
$R_1$ represents a hydrogen atom or an $R_5$ - CO - radical in which $R_8$ represents:
- a linear or branched alkyl, alkenyl or alkynyl radical containing at most 6 carbon atoms and optionally substituted,
- a monocyclic radical containing 5 to 7 members or a radical constituted by condensed rings containing 8 to 10 members, these radicals being saturated or unsaturated, optionally containing one or more identical or different heteroatoms, chosen from oxygen, nitrogen and sulphur atoms and being optionally substituted,
- an

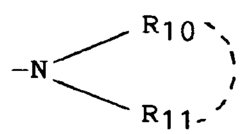

radical

in which $R_{10}$ and $R_{11}$ are such that:
**either** $R_{10}$ and $R_{11}$ form with the nitrogen atom to which they are linked a monocyclic radical containing 5 to 7 members or a radical constituted by condensed rings containing 8 to 10 members, these radicals optionally containing one or more other identical or different heteroatoms, chosen from oxygen, nitrogen and sulphur atoms, and being optionally substituted,
**or** $R_{10}$ and $R_{11}$, identical or different, represent:
- a hydrogen atom,
- a linear or branched alkyl, alkenyl or alkynyl radical containing at most 6 carbon atoms and optionally substituted,
- an aryl radical in which the aryl radical represents a monocyclic radical containing 5 to 7 members or constituted by condensed rings containing 8 to 10 members, these radicals optionally containing one or more identical or different heteroatoms, chosen from oxygen, nitrogen and sulphur atoms, and being optionally substituted,

A represents:
- either a linear or branched alkylene or alkenylene radical containing at most 6 carbon atoms and optionally substituted by a hydroxyl or alkoxy radical containing at most 6 carbon atoms,
- or a single bond,

$R_2$ represents a monocyclic radical containing 5 to 7 members or a radical constituted by condensed rings containing 8 to 10 members, these radicals being saturated or unsaturated, optionally containing one or more identical or different heteroatoms, chosen from oxygen, nitrogen and sulphur atoms and being optionally substituted,
X represents an oxygen or sulphur atom,
$R_3$ and $R_4$ are such that:
**either** $R_3$ and $R_4$ form with the nitrogen atom to which they are linked a monocyclic radical containing 5 to 7 members or a radical constituted by condensed rings containing 8 to 10 members, these radicals optionally containing one or more other identical or different heteroatoms, chosen from oxygen, nitrogen and sulphur atoms, and being optionally substituted,
**or** $R_3$ and $R_4$, identical or different, represent:

- a hydrogen atom,
- one of the following radicals: hydroxyl; alkoxy, acyl or acyloxy containing at most 6 carbon atoms; salified or esterified carboxy; cyano;
- a linear or branched alkyl, alkenyl or alkynyl radical containing at most 6 carbon atoms and optionally substituted,
- an aryl or aryloxy radical in which the aryl radical represents a monocyclic radical containing 5 to 7 members or constituted by condensed rings containing 8 to 10 members, these radicals optionally containing one or more identical or different heteroatoms, chosen from oxygen, nitrogen and sulphur atoms, and being optionally substituted,
- the

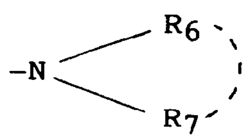

or

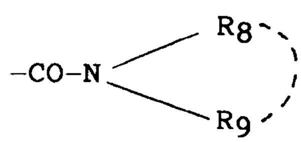

radical
in which $R_6$, $R_7$, $R_8$ and $R_9$, identical or different, represent a hydrogen atom, one of the following radicals: hydroxyl, alkyl, alkoxy, acyloxy or acyl, these radicals containing at most 6 carbon atoms, free, salified or esterified carboxy, phenyl, benzyl, phenethyl, azepine, piperidyl, morpholine, pyrrolidinyl, piperazinyl, optionally substituted on the second nitrogen atom by a hydroxyl, alkyl, alkoxy or phenyl radical, these last three radicals being themselves optionally substituted by one or more radicals chosen from halogen atoms, hydrogen, trifluoromethyl, methoxy, ethoxy, methyl and ethyl radicals,

or on the one hand $R_6$ and $R_7$ and on the other hand $R_8$ and $R_9$ form respectively with the nitrogen atom to which they are linked one of the following radicals: imidazolyl, pyrrolyl, pyrrolinyl, pyrrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepinyl, indolyl, these radicals being optionally substituted by one or more identical or different radicals chosen from halogen atoms, hydroxyl, trifluoromethyl, alkyl and alkoxy radicals, these radicals containing at most 6 carbon atoms,
the said products of formula (I) being in all the possible racemic, enantiomeric and diastereoisomeric isomer forms, as well as the addition salts with mineral and organic acids of the said products of formula (I).

**3.** Products of formulae ($I_A$) and (I) as defined in claims 1 and 2, characterized in that the identical or different substituent or substituents that can be carried by:
a) the alkyl, alkenyl, alkynyl radicals,
b) the monocyclic radicals or radicals constituted by condensed rings, aryl and aryloxy radicals,
are chosen from the group formed by:

- halogen atoms; the following radicals: hydroxyl; cyano; mercapto; nitro; acyl and acyloxy having at most 6 carbon atoms; free, salified or esterified carboxy; alkoxycarbonyl;
- linear and branched alkoxy radicals containing at most 6 carbon atoms;
- the following radicals: alkyl, alkenyl, alkynyl, alkylthio, cycloalkyl, cycloalkenyl, heterocyclic hydrocarbon radicals, aryl, aryloxy, arylthio, phenylalkyl, phenylalkoxy, themselves optionally substituted by one or more identical or different substituents chosen from halogen atoms, the following radicals: hydroxyl, trifluoromethyl, nitro, alkyl, alkenyl, aryl, alkoxy and acyl, these radicals containing at most 6 carbon atoms, free, salified or esterified carboxy, acylamido in which the acyl radical contains at most 6 carbon atoms, carbamoyl and amino, these radicals being optionally substituted on the nitrogen atom by one or two identical or different radicals chosen from hydroxyl radicals, alkyl and alkoxy

radicals containing at most 6 carbon atoms;
- the acylamido radical in which the acyl radical contains at most 6 carbon atoms; the carbamoyl and amino radicals, these radicals being optionally substituted on the nitrogen atom by one or two identical or different radicals chosen from hydroxyl, alkyl, alkenyl and alkoxy radicals containing at most 6 carbon atoms;

the said products of formulae ($I_A$) and (I) being in all the possible racemic, enantiomeric and diastereoisomeric isomer forms, as well as the addition salts with mineral and organic acids of the said products of formulae ($I_A$) and (I).

**4.** Products of formula ($I_A$) as defined in claim 1, corresponding to formula ($I'_A$):

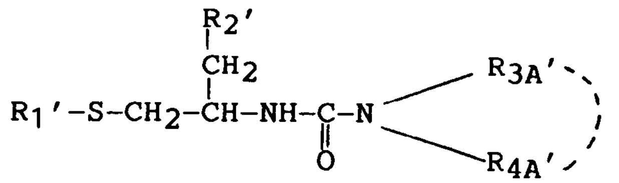

($I'_A$)

in which:

$R_{1A}$' represents a hydrogen atom or an $R_{5A}$'-CO- radical in which $R_{5A}$' represents a phenyl, cycloalkyl, cycloalkenyl, alkyl or alkenyl radical containing at most 6 carbon atoms, these radicals being optionally substituted, or an

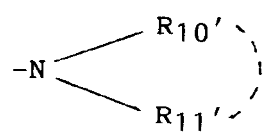

radical

in which $R_{10}$' and $R_{11}$' are such that:

**either** $R_{10}$' and $R_{11}$' form with the nitrogen atom to which they are linked, one of the following radicals: imidazolyl, pyrrolyl, pyrrolinyl, pyrolidinyl, pyridyl, piperidinyl, pyrimidinyl, pyridazinyl, pyrazinyl, piperazinyl, phenylpiperazinyl, methylpiperazinyl, piperidyl, oxazolyl, morpholinyl and thiomorpholinyl, azepine, indolyl, these radicals being optionally substituted by one or more identical or different radicals chosen from halogen atoms, the following radicals: hydroxyl, mercapto, trifluoromethyl, alkyl, alkenyl, alkynyl, alkylthio, alkoxy and phenyl optionally substituted by one or more radicals chosen from halogen atoms and trifluoromethyl, hydroxyl, alkyl and alkoxy radicals, the alkyl and alkoxy radicals containing at most 6 carbon atoms,

**or** $R_{10}$' and $R_{11}$', identical or different, represent:
- a hydrogen atom,
- a linear or branched alkyl, alkenyl or alkynyl radical containing at most 6 carbon atoms and optionally substituted,
- one of the following radicals: phenyl, benzyl, naphthyl, indenyl, imidazolyl, pyrrolyl, pyrrolinyl, pyrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepinyl, indolyl, these radicals being optionally substituted by one or more identical or different radicals chosen from halogen atoms, the following radicals: hydroxyl, mercapto, trifluoromethyl, alkyl, alkenyl, alkynyl, alkylthio and alkoxy containing at most 6 carbon atoms,

$R_2$' represents a phenyl, naphthyl, benzyl, phenethyl, indolyl, cycloalkyl, or cycloalkenyl radical, these radicals being optionally substituted,

$R_{3A}$' and $R_{4A}$' are such that: either $R_{3A}$' and $R_{4A}$' form with the nitrogen atom to which they are linked, one of the following radicals: imidazolyl, pyrrolyl, pyrrolinyl, pyrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepinyl, indolyl, these radicals being optionally substituted by one or more identical or different radicals chosen from halogen atoms, the following radicals: hydroxyl, trifluoromethyl, alkyl, alkenyl, alkynyl, alkoxy and phenyl optionally substituted by one or more radicals chosen from halogen atoms and trifluoromethyl,

hydroxyl, alkyl and alkoxy radicals, the alkyl and alkoxy radicals containing at most 6 carbon atoms and being optionally substituted by a phenyl radical,
or $R_{3A}$' and $R_{4A}$', identical or different, represent:

- a hydrogen atom,
- one of the following radicals: hydroxyl; alkoxy, acyloxy or acyl containing at most 6 carbon atoms; salified or esterified carboxy; cyano;
- a linear or branched alkyl, alkenyl or alkynyl radical containing at most 6 carbon atoms and optionally substituted,
- one of the following radicals: phenyl, benzyl, naphthyl, indenyl, imidazolyl, pyrrolyl, pyrrolinyl, pyrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepinyl, diazepine, benzodiazepine, indolyl, these radicals being optionally substituted by one or more identical or different radicals chosen from halogen atoms, hydroxyl, trifluoromethyl radicals, alkyl and alkoxy radicals containing at most 6 carbon atoms, themselves optionally substituted by one or more radicals chosen from halogen atoms, the following radicals: hydroxyl, alkyl, alkoxy and carboxy, free, salified or esterified by an alkyl, aryl or arylalkyl radical,
- the

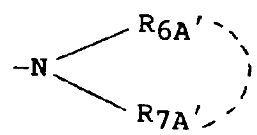 or 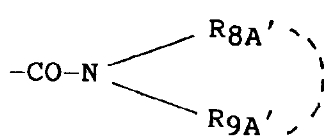

in which $R_{6A}$', $R_{7A}$', $R_{8A}$' and $R_{9A}$', identical or different, represent a hydrogen atom, one of the following radicals: hydroxyl, alkyl, alkoxy, acyloxy or acyl, these radicals containing at most 6 carbon atoms, free, salified or esterified carboxy, phenyl, benzyl, phenethyl, azepinyl, piperidyl, morpholine, pyrrolidinyl, piperazinyl, or on the one hand $R_{6A}$' and $R_{7A}$' and on the other hand $R_{8A}$' and $R_{9A}$' form respectively with the nitrogen atom to which they are linked, one of the following radicals: imidazolyl, pyrrolyl, pyrrolinyl, pyrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepinyl, indolyl, these radicals being optionally substituted by one or more identical or different radicals chosen from halogen atoms, hydroxyl, trifluoromethyl, alkyl and alkoxy radicals, these radicals containing at most 6 carbon atoms, it being understood that the optional identical or different substituent or substituents that can be carried by the radicals that can be represented by $R_1$', $R_2$' and $R_5$' are chosen from the group formed by:

- halogen atoms; the following radicals: hydroxyl; cyano; nitro; acyl and acyloxy having at most 6 carbon atoms; benzoyl; free, salified or esterified carboxy;
- linear and branched alkoxy radicals containing at most 6 carbon atoms;
- the following radicals: alkyl, alkenyl, cyclopentyl, cyclohexyl, cyclopentenyl, cyclohexenyl, phenyl, naphthyl, indolyl, phenoxy, benzyl, phenethyl, benzyloxy, themselves optionally substituted by one or more identical or different substituents chosen from halogen atoms, the following radicals: hydroxyl, trifluoromethyl, nitro, alkyl, alkenyl, alkoxy, these radicals containing at most 6 carbon atoms, formyl, acetyl, benzoyl, free, salified or esterified carboxy, acetamido, benzoylamido, carbamoyl and amino, these radicals being optionally substituted on the nitrogen atom by one or two identical or different radicals chosen from hydroxyl, methyl, ethyl, propyl, methoxy, ethoxy and propoxy radicals;
- the acylamido radical in which the acyl radical contains at most 6 carbon atoms; carbamoyl and amino radicals, these radicals optionally being substituted on the nitrogen atom by one or two identical or different radicals chosen from hydroxyl, alkyl, alkenyl, alkoxy and acyl radicals containing at most 6 carbon atoms; the said products of formula ($I'_A$) being in all the possible racemic, enantiomeric and diastereoisomeric isomer forms, as well as the addition salts with mineral and organic acids of the said products of formula ($I'_A$).

5. Products of formulae ($I_A$) and (I) as defined in claims 1 and 2, corresponding to formula (I'):

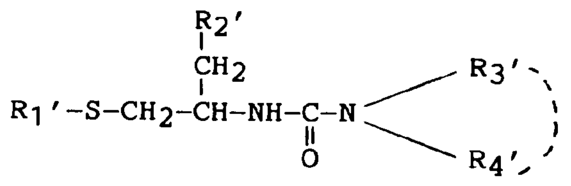

(I')

in which:

$R_1$' represents a hydrogen atom or an $R_5$' - CO - radical in which $R_5$' represents a phenyl, cycloalkyl, cycloalkenyl, alkyl and alkenyl radical containing at most 6 carbon atoms, these radicals being optionally substituted,

or an

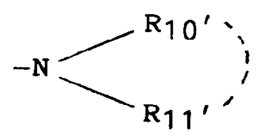

radical

in which $R_{10}$' and $R_{11}$' are such that:

**either** $R_{10}$' and $R_{11}$' form with the nitrogen atom to which they are linked, one of the following radicals: imidazolyl, pyrrolyl, pyrrolinyl, pyrrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepine, indolyl, these radicals being optionally substituted by one or more identical or different radicals chosen from halogen atoms, hydroxyl, trifluoromethyl, alkyl, alkoxy and phenyl radicals optionally substituted by one or more radicals chosen from halogen atoms and trifluoromethyl, hydroxyl, alkyl and alkoxy radicals, the alkyl and alkoxy radicals containing at most 6 carbon atoms,

**or** $R_{10}$' and $R_{11}$', identical or different, represent:

- a hydrogen atom,
- a linear or branched alkyl, alkenyl or alkynyl radical containing at most 6 carbon atoms and optionally substituted,
- one of the following radicals: phenyl, benzoyl, naphthyl, indenyl, imidazolyl, pyrrolyl, pyrrolinyl, pyrrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepine, indolyl, these radicals being optionally substituted by one or more identical or different radicals chosen from halogen atoms, hydroxyl, trifluoromethyl radicals, alkyl and alkoxy radicals containing at most 6 carbon atoms,

$R_2$' represents a phenyl, naphthyl, benzyl, phenethyl, indolyl, cycloalkyl, cycloalkenyl radical, these radicals being optionally substituted,

$R_3$' and $R_4$' are such that:

either $R_3$' and $R_4$' form with the nitrogen atom to which they are linked, one of the following radicals: imidazolyl, pyrrolyl, pyrrolinyl, pyrrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepine, indolyl, these radicals being optionally substituted by one or more identical or different radicals chosen from halogen atoms, hydroxyl, trifluoromethyl, alkyl, alkoxy and phenyl radicals optionally substituted by one or more radicals chosen from halogen atoms and trifluoromethyl, hydroxyl, alkyl and alkoxy radicals, the alkyl and alkoxy radicals containing at most 6 carbon atoms,

or $R_3$' and $R_4$', identical or different, represent:

- a hydrogen atom,
- a hydroxyl radical; an alkoxy, acyloxy or acyl radical containing at most 6 carbon atoms; a salified or esterified carboxy radical; a cyano radical,
- a linear or branched alkyl, alkenyl or alkynyl radical containing at most 6 carbon atoms and optionally substituted,
- one of the following radicals: phenyl, benzoyl, naphthyl, indenyl, imidazolyl, pyrrolyl, pyrrolinyl, pyrrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepine, indolyl, these radicals being optionally substituted by one or more identical or different radicals chosen from halogen atoms, hydroxyl, trifluoromethyl radicals, alkyl and alkoxy radicals containing at most 6 carbon atoms,

- the

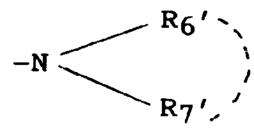 or 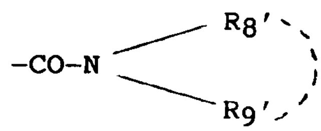

radical
in which $R_6$', $R_7$', $R_8$' and $R_9$', identical or different, represent a hydrogen atom, a hydroxyl, alkyl, alkoxy, acyloxy or acyl radical, these radicals containing at most 6 carbon atoms, a free, salified or esterified carboxy radical, a phenyl, benzyl, phenethyl, azepine, piperidyl, morpholine, pyrrolidinyl, piperazinyl radical,

or on the one hand $R_8$ and $R_7$ and on the other hand $R_8$ and $R_9$ form respectively with the nitrogen atom to which they are linked, one of the following radicals: imidazolyl, pyrrolyl, pyrrolinyl, pyrrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepine, indolyl, these radicals being optionally substituted by one or more identical or different radicals chosen from halogen atoms, hydroxyl, trifluoromethyl, alkyl and alkoxy radicals, these radicals containing at most 6 carbon atoms, it being understood that the optional identical or different substituents that can be carried by the radicals that can be represented by $R_1$', $R_2$' and $R_8$' are chosen from the group formed by:

- halogen atoms; hydroxyl radicals; cyano, nitro radicals; acyl and acyloxy radicals having at most 6 carbon atoms; benzoyl radicals; free, salified or esterified carboxy radicals,
- linear or branched alkoxy radicals containing at most 6 carbon atoms;
- the following radicals: alkyl, alkenyl, cyclopentyl, cyclohexyl, cyclopentenyl, cyclohexenyl, phenyl, naphthyl, indolyl, phenoxy, benzyl, phenethyl, benzyloxy, themselves optionally substituted by one or more identical or different substituents chosen from halogen atoms, the following radicals: hydroxyl, trifluoromethyl, nitro, alkyl, alkenyl, alkoxy, these radicals containing at most 6 carbon atoms, formyl, acetyl, benzoyl, free, salified or esterified carboxy, acetamido, benzoylamido, carbamoyl and amino, these radicals being optionally substituted on the nitrogen atom by one or two identical or different radicals chosen from hydroxyl, methyl, ethyl, propyl, methoxy, ethoxy and propoxy radicals;
- the acylamido radical in which the acyl radical contains at most 6 carbon atoms; carbamoyl and amino radicals, these radicals optionally being substituted on the nitrogen atom by one or two identical or different radicals chosen from hydroxyl, alkyl, alkenyl, alkoxy and acyl radicals containing at most 6 carbon atoms; the said products of formula (I') being in all the possible racemic, enantiomeric and diastereoisomeric isomer forms, as well as the addition salts with mineral and organic acids of the said products of formula (I').

**6.** Products of formula ($I'_A$) as defined in claim 4, characterized in that:
$R_1$' represents a hydrogen atom or an acetyl radical,
$R_2$' represents a phenyl radical,
$R_{3A}$' and $R_{4A}$' are such that:

**either** $R_{3A}$' and $R_{4A}$' form with the nitrogen atom to which they are linked, one of the following radicals: piperidinyl, morpholinyl, dimethylmorpholinyl, thiomorpholinyl, pyrrolidinyl, azepine, diazepine optionally substituted on the second nitrogen atom by an alkyl radical containing 1 or 2 carbon atoms, piperazinyl optionally substituted on the second nitrogen atom either by a phenyl radical optionally substituted by a halogen atom or a trifluoromethyl radical, or by an alkyl, alkenyl or alkynyl radical containing 1 to 4 carbon atoms optionally substituted by a phenyl or acyl radical, or by an acyl radical containing at most 6 carbon atoms,

**or** $R_{3A}$' and $R_{4A}$', identical or different, represent a hydrogen atom; one of the following radicals: hydroxyl; phenyl; phenyl substituted by one of the following radicals: carboxy, piperidinyl optionally substituted on the nitrogen atom by a benzyl radical, piperazinyl optionally substituted on the second nitrogen atom by an alkyl radical containing 1 or 2 carbon atoms, carbamoyl; alkoxy containing at most 4 carbon atoms; alkyl containing at most 4 carbon atoms optionally substituted by one or more identical or different radicals chosen from the following radicals: hydroxyl, alkoxy, free, salified or esterified carboxy, amino, alkylamino, dialkylamino, phenyl, morpholinyl, alkylthio containing at most 4 carbon atoms, optionally substituted by a free, salified or esterified carboxy radical, optionally substituted benzodiazepine, the said products of formula ($I'_A$) being in all the possible racemic, enantiomeric and diastereoisomeric isomer

forms, as well as the addition salts with mineral and organic acids of the said products of formula ($I'_A$).

7. Products of formula (I') as defined in claim 5, characterized in that:
$R_1$' represents a hydrogen atom or an acetyl radical,
$R_2$' represents a phenyl radical,
$R_3$' and $R_4$' are such that:
**either** $R_3$' and $R_4$' form with the nitrogen atom to which they are linked, one of the following radicals: piperidinyl, morpholinyl, dimethylmorpholinyl, thiomorpholinyl, pyrrolidinyl, azepine, piperazinyl optionally substituted on the second nitrogen atom by an alkyl radical containing 1 or 2 carbon atoms or a phenyl radical itself optionally substituted by a halogen atom or a trifluoromethyl radical,
**or** $R_3$' and $R_4$', identical or different, represent a hydrogen atom; one of the following radicals: hydroxyl; phenyl; carbamoyl; alkoxy containing at most 4 carbon atoms; alkyl containing at most 4 carbon atoms optionally substituted by one or more identical or different radicals chosen from hydroxyl, alkoxy, free, salified or esterified carboxy radicals,
the said products of formula (I') being in all the possible racemic, enantiomeric and diastereoisomeric isomer forms, as well as the addition salts with mineral and organic acids of the said products of formula (I').

8. The products of formula ($I_A$) corresponding to the following formulae:
(±) 4-methyl-N-[2-mercapto 1-(phenylmethyl) ethyl] 1-piperazinecarboxamide hydrochloride,
(±) N-[2-mercapto 1-(phenylmethyl) ethyl] 4-morpholinecarboxamide,
(±) N-[2-mercapto 1-(phenylmethyl) ethyl] 1-pyrrolidinecarboxamide,
(±) N-[[[2-mercapto 1-(phenylmethyl) ethyl] amino] carbonyl] glycine,
N,N-bis(2-methoxy ethyl)-N'-[2-mercapto 1-(phenylmethyl) ethyl] urea,
(±) cis-2,6-dimethyl N-[2-mercapto 1-(phenylmethyl) ethyl] 4-morpholinecarboxamide (isomer A),
(±) N-[2-mercapto 1-(phenylmethyl) ethyl] 4-thiomorpholinecarboxamide,
(±) N-[2-mercapto 1-(phenylmethyl) ethyl] N'-methoxy urea,
(±) N-hydroxy N'-[2-mercapto 1-(phenylmethyl) ethyl] N-methyl urea,
(±) N-(1-mercaptomethyl) 2-(phenylethyl) 4-methyl 1-piperazine acetamide,
as well as their salts with mineral or organic acids.

9. Preparation process for products of formula ($I_A$) as defined in claim 1, characterized in that:
- in order to prepare the products of formula ($I_A$) in which n = 0, a compound of formula (II):

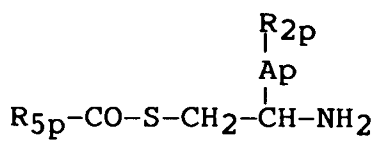

(II)

in which $R_{5p}$, $R_{2p}$ and Ap have the meanings indicated above for $R_5$, $R_2$ and A respectively in which the optional reactive functions are optionally protected by protective groups, is subjected to the action of a reagent such as phosgene or thiophosgene in order to obtain the corresponding isocyanate or isothiocyanate respectively of formula (III):

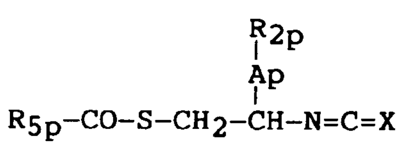

(III)

in which $R_{5p}$, $R_{2p}$ and Ap have the meanings indicated previously and X represents an oxygen atom or a sulphur atom, which is reacted with a compound of formula (IV):

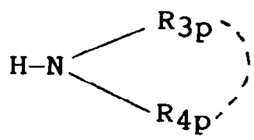

(IV)

in which $R_{3p}$ and $R_{4p}$ have the meanings indicated above for $R_3$ and $R_4$ respectively in which the optional reactive functions are optionally protected by protective groups, in order to obtain a product of formula (V):

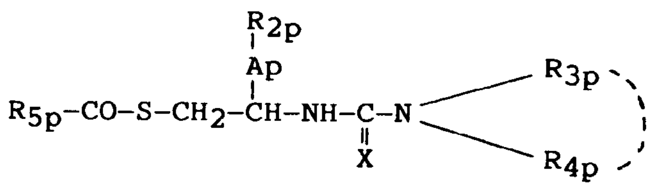

(V)

in which $R_{5p}$, $R_{2p}$, Ap, $R_{3p}$, $R_{4p}$ and X have the meanings indicated previously, which is subjected, if necessary and if desired, to an elimination reaction of the $R_{5p}$-CO- radical in order to obtain a product of formula (VI):

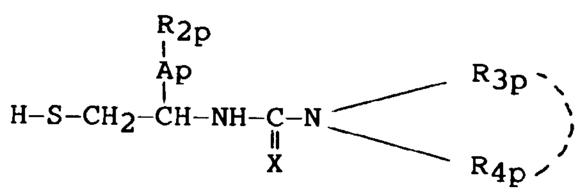

(VI)

in which $R_{2p}$, Ap, $R_{3p}$, $R_{4p}$ and X have the meanings indicated previously,

- in order to prepare the products of formula ($I_A$) in which n is different from 0 and X represents an oxygen atom, a product of formula (II'):

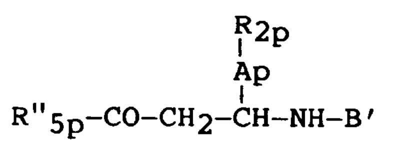

(II')

in which B' represents a hydrogen atom or a protective group of the amine function and R"$_5$ represents an alkyl radical containing 1 to 4 carbon atoms substituted by an

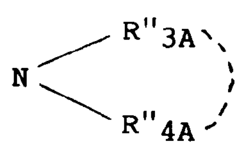

radical in which R"$_{3A}$ and R"$_{4A}$ have the value indicated above for $R_{3A}$ and $R_{4A}$ respectively, is subjected, if appropriate, to the action of an elimination agent of the protective group carried by the amine function, then to the action of an alkaline agent in order to obtain by molecular transposition a product of formula (VII):

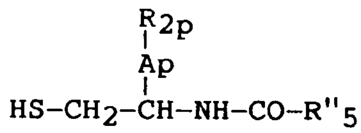

(VII)

in which $R_{2p}$, Ap and $R''_5$ have the meaning indicated above, which products of formulae (V), (VI) and (VII) are treated, if desired and if necessary, with one or more of the following reactions, in any order:

- an elimination reaction of the protective groups that can be carried by the protected reactive functions,
- a salification reaction by a mineral or organic acid in order to obtain the corresponding salt,
- a reduction reaction of the esterified carboxy function into the alcohol function,
- a saponification reaction of the ester function into the acid function,
- a conversion reaction of the cyano function into the acid function,
- a conversion reaction of the alkoxy function into the hydroxyl function,
- an esterification, salification or carbamoylation reaction of the acid function,
- an esterification reaction of the thiol function by an acid or an acid function, or by an isocyanate or by a carbamoyl chloride,

the said products of formula (I) thus obtained being in all the possible racemic, enantiomeric and diastereoisomeric isomer forms.

**10.** As medicaments, the products as defined by formula ($I_A$) of claim 1, the said products of formula ($I_A$) being in all the possible racemic, enantiomeric and diastereoisomeric isomer forms, as well as the addition salts with pharmaceutically acceptable mineral or organic acids of the said products of formula ($I_A$).

**11.** As medicaments, the products of formula ($I_A$) as defined in claims 2 to 8, as well as the addition salts with pharmaceutically acceptable mineral or organic acids of the said products of formula ($I_A$).

**12.** The pharmaceutical compositions containing as active ingredient at least one of the medicaments as defined in any one of claims 11 and 12.

**13.** Preparation process for the starting products of formula (II) as defined in claim 9. characterized in that a compound of formula (IIa):

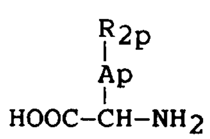

(IIa)

in which $R_{2p}$ and Ap have the meanings indicated in claim 1 for $R_2$ and A respectively in which the optional reactive functions are optionally protected by protective groups, is subjected to the action of a reducing agent of the acid function, in order to obtain the corresponding alcohol of formula (IIb):

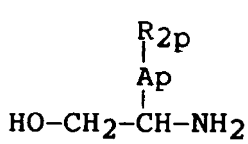

(IIb)

in which $R_{2p}$ and Ap have the meanings indicated previously, which is cyclized by the action of a carbonylated derivative, in order to obtain the corresponding oxazolidone of formula (IIc):

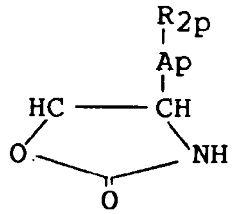

(IIc)

in which $R_{2p}$ and Ap have the meanings indicated previously, which is subjected to the action of a protective agent, in order to obtain a product of formula (IId):

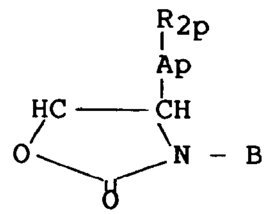

(IId)

in which $R_{2p}$ and Ap have the meanings indicated previously and B represents a protective group of the amine function, which is reacted with a compound of formula (IIe):

$R_{5p}$-CO-SH (IIe)

in which $R_{5p}$ has the meaning indicated above for $R_5$ in which the optional reactive functions are optionally protected by protective groups, in order to obtain a compound of formula (IIf):

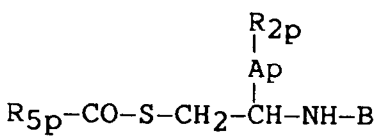

(IIf)

in which $R_{2p}$, $R_{5p}$, Ap and B have the meanings indicated previously, which is subjected, if desired, to an elimination reaction of B, the said products of formula (II) thus obtained being in all the possible racemic, enantiomeric and diastereoisomer isomer forms.

14. As new industrial products, the compounds of formula (III).

**Claims for the following Contracting State : ES**

1. Process for preparing products of formula ($I_A$):

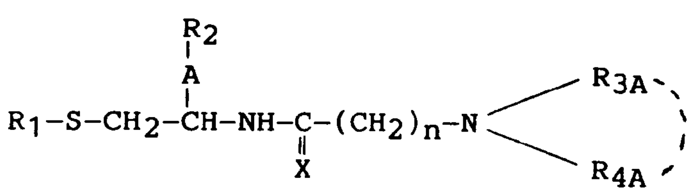

($I_A$)

in which:
n represents the values 0 to 4,
$R_1$ represents a hydrogen atom or an $R_5$ - CO - radical in which $R_5$ represents:
- a linear or branched alkyl, alkenyl or alkynyl radical containing at most 6 carbon atoms and optionally substituted,
- a monocyclic radical containing 5 to 7 members or a radical constituted by condensed rings containing 8 to 10 members, these radicals being saturated or unsaturated, optionally containing one or more identical or different heteroatoms, chosen from oxygen, nitrogen and sulphur atoms and being op-

tionally substituted,
- an

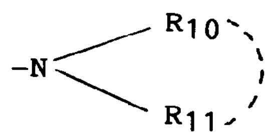

radical
in which $R_{10}$ and $R_{11}$ are such that:
**either** $R_{10}$ and $R_{11}$ form with the nitrogen atom to which they are linked a monocyclic radical containing 5 to 7 members or a radical constituted by condensed rings containing 8 to 10 members, these radicals optionally containing one or more other identical or different heteroatoms, chosen from oxygen, nitrogen and sulphur atoms, and being optionally substituted,
**or** $R_{10}$ and $R_{11}$, identical or different, represent:
- a hydrogen atom,
- a linear or branched alkyl, alkenyl or alkynyl radical containing at most 6 carbon atoms and optionally substituted,
- an aryl radical in which the aryl radical represents a monocyclic radical containing 5 to 7 members or constituted by condensed rings containing 8 to 10 members, these radicals optionally containing one or more identical or different heteroatoms, chosen from oxygen, nitrogen and sulphur atoms, and being optionally substituted,

A represents:
- either a linear or branched alkylene or alkenylene radical containing at most 6 carbon atoms and optionally substituted by a hydroxyl or alkoxy radical containing at most 6 carbon atoms,
- or a single bond,

$R_2$ represents a monocyclic radical containing 5 to 7 members or a radical constituted by condensed rings containing 8 to 10 members, these radicals being saturated or unsaturated, optionally containing one or more identical or different heteroatoms, chosen from oxygen, nitrogen and sulphur atoms and being optionally substituted,
X represents an oxygen or sulphur atom,
$R_{3A}$ and $R_{4A}$ are such that:
**either** $R_{3A}$ and $R_{4A}$ form with the nitrogen atom to which they are linked a monocyclic radical containing 5 to 7 members or a radical constituted by condensed rings containing 8 to 10 members, these radicals optionally containing one or more other identical or different heteroatoms, chosen from oxygen, nitrogen and sulphur atoms, and being optionally substituted,
**or** $R_{3A}$ and $R_{4A}$, identical or different, represent:
- a hydrogen atom,
- one of the following radicals: hydroxyl; alkoxy, acyl or acyloxy containing at most 6 carbon atoms; salified or esterified carboxy; cyano;
- a linear or branched alkyl, alkenyl or alkynyl radical containing at most 6 carbon atoms and optionally substituted;
- an $R_c$ or $R_c$-O- radical in which the $R_c$ radical represents a monocyclic aliphatic or aryl radical containing 5 to 7 members or constituted by condensed rings containing 8 to 10 members, these radicals optionally containing one or more identical or different heteroatoms, chosen from oxygen, nitrogen and sulphur atoms, and being optionally substituted,
- the

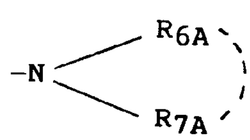
or
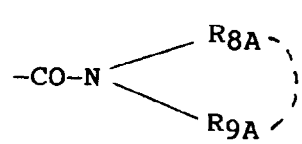

radical
in which $R_{6A}$, $R_{7A}$, $R_{8A}$ and $R_{9A}$, identical or different, represent a hydrogen atom, one or the following radicals: hydroxyl, alkyl, alkoxy, alkyloxy or acyl, these radicals containing at most 6 carbon atoms,

free, salified or esterified carboxy, phenoxy, phenyl, benzyl, phenethyl, azepine, piperidyl, morpholine, pyrrolidinyl, piperazinyl optionally substituted on the second nitrogen atom by a hydroxyl, alkyl, alkoxy or phenyl radical, these last three radicals being themselves optionally substituted by one or more radicals chosen from halogen atoms, hydroxyl, trifluoromethyl, methoxy, ethoxy, methyl and ethyl radicals, or on the one hand $R_{6A}$ and $R_{7A}$ and on the other hand $R_{8A}$ and $R_{9A}$ form respectively with the nitrogen atom to which they are linked, one of the following radicals: imidazolyl, pyrrolyl, pyrrolinyl, pyrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepinyl, indolyl, these radicals being optionally substituted by one or more identical or different radicals chosen from halogen atoms, hydroxyl, trifluoromethyl, alkyl and alkoxy radicals, these radicals containing at most 6 carbon atoms,

the said products of formula ($I_A$) being in all the possible racemic, enantiomeric and diastereoisomeric isomer forms, as well as the addition salts with mineral and organic acids of the said products of formula ($I_A$), characterized in that:

- in order to prepare the products of formula ($I_A$) in which n = 0, a compound of formula (II):

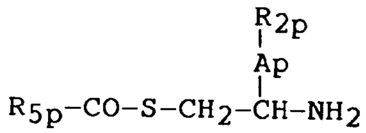

(II)

in which $R_{8p}$, $R_{2p}$ and Ap have the meanings indicated above for $R_8$, $R_2$ and A respectively in which the optional reactive functions are optionally protected by protective groups, is subjected to the action of a reagent such as phosgene or thiophosgene in order to obtain the corresponding isocyanate or isothiocyanate respectively of formula (III):

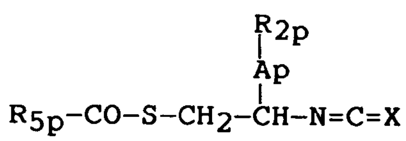

(III)

in which $R_{8p}$, $R_{2p}$ and Ap have the meanings indicated previously and X represents an oxygen atom or a sulphur atom, which is reacted with a compound of formula (IV):

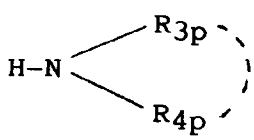

(IV)

in which $R_{3p}$ and $R_{4p}$ have the meanings indicated above for $R_3$ and $R_4$ respectively in which the optional reactive functions are optionally protected by protective groups, in order to obtain a product of formula (V):

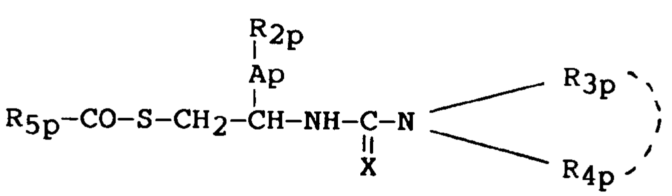

(V)

in which $R_{5p}$, $R_{2p}$, Ap, $R_{3p}$, $R_{4p}$ and X have the meanings indicated previously, which is subjected, if necessary and if desired, to an elimination reaction of the $R_{5p}$-CO- radical in order to obtain a product of formula (VI):

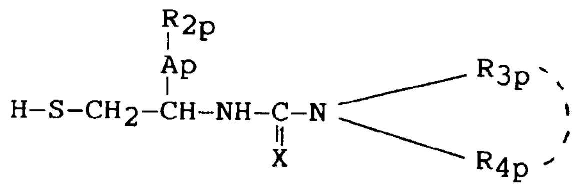

(VI)

in which $R_{2p}$, Ap, $R_{3p}$, $R_{4p}$ and X have the meanings indicated previously,

- in order to prepare the products of formula ($I_A$) in which n is different from 0 and X represents an oxygen atom, a product of formula (II'):

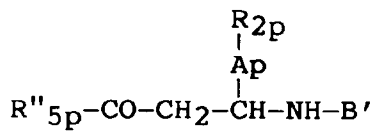

(II')

in which B' represents a hydrogen atom or a protective group of the amine function and R"$_5$ represents an alkyl radical containing 1 to 4 carbon atoms substituted by an

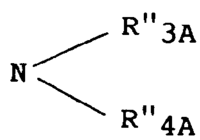

radical in which R"$_{3A}$ and R"$_{4A}$ have the value indicated above for $R_{3A}$ and $R_{4A}$ respectively, is subjected, if appropriate, to the action of an elimination agent of the protective group carried by the amine function, then to the action of an alkaline agent in order to obtain by molecular transposition a product of formula (VII):

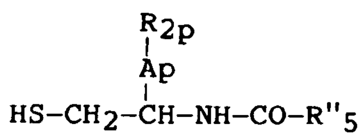

(VII)

in which $R_{2p}$, Ap and R"$_5$ have the meaning indicated above, which products of formulae (V), (VI) and (VII) are treated, if desired and if necessary, with one or more of the following reactions, in any order:

- an elimination reaction of the protective groups that can be carried by the protected reactive functions,
- a salification reaction by a mineral or organic acid in order to obtain the corresponding salt,
- a reduction reaction of the esterified carboxy function into the alcohol function,
- a saponification reaction of the ester function into the acid function,
- a conversion reaction of the cyano function into the acid function,
- a conversion reaction of the alkoxy function into the hydroxyl function,
- an esterification, salification or carbamoylation reaction of the acid function,
- an esterification reaction of the thiol function by an acid or an acid function, or by an isocyanate or by a carbamoyl chloride,

the said products of formula (I) thus obtained being in all the possible racemic, enantiomeric and diastereoisomeric isomer forms.

**2.** Process according to claim 1, characterized in that the identical or different substituent or substituents that can be carried by:

a) the alkyl, alkenyl, alkynyl radicals,

b) the monocyclic radicals or radicals constituted by condensed rings, aryl and aryloxy radicals,

are chosen from the group formed by:

- halogen atoms; the following radicals: hydroxyl; cyano; mercapto; nitro; acyl and acyloxy having at most 6 carbon atoms; free, salified or esterified carboxy; alkoxycarbonyl;
- linear and branched alkoxy radicals containing at most 6 carbon atoms;
- the following radicals: alkyl, alkenyl, alkynyl, alkylthio, cycloalkyl, cycloalkenyl, heterocyclic hydrocarbon radicals, aryl, aryloxy, arylthio, phenylalkyl, phenylalkoxy, themselves optionally substituted by one or more identical or different substituents chosen from halogen atoms, the following radicals: hydroxyl, trifluoromethyl, nitro, alkyl, alkenyl, aryl, alkoxy and acyl, these radicals containing at most 6 carbon atoms, free, salified or esterified carboxy, acylamido in which the acyl radical contains at most 6 carbon atoms, carbamoyl and amino, these radicals being optionally substituted on the nitrogen atom by one or two identical or different radicals chosen from hydroxyl radicals, alkyl and alkoxy radicals containing at most 6 carbon atoms;
- the acylamido radical in which the acyl radical contains at most 6 carbon atoms; the carbamoyl and amino radicals, these radicals being optionally substituted on the nitrogen atom by one or two identical or different radicals chosen from hydroxyl, alkyl, alkenyl and alkoxy radicals containing at most 6 carbon atoms.

**3.** Process according to claim 1 or 2, characterized in that at the start products of formulae (II) and (IV) are used, chosen so as to prepare a product corresponding to formula ($I'_A$):

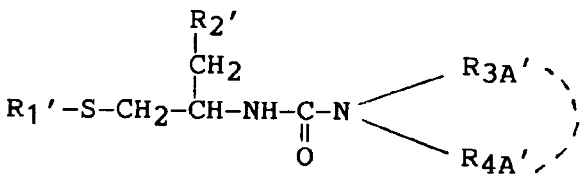

($I'_A$)

in which:

$R_{1A}$' represents a hydrogen atom or an $R_{5A}$'-CO- radical in which $R_{5A}$' represents a phenyl, cycloalkyl, cycloalkenyl, alkyl or alkenyl radical containing at most 6 carbon atoms, these radicals being optionally substituted,
or an

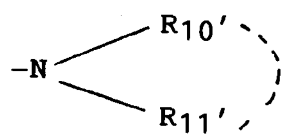

radical
in which $R_{10}$' and $R_{11}$' are such that:
**<u>either</u>** $R_{10}$' and $R_{11}$' form with the nitrogen atom to which they are linked, one of the following radicals: imidazolyl, pyrrolyl, pyrrolinyl, pyrolidinyl, pyridyl, piperidinyl, pyrimidinyl, pyridazinyl, pyrazinyl, piperazinyl, phenylpiperazinyl, methylpiperazinyl, piperidyl, oxazolyl, morpholinyl and thiomorpholinyl, azepine, indolyl, these radicals being optionally substituted by one or more identical or different radicals chosen from halogen atoms, the following radicals: hydroxyl, mercapto, trifluoromethyl, alkyl, alkenyl, alkynyl, alkylthio, alkoxy and phenyl optionally substituted by one or more radicals chosen from halogen atoms and trifluoromethyl, hydroxyl, alkyl and alkoxy radicals, the alkyl and alkoxy radicals containing at most 6 carbon atoms,
**<u>or</u>** $R_{10}$' and $R_{11}$', identical or different, represent:

- a hydrogen atom,
- a linear or branched alkyl, alkenyl or alkynyl radical containing at most 6 carbon atoms and optionally substituted,
- one of the following radicals: phenyl, benzyl, naphthyl, indenyl, imidazolyl, pyrrolyl, pyrrolinyl, pyrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepinyl, indolyl, these radicals being optionally substituted by one or more identical or different radicals chosen from halogen atoms, the following radicals: hydroxyl, mercapto, trifluoromethyl, alkyl, alkenyl, alkynyl, alkylthio and alkoxy containing at most 6

carbon atoms,

$R_2$' represents a phenyl, naphthyl, benzyl, phenethyl, indolyl, cycloalkyl, or cycloalkenyl radical, these radicals being optionally substituted,

$R_{3A}$' and $R_{4A}$' are such that:

either $R_{3A}$' and $R_{4A}$' form with the nitrogen atom to which they are linked, one of the following radicals: imidazolyl, pyrrolyl, pyrrolinyl, pyrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepinyl, indolyl, these radicals being optionally substituted by one or more identical or different radicals chosen from halogen atoms, the following radicals: hydroxyl, trifluoromethyl, alkyl, alkenyl, alkynyl, alkoxy and phenyl optionally substituted by one or more radicals chosen from halogen atoms and trifluoromethyl, hydroxyl, alkyl and alkoxy radicals, the alkyl and alkoxy radicals containing at most 6 carbon atoms and being optionally substituted by a phenyl radical,

or $R_{3A}$' and $R_{4A}$', identical or different, represent:

- a hydrogen atom,
- one of the following radicals: hydroxyl; alkoxy, acyloxy or acyl containing at most 6 carbon atoms; salified or esterified carboxy; cyano;
- a linear or branched alkyl, alkenyl or alkynyl radical containing at most 6 carbon atoms and optionally substituted,
- one of the following radicals: phenyl, benzyl, naphthyl, indenyl, imidazolyl, pyrrolyl, pyrrolinyl, pyrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepinyl, diazepine, benzodiazepine, indolyl, these radicals being optionally substituted by one or more identical or different radicals chosen from halogen atoms, hydroxyl, trifluoromethyl radicals, alkyl and alkoxy radicals containing at most 6 carbon atoms, themselves optionally substituted by one or more radicals chosen from halogen atoms, the following radicals: hydroxyl, alkyl, alkoxy and carboxy, free, salified or esterified by an alkyl, aryl or arylalkyl radical,
- the

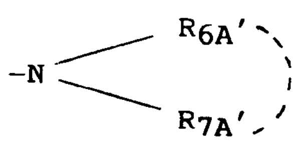

or

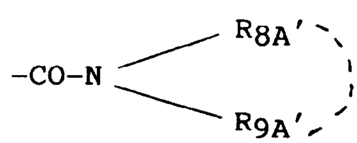

radical

in which $R_{6A}$', $R_{7A}$', $R_{8A}$' and $R_{9A}$', identical or different, represent a hydrogen atom, one of the following radicals: hydroxyl, alkyl, alkoxy, acyloxy or acyl, these radicals containing at most 6 carbon atoms, free, salified or esterified carboxy, phenyl, benzyl, phenethyl, azepinyl, piperidyl, morpholine, pyrrolidinyl, piperazinyl, or on the one hand $R_{6A}$' and $R_{7A}$' and on the other hand $R_{8A}$' and $R_{9A}$' form respectively with the nitrogen atom to which they are linked, one of the following radicals: imidazolyl, pyrrolyl, pyrrolinyl, pyrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepinyl, indolyl, these radicals being optionally substituted by one or more identical or different radicals chosen from halogen atoms, hydroxyl, trifluoromethyl, alkyl and alkoxy radicals, these radicals containing at most 6 carbon atoms,

it being understood that the optional identical or different substituent or substituents that can be carried by the radicals that can be represented by $R_1$', $R_2$' and $R_5$' are chosen from the group formed by:

- halogen atoms; the following radicals: hydroxyl; cyano; nitro; acyl and acyloxy having at most 6 carbon atoms; benzoyl; free, salified or esterified carboxy;
- linear and branched alkoxy radicals containing at most 6 carbon atoms;
- the following radicals: alkyl, alkenyl, cyclopentyl, cyclohexyl, cyclopentenyl, cyclohexenyl, phenyl, naphthyl, indolyl, phenoxy, benzyl, phenethyl, benzyloxy, themselves optionally substituted by one or more identical or different substituents chosen from halogen atoms, the following radicals: hydroxyl, trifluoromethyl, nitro, alkyl, alkenyl, alkoxy, these radicals containing at most 6 carbon atoms, formyl, acetyl, benzoyl, free, salified or esterified carboxy, acetamido, benzoylamido, carbamoyl and amino, these radicals being optionally substituted on the nitrogen atom by one or two identical or different radicals chosen from hydroxyl, methyl, ethyl, propyl, methoxy, ethoxy and propoxy radicals;
- the acylamido radical in which the acyl radical contains at most 6 carbon atoms; carbamoyl and amino

radicals, these radicals optionally being substituted on the nitrogen atom by one or two identical or different radicals chosen from hydroxyl, alkyl, alkenyl, alkoxy and acyl radicals containing at most 6 carbon atoms; the said products of formula ($I'_A$) being in all the possible racemic, enantiomeric and diastereoisomeric isomer forms, as well as the addition salts with mineral and organic acids of the said products of formula ($I'_A$).

**4.** Process according to any one of claims 1 to 3, characterized in that at the start a product of formula (II) is used in which $R_{5p}$ represents a methyl radical and $R_{2p}$ represents a phenyl radical and a product of formula (IV) is used in which $R_{3p}$ and $R_{4p}$ are such that

**either** $R_{3p}$ and $R_{4p}$ form with the nitrogen atom to which they are linked, one of the following radicals: piperidinyl, morpholinyl, dimethylmorpholinyl, thiomorpholinyl, pyrrolidinyl, azepine, diazepine optionally substituted on the second nitrogen atom by an alkyl radical containing 1 or 2 carbon atoms, piperazinyl optionally substituted on the second nitrogen atom either by a phenyl radical optionally substituted by a halogen atom or a trifluoromethyl radical, or by an alkyl, alkenyl or alkynyl radical containing 1 to 4 carbon atoms optionally substituted by a phenyl or acyl radical, or by an acyl radical containing at most 6 carbon atoms,

**or** $R_{3p}$ and $R_{4p}$, identical or different, represent a hydrogen atom; one of the following radicals: hydroxyl; phenyl; phenyl substituted by a carboxy radical, piperidinyl optionally substituted on the nitrogen atom by a benzyl radical, piperazinyl optionally substituted on the second nitrogen atom by an alkyl radical containing 1 or 2 carbon atoms, carbamoyl; alkoxy containing at most 4 carbon atoms; alkyl containing at most 4 carbon atoms optionally substituted by one or more identical or different radicals chosen from the following radicals: hydroxyl, alkoxy, free, salified or esterified carboxy, amino, alkylamino, dialkylamino, phenyl, morpholinyl, alkylthio containing at most 4 carbon atoms, optionally substituted by a free, salified or esterified carboxy radical, optionally substituted benzodiazepine, and in which the reactive functions are optionally protected.

**5.** Process according to any one of claims 1 to 4, characterized in that at the start products of formulae (II) and (IV) are used which are chosen so as to prepare a product corresponding to the following formulae:
(±) 4-methyl-N-[2-mercapto 1-(phenylmethyl) ethyl] 1-piperazinecarboxamide hydrochloride,
(±) N-[2-mercapto 1-(phenylmethyl) ethyl] 4-morpholinecarboxamide,
(±) N-[2-mercapto 1-(phenylmethyl) ethyl] 1-pyrrolidinecarboxamide,
(±) N-[[[2-mercapto 1-(phenylmethyl) ethyl] amino] carbonyl] glycine,
N,N-bis(2-methoxy ethyl)-N'-[2-mercapto 1-(phenylmethyl) ethyl] urea,
(±) cis-2,6-dimethyl N-[2-mercapto 1-(phenylmethyl) ethyl] 4-morpholinecarboxamide (isomer A),
(±) N-[2-mercapto 1-(phenylmethyl) ethyl] 4-thiomorpholinecarboxamide,
(±) N-[2-mercapto 1-(phenylmethyl) ethyl] N'-methoxy urea,
(±) N-hydroxy N'-[2-mercapto 1-(phenylmethyl) ethyl] N-methyl urea,
(±) N-(1-mercaptomethyl) 2-(phenylethyl) 4-methyl 1-piperazine acetamide,
as well as their salts with mineral or organic acids.

**6.** Preparation process for the starting products of formula (II) as defined in claim 1, characterized in that a compound of formula (IIa):

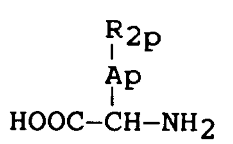

(IIa)

in which $R_{2p}$ and Ap have the meanings indicated in claim 1 for $R_2$ and A respectively in which the optional reactive functions are optionally protected by protective groups, is subjected to the action of a reducing agent of the acid function, in order to obtain the corresponding alcohol of formula (IIb):

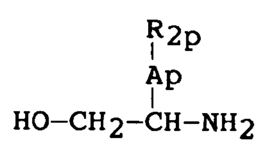

(IIb)

in which $R_{2p}$ and Ap have the meanings indicated previously, which is cyclized by the action of a carbonylated derivative, in order to obtain the corresponding oxazolidone of formula (IIc):

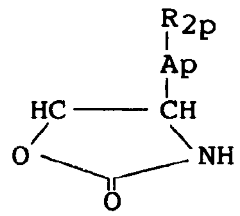

(IIc)

in which $R_{2p}$ and Ap have the meanings indicated previously, which is subjected to the action of a protective agent, in order to obtain a product of formula (IId):

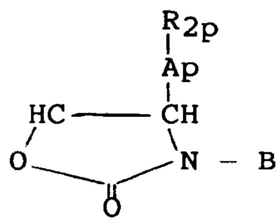

(IId)

in which $R_{2p}$ and Ap have the meanings indicated previously and B represents a protective group of the amine function, which is reacted with a compound of formula (IIe):

$R_{5p}$-CO-SH (IIe)

in which $R_{5p}$ has the meaning indicated above for $R_5$ in which the optional reactive functions are optionally protected by protective groups, in order to obtain a compound of formula (IIf):

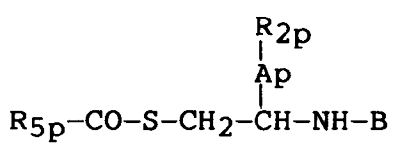

(IIf)

in which $R_{2p}$, $R_{5p}$, Ap and B have the meanings indicated previously, which is subjected, if desired, to an elimination reaction of B, the said products of formula (II) thus obtained being in all the possible racemic, enantiomeric and diastereoisomer isomer forms.

**Claims for the following Contracting State : GR**

**1.** Process for preparing products of formula ($I_A$):

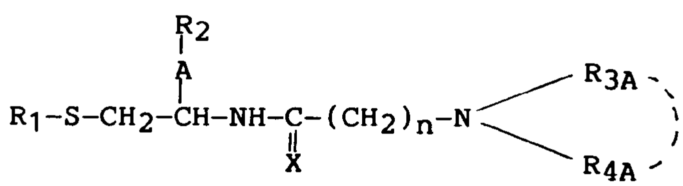

($I_A$)

in which:

n represents the values 0 to 4,

$R_1$ represents a hydrogen atom or an $R_5$ - CO - radical in which $R_5$ represents:

- a linear or branched alkyl, alkenyl or alkynyl radical containing at most 6 carbon atoms and optionally substituted,

- a monocyclic radical containing 5 to 7 members or a radical constituted by condensed rings containing 8 to 10 members, these radicals being saturated or unsaturated, optionally containing one or more identical or different heteroatoms, chosen from oxygen, nitrogen and sulphur atoms and being optionally substituted,
- an

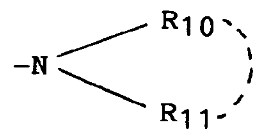

radical
in which $R_{10}$ and $R_{11}$ are such that:

**either** $R_{10}$ and $R_{11}$ form with the nitrogen atom to which they are linked a monocyclic radical containing 5 to 7 members or a radical constituted by condensed rings containing 8 to 10 members, these radicals optionally containing one or more other identical or different heteroatoms, chosen from oxygen, nitrogen and sulphur atoms, and being optionally substituted,

**or** $R_{10}$ and $R_{11}$, identical or different, represent:

- a hydrogen atom,
- a linear or branched alkyl, alkenyl or alkynyl radical containing at most 6 carbon atoms and optionally substituted,
- an aryl radical in which the aryl radical represents a monocyclic radical containing 5 to 7 members or constituted by condensed rings containing 8 to 10 members, these radicals optionally containing one or more identical or different heteroatoms, chosen from oxygen, nitrogen and sulphur atoms, and being optionally substituted,

A represents:

- either a linear or branched alkylene or alkenylene radical containing at most 6 carbon atoms and optionally substituted by a hydroxyl or alkoxy radical containing at most 6 carbon atoms,
- or a single bond,

$R_2$ represents a monocyclic radical containing 5 to 7 members or a radical constituted by condensed rings containing 8 to 10 members, these radicals being saturated or unsaturated, optionally containing one or more identical or different heteroatoms, chosen from oxygen, nitrogen and sulphur atoms and being optionally substituted,

X represents an oxygen or sulphur atom,

$R_{3A}$ and $R_{4A}$ are such that:

**either** $R_{3A}$ and $R_{4A}$ form with the nitrogen atom to which they are linked a monocyclic radical containing 5 to 7 members or a radical constituted by condensed rings containing 8 to 10 members, these radicals optionally containing one or more other identical or different heteroatoms, chosen from oxygen, nitrogen and sulphur atoms, and being optionally substituted,

**or** $R_{3A}$ and $R_{4A}$, identical or different, represent:

- a hydrogen atom,
- one of the following radicals: hydroxyl; alkoxy, acyl or acyloxy containing at most 6 carbon atoms; salified or esterified carboxy; cyano;
- a linear or branched alkyl, alkenyl or alkynyl radical containing at most 6 carbon atoms and optionally substituted;
- an $R_c$ or $R_c$-O- radical in which the $R_c$ radical represents a monocyclic aliphatic or aryl radical containing 5 to 7 members or constituted by condensed rings containing 8 to 10 members, these radicals optionally containing one or more identical or different heteroatoms, chosen from oxygen, nitrogen and sulphur atoms, and being optionally substituted,
- the

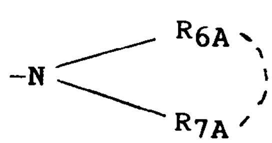

or

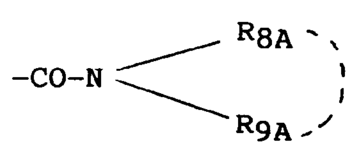

in which $R_{6A}$, $R_{7A}$, $R_{8A}$ and $R_{9A}$, identical or different, represent a hydrogen atom, one or the following radicals: hydroxyl, alkyl, alkoxy, alkyloxy or acyl, these radicals containing at most 6 carbon atoms, free, salified or esterified carboxy, phenoxy, phenyl, benzyl, phenethyl, azepine, piperidyl, morpholine, pyrrolidinyl, piperazinyl optionally substituted on the second nitrogen atom by a hydroxyl, alkyl, alkoxy or phenyl radical, these last three radicals being themselves optionally substituted by one or more radicals chosen from halogen atoms, hydroxyl, trifluoromethyl, methoxy, ethoxy, methyl and ethyl radicals, or on the one hand $R_{6A}$ and $R_{7A}$ and on the other hand $R_{8A}$ and $R_{9A}$ form respectively with the nitrogen atom to which they are linked, one of the following radicals: imidazolyl, pyrrolyl, pyrrolinyl, pyrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepinyl, indolyl, these radicals being optionally substituted by one or more identical or different radicals chosen from halogen atoms, hydroxyl, trifluoromethyl, alkyl and alkoxy radicals, these radicals containing at most 6 carbon atoms,

the said products of formula ($I_A$) being in all the possible racemic, enantiomeric and diastereoisomeric isomer forms, as well as the addition salts with mineral and organic acids of the said products of formula ($I_A$), characterized in that:

- in order to prepare the products of formula ($I_A$) in which n = 0, a compound of formula (II):

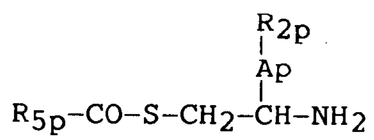

(II)

in which $R_{8p}$, $R_{2p}$ and Ap have the meanings indicated above for $R_8$, $R_2$ and A respectively in which the optional reactive functions are optionally protected by protective groups, is subjected to the action of a reagent such as phosgene or thiophosgene in order to obtain the corresponding isocyanate or isothiocyanate respectively of formula (III):

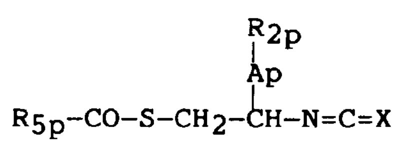

(III)

in which $R_{8p}$, $R_{2p}$ and Ap have the meanings indicated previously and X represents an oxygen atom or a sulphur atom, which is reacted with a compound of formula (IV):

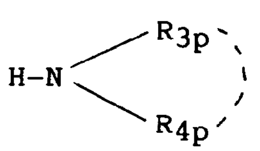

(IV)

in which $R_{3p}$ and $R_{4p}$ have the meanings indicated above for $R_3$ and $R_4$ respectively in which the optional reactive functions are optionally protected by protective groups, in order to obtain a product of formula (V):

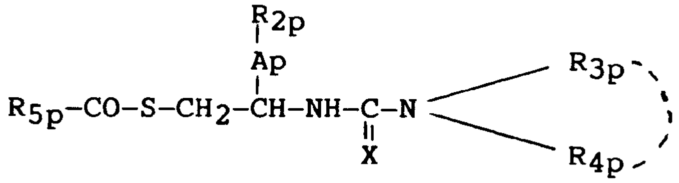

(V)

in which $R_{5p}$, $R_{2p}$, Ap, $R_{3p}$, $R_{4p}$ and X have the meanings indicated previously, which is subjected, if necessary and if desired, to an elimination reaction of the $R_{5p}$-CO- radical in order to obtain a product

of formula (VI):

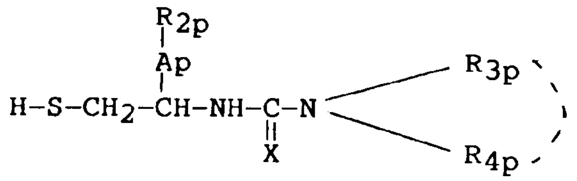

(VI)

in which $R_{2p}$, Ap, $R_{3p}$, $R_{4p}$ and X have the meanings indicated previously,

- in order to prepare the products of formula ($I_A$) in which n is different from 0 and X represents an oxygen atom, a product of formula (II'):

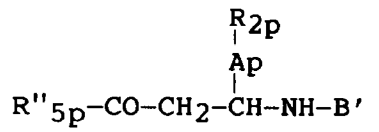

(II')

in which B' represents a hydrogen atom or a protective group of the amine function and $R"_5$ represents an alkyl radical containing 1 to 4 carbon atoms substituted by an

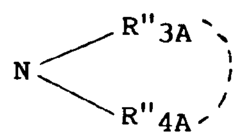

radical in which $R"_{3A}$ and $R"_{4A}$ have the value indicated above for $R_{3A}$ and $R_{4A}$ respectively, is subjected, if appropriate, to the action of an elimination agent of the protective group carried by the amine function, then to the action of an alkaline agent in order to obtain by molecular transposition a product of formula (VII):

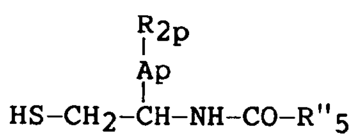

(VII)

in which $R_{2p}$, Ap and $R"_5$ have the meaning indicated above, which products of formulae (V), (VI) and (VII) are treated, if desired and if necessary, with one or more of the following reactions, in any order:

- an elimination reaction of the protective groups that can be carried by the protected reactive functions,
- a salification reaction by a mineral or organic acid in order to obtain the corresponding salt,
- a reduction reaction of the esterified carboxy function into the alcohol function,
- a saponification reaction of the ester function into the acid function,
- a conversion reaction of the cyano function into the acid function,
- a conversion reaction of the alkoxy function into the hydroxyl function,
- an esterification, salification or carbamoylation reaction of the acid function,
- an esterification reaction of the thiol function by an acid or an acid function, or by an isocyanate or by a carbamoyl chloride, the said products of formula (I) thus obtained being in all the possible racemic, enantiomeric and diastereoisomeric isomer forms.

**2.** Process according to claim 1, characterized in that the identical or different substituent or substituents that can be carried by:

a) the alkyl, alkenyl, alkynyl radicals,

b) the monocyclic radicals or radicals constituted by condensed rings, aryl and aryloxy radicals,

are chosen from the group formed by:

- halogen atoms; the following radicals: hydroxyl; cyano; mercapto; nitro; acyl and acyloxy having at most 6 carbon atoms; free, salified or esterified carboxy; alkoxycarbonyl;
- linear and branched alkoxy radicals containing at most 6 carbon atoms;
- the following radicals: alkyl, alkenyl, alkynyl, alkylthio, cycloalkyl, cycloalkenyl, heterocyclic hydrocarbon radicals, aryl, aryloxy, arylthio, phenylalkyl, phenylalkoxy, themselves optionally substituted by one or more identical or different substituents chosen from halogen atoms, the following radicals: hydroxyl, trifluoromethyl, nitro, alkyl, alkenyl, aryl, alkoxy and acyl, these radicals containing at most 6 carbon atoms, free, salified or esterified carboxy, acylamido in which the acyl radical contains at most 6 carbon atoms, carbamoyl and amino, these radicals being optionally substituted on the nitrogen atom by one or two identical or different radicals chosen from hydroxyl radicals, alkyl and alkoxy radicals containing at most 6 carbon atoms;
- the acylamido radical in which the acyl radical contains at most 6 carbon atoms; the carbamoyl and amino radicals, these radicals being optionally substituted on the nitrogen atom by one or two identical or different radicals chosen from hydroxyl, alkyl, alkenyl and alkoxy radicals containing at most 6 carbon atoms.

3. Process according to claim 1 or 2, characterized in that at the start products of formulae (II) and (IV) are used, chosen so as to prepare a product corresponding to formula ($I'_A$):

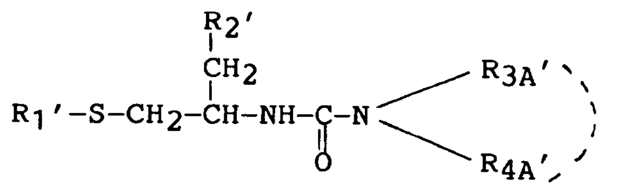

($I'_A$)

in which:
$R_{1A}$' represents a hydrogen atom or an $R_{5A}$'-CO- radical in which $R_{5A}$' represents a phenyl, cycloalkyl, cycloalkenyl, alkyl or alkenyl radical containing at most 6 carbon atoms, these radicals being optionally substituted, or an

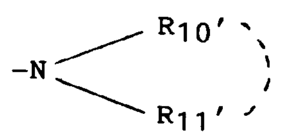

radical
in which $R_{10}$' and $R_{11}$' are such that:
**<u>either</u>** $R_{10}$' and $R_{11}$' form with the nitrogen atom to which they are linked, one of the following radicals: imidazolyl, pyrrolyl, pyrrolinyl, pyrolidinyl, pyridyl, piperidinyl, pyrimidinyl, pyridazinyl, pyrazinyl, piperazinyl, phenylpiperazinyl, methylpiperazinyl, piperidyl, oxazolyl, morpholinyl and thiomorpholinyl, azepinyl, indolyl, these radicals being optionally substituted by one or more identical or different radicals chosen from halogen atoms, the following radicals: hydroxyl, mercapto, trifluoromethyl, alkyl, alkenyl, alkynyl, alkylthio, alkoxy and phenyl optionally substituted by one or more radicals chosen from halogen atoms and trifluoromethyl, hydroxyl, alkyl and alkoxy radicals, the alkyl and alkoxy radicals containing at most 6 carbon atoms,
**<u>or</u>** $R_{10}$' and $R_{11}$', identical or different, represent:

- a hydrogen atom,
- a linear or branched alkyl, alkenyl or alkynyl radical containing at most 6 carbon atoms and optionally substituted,
- one of the following radicals: phenyl, benzyl, naphthyl, indenyl, imidazolyl, pyrrolyl, pyrrolinyl, pyrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepinyl, indolyl, these radicals being optionally substituted by one or more identical or different radicals chosen from halogen atoms, the following radicals: hydroxyl, mercapto, trifluoromethyl, alkyl, alkenyl, alkynyl, alkylthio and alkoxy containing at most 6

carbon atoms,

$R_2$' represents a phenyl, naphthyl, benzyl, phenethyl, indolyl, cycloalkyl, or cycloalkenyl radical, these radicals being optionally substituted,

$R_{3A}$' and $R_{4A}$' are such that:

either $R_{3A}$' and $R_{4A}$' form with the nitrogen atom to which they are linked, one of the following radicals: imidazolyl, pyrrolyl, pyrrolinyl, pyrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepinyl, indolyl, these radicals being optionally substituted by one or more identical or different radicals chosen from halogen atoms, the following radicals: hydroxyl, trifluoromethyl, alkyl, alkenyl, alkynyl, alkoxy and phenyl optionally substituted by one or more radicals chosen from halogen atoms and trifluoromethyl, hydroxyl, alkyl and alkoxy radicals, the alkyl and alkoxy radicals containing at most 6 carbon atoms and being optionally substituted by a phenyl radical,

or $R_{3A}$' and $R_{4A}$', identical or different, represent:

- a hydrogen atom,
- one of the following radicals: hydroxyl; alkoxy, acyloxy or acyl containing at most 6 carbon atoms; salified or esterified carboxy; cyano;
- a linear or branched alkyl, alkenyl or alkynyl radical containing at most 6 carbon atoms and optionally substituted,
- one of the following radicals: phenyl, benzyl, naphthyl, indenyl, imidazolyl, pyrrolyl, pyrrolinyl, pyrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepinyl, diazepine, benzodiazepine, indolyl, these radicals being optionally substituted by one or more identical or different radicals chosen from halogen atoms, hydroxyl, trifluoromethyl radicals, alkyl and alkoxy radicals containing at most 6 carbon atoms, themselves optionally substituted by one or more radicals chosen from halogen atoms, the following radicals: hydroxyl, alkyl, alkoxy and carboxy, free, salified or esterified by an alkyl, aryl or arylalkyl radical,
- the

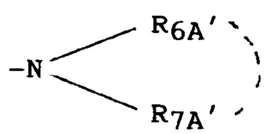 or 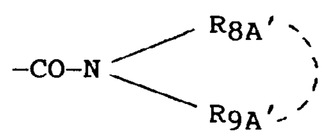

radical

in which $R_{6A}$', $R_{7A}$', $R_{8A}$' and $R_{9A}$', identical or different, represent a hydrogen atom, one of the following radicals: hydroxyl, alkyl, alkoxy, acyloxy or acyl, these radicals containing at most 6 carbon atoms, free, salified or esterified carboxy, phenyl, benzyl, phenethyl, azepinyl, piperidyl, morpholine, pyrrolidinyl, piperazinyl, or on the one hand $R_{6A}$' and $R_{7A}$' and on the other hand $R_{8A}$' and $R_{9A}$' form respectively with the nitrogen atom to which they are linked, one of the following radicals: imidazolyl, pyrrolyl, pyrrolinyl, pyrolidinyl, pyridyl, piperidinyl, pyrimidinyl, pyridazinyl, pyrazinyl, piperazinyl, phenylpiperazinyl, piperidyl, oxazolyl, morpholinyl and thiomorpholinyl, azepinyl, indolyl, these radicals being optionally substituted by one or more identical or different radicals chosen from halogen atoms, hydroxyl, trifluoromethyl, alkyl and alkoxy radicals, these radicals containing at most 6 carbon atoms, it being understood that the optional identical or different substituent or substituents that can be carried by the radicals that can be represented by $R_1$', $R_2$' and $R_5$' are chosen from the group formed by:

- halogen atoms; the following radicals: hydroxyl; cyano; nitro; acyl and acyloxy having at most 6 carbon atoms; benzoyl; free, salified or esterified carboxy;
- linear and branched alkoxy radicals containing at most 6 carbon atoms;
- the following radicals: alkyl, alkenyl, cyclopentyl, cyclohexyl, cyclopentenyl, cyclohexenyl, phenyl, naphthyl, indolyl, phenoxy, benzyl, phenethyl, benzyloxy, themselves optionally substituted by one or more identical or different substituents chosen from halogen atoms, the following radicals: hydroxyl, trifluoromethyl, nitro, alkyl, alkenyl, alkoxy, these radicals containing at most 6 carbon atoms, formyl, acetyl, benzoyl, free, salified or esterified carboxy, acetamido, benzoylamido, carbamoyl and amino, these radicals being optionally substituted on the nitrogen atom by one or two identical or different radicals chosen from hydroxyl, methyl, ethyl, propyl, methoxy, ethoxy and propoxy radicals;
- the acylamido radical in which the acyl radical contains at most 6 carbon atoms; carbamoyl and amino

radicals, these radicals optionally being substituted on the nitrogen atom by one or two identical or different radicals chosen from hydroxyl, alkyl, alkenyl, alkoxy and acyl radicals containing at most 6 carbon atoms; the said products of formula ($I'_A$) being in all the possible racemic, enantiomeric and diastereoisomeric isomer forms, as well as the addition salts with mineral and organic acids of the said products of formula ($I'_A$).

**4.** Process according to any one of claims 1 to 3, characterized in that at the start a product of formula (II) is used in which $R_{5p}$ represents a methyl radical and $R_{2p}$ represents a phenyl radical and a product of formula (IV) is used in which $R_{3p}$ and $R_{4p}$ are such that

**either** $R_{3p}$ and $R_{4p}$ form with the nitrogen atom to which they are linked, one of the following radicals: piperidinyl, morpholinyl, dimethylmorpholinyl, thiomorpholinyl, pyrrolidinyl, azepine, diazepine optionally substituted on the second nitrogen atom by an alkyl radical containing 1 or 2 carbon atoms, piperazinyl optionally substituted on the second nitrogen atom either by a phenyl radical optionally substituted by a halogen atom or a trifluoromethyl radical, or by an alkyl, alkenyl or alkynyl radical containing 1 to 4 carbon atoms optionally substituted by a phenyl or acyl radical, or by an acyl radical containing at most 6 carbon atoms,

**or** $R_{3p}$ and $R_{4p}$, identical or different, represent a hydrogen atom; one of the following radicals: hydroxyl; phenyl; phenyl substituted by a carboxy radical, piperidinyl optionally substituted on the nitrogen atom by a benzyl radical, piperazinyl optionally substituted on the second nitrogen atom by an alkyl radical containing 1 or 2 carbon atoms, carbamoyl; alkoxy containing at most 4 carbon atoms; alkyl containing at most 4 carbon atoms optionally substituted by one or more identical or different radicals chosen from the following radicals: hydroxyl, alkoxy, free, salified or esterified carboxy, amino, alkylamino, dialkylamino, phenyl, morpholinyl, alkylthio containing at most 4 carbon atoms, optionally substituted by a free, salified or esterified carboxy radical, optionally substituted benzodiazepine, and in which the reactive functions are optionally protected.

**5.** Process according to any one of claims 1 to 4, characterized in that at the start products of formulae (II) and (IV) are used which are chosen so as to prepare a product corresponding to the following formulae:
(±) 4-methyl-N-[2-mercapto 1-(phenylmethyl) ethyl] 1-piperazinecarboxamide hydrochloride,
(±) N-[2-mercapto 1-(phenylmethyl) ethyl] 4-morpholinecarboxamide,
(±) N-[2-mercapto 1-(phenylmethyl) ethyl] 1-pyrrolidinecarboxamide,
(±) N-[[[2-mercapto 1-(phenylmethyl) ethyl] amino] carbonyl] glycine,
N,N-bis(2-methoxy ethyl)-N'-[2-mercapto 1-(phenylmethyl) ethyl] urea,
(±) cis-2,6-dimethyl N-[2-mercapto 1-(phenylmethyl) ethyl] 4-morpholinecarboxamide (isomer A),
(±) N-[2-mercapto 1-(phenylmethyl) ethyl] 4-thiomorpholinecarboxamide,
(±) N-[2-mercapto 1-(phenylmethyl) ethyl] N'-methoxy urea,
(±) N-hydroxy N'-[2-mercapto 1-(phenylmethyl) ethyl] N-methyl urea,
(±) N-(1-mercaptomethyl) 2-(phenylethyl) 4-methyl 1-piperazine acetamide,
as well as their salts with mineral or organic acids.

**6.** Preparation process for the starting products of formula (II) as defined in claim 1, characterized in that a compound of formula (IIa):

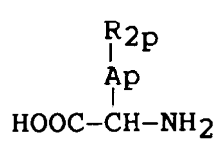

(IIa)

in which $R_{2p}$ and Ap have the meanings indicated in claim 1 for $R_2$ and A respectively in which the optional reactive functions are optionally protected by protective groups, is subjected to the action of a reducing agent of the acid function, in order to obtain the corresponding alcohol of formula (IIb):

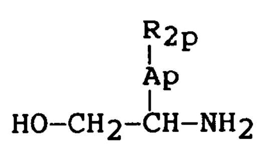

(IIb)

in which $R_{2p}$ and Ap have the meanings indicated previously, which is cyclized by the action of a carbonylated derivative, in order to obtain the corresponding oxazolidone of formula (IIc):

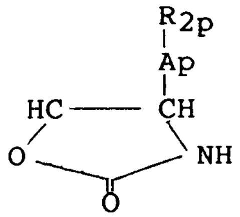

(IIc)

in which $R_{2p}$ and Ap have the meanings indicated previously, which is subjected to the action of a protective agent, in order to obtain a product of formula (IId):

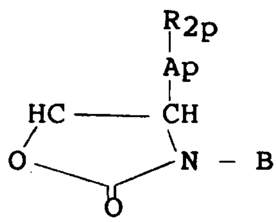

(IId)

in which $R_{2p}$ and Ap have the meanings indicated previously and B represents a protective group of the amine function, which is reacted with a compound of formula (IIe):

$R_{5p}$-CO-SH (IIe)

in which $R_{5p}$ has the meaning indicated above for $R_5$ in which the optional reactive functions are optionally protected by protective groups, in order to obtain a compound of formula (IIf):

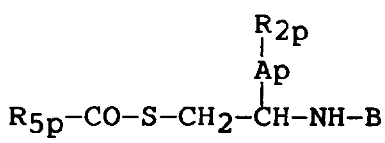

(IIf)

in which $R_{2p}$, $R_{5p}$, Ap and B have the meanings indicated previously, which is subjected, if desired, to an elimination reaction of B, the said products of formula (II) thus obtained being in all the possible racemic, enantiomeric and diastereoisomer isomer forms.

**7.** As new industrial products, the compounds of formula (III).

## Patentansprüche

### Patenstansprüche für folgende Vertragsstaaten : AT, BE, CH, DE, DK, FR, GB, IT, LI, LU, NL, SE

**1.** Produkte der Formel ($I_A$)

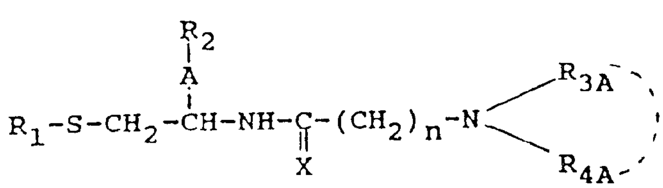

($I_A$)

in der

n die Werte 0 bis 4 besitzt,

$R_1$ ein Wasserstoffatom oder einen Rest $R_5$-CO- darstellt, worin $R_5$ bedeutet:

- einen linearen oder verzweigten Alkyl-, Alkenyl- oder Alkinylrest mit höchstens 6 Kohlenstoffatomen und gegebenenfalls substituiert,
- einen monocyclischen Rest mit 5 bis 7 Ringgliedern oder einen Rest, der aus kondensierten Ringen gebildet wird, die 8 bis 10 Ringglieder umfassen, wobei diese Reste gesättigt oder ungesättigt sind und gegebenenfalls ein oder mehrere, gleiche oder verschiedene Heteroatome umfassen, ausgewählt unter den Atomen von Sauerstoff, Stickstoff und Schwefel, und die gegebenenfalls substituiert sind,
- einen Rest

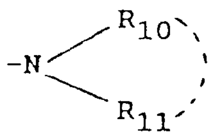

worin $R_{10}$ und $R_{11}$ bedeuten:

entweder bilden $R_{10}$ und $R_{11}$ mit dem Stickstoffatom, an das sie gebunden sind, einen monocyclischen Rest mit 5 bis 7 Ringgliedern oder einen Rest, der aus kondensierten Ringen gebildet wird, die 8 bis 10 Ringglieder umfassen, wobei diese Reste gegebenenfalls ein anderes oder mehrere andere, gleiche oder verschiedene Heteroatome umfassen, ausgewählt unter den Atomen von Sauerstoff, Stickstoff und Schwefel, und die gegebenenfalls substituiert sind,

oder $R_{10}$ und $R_{11}$, gleich oder verschieden, bedeuten:

- ein Wasserstoffatom;
- einen linearen oder verzweigten Alkyl-, Alkenyl- oder Alkinylrest mit höchstens 6 Kohlenstoffatomen und gegebenenfalls substituiert;
- einen Arylrest, in dem der Arylrest einen monocyclischen Rest mit 5 bis 7 Ringgliedern darstellt oder einen Rest, der aus kondensierten Ringen gebildet wird, die 8 bis 10 Ringglieder umfassen, wobei diese Reste gegebenenfalls ein oder mehrere, gleiche oder verschiedene Heteroatome umfassen, ausgewählt unter den Atomen von Sauerstoff, Stickstoff und Schwefel, und die gegebenenfalls substituiert sind;

A bedeutet:

- entweder einen linearen oder verzweigten Alkylen- oder Alkenylenrest mit höchstens 6 Kohlenstoffatomen und gegebenenfalls durch einen Hydroxylrest oder einen Alkoxyrest mit höchstens 6 Kohlenstoffatomen substituiert,
- oder eine einfache Bindung,

$R_2$ einen monocyclischen Rest mit 5 bis 7 Ringgliedern darstellt oder einen Rest, der aus kondensierten Ringen gebildet wird, die 8 bis 10 Ringglieder umfassen, wobei diese Reste gesättigt oder ungesättigt sind und gegebenenfalls ein oder mehrere, gleiche oder verschiedene Heteroatome umfassen, ausgewählt unter den Atomen von Sauerstoff, Stickstoff und Schwefel, und die gegebenenfalls substituiert sind;

X ein Sauerstoff- oder Schwefelatom bedeutet,

$R_{3A}$ und $R_{4A}$ bedeuten:

entweder bilden $R_{3A}$ und $R_{4A}$ mit dem Stickstoffatom, an das sie gebunden sind, einen monocyclischen Rest mit 5 bis 7 Ringgliedern oder einen Rest, der aus kondensierten Ringen gebildet wird, die 8 bis 10 Ringglieder umfassen, wobei diese Reste gegebenenfalls ein anderes oder mehrere andere, gleiche oder verschiedene Heteroatome umfassen, ausgewählt unter den Atomen von Sauerstoff, Stickstoff und Schwefel, und die gegebenenfalls substituiert sind,

oder $R_{3A}$ und $R_{4A}$, gleich oder verschieden, bedeuten:

- ein Wasserstoffatom;
- einen Hydroxylrest, Alkoxy-, Acyl- oder Acyloxyrest mit höchstens 6 Kohlenstoffatomen; Carboxy, in Salz überführt oder verestert; Cyano;
- einen linearen oder verzweigten Alkyl-, Alkenyl- oder Alkinylrest mit höchstens 6 Kohlenstoffatomen und gegebenenfalls substituiert;
- einen Rest $R_c$ oder $R_c$-O- worin der Rest $R_c$ einen aliphatischen oder Arylrest darstellt oder einen monocyclischen Rest mit 5 bis 7 Ringgliedern oder einen Rest, der aus kondensierten Ringen gebildet wird, die 8 bis 10 Ringglieder umfassen, wobei diese Reste gegebenenfalls ein oder mehrere, gleiche oder verschiedene Heteroatome umfassen, ausgewählt unter den Atomen von Sauerstoff,

Stickstoff und Schwefel, und die gegebenenfalls substituiert sind;

- den Rest

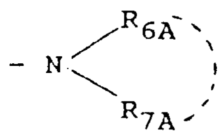

oder

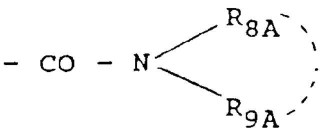

worin $R_{6A}$, $R_{7A}$, $R_{6A}$ und $R_{9A}$, gleich oder verschieden, ein Wasserstoffatom, einen der Reste Hydroxyl, Alkyl, Alkoxy, Acyloxy oder Acyl, wobei diese Reste höchstens 6 Kohlenstoffatome umfassen, freies Carboxy, in Salz überführt oder verestert, Phenoxy, Phenyl, Benzyl, Phenethyl, Azepin, Piperidyl, Morpholin, Pyrrolidinyl oder Piperazinyl bedeuten, gegebenenfalls substituiert an dem zweiten Stickstoffatom durch einen Hydroxyl-, Alkyl-, Alkoxy- oder Phenylrest, wobei diese drei letzten Reste gegebenenfalls selbst durch ein oder mehrere Reste substituiert sind, ausgewählt unter den Halogenatomen und den Resten Hydroxyl, Trifluormethyl, Methoxy, Ethoxy, Methyl und Ethyl, oder einerseits $R_{6A}$ und $R_{7A}$ und andererseits $R_{8A}$ und $R_{9A}$ jeweils mit dem Stickstoffatom, an das sie gebunden sind, einen der Reste Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepinyl oder Indolyl bilden, wobei diese Reste gegebenenfalls substituiert sind durch ein oder mehrere, gleiche oder verschiedene Reste, ausgewählt unter den Halogenatomen und den Resten Hydroxyl, Trifluormethyl, Alkyl und Alkoxy, wobei diese Reste höchstens 6 Kohlenstoffatome umfassen,

wobei die genannten Produkte der Formel ($I_A$) in allen möglichen isomeren, racemischen, enantiomeren und diastereoisomeren Formen vorliegen, sowie die Additionssalze der genannten Produkte der Formel ($I_A$) mit Mineralsäuren und organischen Säuren.

**2.** Produkte der Formel ($I_A$) wie in Anspruch 1 definiert, die der Formel (I) entsprechen

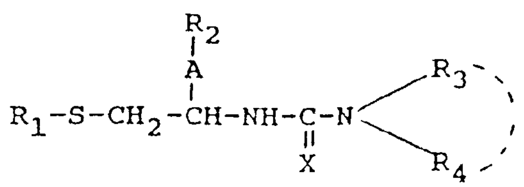

(I)

in der:

$R_1$ ein Wasserstoffatom oder einen Rest $R_6$-CO- darstellt, worin $R_5$ bedeutet:

- einen linearen oder verzweigten Alkyl-, Alkenyl- oder Alkinylrest mit höchstens 6 Kohlenstoffatomen und gegebenenfalls substituiert,
- einen monocyclischen Rest mit 5 bis 7 Ringgliedern oder einen Rest, der aus kondensierten Ringen gebildet wird, die 8 bis 10 Ringglieder umfassen, wobei diese Reste gesättigt oder ungesättigt sind und gegebenenfalls ein oder mehrere, gleiche oder verschiedene Heteroatome umfassen, ausgewählt unter den Atomen von Sauerstoff, Stickstoff und Schwefel, und die gegebenenfalls substituiert sind,
- einen Rest

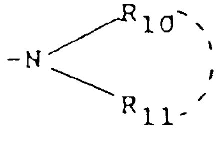

worin $R_{10}$ und $R_{11}$ bedeuten:

<u>entweder</u> bilden $R_{10}$ und $R_{11}$ mit dem Stickstoffatom, an das sie gebunden sind, einen monocyclischen Rest mit 5 bis 7 Ringgliedern oder einen Rest, der aus kondensierten Ringen gebildet wird, die 8 bis 10 Ringglieder umfassen, wobei diese Reste gegebenenfalls ein anderes oder mehrere andere, gleiche oder

verschiedene Heteroatome umfassen, ausgewählt unter den Atomen von Sauerstoff, Stickstoff und Schwefel, und die gegebenenfalls substituiert sind,

oder $R_{10}$ und $R_{11}$, gleich oder verschieden, bedeuten:

- ein Wasserstoffatom;
- einen linearen oder verzweigten Alkyl-, Alkenyl- oder Alkinylrest mit höchstens 6 Kohlenstoffatomen und gegebenenfalls substituiert;
- einen Arylrest, in dem der Arylrest einen monocyclischen Rest mit 5 bis 7 Ringgliedern darstellt oder einen Rest, der aus kondensierten Ringen gebildet wird, die 8 bis 10 Ringglieder umfassen, wobei diese Reste gegebenenfalls ein oder mehrere, gleiche oder verschiedene Heteroatome umfassen, ausgewählt unter den Atomen von Sauerstoff, Stickstoff und Schwefel, und die gegebenenfalls substituiert sind;

A bedeutet:

- entweder einen linearen oder verzweigten Alkylen- oder Alkenylenrest mit höchstens 6 Kohlenstoffatomen und gegebenenfalls durch einen Hydroxylrest oder einen Alkoxyrest mit höchstens 6 Kohlenstoffatomen substituiert,
- oder eine einfache Bindung,

$R_2$ einen monocyclischen Rest mit 5 bis 7 Ringgliedern darstellt oder einen Rest, der aus kondensierten Ringen gebildet wird, die 8 bis 10 Ringglieder umfassen, wobei diese Reste gesättigt oder ungesättigt sind und gegebenenfalls ein oder mehrere, gleiche oder verschiedene Heteroatome umfassen, ausgewählt unter den Atomen von Sauerstoff, Stickstoff und Schwefel, und die gegebenenfalls substituiert sind;

X ein Sauerstoff- oder Schwefelatom bedeutet,

$R_3$ und $R_4$ bedeuten:

entweder bilden $R_3$ und $R_4$ mit dem Stickstoffatom, an das sie gebunden sind, einen monocyclischen Rest mit 5 bis 7 Ringgliedern oder einen Rest, der aus kondensierten Ringen gebildet wird, die 8 bis 10 Ringglieder umfassen, wobei diese Reste gegebenenfalls ein anderes oder mehrere andere, gleiche oder verschiedene Heteroatome umfassen, ausgewählt unter den Atomen von Sauerstoff, Stickstoff und Schwefel, und die gegebenenfalls substituiert sind,

oder $R_3$ und $R_4$, gleich oder verschieden, bedeuten:

- ein Wasserstoffatom;
- einen Hydroxylrest, Alkoxy-, Acyl- oder Acyloxyrest mit höchstens 6 Kohlenstoffatomen; Carboxy, in Salz überführt oder verestert; Cyano;
- einen linearen oder verzweigten Alkyl-, Alkenyl- oder Alkinylrest mit höchstens 6 Kohlenstoffatomen und gegebenenfalls substituiert;
- einen Aryl- oder Aryloxidrest, worin der Arylrest einen monocyclischen Rest mit 5 bis 7 Ringgliedern oder einen Rest darstellt, der aus kondensierten Ringen gebildet wird, die 8 bis 10 Ringglieder umfassen, wobei diese Reste gegebenenfalls ein oder mehrere, gleiche oder verschiedene Heteroatome umfassen, ausgewählt unter den Atomen von Sauerstoff, Stickstoff und Schwefel, und die gegebenenfalls substituiert sind;
- den Rest

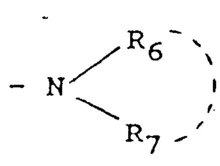

oder

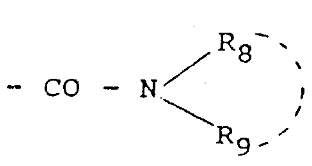

worin $R_6$, $R_7$, $R_6$ und $R_9$, gleich oder verschieden, ein Wasserstoffatom, einen der Reste Hydroxyl, Alkyl, Alkoxy, Acyloxy oder Acyl, wobei diese Reste höchstens 6 Kohlenstoffatome umfassen, freies Carboxy, in Salz überführt oder verestert, Phenyl, Benzyl, Phenethyl, Azepin, Piperidyl, Morpholin, Pyrrolidinyl oder Piperazinyl bedeuten, gegebenenfalls substituiert an dem zweiten Stickstoffatom durch einen Hydroxyl-, Alkyl-, Alkoxy- oder Phenylrest, wobei diese drei letzten Reste gegebenenfalls selbst durch ein oder mehrere Reste substituiert sind, ausgewählt unter den Halogenatomen und den Resten Hydroxyl, Trifluormethyl, Methoxy, Ethoxy, Methyl und Ethyl, oder einerseits $R_6$ und $R_7$ und andererseits $R_6$ und $R_9$ jeweils mit dem Stickstoffatom, an das sie gebunden sind, einen der Reste Imidazolyl, Pyrrolyl , Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepinyl oder Indolyl bilden, wobei diese Reste gegebenenfalls substituiert sind durch ein oder mehrere, gleiche

oder verschiedene Reste, ausgewählt unter den Halogenatomen und den Resten Hydroxyl, Trifluormethyl, Alkyl und Alkoxy, wobei diese Reste höchstens 6 Kohlenstoffatome umfassen,

wobei die genannten Produkte der Formel (I) in allen möglichen isomeren, racemischen, enantiomeren und diastereoisomeren Formen vorliegen, sowie die Additionssalze der genannten Produkte der Formel (I) mit Mineralsäuren und organischen Säuren.

3. Produkte der Formeln ($I_A$) und (I) wie in den Ansprüchen 1 und 2 definiert, dadurch gekennzeichnet, daß der oder die Substituenten, gleich oder verschieden, die tragen können:

a) die Alkyl-, Alkenyl- oder Alkinylreste,

b) die monocyclischen Reste oder die aus kondensierten Ringen sowie aus Aryl und Aryloxy gebildeten Reste,

ausgewählt werden aus der Gruppe, gebildet durch:

- die Halogenatome; die Hydroxylreste; Cyano; Mercapto; Nitro; Acyl und Acyloxy mit höchstens 6 Kohlenstoffatomen; freies Carboxy, in Salz überführt oder verestert; Alkoxycarbonyl;
- die linearen und verzweigten Alkoxyreste mit höchstens 6 Kohlenstoffatomen;
- die Reste Alkyl, Alkenyl, Alkinyl, Alkylthio, Cycloalkyl, Cycloalkenyl, die heterocyclischen Kohlenwasserstoffreste, Aryl, Aryloxy, Arylthio, Phenylalkyl, Phenylalkoxy, die gegebenenfalls selbst durch ein oder mehrere, gleiche oder verschiedene Substituenten substituiert sind, ausgewählt unter den Halogenatomen, den Resten Hydroxyl, Trifluormethyl, Nitro, Alkyl, Alkenyl, Aryl, Alkoxy und Acyl, wobei diese Reste höchstens 6 Kohlenstoffatome umfassen, freies Carboxy, in Salz überführt oder verestert, Acylamido, worin der Acylrest höchstens 6 Kohlenstoffatome umfaßt, Carbamoyl und Amino, wobei diese Reste gegebenenfalls am Stickstoffatom durch ein oder zwei gleiche oder verschiedene Reste substituiert sind, ausgewählt unter den Resten Hydroxyl, Alkyl, und Alkoxy, die höchstens 6 Kohlenstoffatome umfassen;
- den Acylamidorest, worin der Acylrest höchstens 6 Kohlenstoffatome umfaßt; die Reste Carbamoyl und Amino, die gegebenenfalls am Stickstoffatom durch ein oder zwei gleiche oder verschiedene Reste substituiert sind, ausgewählt unter den Resten Hydroxyl, Alkyl, Alkenyl und Alkoxy, die höchstens 6 Kohlenstoffatome umfassen;

wobei die genannten Produkte der Formel ($I_A$) und (I) in allen möglichen isomeren, racemischen, enantiomeren und diastereoisomeren Formen vorliegen, sowie die Additionssalze der genannten Produkte der Formel ($I_A$) und (I) mit Mineralsäuren und organischen Säuren.

4. Produkte der Formel ($I_A$) wie in Anspruch 1 definiert, die der Formel ($I'_A$) entsprechen

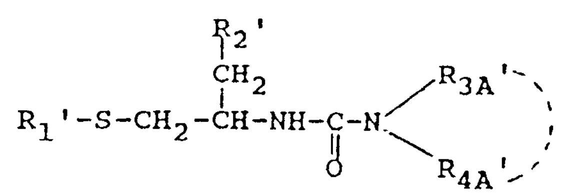

($I'_A$)

in der

$R_{1A}'$ ein Wasserstoffatom oder einen Rest $R_{5A}'$-CO- darstellt, worin $R_{5A}'$ einen Phenyl-, Cycloalkyl-, Cycloalkenyl-, Alkyl- oder Alkenylrest mit höchstens 6 Kohlenstoffatomen bedeutet, wobei diese Reste gegebenenfalls substituiert sind, oder

- einen Rest

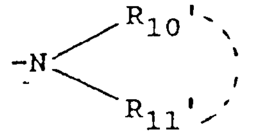

worin $R_{10}'$ und $R_{11}'$ bedeuten:

<u>entweder</u> bilden $R_{10}'$ und $R_{11}'$ mit dem Stickstoffatom, an das sie gebunden sind, einen der Reste Imidazolyl, Pyrrolyl, Pyrrolinyl, Pyrrol idinyl, Pyridyl, Piperidinyl, Pyrimidinyl, Pyridazinyl, Pyrazinyl, Piperazinyl, Phenylpiperazinyl, Methylpiperazinyl, Piperidyl, Oxazolyl, Morpholinyl und Thiomorpholinyl,

Azepin oder Indolyl, wobei diese Reste gegebenenfalls substituiert sind durch ein oder mehrere, gleiche oder verschiedene Reste, ausgewählt unter den Halogenatomen und den Resten Hydroxyl, Mercapto, Trifluormethyl, Alkyl, Alkenyl, Alkinyl, Alkylthio, Alkoxy und Phenyl, gegebenenfalls substituiert durch einen oder mehrere Reste, ausgewählt unter den Halogenatomen und den Resten Trifluormethyl, Hydroxyl, Alkyl und Alkoxy, wobei die Alkyl- und Alkoxyreste höchstens 6 Kohlenstoffatome umfassen,
oder $R_{10}$' und $R_{11}$', gleich oder verschieden, bedeuten:

- ein Wasserstoffatom;
- einen linearen oder verzweigten Alkyl-, Alkenyl- oder Alkinylrest mit höchstens 6 Kohlenstoffatomen und gegebenenfalls substituiert;
- einen der Reste Phenyl, Benzyl, Naphthyl, Indenyl, Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepinyl oder Indolyl, wobei diese Reste gegebenenfalls substituiert sind durch ein oder mehrere, gleiche oder verschiedene Reste, ausgewählt unter den Halogenatomen und den Resten Hydroxyl, Mercapto, Trifluormethyl, Alkyl, Alkenyl, Alkinyl, Alkylthio und Alkoxy, die höchstens 6 Kohlenstoffatome umfassen.

$R_2$' einen der Reste Phenyl, Naphthyl, Benzyl, Phenethyl, Indolyl, Cycloalkyl oder Cycloalkenyl darstellt, wobei diese Reste gegebenenfalls substituiert sind;
$R_{3A}$' und $R_{4A}$' bedeuten:
entweder bilden $R_{3A}$' und $R_{4A}$' mit dem Stickstoffatom, an das sie gebunden sind, einen der Reste Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepinyl oder Indolyl, wobei diese Reste gegebenenfalls substituiert sind durch ein oder mehrere, gleiche oder verschiedene Reste, ausgewählt unter den Halogenatomen und den Resten Hydroxyl, Trifluormethyl, Alkyl, Alkenyl, Alkinyl, Alkoxy und Phenyl, gegebenenfalls substituiert durch einen oder mehrere Reste, ausgewählt unter den Halogenatomen und den Resten Trifluormethyl, Hydroxyl, Alkyl und Alkoxy, wobei die Reste Alkyl und Alkoxy höchstens 6 Kohlenstoffatome umfassen und gegebenenfalls durch einen Phenylrest substituiert sind, oder $R_{3A}$' und $R_{4A}$', gleich oder verschieden, bedeuten:

- ein Wasserstoffatom;
- einen Hydroxylrest, Alkoxy-, Acyloxy- oder Acylrest mit höchstens 6 Kohlenstoffatomen; Carboxy, in Salz überführt oder verestert; Cyano;
- einen linearen oder verzweigten Alkyl-, Alkenyl- oder Alkinylrest mit höchstens 6 Kohlenstoffatomen und gegebenenfalls substituiert;
- einen der Reste Phenyl, Benzoyl, Naphthyl, Indenyl, Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepinyl, Diazepin, Benzodiazepin oder Indolyl, wobei diese Reste gegebenenfalls substituiert sind durch ein oder mehrere, gleiche oder verschiedene Reste, ausgewählt unter den Halogenatomen und den Resten Hydroxyl, Trifluormethyl, Alkyl und Alkoxy, die höchstens 6 Kohlenstoffatome umfassen und gegebenenfalls selbst substituiert sind durch einen oder mehrere Reste, ausgewählt unter den Halogenatomen und den Resten Hydroxyl, Alkyl, Alkoxy und Carboxy, frei, in ein Salz überführt oder verestert durch einen Alkyl-, Aryl- oder Arylalkylrest,
- den Rest

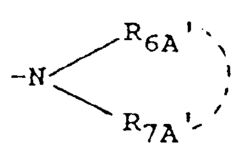 oder 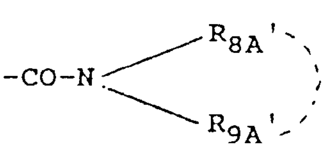

worin $R_{6A}$', $R_{7A}$', $R_{8A}$' und $R_{9A}$', gleich oder verschieden, ein Wasserstoffatom, einen der Reste Hydroxyl, Alkyl, Alkoxy, Acyloxy oder Acyl, wobei diese Reste höchstens 6 Kohlenstoffatome umfassen, freies Carboxy, in Salz überführt oder verestert, Phenyl, Benzyl, Phenethyl, Azepinyl, Piperidyl, Morpholin, Pyrrolidinyl oder Piperazinyl bedeuten,

oder einerseits $R_{6A}$' und $R_{7A}$' und andererseits $R_{8A}$' und $R_{9A}$' jeweils mit dem Stickstoffatom, an das sie gebunden sind, einen der Reste Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepinyl oder Indolyl bilden, wobei diese Reste gegebenenfalls substituiert sind durch ein oder mehrere, gleiche oder verschiedene Reste, ausgewählt unter den Halogenatomen und den Resten Hydroxyl, Trifluormethyl, Alkyl und Alkoxy, wobei diese Reste höchstens 6 Kohlenstoffatome umfassen, mit der Maßgabe, daß der oder die eventuellen Substituenten, gleich oder verschieden, die die Reste tragen können, die $R_1$', $R_2$' und $R_5$' darstellen, ausgewählt werden aus der Gruppe, die gebildet wird aus:

- den Halogenatomen; den Hydroxylresten; Cyano; Nitro; Acyl und Acyloxy mit höchstens 6 Kohlenstoffatomen;Benzoyl,freies Carboxy,in Salz überführt oder verestert;
- den linearen und verzweigten Alkoxyresten mit höchstens 6 Kohlenstoffatomen;
- den Resten Alkyl, Alkenyl, Cyclopentyl, Cyclohexyl, Cyclopentenyl, Cyclohexenyl, Phenyl, Naphthyl, Indolyl, Phenoxy, Benzyl, Phenethyl und Benzyloxy, die gegebenenfalls selbst durch einen oder mehrere gleiche oder verschiedene Substituenten substituiert sind, ausgewählt unter den Halogenatomen und den Resten Hydroxyl, Trifluormethyl, Nitro, Alkyl, Alkenyl und Alkoxy, wobei diese Reste höchstens 6 Kohlenstoffatome umfassen, Formyl, Acetyl, Benzoyl, freies Carboxy, in Salz überführt oder verestert, Acetamido, Benzoylamido, Carbamoyl und Amino, wobei diese Reste gegebenenfalls am Stickstoffatom durch ein oder zwei gleiche oder verschiedene Reste substituiert sind, ausgewählt unter den Resten Hydroxyl, Methyl, Ethyl, Propyl, Methoxy, Ethoxy und Propoxy;
- dem Acylamidorest, worin der Acylrest höchstens 6 Kohlenstoffatome umfaßt; den Resten Carbamoyl und Amino, die gegebenenfalls am Stickstoffatom durch ein oder zwei gleiche oder verschiedene Reste substituiert sind, ausgewählt unter den Resten Hydroxyl, Alkyl, Alkenyl, Alkoxy und Acyl, die höchstens 6 Kohlenstoffatome umfassen;

wobei die genannten Produkte der Formel ($I_A$') in allen möglichen isomeren, racemischen, enantiomeren und diastereoisomeren Formen vorliegen,
sowie die Additionssalze der genannten Produkte der Formel ($I_A$') mit Mineralsäuren und organischen Säuren.

**5.** Produkte der Formel ($I_A$) und (I) wie in Anspruch 1 und 2 definiert, die der Formel (I') entsprechen

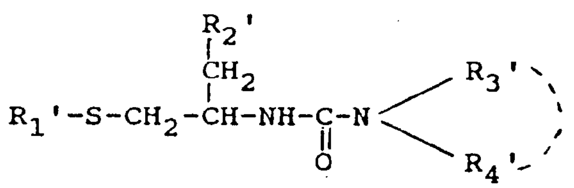

(I')

in der:
$R_1$' ein Wasserstoffatom oder einen Rest $R_5$'-CO- darstellt, worin $R_5$' einen der Reste Phenyl, Cycloalkyl, Cycloalkenyl, Alkyl oder Alkenyl mit höchstens 6 Kohlenstoffatomen bedeutet, und die gegebenenfalls substituiert sind, oder einen Rest

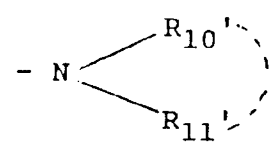

worin $R_{10}$' und $R_{11}$' bedeuten: <u>entweder</u> bilden $R_{10}$' und $R_{11}$' mit dem Stickstoffatom, an das sie gebunden sind, einen der Reste Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepin oder Indolyl, wobei diese Reste gegebenenfalls substituiert sind durch ein oder mehrere, gleiche oder verschiedene Reste, ausgewählt unter den Halogenatomen und den Resten Hydroxyl, Trifluormethyl, Alkyl, Alkoxy und Phenyl, gegebenenfalls substituiert durch einen oder mehrere Reste, ausgewählt unter den Halogenatomen und den Resten Trifluormethyl, Hydroxyl, Alkyl und Alkoxy, wobei diese Reste höchstens 6 Kohlenstoffatome umfassen,
<u>oder</u> $R_{10}$' und $R_{11}$', gleich oder verschieden, bedeuten:

- ein Wasserstoffatom;
- einen linearen oder verzweigten Alkyl-, Alkenyl- oder Alkinylrest mit höchstens 6 Kohlenstoffatomen und gegebenenfalls substituiert;
- einen der Reste Phenyl, Benzoyl, Naphthyl, Indenyl, Imidazolyl, Pyrrolyl Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl,

Oxazolyl, Morpholinyl und Thiomorpholinyl, Azepin oder Indolyl, wobei diese Reste gegebenenfalls substituiert sind durch ein oder mehrere, gleiche oder verschiedene Reste, ausgewählt unter den Halogenatomen und den Resten Hydroxyl, Trifluormethyl, Alkyl und Alkoxy, die höchstens 6 Kohlenstoffatome umfassen,

$R_2$' einen der Reste Phenyl, Naphthyl, Benzyl, Phenethyl, Indolyl, Cycloalkyl oder Cycloalkenyl darstellt, wobei diese Reste gegebenenfalls substituiert sind;

$R_3$' und $R_4$' bedeuten:

entweder bilden $R_3$' und $R_4$' mit dem Stickstoffatom, an das sie gebunden sind, einen der Reste Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepin oder Indolyl, wobei diese Reste gegebenenfalls substituiert sind durch ein oder mehrere, gleiche oder verschiedene Reste, ausgewählt unter den Halogenatomen und den Resten Hydroxyl, Trifluormethyl, Alkyl, Alkoxy und Phenyl, gegebenenfalls substituiert durch einen oder mehrere Reste, ausgewählt unter den Halogenatomen und den Resten Trifluormethyl, Hydroxyl, Alkyl und Alkoxy, wobei diese Reste höchstens 6 Kohlenstoffatome umfassen,

oder $R_3$' und $R_4$', gleich oder verschieden, bedeuten:

- ein Wasserstoffatom;
- einen Hydroxylrest, Alkoxy-, Acyloxy- oder Acylrest mit höchstens 6 Kohlenstoffatomen; Carboxy, in Salz überführt oder verestert; Cyano;
- einen linearen oder verzweigten Alkyl-, Alkenyl- oder Alkinylrest mit höchstens 6 Kohlenstoffatomen und gegebenenfalls substituiert;
- einen der Reste Phenyl, Benzoyl, Naphthyl, Indenyl, Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepin oder Indolyl, wobei diese Reste gegebenenfalls substituiert sind durch ein oder mehrere, gleiche oder verschiedene Reste, ausgewählt unter den Halogenatomen und den Resten Hydroxyl, Trifluormethyl, Alkyl und Alkoxy, die höchstens 6 Kohlenstoffatome umfassen,
- den Rest

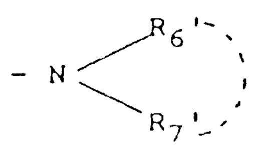 oder 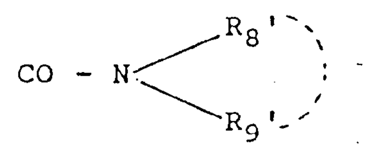

worin $R_6$', $R_7$', $R_8$' und $R_9$', gleich oder verschieden, ein Wasserstoffatom, einen der Reste Hydroxyl, Alkyl, Alkoxy, Acyloxy oder Acyl, wobei diese Reste höchstens 6 Kohlenstoffatome umfassen, freies Carboxy, in Salz überführt oder verestert, Phenyl, Benzyl, Phenethyl, Azepin, Piperidyl, Morpholin, Pyrrolidinyl oder Piperazinyl bedeuten,

oder einerseits $R_6$ und $R_7$ und andererseits $R_8$ und $R_9$ jeweils mit dem Stickstoffatom, an das sie gebunden sind, einen der Reste Imidazolyl, Pyrrolyl, Pyrrolinyl, Pyrrolidinyl, Pyridyl, Piperidinyl,-Pyrimidinyl, Pyridazinyl, Pyrazinyl, Piperazinyl, Phenylpiperazinyl, Piperidyl, Oxazolyl, Morpholinyl und Thiomorpholinyl, Azepin oder Indolyl bilden, wobei diese Reste gegebenenfalls substituiert sind durch ein oder mehrere, gleiche oder verschiedene Reste, ausgewählt unter den Halogenatomen und den Resten Hydroxyl, Trifluormethyl, Alkyl und Alkoxy, wobei diese Reste höchstens 6 Kohlenstoffatome umfassen, mit der Maßgabe, daß der oder die eventuellen Substituenten, gleich oder verschieden, die die Reste tragen können, die $R_1$', $R_2$' und $R_8$' darstellen, ausgewählt werden aus der Gruppe, die gebildet wird aus:

- den Halogenatomen; den Hydroxylresten; Cyano; Nitro; Acyl und Acyloxy mit höchstens 6 Kohlenstoffatomen; Benzoyl; freies Carboxy, in Salz überführt oder verestert;
- den linearen und verzweigten Alkoxyresten mit höchstens 6 Kohlenstoffatomen;
- den Resten Alkyl, Alkenyl, Cyclopentyl, Cyclohexyl, Cyclopentenyl, Cyclohexenyl, Phenyl, Naphthyl, Indolyl, Phenoxy, Benzyl, Phenethyl und Benzyloxy, die gegebenenfalls selbst durch einen oder mehrere gleiche oder verschiedene Substituenten substituiert sind, ausgewählt unter den Halogenatomen und den Resten Hydroxyl, Trifluormethyl, Nitro, Alkyl, Alkenyl und Alkoxy, wobei diese Reste höchstens 6 Kohlenstoffatome umfassen, Formyl, Acetyl, Benzoyl, freies Carboxy, in Salz überführt oder verestert, Acetamido, Benzoylamido, Carbamoyl und Amino, wobei diese Reste gegebenenfalls am Stickstoffatom durch ein oder zwei gleiche oder verschiedene Reste substituiert sind, ausgewählt unter den Resten Hydroxyl, Methyl, Ethyl, Propyl, Methoxy, Ethoxy und Propoxy;

- dem Acylamidorest, worin der Acylrest höchstens 6 Kohlenstoffatome umfaßt; den Resten Carbamoyl und Amino, die gegebenenfalls am Stickstoffatom durch ein oder zwei gleiche oder verschiedene Reste substituiert sind, ausgewählt unter den Resten Hydroxyl, Alkyl, Alkenyl, Alkoxy und Acyl, die höchstens 6 Kohlenstoffatome umfassen;

wobei die genannten Produkte der Formel (I') in allen möglichen isomeren, racemischen, enantiomeren und diastereoisomeren Formen vorliegen, sowie die Additionssalze der genannten Produkte der Formel (I') mit Mineralsäuren und organischen Säuren.

6. Produkte der Formel ($I'_A$) wie in Anspruch 4 definiert, dadurch gekennzeichnet, daß
$R_1$' ein Wasserstoffatom oder einen Acetylrest darstellt,
$R_2$' einen Phenylrest bedeutet;
$R_{3A}$' und $R_{4A}$' bedeuten:
<u>entweder</u> bilden $R_{3A}$' und $R_{4A}$' mit dem Stickstoffatom, an das sie gebunden sind, einen der Reste Piperidinyl, Morpholinyl, Dimethylmorpholinyl, Thiomorpholinyl, Pyrrolidinyl, Azepin oder Diazepin, gegebenenfalls substituiert am zweiten Stickstoffatom durch einen Alkylrest mit 1 oder 2 Kohlenstoffatomen, Piperazinyl, gegebenenfalls substituiert am zweiten Stickstoffatom entweder durch einen Phenylrest, gegebenenfalls substituiert durch ein Halogenatom oder einen Trifluormethylrest, oder durch einen Alkyl-, Alkenyl- oder Alkinylrest mit 1 bis 4 Kohlenstoffatomen, gegebenenfalls substituiert durch einen Phenyl- oder Acylrest, oder durch einen Acylrest mit höchstens 6 Kohlenstoffatomen,
oder $R_{3A}$' und $R_{4A}$', gleich oder verschieden, bedeuten:
- ein Wasserstoffatom; einen Hydroxylrest; Phenyl; Phenyl, substituiert durch einen Carboxyrest, Piperidinyl, gegebenenfalls substituiert am Stickstoffatom durch einen Benzylrest, Piperazinyl, gegebenenfalls substituiert am zweiten Stickstoffatom durch einen Alkylrest mit 1 oder 2 Kohlenstoffatomen, Carbamoyl; Alkoxy mit höchstens 4 Kohlenstoffatomen, Alkyl mit höchstens 4 Kohlenstoffatomen, gegebenenfalls substituiert durch einen oder mehrere gleiche oder verschiedene Reste, ausgewählt unter den Resten Hydroxyl, Alkoxy, freies Carboxy, in Salz überführt oder verestert, Amino, Alkylamino, Dialkylamino, Phenyl, Morpholinyl, Alkylthio mit höchstens 4 Kohlenstoffatomen, gegebenenfalls substituiert durch einen freien Carboxyrest, in Salz überführt oder verestert, Benzodiazepin, gegebenenfalls substituiert,
wobei die genannten Produkte der Formel ($I'_A$) in allen möglichen isomeren, racemischen, enantiomeren und diastereoisomeren Formen vorliegen, sowie die Additionssalze der genannten Produkte der Formel ($I'_A$) mit Mineralsäuren und organischen Säuren.

7. Produkte der Formel (I') wie in Anspruch 5 definiert, dadurch gekennzeichnet, daß
$R_1$' ein Wasserstoffatom oder einen Acetylrest darstellt,
$R_2$' einen Phenylrest darstellt,
$R_3$' und $R_4$' bedeuten:
<u>entweder</u> bilden $R_3$' und $R_4$' mit dem Stickstoffatom, an das sie gebunden sind, einen der Reste Piperidinyl, Morpholinyl, Dimethylmorpholinyl, Thiomorpholinyl, Pyrrolidinyl, Azepin, Piperazinyl, gegebenenfalls substituiert am zweiten Stickstoffatom durch einen Alkylrest mit 1 oder 2 Kohlenstoffatomen oder einen Phenylrest, der selbst gegebenenfalls durch ein Halogenatom oder einen Trifluormethylrest substituiert ist,
<u>oder</u> $R_3$' und $R_4$', gleich oder verschieden, bedeuten:
ein Wasserstoffatom; einen Hydroxylrest, Phenyl; Carbamoyl; Alkoxy mit höchstens 4 Kohlenstoffatomen, Alkyl mit höchstens 4 Kohlenstoffatomen, gegebenenfalls substituiert durch einen oder mehrere gleiche oder verschiedene Reste, ausgewählt unter den Resten Hydroxyl, Alkoxy, freies Carboxy, in Salz überführt oder verestert,
wobei die genannten Produkte der Formel (I') in allen möglichen isomeren, racemischen, enantiomeren und diastereoisomeren Formen vorliegen,
sowie die Additionssalze der genannten Produkte der Formel (I') mit Mineralsäuren und organischen Säuren.

8. Produkte der Formel ($I_A$), die den folgenden Formeln entsprechen:
(±)4-Methyl-N-[2-mercapto-1-(phenylmethyl)-ethyl]-1-piperazin-carboxamid-Hydrochlorid,
(±)N-[2-Mercapto-1-(phenylmethyl)-ethyl]-4-morpholin-carboxamid,
(±)N-[2-Mercapto-1-(phenylmethyl)-ethyl]-1-pyrrolidin-carboxamid,
(±)N-[[[2-Mercapto-1-(phenylmethyl)-ethyl]-amino]-carbonyl]-glycin,
N,N-bis(2-Methoxy-ethyl)-N'-[2-mercapto-1-(phenylmethyl)-ethyll-harnstoff,

(±)cis-2,6-Dimethyl-N-[2-mercapto-1-(phenylmethyl)-ethyl]-4-morpholin- carboxamid (Isomer A),
(±)N-[2-Mercapto-1-(phenylmethyl)-ethyl]-4-thiomorpholin-carboxamid,
(±)N-[2-Mercapto-1-(phenylmethyl)-ethyl]-N'-methoxy-harnstoff,
(±)N-Hydroxy-N'-[2-mercapto-1-(phenylmethyl)-ethyl]-N'-methyl-harnstoff,
(±)N-(1-Mercapto-methyl)-2-(phenylethyl)-4-methyl-1-piperazin-acetamid,
sowie ihre Salze mit Mineralsäuren oder organischen Säuren.

**9.** Verfahren zur Herstellung der Produkte der Formel ($I_A$) wie in Anspruch 1 definiert, dadurch gekennzeichnet, daß man

- zur Herstellung der Produkte der Formel ($I_A$), in der n = 0 ist, eine Verbindung der Formel (II)

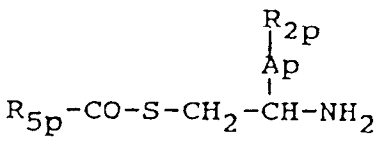

(II)

in der $R_{5p}$, $R_{2p}$ und Ap die vorstehend jeweils für $R_5$, $R_2$ und A angegebenen Bedeutungen besitzen und in denen die eventuellen reaktiven Funktionen gegebenenfalls durch Schutzgruppen geschützt sind, der Einwirkung eines Reaktanden, wie Phosgen oder Thiophosgen unterzieht, um jeweils das entsprechende Isocyanat oder Isothiocyanat der Formel (III)

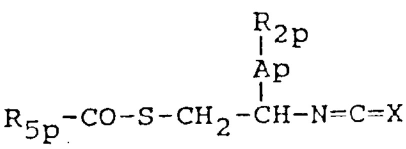

(III)

zu erhalten, in der $R_{5p}$, $R_{2p}$ und Ap die vorstehend angegebenen Bedeutungen besitzen und X ein Sauerstoffatom oder ein Schwefelatom darstellt, das man mit einer Verbindung der Formel (IV)

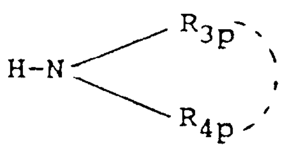

(IV)

zur Reaktion bringt, in der $R_{3p}$ und $R_{4p}$ die vorstehend jeweils für $R_3$ und $R_4$ angegebenen Bedeutungen besitzen und in denen die eventuellen reaktiven Funktionen gegebenenfalls durch Schutzgruppen geschützt sind, um ein Produkt der Formel (V)

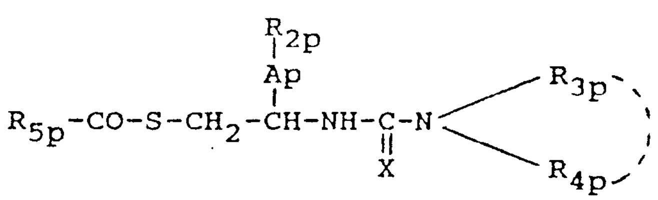

(V)

zu erhalten, in der $R_{5p}$, $R_{2p}$, Ap, $R_{3p}$, $R_{4p}$ und X die vorstehend angegebenen Bedeutungen besitzen, das man, wenn erforderlich und wenn gewünscht, einer Reaktion zur Entfernung des Restes $R_{5p}$-CO- unterzieht, um ein Produkt der Formel (VI)

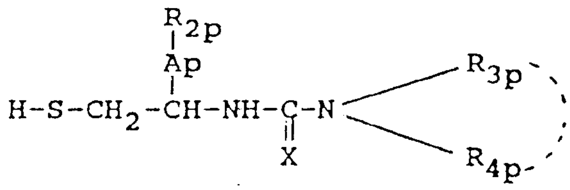

(VI)

zu erhalten, in der $R_{2p}$, Ap, $R_{3p}$, $R_{4p}$ und X die vorstehend angegebenen Bedeutungen besitzen,

- zur Herstellung der Produkte der Formel ($I_A$), in der n von 0 verschieden ist und X ein Sauerstoffatom darstellt, eine Verbindung der Formel (II')

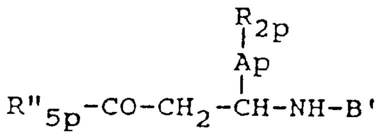

(II')

in der B' ein Wasserstoffatom oder eine Schutzgruppe für die Aminfunktion bedeutet und $R"_5$ einen Alkylrest mit 1 bis 4 Kohlenstoffatomen darstellt, substituiert durch einen Rest

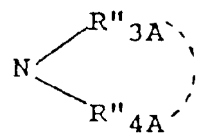

in dem $R"_{3A}$ und $R"_{4A}$ den vorstehend jeweils für $R_{3A}$ und $R_{4A}$ angegebenen Wert besitzen, gegebenenfalls der Einwirkung eines Mittels zur Entfernung der von der Aminfunktion getragenen Schutzgruppe unterzieht und anschließend der Einwirkung eines alkalischen Mittels unterwirft, um durch molekulare Umlagerung ein Produkt der Formel (VII)

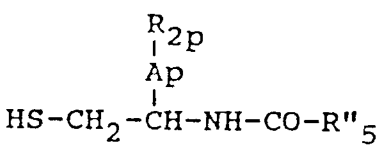

(VII)

zu erhalten, in der $R_{2p}$, Ap und $R"_5$ die vorstehend angegebenen Bedeutungen besitzen, wobei man, wenn erforderlich und wenn gewünscht, das Produkt der Formel (V), (VI) und (VII) durch eine oder mehrere der folgenden Reaktionen, in beliebiger Reihenfolge, behandelt:

- eine Reaktion zur Entfernung der Schutzgruppen, die von den geschützten, reaktiven Funktionen getragen werden können,
- eine Reaktion zur Salzbildung durch eine Mineralsäure oder organische Säure, um das entsprechende Salz zu erhalten,
- eine Reaktion zur Reduktion der veresterten Carboxyfunktion zur Alkoholfunktion,
- eine Reaktion zur Verseifung der Esterfunktion zur Säurefunktion,
- eine Reaktion zur Umwandlung der Cyanofunktion zur Säurefunktion,
- eine Reaktion zur Umwandlung der Alkoxyfunktion zur Hydroxylfunktion,
- eine Reaktion zur Veresterung, Salzbildung oder Carbamoylierung der Säurefunktion,
- eine Reaktion zur Veresterung der Thiolfunktion durch eine Säure oder eine Säurefunktion, oder durch ein Isocyanat oder ein Carbamoylchlorid, wobei die auf diese Weise erhaltenen Produkte der Formel (I) in allen möglichen isomeren, racemischen, enantiomeren und diastereoisomeren Formen vorliegen,

**10.** Als Medikamente die Produkte der Formel ($I_A$) wie in Anspruch 1 definiert, wobei die genannten Produkte der Formel ($I_A$) in allen möglichen isomeren, racemischen, enantiomeren und diastereoisomeren Formen vorliegen, sowie die Additionssalze der genannten Produkte der Formel ($I_A$) mit pharmazeutisch akzeptablen Mineralsäuren und organischen Säuren.

**11.** Als Medikamente die Produkte der Formel ($I_A$) wie in Anspruch 2 bis 8 definiert, sowie die Additionssalze der genannten Produkte der Formel ($I_A$) mit pharmazeutisch akzeptablen Mineralsäuren und organischen Säuren.

**12.** Pharmazeutische Zusammensetzungen, die als Wirkstoff mindestens eines der Medikamente enthalten, wie in irgendeinem der Ansprüche 11 und 12 definiert.

**13.** Verfahren zur Herstellung der Ausgangsprodukte der Formel (II), wie in Anspruch 9 definiert, dadurch gekennzeichnet, daß man eine Verbindung der Formel (IIa)

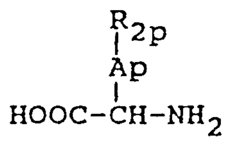

(IIa)

in der $R_{2p}$ und A die in Anspruch 1 jeweils für $R_2$ und A angegebenen Bedeutungen besitzen und in denen die-eventuellen reaktiven Funktionen gegebenenfalls durch Schutzgruppen geschützt sind, der Einwirkung eines Reduktionsmittels für die Säurefunktion unterzieht, um den entsprechenden Alkohol der Formel (IIb)

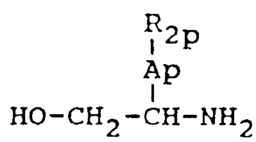

(IIb)

zu erhalten, in der $R_{2p}$ und Ap die vorstehend angegebenen Bedeutungen besitzen, den man durch Einwirkung eines Carbonyl-Derivates cyclisiert, um das entsprechende Oxazolidon der Formel (IIc)

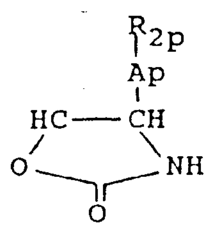

(IIc)

zu erhalten, in der $R_{2p}$ und Ap die vorstehend angegebenen Bedeutungen besitzen, das man der Einwirkung eines Schutzmittels unterzieht, um ein Produkt der Formel (IId)

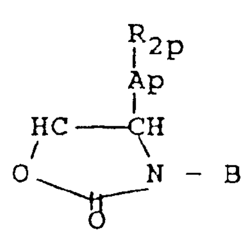

(IId)

zu erhalten, in der $R_{2p}$ und Ap die vorstehend angegebenen Bedeutungen besitzen und B eine Schutzgruppe für die Aminfunktion darstellt, das man mit einer Verbindung der Formel (IIe)

$$R_{5p} - CO - SH \qquad (IIe)$$

zur Reaktion bringt, in der $R_{5p}$ die vorstehend für $R_5$ angegebene Bedeutung besitzt und in der die eventuellen reaktiven Funktionen gegebenenfalls durch Schutzgruppen geschützt sind, um eine Verbindung der Formel (IIf)

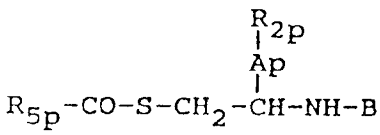

(IIf)

zu erhalten, in der $R_{2p}$, $R_{5p}$, Ap und B die vorstehend angegebenen Bedeutungen besitzen, die man, wenn gewünscht, einer Reaktion zur Entfernung von B unterzieht, wobei die auf diese Weise erhaltenen Produkte der Formel (II) in allen möglichen isomeren, racemischen, enantiomeren und diastereoisomeren Formen vorliegen,

**14.** Als neue industrielle Produkte die Verbindungen der Formel (III).

**Patentansprüche für folgenden Vertragsstaat : ES**

**1.** Verfahren zur Herstellung von Produkten der Formel ($I_A$)

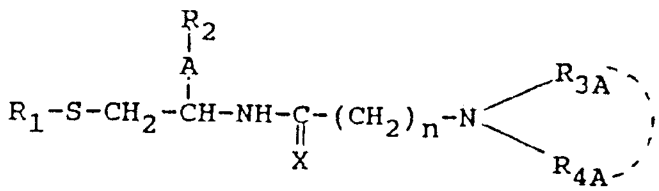

($I_A$)

in der
n die Werte 0 bis 4 besitzt,
$R_1$ ein Wasserstoffatom oder einen Rest $R_5$-CO- darstellt, worin $R_5$ bedeutet:

- einen linearen oder verzweigten Alkyl-, Alkenyl- oder Alkinylrest mit höchstens 6 Kohlenstoffatomen und gegebenenfalls substituiert,
- einen monocyclischen Rest mit 5 bis 7 Ringgliedern oder einen Rest, der aus kondensierten Ringen gebildet wird, die 8 bis 10 Ringglieder umfassen, wobei diese Reste gesättigt oder ungesättigt sind und gegebenenfalls ein oder mehrere, gleiche oder verschiedene Heteroatome umfassen, ausgewählt unter den Atomen von Sauerstoff, Stickstoff und Schwefel, und die gegebenenfalls substituiert sind
- einen Rest

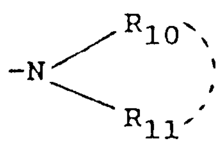

worin $R_{10}$ und $R_{11}$ bedeuten:

entweder bilden $R_{10}$ und $R_{11}$ mit dem Stickstoffatom, an das sie gebunden sind, einen monocyclischen Rest mit 5 bis 7 Ringgliedern oder einen Rest, der aus kondensierten Ringen gebildet wird, die 8 bis 10 Ringglieder umfassen, wobei diese Reste gegebenenfalls ein anderes oder mehrere andere, gleiche oder verschiedene Heteroatome umfassen, ausgewählt unter den Atomen von Sauerstoff, Stickstoff und Schwefel, und die gegebenenfalls substituiert sind,

oder $R_{10}$ und $R_{11}$, gleich oder verschieden, bedeuten:

- ein Wasserstoffatom;
- einen linearen oder verzweigten Alkyl-, Alkenyl- oder Alkinylrest mit höchstens 6 Kohlenstoffatomen und gegebenenfalls substituiert;
- einen Arylrest, in dem der Arylrest einen monocyclischen Rest mit 5 bis 7 Ringgliedern darstellt oder einen Rest, der aus kondensierten Ringen gebildet wird, die 8 bis 10 Ringglieder umfassen, wobei diese Reste gegebenenfalls ein oder mehrere, gleiche oder verschiedene Heteroatome umfassen, ausgewählt unter den Atomen von Sauerstoff, Stickstoff und Schwefel, und die gegebenenfalls substituiert sind;

A bedeutet:

- entweder einen linearen oder verzweigten Alkylen- oder Alkenylenrest mit höchstens 6 Kohlenstoffatomen und gegebenenfalls durch einen Hydroxylrest oder einen Alkoxyrest mit höchstens 6 Kohlenstoffatomen substituiert,
- oder eine einfache Bindung.

$R_2$ einen monocyclischen Rest mit 5 bis 7 Ringgliedern darstellt oder einen Rest, der aus kondensierten Ringen gebildet wird, die 8 bis 10 Ringglieder umfassen, wobei diese Reste gesättigt oder ungesättigt sind und gegebenenfalls ein oder mehrere, gleiche oder verschiedene Heteroatome umfassen, ausgewählt unter den Atomen von Sauerstoff, Stickstoff und Schwefel, und die gegebenenfalls substituiert sind;

X ein Sauerstoff- oder Schwefelatom bedeutet,

$R_{3A}$ und $R_{4A}$ bedeuten:

entweder bilden $R_{3A}$ und $R_{4A}$ mit dem Stickstoffatom, an das sie gebunden sind, einen monocyclischen Rest mit 5 bis 7 Ringgliedern oder einen Rest, der aus kondensierten Ringen gebildet wird, die 8 bis 10 Ringglieder umfassen, wobei diese Reste gegebenenfalls ein anderes oder mehrere andere, gleiche oder verschiedene Heteroatome umfassen, ausgewählt unter den Atomen von Sauerstoff, Stickstoff und Schwefel, und die gegebenenfalls substituiert sind,

oder $R_{3A}$ und $R_{4A}$, gleich oder verschieden, bedeuten:

- ein Wasserstoffatom;
- einen Hydroxylrest, Alkoxy-, Acyl- oder Acyloxyrest mit höchstens 6 Kohlenstoffatomen; Carboxy, in Salz überführt oder verestert; Cyano;
- einen linearen oder verzweigten Alkyl-, Alkenyl- oder Alkinylrest mit höchstens 6 Kohlenstoffatomen und gegebenenfalls substituiert;
- einen Rest $R_c$ oder $R_c$-O- worin der Rest $R_c$ einen aliphatischen oder Arylrest darstellt oder einen monocyclischen Rest mit 5 bis 7 Ringgliedern oder einen Rest, der aus kondensierten Ringen gebildet wird, die 8 bis 10 Ringglieder umfassen, wobei diese Reste gegebenenfalls ein oder mehrere, gleiche oder verschiedene Heteroatome umfassen, ausgewählt unter den Atomen von Sauerstoff, Stickstoff und Schwefel, und die gegebenenfalls substituiert sind;
- den Rest

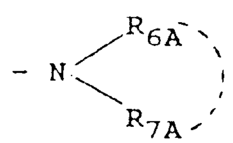 oder 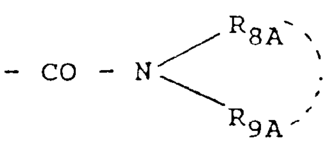

worin $R_{6A}$, $R_{7A}$, $R_{6A}$ und $R_{9A}$, gleich oder verschieden, ein Wasserstoffatom, einen der Reste Hydroxyl, Alkyl, Alkoxy, Acyloxy oder Acyl, wobei diese Reste höchstens 6 Kohlenstoffatome umfassen, freies Carboxy, in Salz überführt oder verestert, Phenoxy, Phenyl, Benzyl, Phenethyl, Azepin, Piperidyl, Morpholin, Pyrrolidinyl oder Piperazinyl bedeuten, gegebenenfalls substituiert an dem zweiten Stickstoffatom durch einen Hydroxyl-, Alkyl-, Alkoxy- oder Phenylrest, wobei diese drei letzten Reste gegebenenfalls selbst durch ein oder mehrere Reste substituiert sind, ausgewählt unter den Halogenatomen und den Resten Hydroxyl, Trifluormethyl, Methoxy, Ethoxy, Methyl und Ethyl, oder einerseits $R_{6A}$ und $R_{7A}$ und andererseits $R_{8A}$ und $R_{9A}$ jeweils mit dem Stickstoffatom, an das sie gebunden sind, einen der Reste Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepinyl oder Indolyl bilden, wobei diese Reste gegebenenfalls substituiert sind durch ein oder mehrere, gleiche oder verschiedene Reste, ausgewählt unter den Halogenatomen und den Resten Hydroxyl, Trifluormethyl, Alkyl und Alkoxy, wobei diese Reste höchstens 6 Kohlenstoffatome umfassen,

wobei die genannten Produkte der Formel ($I_A$) in allen möglichen isomeren, racemischen, enantiomeren und diastereoisomeren Formen vorliegen,

sowie den Additionssalzen der genannten Produkte der Formel ($I_A$) mit Mineralsäuren und organischen Säuren, dadurch gekennzeichnet, daß man

- zur Herstellung der Produkte der Formel ($I_A$), in der n = 0 ist, eine Verbindung der Formel (II)

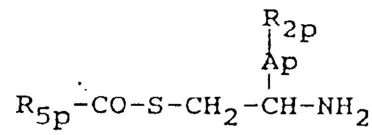

(II)

in der $R_{5p}$, $R_{2p}$ und Ap die vorstehend jeweils für $R_5$, $R_2$ und A angegebenen Bedeutungen besitzen und in denen die eventuellen reaktiven Funktionen gegebenenfalls durch Schutzgruppen geschützt sind, der Einwirkung eines Reaktanden, wie Phosgen oder Thiophosgen unterzieht, um jeweils das entsprechende Isocyanat oder Isothiocyanat der Formel (III)

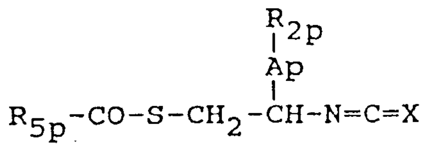

(III)

zu erhalten, in der $R_{5p}$, $R_{2p}$ und Ap die vorstehend angegebenen Bedeutungen besitzen und X ein Sauerstoffatom oder ein Schwefelatom darstellt, das man mit einer Verbindung der Formel (IV)

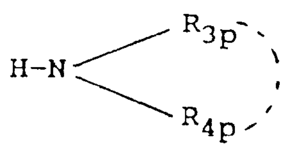

(IV)

zur Reaktion bringt, in der $R_{3p}$ und $R_{4p}$ die vorstehend jeweils für $R_3$ und $R_4$ angegebenen Bedeutungen besitzen und in denen die eventuellen reaktiven Funktionen gegebenenfalls durch Schutzgruppen geschützt sind, um ein Produkt der Formel (V)

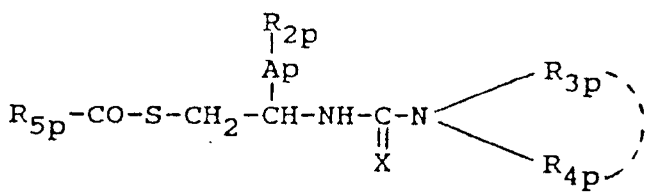

(V)

zu erhalten, in der $R_{5p}$, $R_{2p}$, Ap, $R_{3p}$, $R_{4p}$ und X die vorstehend angegebenen Bedeutungen besitzen, das man, wenn erforderlich und wenn gewünscht, einer Reaktion zur Entfernung des Restes $R_{5p}$-CO- unterzieht, um ein Produkt der Formel (VI)

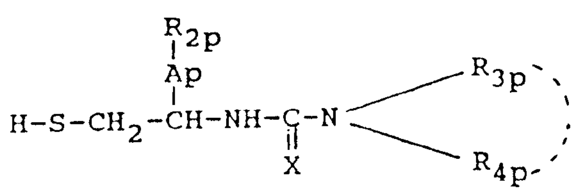

(VI)

zu erhalten, in der $R_{2p}$, Ap, $R_{3p}$, $R_{4p}$ und X die vorstehend angegebenen Bedeutungen besitzen,

- zur Herstellung der Produkte der Formel ($I_A$), in der n von 0 verschieden ist und X ein Sauerstoffatom darstellt, eine Verbindung der Formel (II')

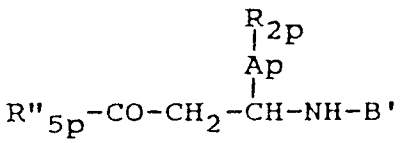

(II')

in der B' ein Wasserstoffatom oder eine Schutzgruppe für die Aminfunktion bedeutet und $R''_5$ einen Alkylrest mit 1 bis 4 Kohlenstoffatomen darstellt, substituiert durch einen Rest

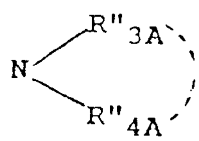

in dem $R''_{3A}$ und $R''_{4A}$ den vorstehend jeweils für $R_{3A}$ und $R_{4A}$ angegebenen Wert besitzen, gegebenenfalls der Einwirkung eines Mittels zur Entfernung der von der Aminfunktion getragenen Schutzgruppe unterzieht und anschließend der Einwirkung eines alkalischen Mittels unterwirft, um durch molekulare Umlagerung ein Produkt der Formel (VII)

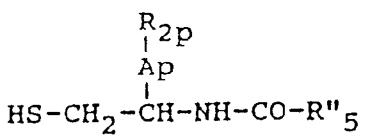

(VII)

zu erhalten, in der $R_{2p}$, Ap und $R''_5$ die vorstehend angegebenen Bedeutungen besitzen, wobei man, wenn erforderlich und wenn gewünscht, das Produkt der Formel (V), (VI) und (VII) durch eine oder mehrere der folgenden Reaktionen, in beliebiger Reihenfolge, behandelt:

- eine Reaktion zur Entfernung der Schutzgruppen, die von den geschützten, reaktiven Funktionen getragen werden können,
- eine Reaktion zur Salzbildung durch eine Mineralsäure oder organische Säure, um das entsprechende Salz zu erhalten,
- eine Reaktion zur Reduktion der veresterten Carboxyfunktion zur Alkoholfunktion,
- eine Reaktion zur Verseifung der Esterfunktion zur Säurefunktion,
- eine Reaktion zur Umwandlung der Cyanofunktion zur Säurefunktion,
- eine Reaktion zur Umwandlung der Alkoxyfunktion zur Hydroxylfunktion,
- eine Reaktion zur Veresterung, Salzbildung oder Carbamoylierung der Säurefunktion,
- eine Reaktion zur Veresterung der Thiolfunktion durch eine Säure oder eine Säurefunktion, oder durch ein Isocyanat oder ein Carbamoylchlorid, wobei die auf diese Weise erhaltenen Produkte der Formel (I) in allen möglichen isomeren, racemischen, enantiomeren und diastereoisomeren Formen vorliegen,

2. Verfahren nach Anspruch 1, dadurch gekennzeichnet, daß der oder die Substituenten, gleich oder verschieden, die tragen können:

   a) die Alkyl-, Alkenyl- oder Alkinylreste,

   b) die monocyclischen Reste oder die aus kondensierten Ringen sowie aus Aryl und Aryloxy gebildeten Reste,

   ausgewählt werden aus der Gruppe, gebildet durch:

   - die Halogenatome; die Hydroxylreste; Cyano; Mercapto; Nitro; Acyl und Acyloxy mit höchstens 6 Kohlenstoffatomen; freies Carboxy, in Salz überführt oder verestert; Alkoxycarbonyl;
   - die linearen und verzweigten Alkoxyreste mit höchstens 6 Kohlenstoffatomen;
   - die Reste Alkyl, Alkenyl, Alkinyl, Alkylthio, Cycloalkyl, Cycloalkenyl, die heterocyclischen Kohlenwasserstoffreste, Aryl, Aryloxy, Arylthio, Phenylalkyl, Phenylalkoxy, die gegebenenfalls selbst durch ein oder mehrere, gleiche oder verschiedene Substituenten substituiert sind, ausgewählt unter den Halogenatomen, den Resten Hydroxyl, Trifluormethyl, Nitro, Alkyl, Alkenyl, Aryl, Alkoxy und Acyl, wobei diese Reste höchstens 6 Kohlenstoffatome umfassen, freies Carboxy, in Salz überführt oder verestert, Acylamido, worin der Acylrest höchstens 6 Kohlenstoffatome umfaßt, Carbamoyl und Amino,

wobei diese Reste gegebenenfalls am Stickstoffatom durch ein oder zwei gleiche oder verschiedene Reste substituiert sind, ausgewählt unter den Resten Hydroxyl, Alkyl, und Alkoxy, die höchstens 6 Kohlenstoffatome umfassen;

- den Acylamidorest, worin der Acylrest höchstens 6 Kohlenstoffatome umfaßt; die Reste Carbamoyl und Amino, die gegebenenfalls am Stickstoffatom durch ein oder zwei gleiche oder verschiedene Reste substituiert sind, ausgewählt unter den Resten Hydroxyl, Alkyl, Alkenyl und Alkoxy, die höchstens 6 Kohlenstoffatome umfassen.

3. Verfahren nach Anspruch 1 oder 2, dadurch gekennzeichnet, daß man die Ausgangsprodukte der Formel (II) und (IV) verwendet, die in der Weise ausgewählt werden, daß man ein Produkt herstellt, das der Formel ($I'_A$) entspricht

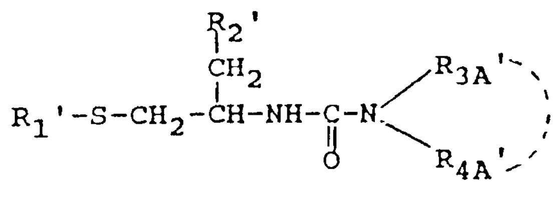

($I'_A$)

in der

$R_{1A}'$ ein Wasserstoffatom oder einen Rest $R_{5A}'$-CO- darstellt, worin $R_{5A}'$ einen Phenyl-, Cycloalkyl-, Cycloalkenyl-, Alkyl- oder Alkenylrest mit höchstens 6 Kohlenstoffatomen bedeutet, wobei diese Reste gegebenenfalls substituiert sind, oder

- einen Rest

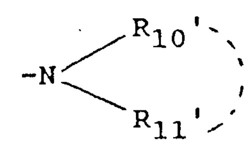

worin $R_{10}'$ und $R_{11}'$ bedeuten:

entweder bilden $R_{10}'$ und $R_{11}'$ mit dem Stickstoffatom, an das sie gebunden sind, einen der Reste Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Methylpiperazinyl, Piperidyl, Oxazolyl, Morpholinyl und Thiomorpholinyl, Azepin oder Indolyl, wobei diese Reste gegebenenfalls substituiert sind durch ein oder mehrere, gleiche oder verschiedene Reste, ausgewählt unter den Halogenatomen und den Resten Hydroxyl, Mercapto, Trifluormethyl, Alkyl, Alkenyl, Alkinyl, Alkylthio, Alkoxy und Phenyl, gegebenenfalls substituiert durch einen oder mehrere Reste, ausgewählt unter den Halogenatomen und den Resten Trifluormethyl, Hydroxyl, Alkyl und Alkoxy, wobei die Alkyl- und Alkoxyreste höchstens 6 Kohlenstoffatome umfassen,

oder $R_{10}'$ und $R_{11}'$, gleich oder verschieden, bedeuten:

- ein Wasserstoffatom;
- einen linearen oder verzweigten Alkyl-, Alkenyl- oder Alkinylrest mit höchstens 6 Kohlenstoffatomen und gegebenenfalls substituiert;
- einen der Reste Phenyl, Benzyl, Naphthyl, Indenyl, Imidazolyl, Pyrrolyl, Pyrrolinyl Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepinyl oder Indolyl, wobei diese Reste gegebenenfalls substituiert sind durch ein oder mehrere, gleiche oder verschiedene Reste, ausgewählt unter den Halogenatomen und den Resten Hydroxyl, Mercapto, Trifluormethyl, Alkyl, Alkenyl, Alkinyl, Alkylthio und Alkoxy, die höchstens 6 Kohlenstoffatome umfassen,

$R_2'$ einen der Reste Phenyl, Naphthyl, Benzyl, Phenethyl, Indolyl, Cycloalkyl oder Cycloalkenyl darstellt, wobei diese Reste gegebenenfalls substituiert sind;

$R_{3A}'$ und $R_{4A}'$ bedeuten:

entweder bilden $R_{3A}'$ und $R_{4A}'$ mit dem Stickstoffatom, an das sie gebunden sind, einen der Reste Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepinyl oder Indolyl, wobei diese Reste gegebenenfalls substituiert sind durch ein oder mehrere, gleiche oder verschiedene Reste, ausgewählt unter den Halogenatomen und den Resten Hydroxyl, Trifluormethyl, Alkyl, Alkenyl, Alkinyl, Alkoxy und Phenyl, gegebenenfalls substituiert durch einen oder mehrere Reste, ausgewählt unter den Halogenatomen und den Resten Trifluormethyl, Hydroxyl, Alkyl und Alkoxy, wobei die Reste Alkyl und Alkoxy höchstens 6 Kohlenstoffatome umfassen und gegebenenfalls durch einen Phenylrest substituiert sind, oder $R_{3A}$' und $R_{4A}$', gleich oder verschieden, bedeuten:

- ein Wasserstoffatom:
- einen Hydroxylrest, Alkoxy-, Acyloxy- oder Acylrest mit höchstens 6 Kohlenstoffatomen; Carboxy, in Salz überführt oder verestert; Cyano;
- einen linearen oder verzweigten Alkyl-, Alkenyl- oder Alkinylrest mit höchstens 6 Kohlenstoffatomen und gegebenenfalls substituiert;
- einen der Reste Phenyl, Benzoyl, Naphthyl, Indenyl, Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepinyl, Diazepin, Benzodiazepin oder Indolyl, wobei diese Reste gegebenenfalls substituiert sind durch ein oder mehrere, gleiche oder verschiedene Reste, ausgewählt unter den Halogenatomen und den Resten Hydroxyl, Trifluormethyl, Alkyl und Alkoxy, die höchstens 6 Kohlenstoffatome umfassen und gegebenenfalls selbst substituiert sind durch einen oder mehrere Reste, ausgewählt unter den Halogenatomen und den Resten Hydroxyl, Alkyl, Alkoxy und Carboxy, frei, in ein Salz überführt oder verestert durch einen Alkyl-, Aryl- oder Arylalkylrest,
- den Rest

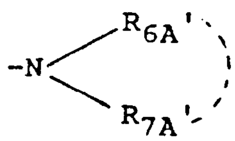 oder 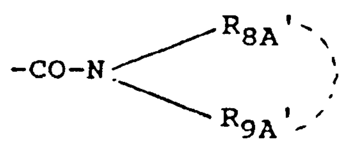

worin $R_{6A}$', $R_{7A}$', $R_{8A}$' und $R_{9A}$', gleich oder verschieden, ein Wasserstoffatom, einen der Reste Hydroxyl, Alkyl, Alkoxy, Acyloxy oder Acyl, wobei diese Reste höchstens 6 Kohlenstoffatome umfassen, freies Carboxy, in Salz überführt oder verestert, Phenyl, Benzyl, Phenethyl, Azepinyl, Piperidyl, Morpholin, Pyrrolidinyl oder Piperazinyl bedeuten,

oder einerseits $R_{6A}$' und $R_{7A}$' und andererseits $R_{8A}$' und $R_{9A}$' jeweils mit dem Stickstoffatom, an das sie gebunden sind, einen der Reste Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepinyl oder Indolyl bilden, wobei diese Reste gegebenenfalls substituiert sind durch ein oder mehrere, gleiche oder verschiedene Reste, ausgewählt unter den Halogenatomen und den Resten Hydroxyl, Trifluormethyl, Alkyl und Alkoxy, wobei diese Reste höchstens 6 Kohlenstoffatome umfassen, mit der Maßgabe, daß der oder die eventuellen Substituenten, gleich oder verschieden, die die Reste tragen können, die $R_1$', $R_2$' und $R_6$' darstellen, ausgewählt werden aus der Gruppe, die gebildet wird aus:

- den Halogenatomen; den Hydroxylresten; Cyano; Nitro; Acyl und Acyloxy mit höchstens 6 Kohlenstoffatomen;Benzoyl,freies Carboxy,in Salz überführt oder verestert;
- den linearen und verzweigten Alkoxyresten mit höchstens 6 Kohlenstoffatomen;
- den Resten Alkyl, Alkenyl, Cyclopentyl, Cyclohexyl, Cyclopentenyl, Cyclohexenyl, Phenyl, Naphthyl, Indolyl, Phenoxy, Benzyl, Phenethyl und Benzyloxy, die gegebenenfalls selbst durch einen oder mehrere gleiche oder verschiedene Substituenten substituiert sind, ausgewählt unter den Halogenatomen und den Resten Hydroxyl, Trifluormethyl, Nitro, Alkyl, Alkenyl und Alkoxy, wobei diese Reste höchstens 6 Kohlenstoffatome umfassen, Formyl, Acetyl, Benzoyl, freies Carboxy, in Salz überführt oder verestert, Acetamido, Benzoylamido, Carbamoyl und Amino, wobei diese Reste gegebenenfalls am Stickstoffatom durch ein oder zwei gleiche oder verschiedene Reste substituiert sind, ausgewählt unter den Resten Hydroxyl, Methyl, Ethyl, Propyl, Methoxy, Ethoxy und Propoxy;
- dem Acylamidorest, worin der Acylrest höchstens 6 Kohlenstoffatome umfaßt; den Resten Carbamoyl und Amino, die gegebenenfalls am Stickstoffatom durch ein oder zwei gleiche oder verschiedene Reste substituiert sind, ausgewählt unter den Resten Hydroxyl, Alkyl, Alkenyl, Alkoxy und Acyl, die höchstens 6 Kohlenstoffatome umfassen;

wobei die genannten Produkte der Formel ($I_A$') in allen möglichen isomeren, racemischen, enantiomeren und diastereoisomeren Formen vorliegen, sowie die Additionssalze der genannten Produkte der Formel ($I_A$') mit Mineralsäuren und organischen Säuren.

4. Verfahren nach irgendeinem der Ansprüche 1 bis 3, dadurch gekennzeichnet, daß man als Ausgangsprodukte ein Produkt der Formel (II) verwendet, in der $R_{5p}$ einen Methylrest darstellt und $R_{2p}$ einen Phenylrest bedeutet, und ein Produkt der Formel (IV), in der $R_{3p}$ und $R_{4p}$ bedeuten:
entweder bilden $R_{3p}$ und $R_{4p}$ mit dem Stickstoffatom, an das sie gebunden sind, einen der Reste Piperidinyl, Morpholinyl, Dimethylmorpholinyl, Thiomorpholinyl, Pyrrolidinyl, Azepin oder Diazepin, gegebenenfalls substituiert am zweiten Stickstoffatom durch einen Alkylrest mit 1 oder 2 Kohlenstoffatomen, Piperazinyl, gegebenenfalls substituiert am zweiten Stickstoffatom entweder durch einen Phenylrest, gegebenenfalls substituiert durch ein Halogenatom oder einen Trifluormethylrest oder durch einen Alkyl-, Alkenyl- oder Alkinylrest mit 1 bis 4 Kohlenstoffatomen, gegebenenfalls substituiert durch einen Phenyl- oder Acylrest, oder durch einen Acylrest mit höchstens 6 Kohlenstoffatomen,
oder $R_{3p}$ und $R_{4p}$, gleich oder verschieden, bedeuten:
ein Wasserstoffatom; einen Hydroxylrest, Phenyl; Phenyl, substituiert durch einen Carboxyrest; Piperidinyl, gegebenenfalls substituiert am Stickstoffatom durch einen Benzylrest, Piperazinyl, gegebenenfalls substituiert am zweiten Stickstoffatom durch einen Alkylrest mit 1 oder 2 Kohlenstoffatomen, Carbamoyl; Alkoxy mit höchstens 4 Kohlenstoffatomen, Alkyl mit höchstens 4 Kohlenstoffatomen, gegebenenfalls substituiert durch einen oder mehrere gleiche oder verschiedene Reste, ausgewählt unter den Resten Hydroxyl, Alkoxy, freies Carboxy, in Salz überführt oder verestert, Amino, Alkylamino, Dialkylamino, Phenyl, Morpholino, Alkylthio mit höchstens 4 Kohlenstoffatomen, gegebenenfalls substituiert durch einen freien Carboxyrest, in Salz überführt oder verestert, Benzodiazepin, gegebenenfalls substituiert, und in denen die reaktiven Funktionen gegebenenfalls geschützt sind.

5. Verfahren nach irgendeinem der Ansprüche 1 bis 4, dadurch gekennzeichnet, daß man die Ausgangsprodukte der Formel (II) und (IV) in der Weise auswählt, daß man ein Produkt der folgenden Formeln herstellt:
(±)4-Methyl-N-[2-mercapto-1-(phenylmethyl)-ethyl]-1-piperazin-carboxamid- Hydrochlorid,
(±)N-[2-Mercapto-1-(phenylmethyl)-ethyl]-4-morpholin-carboxamid,
(±)N-[2-Mercapto-1-(phenylmethyl)-ethyl]-1-pyrrolidin-carboxamid,
(±)N-[[[2-Mercapto-1-(phenylmethyl)-ethyl]-amino]-carbonyl]-glycin,
N,N-bis(2-Methoxy-ethyl)-N'-[2-mercapto-1-(phenylmethyl)-ethyl]-harnstoff,
(±)cis-2,6-Dimethyl-N-[2-mercapto-1-(phenylmethyl)-ethyl]-4-morpholin- carboxamid (Isomer A),
(±)N-[2-Mercapto-1-(phenylmethyl)-ethyl]-4-thiomorpholin-carboxamid,
(±)N-[2-Mercapto-1-(phenylmethyl)-ethyl]-N'-methoxy-harnstoff,
(±)N-Hydroxy-N'-[2-mercapto-1-(phenylmethyl)-ethyl]-N'-methyl-harnstoff,
(±)N-(1-Mercapto-methyl)-2-(phenylethyl)-4-methyl-1-piperazin-acetamid,
sowie ihre Salze mit Mineralsäuren oder organischen Säuren.

6. Verfahren zur Herstellung der Ausgangsprodukte der Formel (II), wie in Anspruch 1 definiert, dadurch gekennzeichnet, daß man eine Verbindung der Formel (IIa)

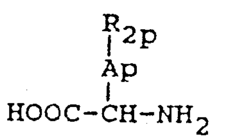

(IIa)

in der $R_{2p}$ und A die in Anspruch 1 jeweils für $R_2$ und A angegebenen Bedeutungen besitzen und in denen die eventuellen reaktiven Funktionen gegebenenfalls durch Schutzgruppen geschützt sind, der Einwirkung eines Reduktionsmittels für die Säurefunktion unterzieht, um den entsprechenden Alkohol der Formel (IIb)

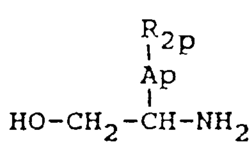

(IIb)

zu erhalten, in der $R_{2p}$ und Ap die vorstehend angegebenen Bedeutungen besitzen, den man durch Einwirkung eines Carbonyl-Derivates cyclisiert, um das entsprechende Oxazolidon der Formel (IIc)

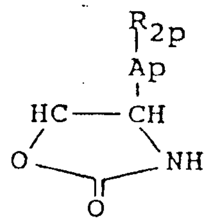

(IIc)

zu erhalten, in der $R_{2p}$ und Ap die vorstehend angegebenen Bedeutungen besitzen, das man der Einwirkung eines Schutzmittels unterzieht, um ein Produkt der Formel (IId)

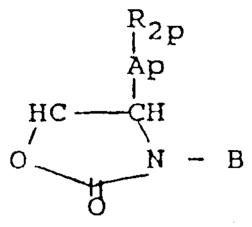

(IId)

zu erhalten, in der $R_{2p}$ und Ap die vorstehend angegebenen Bedeutungen besitzen und B eine Schutzgruppe für die Aminfunktion darstellt, das man mit einer Verbindung der Formel (IIe)

$$R_{5p} - CO - SH \qquad (IIe)$$

zur Reaktion bringt, in der $R_{5p}$ die vorstehend für $R_5$ angegebene Bedeutung besitzt und in der die eventuellen reaktiven Funktionen gegebenenfalls durch Schutzgruppen geschützt sind, um eine Verbindung der Formel (IIf)

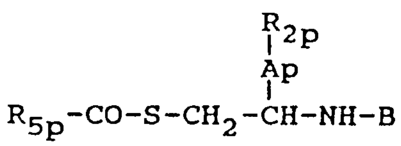

(IIf)

zu erhalten, in der $R_{2p}$, $R_{5p}$, Ap und B die vorstehend angegebenen Bedeutungen besitzen, die man, wenn gewünscht, einer Reaktion zur Entfernung von B unterzieht, wobei die auf diese Weise erhaltenen Produkte der Formel (II) in allen möglichen isomeren, racemischen, enantiomeren und diastereoisomeren Formen vorliegen,

**Patentansprüche für folgenden Vertragsstaat : GR**

1. Verfahren zur Herstellung von Produkten der Formel ($I_A$)

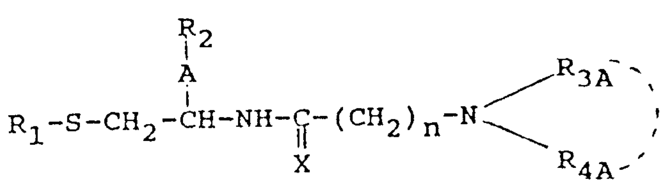

($I_A$)

in der
n die Werte 0 bis 4 besitzt,
$R_1$ ein Wasserstoffatom oder einen Rest $R_5$-CO- darstellt, worin $R_5$ bedeutet:

- einen linearen oder verzweigten Alkyl-, Alkenyl- oder Alkinylrest mit höchstens 6 Kohlenstoffatomen und gegebenenfalls substituiert,
- einen monocyclischen Rest mit 5 bis 7 Ringgliedern oder einen Rest, der aus kondensierten Ringen gebildet wird, die 8 bis 10 Ringglieder umfassen, wobei diese Reste gesättigt oder ungesättigt sind und gegebenenfalls ein oder mehrere, gleiche oder verschiedene Heteroatome umfassen, ausgewählt unter den Atomen von Sauerstoff, Stickstoff und Schwefel, und die gegebenenfalls substituiert sind,

- einen Rest

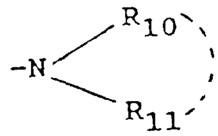

worin $R_{10}$ und $R_{11}$ bedeuten:

entweder bilden $R_{10}$ und $R_{11}$ mit dem Stickstoffatom, an das sie gebunden sind, einen monocyclischen Rest mit 5 bis 7 Ringgliedern oder einen Rest, der aus kondensierten Ringen gebildet wird, die 8 bis 10 Ringglieder umfassen, wobei diese Reste gegebenenfalls ein anderes oder mehrere andere, gleiche oder verschiedene Heteroatome umfassen, ausgewählt unter den Atomen von Sauerstoff, Stickstoff und Schwefel, und die gegebenenfalls substituiert sind,

oder $R_{10}$ und $R_{11}$, gleich oder verschieden, bedeuten:

- ein Wasserstoffatom;
- einen linearen oder verzweigten Alkyl-, Alkenyl- oder Alkinylrest mit höchstens 6 Kohlenstoffatomen und gegebenenfalls substituiert;
- einen Arylrest, in dem der Arylrest einen monocyclischen Rest mit 5 bis 7 Ringgliedern darstellt oder einen Rest, der aus kondensierten Ringen gebildet wird, die 8 bis 10 Ringglieder umfassen, wobei diese Reste gegebenenfalls ein oder mehrere, gleiche oder verschiedene Heteroatome umfassen, ausgewählt unter den Atomen von Sauerstoff, Stickstoff und Schwefel, und die gegebenenfalls substituiert sind;

A bedeutet:

- entweder einen linearen oder verzweigten Alkylen- oder Alkenylenrest mit höchstens 6 Kohlenstoffatomen und gegebenenfalls durch einen Hydroxylrest oder einen Alkoxyrest mit höchstens 6 Kohlenstoffatomen substituiert,
- oder eine einfache Bindung,

$R_2$ einen monocyclischen Rest mit 5 bis 7 Ringgliedern darstellt oder einen Rest, der aus kondensierten Ringen gebildet wird, die 8 bis 10 Ringglieder umfassen, wobei diese Reste gesättigt oder ungesättigt sind und gegebenenfalls ein oder mehrere, gleiche oder verschiedene Heteroatome umfassen, ausgewählt unter den Atomen von Sauerstoff, Stickstoff und Schwefel, und die gegebenenfalls substituiert sind;

X ein Sauerstoff- oder Schwefelatom bedeutet,

$R_{3A}$ und $R_{4A}$ bedeuten:

entweder bilden $R_{3A}$ und $R_{4A}$ mit dem Stickstoffatom, an das sie gebunden sind, einen monocyclischen Rest mit 5 bis 7 Ringgliedern oder einen Rest, der aus kondensierten Ringen gebildet wird, die 8 bis 10 Ringglieder umfassen, wobei diese Reste gegebenenfalls ein anderes oder mehrere andere, gleiche oder verschiedene Heteroatome umfassen, ausgewählt unter den Atomen von Sauerstoff, Stickstoff und Schwefel, und die gegebenenfalls substituiert sind,

oder $R_{3A}$ und $R_{4A}$, gleich oder verschieden, bedeuten:

- ein Wasserstoffatom;
- einen Hydroxylrest, Alkoxy-, Acyl- oder Acyloxyrest mit höchstens 6 Kohlenstoffatomen; Carboxy, in Salz überführt oder verestert; Cyano;
- einen linearen oder verzweigten Alkyl-, Alkenyl- oder Alkinylrest mit höchstens 6 Kohlenstoffatomen und gegebenenfalls substituiert;
- einen Rest $R_c$ oder $R_c$-O- worin der Rest $R_c$ einen aliphatischen oder Arylrest darstellt oder-einen monocyclischen Rest mit 5 bis 7 Ringgliedern oder einen Rest, der aus kondensierten Ringen gebildet wird, die 8 bis 10 Ringglieder umfassen, wobei diese Reste gegebenenfalls ein oder mehrere, gleiche oder verschiedene Heteroatome umfassen, ausgewählt unter den Atomen von Sauerstoff, Stickstoff und Schwefel, und die gegebenenfalls substituiert sind;
- den Rest

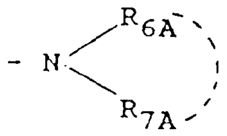 oder 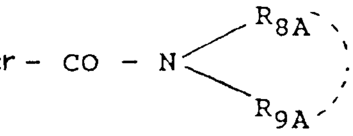

worin $R_{6A}$, $R_{7A}$, $R_{6A}$ und $R_{9A}$, gleich oder verschieden, ein Wasserstoffatom, einen der Reste Hydroxyl, Alkyl, Alkoxy, Acyloxy oder Acyl, wobei diese Reste höchstens 6 Kohlenstoffatome umfassen, freies Carboxy, in Salz überführt oder verestert, Phenoxy, Phenyl, Benzyl, Phenethyl, Azepin, Piperidyl, Morpholin, Pyrrolidinyl oder Piperazinyl bedeuten, gegebenenfalls substituiert an dem zweiten Stickstoffatom durch einen Hydroxyl-, Alkyl-, Alkoxy- oder Phenylrest, wobei diese drei letzten Reste gegebenenfalls selbst durch ein oder mehrere Reste substituiert sind, ausgewählt unter den Halogenatomen und den Resten Hydroxyl, Trifluormethyl, Methoxy, Ethoxy, Methyl und Ethyl, oder einerseits $R_{6A}$ und $R_{7A}$ und andererseits $R_{8A}$ und $R_{9A}$ jeweils mit dem Stickstoffatom, an das sie gebunden sind, einen der Reste Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepinyl oder Indolyl bilden, wobei diese Reste gegebenenfalls substituiert sind durch ein oder mehrere, gleiche oder verschiedene Reste, ausgewählt unter den Halogenatomen und den Resten Hydroxyl, Trifluormethyl, Alkyl und Alkoxy, wobei diese Reste höchstens 6 Kohlenstoffatome umfassen,

wobei die genannten Produkte der Formel ($I_A$) in allen möglichen isomeren, racemischen, enantiomeren und diastereoisomeren Formen vorliegen,

sowie den Additionssalzen der genannten Produkte der Formel ($I_A$) mit Mineralsäuren und organischen Säuren, dadurch gekennzeichnet, daß man

- zur Herstellung der Produkte der Formel ($I_A$), in der n = 0 ist, eine Verbindung der Formel (II)

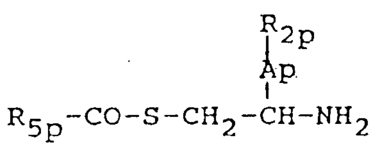 (II)

in der $R_{6p}$, $R_{2p}$ und Ap die vorstehend jeweils für $R_5$, $R_2$ und A angegebenen Bedeutungen besitzen und in denen die eventuellen reaktiven Funktionen gegebenenfalls durch Schutzgruppen geschützt sind, der Einwirkung eines Reaktanden, wie Phosgen oder Thiophosgen unterzieht, um jeweils das entsprechende Isocyanat oder Isothiocyanat der Formel (III)

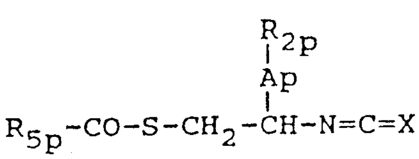 (III)

zu erhalten, in der $R_{6p}$, $R_{2p}$ und Ap die vorstehend angegebenen Bedeutungen besitzen und X ein Sauerstoffatom oder ein Schwefelatom darstellt, das man mit einer Verbindung der Formel (IV)

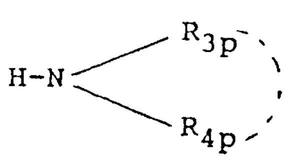 (IV)

zur Reaktion bringt, in der $R_{3p}$ und $R_{4p}$ die vorstehend jeweils für $R_3$ und $R_4$ angegebenen Bedeutungen besitzen und in denen die eventuellen reaktiven Funktionen gegebenenfalls durch Schutzgruppen geschützt sind, um ein Produkt der Formel (V)

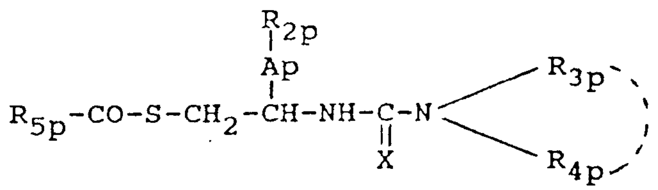

(V)

zu erhalten, in der $R_{5p}$, $R_{2p}$, Ap, $R_{3p}$, $R_{4p}$ und X die vorstehend angegebenen Bedeutungen besitzen, das man, wenn erforderlich und wenn gewünscht, einer Reaktion zur Entfernung des Restes $R_{5p}$-CO- unterzieht, um ein Produkt der Formel (VI)

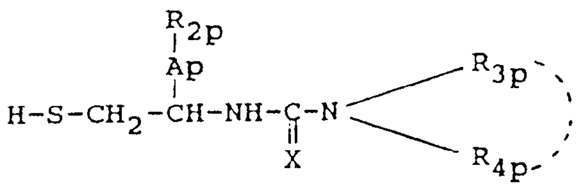

(VI)

zu erhalten, in der $R_{2p}$, Ap, $R_{3p}$, $R_{4p}$ und X die vorstehend angegebenen Bedeutungen besitzen,

- zur Herstellung der Produkte der Formel ($I_A$), in der n von 0 verschieden ist und X ein Sauerstoffatom darstellt, eine Verbindung der Formel (II')

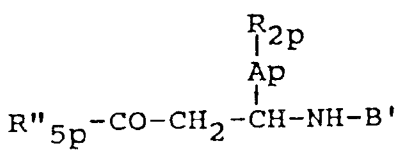

(II')

in der B' ein Wasserstoffatom oder eine Schutzgruppe für die Aminfunktion bedeutet und $R''_5$ einen Alkylrest mit 1 bis 4 Kohlenstoffatomen darstellt, substituiert durch einen Rest

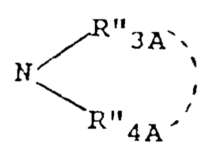

in dem $R''_{3A}$ und $R''_{4A}$ den vorstehend jeweils für $R_{3A}$ und $R_{4A}$ angegebenen Wert besitzen, gegebenenfalls der Einwirkung eines Mittels zur Entfernung der von der Aminfunktion getragenen Schutzgruppe unterzieht und anschließend der Einwirkung eines alkalischen Mittels unterwirft, um durch molekulare Umlagerung ein Produkt der Formel (VII)

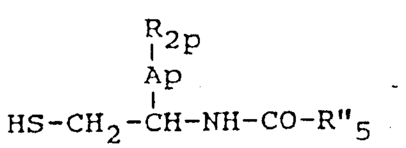

(VII)

zu erhalten, in der $R_{2p}$, Ap und $R''_5$ die vorstehend angegebenen Bedeutungen besitzen, wobei man, wenn erforderlich und wenn gewünscht, das Produkt der Formel (V), (VI) und (VII) durch eine oder mehrere der folgenden Reaktionen, in beliebiger Reihenfolge, behandelt:

- eine Reaktion zur Entfernung der Schutzgruppen, die von den geschützten, reaktiven Funktionen getragen werden können,
- eine Reaktion zur Salzbildung durch eine Mineralsäure oder organische Säure, um das entsprechende Salz zu erhalten,

- eine Reaktion zur Reduktion der veresterten Carboxyfunktion zur Alkoholfunktion,
- eine Reaktion zur Verseifung der Esterfunktion zur Säurefunktion,
- eine Reaktion zur Umwandlung der Cyanofunktion zur Säurefunktion,
- eine Reaktion zur Umwandlung der Alkoxyfunktion zur Hydroxylfunktion,
- eine Reaktion zur Veresterung, Salzbildung oder Carbamoylierung der Säurefunktion,
- eine Reaktion zur Veresterung der Thiolfunktion durch eine Säure oder eine Säurefunktion, oder durch ein Isocyanat oder ein Carbamoylchlorid, wobei die auf diese Weise erhaltenen Produkte der Formel (I) in allen möglichen isomeren, racemischen, enantiomeren und diastereoisomeren Formen vorliegen,

2. Verfahren nach Anspruch 1, dadurch gekennzeichnet, daß der oder die Substituenten, gleich oder verschieden, die tragen können:
   a) die Alkyl-, Alkenyl- oder Alkinylreste,
   b) die monocyclischen Reste oder die aus kondensierten Ringen sowie aus Aryl und Aryloxy gebildeten Reste,

   ausgewählt werden aus der Gruppe, gebildet durch:
   - die Halogenatome; die Hydroxylreste; Cyano; Mercapto; Nitro; Acyl und Acyloxy mit höchstens 6 Kohlenstoffatomen; freies Carboxy, in Salz überführt oder verestert; Alkoxycarbonyl;
   - die linearen und verzweigten Alkoxyreste mit höchstens 6 Kohlenstoffatomen;
   - die Reste Alkyl, Alkenyl, Alkinyl, Alkylthio, Cycloalkyl, Cycloalkenyl, die heterocyclischen Kohlenwasserstoffreste, Aryl, Aryloxy, Arylthio, Phenylalkyl, Phenylalkoxy, die gegebenenfalls selbst durch ein oder mehrere, gleiche oder verschiedene Substituenten substituiert sind, ausgewählt unter den Halogenatomen, den Resten Hydroxyl, Trifluormethyl, Nitro, Alkyl, Alkenyl, Aryl, Alkoxy und Acyl, wobei diese Reste höchstens 6 Kohlenstoffatome umfassen, freies Carboxy, in Salz überführt oder verestert, Acylamido, worin der Acylrest höchstens 6 Kohlenstoffatome umfaßt, Carbamoyl und Amino, wobei diese Reste gegebenenfalls am Stickstoffatom durch ein oder zwei gleiche oder verschiedene Reste substituiert sind, ausgewählt unter den Resten Hydroxyl, Alkyl, und Alkoxy, die höchstens 6 Kohlenstoffatome umfassen;
   - den Acylamidorest, worin der Acylrest höchstens 6 Kohlenstoffatome umfaßt; die Reste Carbamoyl und Amino, die gegebenenfalls am Stickstoffatom durch ein oder zwei gleiche oder verschiedene Reste substituiert sind, ausgewählt unter den Resten Hydroxyl, Alkyl, Alkenyl und Alkoxy, die höchstens 6 Kohlenstoffatome umfassen.

3. Verfahren nach Anspruch 1 oder 2, dadurch gekennzeichnet, daß man die Ausgangsprodukte der Formel (II) und (IV) verwendet, die in der Weise ausgewählt werden, daß man ein Produkt herstellt, das der Formel ($I'_A$) entspricht

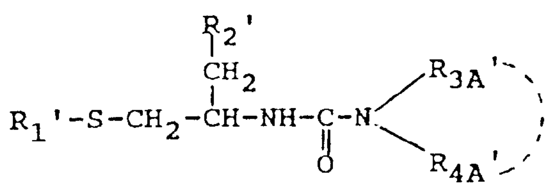

($I'_A$)

in der

$R_{1A}$' ein Wasserstoffatom oder einen Rest $R_{5A}$'-CO- darstellt, worin $R_{5A}$' einen Phenyl -, Cycloalkyl-, Cycloalkenyl-, Alkyl- oder Alkenylrest mit höchstens 6 Kohlenstoffatomen bedeutet, wobei diese Reste gegebenenfalls substituiert sind, oder

- einen Rest

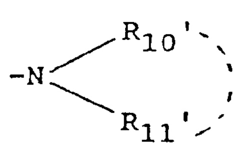

worin $R_{10}$' und $R_{11}$' bedeuten: <u>entweder</u> bilden $R_{10}$' und $R_{11}$' mit dem Stickstoffatom, an das sie gebunden sind, einen der Reste Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Methylpiperazinyl, Piperidyl, Oxazolyl, Morpholinyl und Thiomorpholinyl, Azepin oder Indolyl, wobei diese Reste gegebenenfalls substituiert sind durch ein oder mehrere, gleiche oder verschiedene Reste, ausgewählt unter den Halogenatomen und den Resten Hydroxyl, Mercapto, Trifluormethyl, Alkyl, Alkenyl, Alkinyl, Alkylthio, Alkoxy und Phenyl, gegebenenfalls substituiert durch einen oder mehrere Reste, ausgewählt unter den Halogenatomen und den Resten Trifluormethyl, Hydroxyl, Alkyl und Alkoxy, wobei die Alkyl- und Alkoxyreste höchstens 6 Kohlenstoffatome umfassen,

oder $R_{10}$' und $R_{11}$', gleich oder verschieden, bedeuten:

- ein Wasserstoffatom;
- einen linearen oder verzweigten Alkyl-, Alkenyl- oder Alkinylrest mit höchstens 6 Kohlenstoffatomen und gegebenenfalls substituiert;
- einen der Reste Phenyl, Benzyl, Naphthyl, Indenyl, Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepinyl oder Indolyl, wobei diese Reste gegebenenfalls substituiert sind durch ein oder mehrere, gleiche oder verschiedene Reste, ausgewählt unter den Halogenatomen und den Resten Hydroxyl, Mercapto, Trifluormethyl, Alkyl, Alkenyl, Alkinyl , Alkylthio und Alkoxy, die höchstens 6 Kohlenstoffatome umfassen,

$R_2$' einen der Reste Phenyl, Naphthyl, Benzyl, Phenethyl, Indolyl, Cycloalkyl oder Cycloalkenyl darstellt, wobei diese Reste gegebenenfalls substituiert sind;

$R_{3A}$' und $R_{4A}$' bedeuten:

entweder bilden $R_{3A}$' und $R_{4A}$' mit dem Stickstoffatom, an das sie gebunden sind, einen der Reste Imidazolyl, Pyrrolyl Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepinyl oder Indolyl, wobei diese Reste gegebenenfalls substituiert sind durch ein oder mehrere, gleiche oder verschiedene Reste, ausgewählt unter den Halogenatomen und den Resten Hydroxyl, Trifluormethyl, Alkyl, Alkenyl, Alkinyl, Alkoxy und Phenyl, gegebenenfalls substituiert durch einen oder mehrere Reste, ausgewählt unter den Halogenatomen und den Resten Trifluormethyl, Hydroxyl, Alkyl und Alkoxy, wobei die Reste Alkyl und Alkoxy höchstens 6 Kohlenstoffatome umfassen und gegebenenfalls durch einen Phenylrest substituiert sind, oder $R_{3A}$' und $R_{4A}$', gleich oder verschieden, bedeuten:

- ein Wasserstoffatom;
- einen Hydroxylrest, Alkoxy-, Acyloxy- oder Acylrest mit höchstens 6 Kohlenstoffatomen; Carboxy, in Salz überführt oder verestert; Cyano;
- einen linearen oder verzweigten Alkyl-, Alkenyl- oder Alkinylrest mit höchstens 6 Kohlenstoffatomen und gegebenenfalls substituiert;
- einen der Reste Phenyl, Benzoyl, Naphthyl, Indenyl, Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und Thiomorpholinyl, Azepinyl, Diazepin, Benzodiazepin oder Indolyl, wobei diese Reste gegebenenfalls substituiert sind durch ein oder mehrere, gleiche oder verschiedene Reste, ausgewählt unter den Halogenatomen und den Resten Hydroxyl, Trifluormethyl, Alkyl und Alkoxy, die höchstens 6 Kohlenstoffatome umfassen und gegebenenfalls selbst substituiert sind durch einen oder mehrere Reste, ausgewählt unter den Halogenatomen und den Resten Hydroxyl, Alkyl, Alkoxy und Carboxy, frei, in ein Salz überführt oder verestert durch einen Alkyl-, Aryl- oder Arylalkylrest,
- den Rest

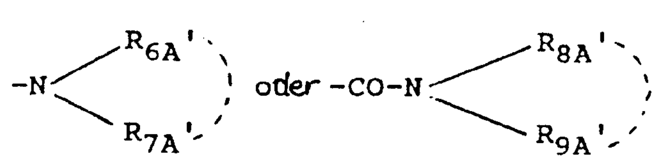

worin $R_{6A}$', $R_{7A}$', $R_{8A}$' und $R_{9A}$', gleich oder verschieden, ein Wasserstoffatom, einen der Reste Hydroxyl, Alkyl, Alkoxy, Acyloxy oder Acyl, wobei diese Reste höchstens 6 Kohlenstoffatome umfassen, freies Carboxy, in Salz überführt oder verestert, Phenyl, Benzyl, Phenethyl, Azepinyl, Piperidyl, Morpholin, Pyrrolidinyl oder Piperazinyl bedeuten,

oder einerseits $R_{6A}$' und $R_{7A}$' und andererseits $R_{8A}$' und $R_{9A}$' jeweils mit dem Stickstoffatom, an das sie gebunden sind, einen der Reste Imidazolyl, Pyrrolyl, Pyrrolinyl, Pyrrolidinyl, Pyridyl, Piperidinyl, Pyrimidinyl, Pyridazinyl, Pyrazinyl, Piperazinyl, Phenylpiperazinyl, Piperidyl, Oxazolyl, Morpholinyl und

Thiomorpholinyl, Azepinyl oder Indolyl bilden, wobei diese Reste gegebenenfalls substituiert sind durch ein oder mehrere, gleiche oder verschiedene Reste, ausgewählt unter den Halogenatomen und den Resten Hydroxyl, Trifluormethyl, Alkyl und Alkoxy, wobei diese Reste höchstens 6 Kohlenstoffatome umfassen, mit der Maßgabe, daß der oder die eventuellen Substituenten, gleich oder verschieden, die die Reste tragen können, die $R_1'$, $R_2'$ und $R_5'$ darstellen, ausgewählt werden aus der Gruppe, die gebildet wird aus:

- den Halogenatomen; den Hydroxylresten; Cyano; Nitro; Acyl und Acyloxy mit höchstens 6 Kohlenstoffatomen;Benzoyl,freies Carboxy,in Salz überführt oder verestert;
- den linearen und verzweigten Alkoxyresten mit höchstens 6 Kohlenstoffatomen;
- den Resten Alkyl, Alkenyl, Cyclopentyl, Cyclohexyl, Cyclopentenyl, Cyclohexenyl, Phenyl, Naphthyl, Indolyl, Phenoxy, Benzyl, Phenethyl und Benzyloxy, die gegebenenfalls selbst durch einen oder mehrere gleiche oder verschiedene Substituenten substituiert sind, ausgewählt unter den Halogenatomen und den Resten Hydroxyl, Trifluormethyl, Nitro, Alkyl, Alkenyl und Alkoxy, wobei diese Reste höchstens 6 Kohlenstoffatome umfassen, Formyl, Acetyl, Benzoyl, freies Carboxy, in Salz überführt oder verestert, Acetamido, Benzoylamido, Carbamoyl und Amino, wobei diese Reste gegebenenfalls am Stickstoffatom durch ein oder zwei gleiche oder verschiedene Reste substituiert sind, ausgewählt unter den Resten Hydroxyl, Methyl, Ethyl, Propyl, Methoxy, Ethoxy und Propoxy;
- dem Acylamidorest, worin der Acylrest höchstens 6 Kohlenstoffatome umfaßt; den Resten Carbamoyl und Amino, die gegebenenfalls am Stickstoffatom durch ein oder zwei gleiche oder verschiedene Reste substituiert sind, ausgewählt unter den Resten Hydroxyl, Alkyl, Alkenyl, Alkoxy und Acyl, die höchstens 6 Kohlenstoffatome umfassen;

wobei die genannten Produkte der Formel ($I_A'$) in allen möglichen isomeren, racemischen, enantiomeren und diastereoisomeren Formen vorliegen, sowie die Additionssalze der genannten Produkte der Formel ($I_A'$) mit Mineralsäuren und organischen Säuren.

**4.** Verfahren nach irgendeinem der Ansprüche 1 bis 3, dadurch gekennzeichnet, daß man als Ausgangsprodukte ein Produkt der Formel (II) verwendet, in der $R_{5p}$ einen Methylrest darstellt und $R_{2p}$ einen Phenylrest bedeutet, und ein Produkt der Formel (IV), in der $R_{3p}$ und $R_{4p}$ bedeuten:
<u>entweder</u> bilden $R_{3p}$ und $R_{4p}$ mit dem Stickstoffatom, an das sie gebunden sind, einen der Reste Piperidinyl, Morpholinyl, Dimethylmorpholinyl, Thiomorpholinyl, Pyrrolidinyl, Azepin oder Diazepin, gegebenenfalls substituiert am zweiten Stickstoffatom durch einen Alkylrest mit 1 oder 2 Kohlenstoffatomen, Piperazinyl, gegebenenfalls substituiert am zweiten Stickstoffatom entweder durch einen Phenylrest, gegebenenfalls substituiert durch ein Halogenatom oder einen Trifluormethylrest oder durch einen Alkyl-, Alkenyl- oder Alkinylrest mit 1 bis 4 Kohlenstoffatomen, gegebenenfalls substituiert durch einen Phenyl- oder Acylrest, oder durch einen Acylrest mit höchstens 6 Kohlenstoffatomen,
<u>oder</u> $R_{3p}$ und $R_{4p}$, gleich oder verschieden, bedeuten:
ein Wasserstoffatom; einen Hydroxylrest, Phenyl; Phenyl, substituiert durch einen Carboxyrest; Piperidinyl, gegebenenfalls substituiert am Stickstoffatom durch einen Benzylrest, Piperazinyl, gegebenenfalls substituiert am zweiten Stickstoffatom durch einen Alkylrest mit 1 oder 2 Kohlenstoffatomen, Carbamoyl; Alkoxy mit höchstens 4 Kohlenstoffatomen, Alkyl mit höchstens 4 Kohlenstoffatomen, gegebenenfalls substituiert durch einen oder mehrere gleiche oder verschiedene Reste, ausgewählt unter den Resten Hydroxyl, Alkoxy, freies Carboxy, in Salz überführt oder verestert, Amino, Alkylamino, Dialkylamino, Phenyl, Morpholino, Alkylthio mit höchstens 4 Kohlenstoffatomen, gegebenenfalls substituiert durch einen freien Carboxyrest, in Salz überführt oder verestert, Benzodiazepin, gegebenenfalls substituiert, und in denen die reaktiven Funktionen gegebenenfalls geschützt sind.

**5.** Verfahren nach irgendeinem der Ansprüche 1 bis 4, dadurch gekennzeichnet, daß man die Ausgangsprodukte der Formel (II) und (IV) in der Weise auswählt, daß man ein Produkt der folgenden Formeln herstellt:
(±)4-Methyl-N-[2-mercapto-1-(phenylmethyl)-ethyl]-1-piperazin-carboxamid-Hydrochlorid,
(±)N-[2-Mercapto-1-(phenylmethyl)-ethyl]-4-morpholin-carboxamid,
(±)N-[2-Mercapto-1-(phenylmethyl)-ethyl]-1-pyrrolidin-carboxamid,
(±)N-[[[2-Mercapto-1-(phenylmethyl)-ethyl]-amino]-carbonyl]-glycin,
N,N-bis(2-Methoxy-ethyl)-N'-[2-mercapto-1-(phenylmethyl)-ethyl]-harnstoff,
(±)cis-2,6-Dimethyl-N-[2-mercapto-1-(phenylmethyl)-ethyl]-4-morpholin- carboxamid (Isomer A),
(±)N-[2-Mercapto-1-(phenylmethyl)-ethyl]-4-thiomorpholin-carboxamid,
(±)N-[2-Mercapto-1-(phenylmethyl)-ethyl]-N'-methoxy-harnstoff,
(±)N-Hydroxy-N'-[2-mercapto-1-(phenylmethyl)-ethyl]-N'-methyl-harnstoff,
(±)N-(1-Mercapto-methyl)-2-(phenylethyl)-4-methyl-1-piperazin-acetamid,
sowie ihre Salze mit Mineralsäuren oder organischen Säuren.

6. Verfahren zur Herstellung der Ausgangsprodukte der Formel (II), wie in Anspruch 1 definiert, dadurch gekennzeichnet, daß man eine Verbindung der Formel (IIa)

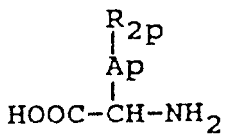

(IIa)

in der $R_{2p}$ und A die in Anspruch 1 jeweils für $R_2$ und A angegebenen Bedeutungen besitzen und in denen die eventuellen reaktiven Funktionen gegebenenfalls durch Schutzgruppen geschützt sind, der Einwirkung eines Reduktionsmittels für die Säurefunktion unterzieht, um den entsprechenden Alkohol der Formel (IIb)

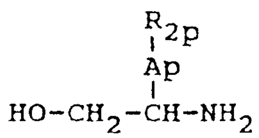

(IIb)

zu erhalten, in der $R_{2p}$ und Ap die vorstehend angegebenen Bedeutungen besitzen, den man durch Einwirkung eines Carbonyl-Derivates cyclisiert, um das entsprechende Oxazolidon der Formel (IIc)

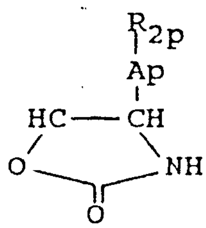

(IIc)

zu erhalten, in der $R_{2p}$ und Ap die vorstehend angegebenen Bedeutungen besitzen, das man der Einwirkung eines Schutzmittels unterzieht, um ein Produkt der Formel (IId)

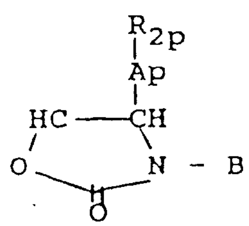

(IId)

zu erhalten, in der $R_{2p}$ und Ap die vorstehend angegebenen Bedeutungen besitzen und B eine Schutzgruppe für die Aminfunktion darstellt, das man mit einer Verbindung der Formel (IIe)

$R_{5p}$ - CO - SH (IIe)

zur Reaktion bringt, in der $R_{5p}$ die vorstehend für $R_5$ angegebene Bedeutung besitzt und in der die eventuellen reaktiven Funktionen gegebenenfalls durch Schutzgruppen geschützt sind, um eine Verbindung der Formel (IIf)

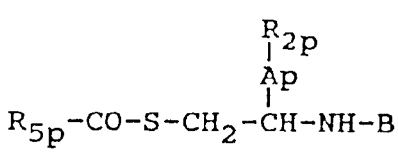

(IIf)

zu erhalten, in der $R_{2p}$, $R_{5p}$, Ap und B die vorstehend angegebenen Bedeutungen besitzen, die man, wenn

gewünscht, einer Reaktion zur Entfernung von B unterzieht, wobei die auf diese Weise erhaltenen Produkte der Formel (II) in allen möglichen isomeren, racemischen, enantiomeren und diastereoisomeren Formen vorliegen,

**7.** Als neue industrielle Produkte die Verbindungen der Formel (III).